(19) Europäisches Patentamt
European Patent Office
Office européen des brevets

(11) EP 4 778 931 A1

(12) EUROPEAN PATENT APPLICATION
published in accordance with Art. 153(4) EPC

(43) Date of publication:
**22.07.2026 Bulletin 2026/30**

(21) Application number: **24864690.3**

(22) Date of filing: **12.09.2024**

(51) International Patent Classification (IPC):
***C07D 498/04*** (2006.01) ***C07D 519/00*** (2006.01)
***C07D 413/04*** (2006.01) ***A61P 35/00*** (2006.01)
***A61K 31/506*** (2006.01) ***A61K 31/551*** (2006.01)

(52) Cooperative Patent Classification (CPC):
**A61K 31/506; A61K 31/551; A61P 35/00; C07D 413/04; C07D 498/04; C07D 519/00**

(86) International application number:
**PCT/CN2024/118472**

(87) International publication number:
**WO 2025/055976 (20.03.2025 Gazette 2025/12)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.09.2023 CN 202311187688**

(71) Applicant: **Dovetree Medicines Unus, Inc.**
**Prides Crossing, MA 01965 (US)**

(72) Inventors:
- **HUA, Zihao**
  **Shenzhen, Guangdong 518017 (CN)**
- **SUN, Yina**
  **Shenzhen, Guangdong 518017 (CN)**
- **ZHAO, Chuanfang**
  **Shenzhen, Guangdong 518017 (CN)**
- **CHEN, Chong**
  **Shenzhen, Guangdong 518017 (CN)**

(74) Representative: **Latscha Schöllhorn Partner AG**
**Grellingerstrasse 60**
**4052 Basel (CH)**

# (54) ULK1 INHIBITOR, PREPARATION METHOD THEREFOR AND USE THEREOF

(57) The present invention relates to a novel ULK1 inhibitor as shown in formula (1), a pharmaceutical composition and kit containing the ULK1 inhibitor, a preparation method for the ULK1 inhibitor, and a use of the ULK1 inhibitor in preparation of a drug for treating ULK1-mediated diseases.

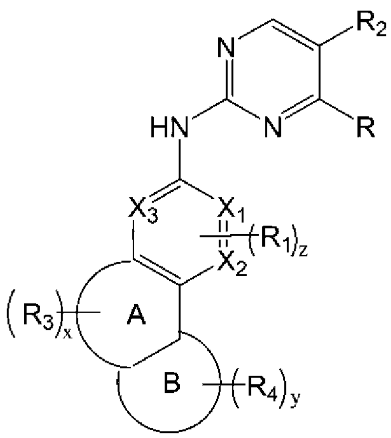

(1)

EP 4 778 931 A1

## Description

Reference to Related Applications

This application claims priority to Chinese Patent Application No. 202311187688.4, filed on September 13, 2023, the contents of which are incorporated herein by reference in their entirety and for all purposes.

## Field of the Invention

The present invention relates to a novel ULK1 inhibitor, a pharmaceutical composition and a kit containing the same, a preparation method therefor and a use thereof in the preparation of a medicament for a ULK1-mediated disease.

## Background

Autophagy is a central cellular mechanism that eliminates damaged proteins, protein complexes, and organelles. This conserved process plays a crucial role in the cellular response to nutrient deprivation and other stresses, in addition to maintaining appropriate cellular and tissue homeostasis during embryonic development and defending against pathogens. Defects in the autophagic pathway are associated with certain human pathologies, including an infectious disease, a neurodegenerative disorder, and a cancer. Despite possessing these highly conserved fundamental cellular functions, little is known about the molecular and biochemical details of how different carriers initiate autophagy, and the coordination of the steps from autophagosome initiation to eventual fusion with a lysosome.

Provided herein is an inhibitor of unc 51-like autophagy-activated kinase (ULK) protein. In many cases, ULK1 and ULK2 are important proteins that regulate autophagy in mammalian cells. In certain cases, ULK1 and ULK2 are activated by several upstream signals under a nutrient deprivation condition, which then initiate autophagy. The demand for ULKl and ULK2 in the initiation of autophagy has been studied in the context of nutrient deprivation. Although ULK1 appears to be the most essential for autophagy, in certain cases, ULK1 and ULK2 exhibit a high functional redundancy. The kinase domains of ULK1 and ULK2 share 78% sequence homology, indicating that in certain cases, ULK2 may compensate for the loss of ULKl. Therefore, nutrient-dependent autophagy may only be eliminated if both ULK1 and ULK2 are inhibited. However, in certain cases, ULK1 inhibition alone is sufficient to provide a therapeutic benefit, such as normalizing autophagy in any of the methods provided herein, or other beneficial outcomes. Inhibition of ULK1 and ULK2 produces a therapeutic benefit, such as tumor shrinkage, tumor cell death, or a reduction in the rate of tumor growth, there remains a need to provide a novel ULK1 inhibitor for the treatment of a ULK1-mediated disease.

## Summary of the Invention

In one aspect, the present invention provides a pyrimidine compound that has a specific inhibitory effect on ULK1, and thus has a good therapeutic effect on a ULK1-mediated disease. The compound is a compound of formula 1 or a pharmaceutically acceptable salt, a stereoisomer, a tautomer, a polymorph, a solvate, a hydrate, an N-oxide, an isotope labeled compound, a metabolite, an ester, or a prodrug thereof:

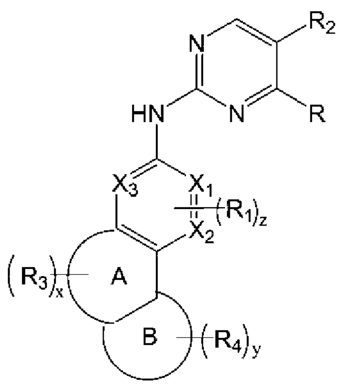

(1)

wherein $X_1$, $X_2$, $X_3$ are independently CH or N;
ring A is selected from 3-10-membered heterocyclyl, $C_3$-$C_{10}$ cycloalkyl, $C_6$-$C_{10}$ aryl, and 5-10-membered heteroaryl;
ring B is selected from 3-10-membered heterocyclyl, $C_3$-$C_{10}$ cycloalkyl, $C_6$-$C_{10}$ aryl, and 5-10-membered heteroaryl;
each $R_1$ is independently selected from H, halogen, cyano, nitro, $C_1$-$C_6$ alkyl, $C_2$-$C_6$ alkenyl, $C_2$-$C_6$ alkynyl, $C_1$-$C_6$

alkoxy, $C_3$-$C_{10}$ cycloalkyl, 3-10-membered heterocyclyl, $C_6$-$C_{10}$ aryl, and 5-10-membered heteroaryl;

$R_2$ is selected from H, cyano, halogen, $C_1$-$C_6$ alkyl, $C_1$-$C_6$ hydroxyalkyl, $C_1$-$C_6$ aminoalkyl, $C_1$-$C_6$ haloalkyl, $C_1$-$C_6$ heteroalkyl, $C_2$-$C_6$ alkenyl, $C_2$-$C_6$ alkynyl, and $C_3$-$C_{10}$ cycloalkyl;

$R_3$, $R_4$ are each independently selected from H, oxo, halogen, cyano, nitro, $C_1$-$C_6$ alkyl, $C_2$-$C_6$ alkenyl, $C_2$-$C_6$ alkynyl, $C_3$-$C_{10}$ cycloalkyl, 3-10-membered heterocyclyl, $C_6$-$C_{10}$ aryl, 5-10-membered heteroaryl, $R_5O$-, $R_6C(O)$-, $-NR_7R_8$ and $-NHCONR_7R_8$, wherein each of the $C_1$-$C_6$ alkyl, $C_2$-$C_6$ alkenyl, $C_2$-$C_6$ alkynyl, $C_3$-$C_{10}$ cycloalkyl, 3-10-membered heterocyclyl, $C_6$-$C_{10}$ aryl, or 5-10-membered heteroaryl is optionally substituted by one or more $R_9$;

$R_5$, $R_6$ are each independently selected from H, $C_1$-$C_6$ alkyl, $C_3$-$C_{10}$ cycloalkyl, $C_6$-$C_{10}$ aryl, and 5-10-membered heteroaryl;

$R_7$, $R_8$ are each independently selected from H, $C_1$-$C_6$ alkyl, $C_3$-$C_{10}$ cycloalkyl, 3-10-membered heterocyclyl, and $C_6$-$C_{10}$ aryl; or $R_7$ and $R_8$, together with N atom that they are attached to, form 5-6-membered heterocycle or 5-6-membered heteroaromatic ring;

each $R_9$ is independently selected from halogen, cyano, nitro, amino, $C_1$-$C_6$ alkyl, $C_2$-$C_6$ alkenyl, $C_2$-$C_6$ alkynyl, $C_3$-$C_{10}$ cycloalkyl, 3-10-membered heterocyclyl, $C_6$-$C_{10}$ aryl, and 5-10-membered heteroaryl;

R is selected from $-NR_7R_8$, $R_5O$-, $R_5S$-, halogen, $C_1$-$C_6$ alkyl, 3-12-membered heterocyclyl, 5-10-membered heteroaryl, and $-L-R_{10}$, wherein each of the 3-12-membered heterocyclyl or 5-10-membered heteroaryl is optionally substituted by one or more $R_{11}$;

L is $-(L_1)_m-(L_2)_n-(L_3)_p-$;

$L_1$ is selected from -O-, $-N(R_{12})$-, $-C(R_{12})(R_{13})-O$-, $-N(R_{12})CO$-, and -S-;

$L_2$ is selected from $C_1$-$C_6$ alkylene, $C_1$-$C_6$ heteroalkylene, $C_3$-$C_{10}$ cycloalkylene, and $C_3$-$C_{10}$ heterocyclylene;

$L_3$ is selected from $-N(R_{12})$-, $-N(R_{12})C(O)$-, and -C(O)-;

m, n, and p are each independently selected from 0, 1 and 2;

$R_{10}$ is selected from $C_3$-$C_{10}$ cycloalkyl, $C_6$-$C_{10}$ aryl, 5-10-membered heteroaryl, and 3-10-membered heterocyclyl, each of the $C_3$-$C_{10}$ cycloalkyl, $C_6$-$C_{10}$ aryl, 5-10-membered heteroaryl, or 3-10-membered heterocyclyl is optionally substituted by one or more $R_{14}$;

each $R_{11}$ is independently selected from $C_1$-$C_6$ alkyl, oxo, 5-10-membered heteroaryl, 3-10-membered heterocyclyl, $R_6C(O)$-, $R_6C(O)N(R_{12})$-, and $-NR_7R_8$, wherein each of the $C_1$-$C_6$ alkyl, 5-10-membered heteroaryl, or 3-10-membered heterocyclyl is optionally substituted by one or more $R_{15}$;

$R_{12}$, $R_{13}$ are each independently selected from H, $C_1$-$C_6$ alkyl, and $C_3$-$C_{10}$ cycloalkyl;

each $R_{14}$ is independently selected from H, halogen, cyano, oxo, $C_1$-$C_6$ alkyl, $C_1$-$C_6$ alkoxy, $-CONR_7R_8$, and $R_6C(O)$-; and

each $R_{15}$ is independently selected from H, halogen, cyano, oxo, $C_1$-$C_6$ alkyl, $C_3$-$C_{10}$ cycloalkyl, and 3-10-membered heterocyclyl, wherein each of the $C_1$-$C_6$ alkyl, $C_3$-$C_{10}$ cycloalkyl, or 3-10-membered heterocyclyl is optionally substituted by one or more substituent selected from $C_1$-$C_6$ alkyl and oxo; and

x, y, z are each independently selected from 1, 2 and 3.

In another aspect, the present invention provides a pharmaceutical composition, comprising an effective amount of the compound or pharmaceutically acceptable salt, stereoisomer, tautomer, polymorph, solvate, hydrate, N-oxide, isotope labeled compound, metabolite, ester, or prodrug thereof of the present invention, and one or more pharmaceutically acceptable carriers.

In yet another aspect, the present invention provides use of the compound or pharmaceutically acceptable salt, stereoisomer, tautomer, polymorph, solvate, hydrate, N-oxide, isotope labeled compound, metabolite, ester, or prodrug thereof of the present invention, or the pharmaceutical composition of the present invention in the preparation of a medicament for treating a ULK1-mediated disease in a subject in need thereof.

In another aspect, the present invention provides a method for treating a ULK1-mediated disease in a subject in need thereof, the method comprising administering to the subject the compound or pharmaceutically acceptable salt, stereoisomer, tautomer, polymorph, solvate, hydrate, N-oxide, isotope labeled compound, metabolite, ester, or prodrug thereof of the present invention, or the pharmaceutical composition of the present invention.

In yet another aspect, the present invention provides the compound or pharmaceutically acceptable salt, stereoisomer, tautomer, polymorph, solvate, hydrate, N-oxide, isotope labeled compound, metabolite, ester, or prodrug thereof of the present invention, or the pharmaceutical composition of the present invention, for use in treating a ULK1-mediated disease in a subject in need thereof.

In a further aspect, the present invention provides a method for preparing the compound of the present invention.

## Detailed Description of the Invention

## Definitions

Unless otherwise defined below, all technical and scientific terms used herein are intended to have the same meaning as commonly understood by those skilled in the art. Reference to a technique used herein is intended to refer to a technique as commonly understood in the art, including those technological changes or equivalent replacements that are obvious to those skilled in the art. While it is believed that the following terms will be well understood by those skilled in the art, the following definitions are set forth in order to better explain the present invention.

As used herein, the terms "including", "comprising", "having", "containing", or "involving" and their other variant forms herein, are inclusive or open-ended and do not exclude other unlisted elements or method steps.

As used herein, the term "alkyl" is defined as a straight or branched chain saturated aliphatic hydrocarbon group. For example, as used herein, the term "$C_{1-6}$ alkyl" refers to a straight or branched chain group having 1 to 6 carbon atoms (for example, methyl, ethyl, n-propyl, isopropyl, n-butyl, isobutyl, sec-butyl, tert-butyl, n-pentyl, or n-hexyl).

As used herein, the term "alkylene" refers to a straight or branched chain divalent alkyl.

As used herein, the term "alkenyl" refers to a straight or branched chain aliphatic hydrocarbon group having 1 and above unsaturated double bond. For example, as used herein, the term "$C_2$-$C_6$ alkenyl" refers to an alkenyl having 2 to 6 carbon atoms, for example, vinyl, 1-propenyl, 2-propenyl, 2-methyl-1-propenyl, 2-methyl-2-propenyl, 2-buten-1-yl, 3-buten-1-yl, 2-penten-1-yl, 3-penten-1-yl, 4-penten-1-yl, 5-hexen-1-yl, 4-hexen-1-yl, 3-hexen-1-yl, 2-hexen-1-yl, 3-methyl-2-buten-1-yl, 3-methyl-3-penten-1-yl, 3-methyl-2-penten-1-yl, 4-methyl-3-penten-1-yl, 4-methyl-2-penten-1-yl, and 2-methyl-2-penten-1-yl, etc. It is preferable to have one double bond.

As used herein, the term "alkynyl" refers to a straight or branched chain aliphatic hydrocarbon group having 1 or more unsaturated triple bonds. For example, as used herein, the term "$C_2$-$C_6$ alkynyl" refers to an alkenyl having 2 to 6 carbon atoms, for example, ethynyl, 1-propyn-1-yl, 2-propyn-1-yl, 2-butyn-1-yl, 3-butyn-1-yl, 2-pentyn-1-yl, 3-pentyn-1-yl, 4-pentyn-1-yl, 5-hexyn-1-yl, 4-hexyn-1-yl, 3-hexyn-1-yl, and 2-hexyn-1-yl, etc. It is preferable to have one triple bond.

As used herein, the term "alkoxy" means a group in which an oxygen atom is inserted at any reasonable position in an alkyl (as defined hereinbefore), for example, $C_{1-8}$ alkoxy, $C_{1-6}$ alkoxy, $C_{1-4}$ alkoxy, or $C_{1-3}$ alkoxy. Representative examples of $C_{1-6}$ alkoxy include, but are not limited to, methoxy, ethoxy, n-propoxy, isopropoxy, n-butoxy, isobutoxy, tert-butoxy, pentyloxy, hexyloxy, etc.

As used herein, the term "halo" or "halogen" group is defined as including fluorine, chlorine, bromine, or iodine.

As used herein, the term "haloalkyl" refers to an alkyl substituted by one or more (such as 1 to 3) identical or different halogen atoms. For example, the term "$C_{1-6}$ haloalkyl" refers to a haloalkyl having 1 to 6 carbon atoms, for example, $-CF_3$, $-C_2F_5$, $-CHF_2$, $-CH_2F$, $-CH_2CF_3$, - $CH_2Cl$, or $-CH_2CH_2CF_3$, etc.

As used herein, the term "hydroxyalkyl" refers to an alkyl substituted by one or more (such as 1 to 3) hydroxyl groups. For example, the term "$C_{1-6}$ hydroxyalkyl" refers to a hydroxyalkyl having 1 to 6 carbon atoms, for example, $-CH_2OH$, $-CH_2CH_2OH$, etc.

As used herein, the term "aminoalkyl" refers to an alkyl substituted by one or more (such as 1 to 3) amino groups. For example, the term "$C_{1-6}$ aminoalkyl" refers to an aminoalkyl having 1 to 6 carbon atoms, for example, $-CH_2NH_2$, $-CH_2CH_2NH_2$, etc.

As used herein, the term "heteroalkyl" refers to a straight or branched chain alkyl interrupted by one or more heteroatoms independently selected from N, O or $S(O)_q$ (wherein q is 0, 1, or 2). For example, the term "$C_{1-6}$ heteroalkyl" refers to a heteroalkyl having 1 to 6 carbon atoms.

As used herein, the term "heteroalkylene" refers to a straight or branched chain alkylene interrupted by one or more heteroatoms independently selected from N, O or $S(O)_q$ (wherein q is 0, 1, or 2). For example, the term "$C_{1-6}$ heteroalkylene" refers to a heteroalkylene having 1 to 6 carbon atoms.

As used herein, the term "cycloalkyl" refers to a saturated or partially unsaturated nonaromatic monocyclic or polycyclic (such as bicyclic) hydrocarbon ring (for example, a monocyclic ring, such as cyclopropyl, cyclobutyl, cyclopentyl, cyclohexyl, cycloheptyl, cyclooctyl, cyclononyl, or a bicyclic ring, including a spiro, fused (fused-ring) or bridged (bridged-ring) system, such as bicyclo[1.1.1]pentyl, bicyclo[2.2.1]heptyl, bicyclo[3.2.1]octyl, or bicyclo[5.2.0]nonyl, decalinyl, etc), which is optionally substituted by one or more (such as 1 to 3) suitable substituents. The cycloalkyl has 3 to 15, for example, 3 to 10 carbon atoms. For example, the term "$C_{3-10}$ cycloalkyl" refers to a saturated or partially unsaturated non-aromatic monocyclic or polycyclic (such as bicyclic) hydrocarbon ring having 3 to 10 ring-forming carbon atoms (for example, cyclopropyl, cyclobutyl, cyclopentyl, or cyclohexyl).

As used herein, the term "heterocyclyl" or "heterocycle" refers to a saturated or partially unsaturated monocyclic or polycyclic (including a spiro, fused (fused-ring) or bridged (bridged-ring) system) group, for example it has 2, 3, 4, 5, 6, 7, 8, or 9 carbon atoms and one or more (e.g., 1, 2, 3, or 4) heteroatoms independently selected from N, O, or $S(O)_q$ (wherein q is 0, 1, or 2) in the ring, for example, a 3-12-membered heterocyclyl, a 3-10-membered heterocyclyl, a 3-6-membered heterocyclyl, a 5-6-membered heterocyclyl, etc. Representative examples of heterocyclyl include, but are not limited to, oxiranyl, aziridinyl, azetidinyl, oxetanyl, tetrahydrofuranyl, pyrrolidinyl, pyrrolidinonyl, imidazolidinyl, pyrazolidinyl, tetrahydropyranyl, piperidinyl, morpholinyl, dithianyl, thiomorpholinyl, piperazinyl, trithianyl, etc. As used herein, the term

"nitrogen-containing heterocycle" refers to a heterocycle having at least one N atom and optionally other heteroatoms in the ring, including a monocyclic or polycyclic (including a spiro, fused (fused-ring) or bridged (bridged-ring) system) heterocycle.

As used herein, the term "aryl" or "aromatic ring" refers to an all-carbon monocyclic or fused-ring polycyclic aromatic group having a conjugated $\pi$-electron system. For example, the term "$C_{6-10}$ aryl" or "$C_{6-10}$ aromatic ring" refer to an aromatic group containing 6 to 10 carbon atoms, such as phenyl (ring) or naphthalenyl (ring).

As used herein, the term "heteroaryl" or "heteroaromatic ring" refers to a monocyclic, bicyclic or tricyclic aromatic ring system containing at least one heteroatom selected from N, O and S, for example having 5, 6, 8, 9, 10, 11, 12, 13, or 14 ring atoms, particularly containing 1 or 2 or 3 or 4 or 5 or 6 or 9 or 10 carbon atoms, and, furthermore, in each case, it can be benzofused. For example, examples of heteroaryl or heteroaromatic ring include, but are not limited to, thienyl (ring), furanyl (ring), pyrrolyl (ring), oxazolyl (ring), thiazolyl (ring), imidazolyl (ring), pyrazolyl (ring), isoxazolyl (ring), isothiazolyl (ring), oxadiazolyl (ring), triazolyl (ring), thiadiazolyl (ring), etc., as well as their benzo derivatives; or pyridinyl (ring), pyridazinyl (ring), pyrimidinyl (ring), pyrazinyl (ring), triazinyl (ring), etc., as well as their benzo derivatives.

The term "substitution" refers to a selective replacement of one or more (e.g., 1, 2, 3, or 4) hydrogens on a specified atom by a designated group, with the proviso that the normal valence of the specified atom in the present case is not exceeded and the substitution forms a stable compound. A combination of substituents and/or variables is permitted only when this combination forms a stable compound.

If a substituent is described as "optionally substituted by ......", the substituent may be (1) unsubstituted or (2) substituted. If the carbon of the substituent is described as being optionally substituted by one or more of the substituents in the list, one or more hydrogens on the carbon (to the extent that any hydrogens are present) may be substituted individually and/or together by an independently selected substituent or not substituted. If the nitrogen of the substituent is described as being optionally substituted by one or more of the substituents in the list, each of one or more hydrogens on the nitrogen (to the extent that any hydrogens are present) may be substituted by an independently selected substituent or not substituted.

If a substituent is described as being "independently selected from" a group of groups, each substituent is selected independently of the other. Therefore, each substituent may be the same as or different from another (other) substituent.

As used herein, the term "one or more" means 1 or more than 1 under a reasonable condition, for example, 2, 3, 4, 5, 6, 7, 8, 9, or 10.

Unless otherwise specified, as used herein, the point of attachment of a substituent may be from any suitable position of the substituent.

When the bond of a substituent is shown as passing through a bond connecting two atoms in a ring, such a substituent may be bonded to any ring-forming atom in the substitutable ring.

The present invention also includes all pharmaceutically acceptable isotope labeled compounds that are identical to the compounds of the present invention, except that one or more atoms are replaced by atoms having the same atomic number but with an atomic mass or mass number different from which predominates in nature. Examples of an isotope suitable for inclusion in the compound of the present invention include, but are not limited to, a hydrogen isotope (e.g., $^{2}H$, $^{3}H$, deuterium D, tritium T); a carbon isotope (e.g., $^{11}C$, $^{13}C$, and $^{14}C$); a chlorine isotope (e.g., $^{37}Cl$); a fluorine isotope (e.g., $^{18}F$); an iodine isotope (e.g., $^{123}I$ and $^{125}I$); a nitrogen isotope (e.g., $^{13}N$ and $^{15}N$); an oxygen isotope (e.g., $^{15}O$, $^{17}O$ and $^{18}O$); a phosphorus isotope (e.g., $^{32}P$); and a sulfur isotope (e.g., $^{35}S$). Certain isotope labeled compounds of the present invention (e.g., those incorporating a radioisotope) can be used in a pharmaceutical and/or substrate tissue distribution study (e.g., analysis). Radioisotope tritium (i.e., $^{3}H$) and carbon-14 (i.e., $^{14}C$) are particularly useful for this purpose due to their ease of incorporation and detection. Substitution with a positron emission isotope (e.g., $^{11}C$, $^{18}F$, $^{15}O$, and $^{13}N$) can be used to examine substrate receptor occupancy in a positron emission tomography (PET) study. An isotope labeled compound of the present invention can be prepared by a method similar to those described in the accompanying routes and/or an example and preparation, by using an appropriate isotope labeled reagent instead of a previously used unlabeled reagent. The pharmaceutically acceptable solvate of the present invention includes those in which a crystallization solvent can be substituted by an isotope, for example, $D_2O$, acetone-$d_6$ or DMSO-$d_6$.

The term "stereoisomer" represents an isomer formed due to at least one asymmetric center. In compounds with one or more (e.g., 1, 2, 3, or 4) asymmetric centers, racemic mixtures, single enantiomers, diastereomer mixtures, and individual diastereomers can be produced. A specific individual molecule can also exist as a geometric isomer (cis/trans).

Similarly, the compound of the present invention can exist as a mixture of two or more structurally different forms in rapid equilibrium (commonly referred to as tautomers). Representative examples of tautomers include keto-enol tautomers, phenol-keto tautomers, nitroso-oxime tautomers, imine-enamine tautomers, etc. It should be understood that the scope of the present application covers all such isomers or mixtures thereof in any proportion (e.g., 60%, 65%, 70%, 75%, 80%, 85%, 90%, 95%, 96%, 97%, 98%, 99%).

The present invention covers all possible crystalline forms or polymorphs of the compound of the present invention, which may be a single polymorph or a mixture of more than one polymorphs in any proportion.

It should also be understood that certain compounds of the present invention may exist in a free form for treatment, or where appropriate, exist in their pharmaceutically acceptable derivative forms. In the present invention, a pharmaceutically acceptable derivative includes, but is not limited to, a pharmaceutically acceptable salt, solvate, metabolite or prodrug that, when administered to a patient in need thereof, can directly or indirectly provide the compound of the present invention or its metabolite or residue. Therefore, when referring to "the compound of the present invention" herein, it is also intended to cover the various derivative forms of the compound described above.

The pharmaceutically acceptable salt of the compound of the present invention includes an acid addition salt and a base addition salt thereof. A suitable acid addition salt is formed from an acid that forms a pharmaceutically acceptable salt. A suitable base addition salt is formed from a base that forms a pharmaceutically acceptable salt. For a review of a suitable salt, see "Handbook of Pharmaceutical Salts: Properties, Selection, and Use" by Stahl and Wermuth (Wiley-VCH, 2002). The methods for preparing a pharmaceutically acceptable salt of the compound of the present invention are known to those skilled in the art.

The compound of the present invention may exist in the form of a solvate (preferably a hydrate), wherein the compound of the present invention contains a polar solvent, particularly, for example, water, methanol, or ethanol, as a structural element of the lattice of the compound. The amount of the polar solvent, particularly water, can exist in a stoichiometric or non-stoichiometric ratio.

Those skilled in the art will understand that not all nitrogen-containing heterocycles are capable of forming N-oxides because nitrogen requires available lone pairs of electrons to be oxidized to an oxide; those skilled in the art will identify nitrogen-containing heterocycles that are capable of forming N-oxides. Those skilled in the art will also recognize that tertiary amines can form N-oxides. The synthetic methods for preparing N-oxides of heterocycles and tertiary amines are well known to those skilled in the art, including the oxidation of heterocycles and tertiary amines with peroxy acids such as peracetic acid and m-chloroperoxybenzoic acid (MCPBA), hydrogen peroxide, alkyl hydrogen peroxide such as tert-butyl hydrogen peroxide, sodium perborate, and dioxirane such as dimethyldioxirane. These methods for preparing N-oxides have been extensively described and reviewed in literatures, see, for example: T. L. Gilchrist, Comprehensive Organic Synthesis, vol. 7, pp 748-750; A. R. Katritzky and A. J. Boulton, Eds., Academic Press; and G. W. H. Cheeseman and E. S. G. Werstiuk, Advances in Heterocyclic Chemistry, vol. 22, pp 390-392, A. R. Katritzky and A. J. Boulton, Eds., Academic Press.

The scope of the present invention also includes a metabolite of the compound of the present invention, i.e., a substance formed in the body upon administration of the compound of the present invention. Such products can be generated, for example, by oxidation, reduction, hydrolysis, amidation, deamidation, esterification, enzymatic hydrolysis, etc., of the administered compound. Therefore, the present invention includes a metabolite of the compound of the present invention, including a compound prepared by a method of contacting the compound of the present invention with a mammal for a time sufficient to produce its metabolite.

The present invention further includes, within its scope, prodrugs of the compound of the present invention, which are certain derivatives of the compound of the present invention that may themselves have a little or no pharmacological activity, and which, when administered to or on the body, can be converted, for example, by hydrolytic cleavage into the compound of the present invention having the desired activity. Typically, such a prodrug will be a functional group derivative of the compound, which is readily converted in vivo into a compound with the desired therapeutic activity. Other information regarding the use of prodrugs can be found in "Pro-drugs as Novel Delivery Systems", Vol. 14, ACS Symposium Series (T. Higuchi and V. Stella), and "Bioreversible Carriers in Drug Design," Pergamon Press, 1987 (edited by E. B. Roche, American Pharmaceutical Association). The prodrug of the present invention can be prepared, for example, by replacing an appropriate functional group present in the compound of the present invention with a certain moiety known to those skilled in the art as "pro-moiety (e.g., described in "Design of Prodrugs", H. Bundgaard (Elsevier, 1985))".

The present invention also covers the compound of the present invention containing a protecting group. In any process for preparing the compound of the present invention, it may be necessary and/or desirable to protect sensitive or reactive groups on any relevant molecules, thereby forming a chemically protected form of the compound of the present invention. This can be achieved by a conventional protecting group, for example, those protecting groups described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991, these references are incorporated herein by reference. The protecting group can be removed at an appropriate subsequent stage using methods known in the art.

The term "about" refers to within the range of $\pm$10%, preferably $\pm$5%, more preferably $\pm$2% of the stated numerical value.

## Compounds

One object of the present invention is to provide a compound of formula 1 or a pharmaceutically acceptable salt, a stereoisomer, a tautomer, a polymorph, a solvate, a hydrate, an N-oxide, an isotope labeled compound, a metabolite, an ester, or a prodrug thereof:

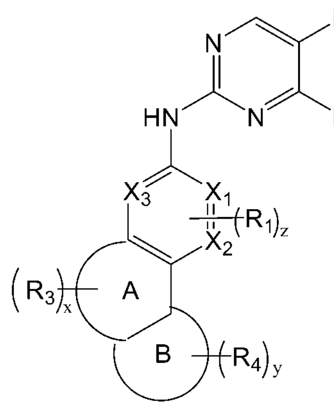

(1)

wherein $X_1$, $X_2$, $X_3$ are independently CH or N;
ring A is selected from 3-10-membered heterocyclyl, $C_3$-$C_{10}$ cycloalkyl, $C_6$-$C_{10}$ aryl, and 5-10-membered heteroaryl;
ring B is selected from 3-10-membered heterocyclyl, $C_3$-$C_{10}$ cycloalkyl, $C_6$-$C_{10}$ aryl, and 5-10-membered heteroaryl;
each $R_1$ is independently selected from H, halogen, cyano, nitro, $C_1$-$C_6$ alkyl, $C_2$-$C_6$ alkenyl, $C_2$-$C_6$ alkynyl, $C_1$-$C_6$ alkoxy, $C_3$-$C_{10}$ cycloalkyl, 3-10-membered heterocyclyl, $C_6$-$C_{10}$ aryl, and 5-10-membered heteroaryl;
$R_2$ is selected from H, cyano, halogen, $C_1$-$C_6$ alkyl, $C_1$-$C_6$ hydroxyalkyl, $C_1$-$C_6$ aminoalkyl, $C_1$-$C_6$ haloalkyl, $C_1$-$C_6$ heteroalkyl, $C_2$-$C_6$ alkenyl, $C_2$-$C_6$ alkynyl, and $C_3$-$C_{10}$ cycloalkyl;
$R_3$, $R_4$ are each independently selected from H, oxo, halogen, cyano, nitro, $C_1$-$C_6$ alkyl, $C_2$-$C_6$ alkenyl, $C_2$-$C_6$ alkynyl, $C_3$-$C_{10}$ cycloalkyl, 3-10-membered heterocyclyl, $C_6$-$C_{10}$ aryl, 5-10-membered heteroaryl, $R_5O$-, $R_6C(O)$-, $-NR_7R_8$ and $-NHCONR_7R_8$, wherein each of the $C_1$-$C_6$ alkyl, $C_2$-$C_6$ alkenyl, $C_2$-$C_6$ alkynyl, $C_3$-$C_{10}$ cycloalkyl, 3-10-membered heterocyclyl, $C_6$-$C_{10}$ aryl, or 5-10-membered heteroaryl is optionally substituted by one or more $R_9$;
$R_5$, $R_6$ are each independently selected from H, $C_1$-$C_6$ alkyl, $C_3$-$C_{10}$ cycloalkyl, $C_6$-$C_{10}$ aryl, and 5-10-membered heteroaryl;
$R_7$, $R_8$ are each independently selected from H, $C_1$-$C_6$ alkyl, $C_3$-$C_{10}$ cycloalkyl, 3-10-membered heterocyclyl, and $C_6$-$C_{10}$ aryl; or $R_7$ and $R_8$, together with N atom that they are attached to, form 5-6-membered heterocycle or 5-6-membered heteroaromatic ring;
each $R_9$ is independently selected from halogen, cyano, nitro, amino, $C_1$-$C_6$ alkyl, $C_2$-$C_6$ alkenyl, $C_2$-$C_6$ alkynyl, $C_3$-$C_{10}$ cycloalkyl, 3-10-membered heterocyclyl, $C_6$-$C_{10}$ aryl, and 5-10-membered heteroaryl;
R is selected from $-NR_7R_8$, $R_5O$-, $R_5S$-, halogen, $C_1$-$C_6$ alkyl, 3-12-membered heterocyclyl, 5-10-membered heteroaryl, and $-L-R_{10}$, wherein each of the 3-12-membered heterocyclyl or 5-10-membered heteroaryl is optionally substituted by one or more $R_{11}$;
L is $-(L_1)_m-(L_2)_n-(L_3)_p-$;
$L_1$ is selected from -O-, $-N(R_{12})$-, $-C(R_{12})(R_{13})-O$-, $-N(R_{12})CO$-, and -S-;
$L_2$ is selected from $C_1$-$C_6$ alkylene, $C_1$-$C_6$ heteroalkylene, $C_3$-$C_{10}$ cycloalkylene, and 3-10-membered heterocyclylene;
$L_3$ is selected from $-N(R_{12})$-, $-N(R_{12})C(O)$-, and -C(O)-;
m, n, and p are each independently selected from 0, 1 and 2;
$R_{10}$ is selected from $C_3$-$C_{10}$ cycloalkyl, $C_6$-$C_{10}$ aryl, 5-10-membered heteroaryl, and 3-10-membered heterocyclyl, each of the $C_3$-$C_{10}$ cycloalkyl, $C_6$-$C_{10}$ aryl, 5-10-membered heteroaryl, or 3-10-membered heterocyclyl is optionally substituted by one or more $R_{14}$;
each $R_{11}$ is independently selected from $C_1$-$C_6$ alkyl, oxo, 5-10-membered heteroaryl, 3-10-membered heterocyclyl, $R_6C(O)$-, $R_6C(O)N(R_{12})$-, and $-NR_7R_8$, wherein each of the $C_1$-$C_6$ alkyl, 5-10-membered heteroaryl, or 3-10-membered heterocyclyl is optionally substituted by one or more $R_{15}$;
$R_{12}$, $R_{13}$ are each independently selected from H, $C_1$-$C_6$ alkyl, and $C_3$-$C_{10}$ cycloalkyl;
each $R_{14}$ is independently selected from H, halogen, cyano, oxo, $C_1$-$C_6$ alkyl, $C_1$-$C_6$ alkoxy, $-CONR_7R_8$, and $R_6C(O)$-; and
each $R_{15}$ is independently selected from H, halogen, cyano, oxo, $C_1$-$C_6$ alkyl, $C_3$-$C_{10}$ cycloalkyl, and 3-10-membered heterocyclyl, wherein each of the $C_1$-$C_6$ alkyl, $C_3$-$C_{10}$ cycloalkyl, or 3-10-membered heterocyclyl is optionally substituted by one or more substituent selected from $C_1$-$C_6$ alkyl and oxo; and
x, y, z are each independently selected from 1, 2 and 3.

According to some embodiments of the present invention, ring A is selected from 4-10-membered heterocyclyl, $C_4$-$C_{10}$ cycloalkyl, $C_6$-$C_{10}$ aryl, and 5-10-membered heteroaryl.

According to some embodiments of the present invention, at most one of $X_1$, $X_2$, $X_3$ is N.

According to some embodiments of the present invention, ring A is 5-8-membered heterocyclyl, preferably 6-7-membered heterocyclyl, more preferably 6-7-membered nitrogen-containing heterocyclyl.

In some embodiments of the present invention, ring A is

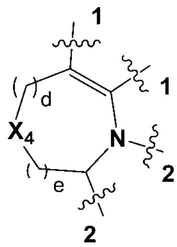,

wherein

$X_4$ is selected from O and NH; d is 0 or 1; e is 1 or 2; the wavy line represents the point of attachment of the group to the rest of the molecule;

symbol 1 represents the point of attachment of ring A to

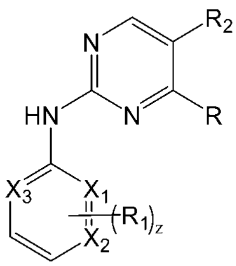

moiety; and symbol 2 represents the point of attachment of ring A to ring B.

In some embodiments of the present invention, ring A is selected from

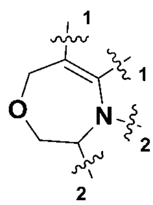,

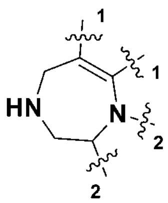, 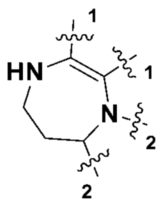, 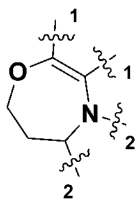, 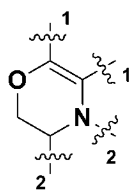, and 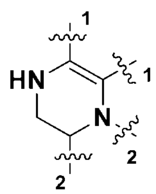,

wherein the wavy line represents the point of attachment of the group to the rest of the molecule; symbol 1 represents the point of attachment of ring A to

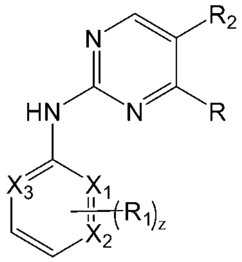

moiety; and symbol 2 represents the point of attachment of ring A to ring B.

According to some embodiments of the present invention, ring B is 5-6-membered heterocycle, more preferably 5-6-membered nitrogen-containing heterocycle.

In some embodiments of the present invention, ring B is

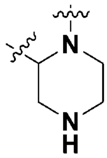 ,

wherein the wavy line 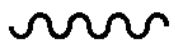 represents the point of attachment of ring B to ring A.

According to some embodiments of the present invention, $R_1$ is H.

According to some embodiments of the present invention, $R_2$ is selected from H and $C_1$-$C_6$ haloalkyl, preferably $C_1$-$C_6$ haloalkyl, further preferably $C_1$-$C_3$ haloalkyl, more preferably trifluoromethyl.

According to some embodiments of the present invention, $R_3$, $R_4$ are each independently selected from H, oxo, $C_1$-$C_6$ alkyl, $C_3$-$C_{10}$ cycloalkyl, 3-10-membered heterocyclyl, and $R_6C(O)$-, wherein each of the $C_1$-$C_6$ alkyl, $C_3$-$C_{10}$ cycloalkyl, or 3-10-membered heterocyclyl is optionally substituted by one or more $R_9$;

$R_6$ is selected from $C_1$-$C_6$ alkyl and $C_3$-$C_{10}$ cycloalkyl; and each $R_9$ is independently selected from halogen and $C_1$-$C_6$ alkyl.

In some embodiments of the present invention, $R_3$, $R_4$ are each independently selected from H, oxo, $C_1$-$C_6$ alkyl, $C_3$-$C_6$ cycloalkyl, 4-6-membered heterocyclyl, and $R_6C(O)$-, wherein the $C_1$-$C_6$ alkyl is optionally substituted by 1, 2, 3, 4, or 5 of halogen, and the 4-6-membered heterocyclyl is optionally substituted by 1, 2, or 3 $C_1$-$C_6$ alkyl; and $R_6$ is $C_1$-$C_6$ alkyl.

In some embodiments of the present invention, $R_3$, $R_4$ are each independently selected from H, oxo, $C_1$-$C_6$ alkyl, $C_3$-$C_6$ cycloalkyl, oxetanyl, piperidinyl, tetrahydropyranyl, and $R_6C(O)$-, wherein the $C_1$-$C_6$ alkyl is optionally substituted by 1, 2, 3, 4, or 5 of halogen, and each of the piperidinyl or tetrahydropyranyl is optionally substituted by 1 or 2 $C_1$-$C_6$ alkyl; and $R_6$ is $C_1$-$C_6$ alkyl.

In some embodiments of the present invention, $R_3$, $R_4$ are each independently selected from H, oxo, methyl, isopropyl, cyclopropyl, cyclobutyl, $CF_3CH_2$-, $CH_3CO$-,

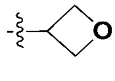 ,

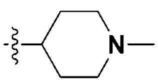 , and 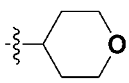 ,

wherein the wavy line 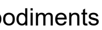 represents the point of attachment of the group to the rest of the molecule.

In some embodiments of the present invention, $R_3$ is selected from H, oxo, and $C_1$-$C_6$ alkyl, preferably selected from H, oxo, and $C_1$-$C_3$ alkyl, more preferably selected from H, oxo, and methyl.

In some embodiments of the present invention, $R_4$ is selected from $C_1$-$C_6$ alkyl, $C_3$-$C_6$ cycloalkyl, 4-6-membered heterocyclyl, and $R_6C(O)$-, wherein the $C_1$-$C_6$ alkyl is optionally substituted by 1, 2, 3, 4, or 5 of halogen, and the 4-6-membered heterocyclyl is optionally substituted by 1, 2, or 3 $C_1$-$C_6$ alkyl; and $R_6$ is $C_1$-$C_6$ alkyl.

In some embodiments of the present invention, $R_4$ is selected from H, $C_1$-$C_6$ alkyl, $C_3$-$C_6$ cycloalkyl, oxetanyl, piperidinyl, tetrahydropyranyl, and $R_6C(O)$-, wherein the $C_1$-$C_6$ alkyl is optionally substituted by 1, 2, 3, 4, or 5 of halogen, and each of the piperidinyl or tetrahydropyranyl is optionally substituted by 1 or 2 $C_1$-$C_6$ alkyl; and $R_6$ is $C_1$-$C_6$ alkyl.

In some embodiments of the present invention, $R_4$ is selected from $C_1$-$C_6$ alkyl, $C_3$-$C_6$ cycloalkyl, oxetanyl,

piperidinyl, tetrahydropyranyl, and $R_6C(O)$-, wherein the $C_1$-$C_6$ alkyl is optionally substituted by 1, 2, 3, 4, or 5 of halogen, and each of the piperidinyl or tetrahydropyranyl is optionally substituted by 1 or 2 $C_1$-$C_6$ alkyl; and
$R_6$ is $C_1$-$C_6$ alkyl.

In some embodiments of the present invention, $R_4$ is selected from methyl, isopropyl, cyclopropyl, cyclobutyl, $CF_3CH_2$-, $CH_3CO$-,

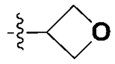, 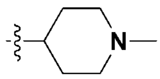, and 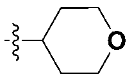,

wherein the wavy line 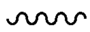 represents the point of attachment of the group to the rest of the molecule.

According to some embodiments of the present invention, x, y, z are each independently selected from 1 and 2.

According to some embodiments of the present invention,

R is selected from 3-12-membered heterocyclyl, 5-10-membered heteroaryl, and -L-$R_{10}$, wherein each of the 3-12-membered heterocyclyl or 5-10-membered heteroaryl is optionally substituted by one or more $R_{11}$;
L is $-(L_1)_m-(L_2)_n-(L_3)_p-$;
$L_1$ is selected from -O-, -NH-, and $-CH_2-O-$;
$L_2$ is selected from $C_1$-$C_6$ alkylene, $C_3$-$C_{10}$ cycloalkylene, and 3-10-membered heterocyclylene;
$L_3$ is selected from -NH-, -NHC(O)-, and -C(O)-;
m, n, and p are each independently selected from 0 and 1, and m, n, and p are not simultaneously 0;
$R_{10}$ is selected from $C_3$-$C_{10}$ cycloalkyl, $C_6$-$C_{10}$ aryl, 5-10-membered heteroaryl, and 3-10-membered heterocyclyl, each of the $C_3$-$C_{10}$ cycloalkyl, $C_6$-$C_{10}$ aryl, 5-10-membered heteroaryl, or 3-10-membered heterocyclyl is optionally substituted by one or more $R_{14}$;
each $R_{11}$ is independently selected from $C_1$-$C_6$ alkyl, oxo, 5-10-membered heteroaryl, 3-10-membered heterocyclyl, and $R_6C(O)$-, wherein each of the 3-10-membered heterocyclyl is optionally substituted by one or more $R_{15}$;
each $R_{14}$ is independently selected from H, halogen, cyano, oxo, and $C_1$-$C_6$ alkyl; and
each $R_{15}$ is independently selected from oxo and $C_1$-$C_6$ alkyl.

According to some embodiments of the present invention,

L is $-(L_1)_m-(L_2)_n-(L_3)_p-$*, wherein the bond marked with "*" is attached to $R_{10}$;
$L_1$ is selected from -O-, $-N(R_{12})-$, $-C(R_{12})(R_{13})-O-$@, $-N(R_{12})CO-$@, and -S-, wherein the bond marked with "@" is attached to $L_2$;
$L_2$ is selected from $C_1$-$C_6$ alkylene, $C_1$-$C_6$ heteroalkylene, $C_3$-$C_{10}$ cycloalkylene, and 3-10-membered heterocyclylene;
$L_3$ is selected from $-N(R_{12})-$, $-N(R_{12})C(O)-$\$, and -C(O)-, wherein the bond marked with "\$" is attached to $R_{10}$;
m, n, and p are each independently selected from 0 and 1, and m, n, and p are not simultaneously 0;
preferably, $L_1$ is selected from -O-, $-N(R_{12})-$;
$L_2$ is selected from $C_1$-$C_6$ alkylene, $C_3$-$C_6$ cycloalkylene, and 3-6-membered heterocyclylene;
$L_3$ is selected from $-N(R_{12})-$, $-N(R_{12})C(O)-$\$, and -C(O)-, more preferably, -C(O)- , wherein the bond marked with "\$" is attached to $R_{10}$;
preferably, $L_1$ is selected from -O-, -NH-;
$L_2$ is selected from ethylene, propylene, cyclohexylidene, piperidylidene;
$L_3$ is selected from -NH-, -NH-C(O)-\$, -C(O)-, wherein the bond marked with "\$" is attached to $R_{10}$;
preferably, L is -O-, -O-cyclohexylidene-*, -NH-cyclohexylidene-*, -NH-piperidylidene-C(O)-*, -O-propylene-*, -NH-propylene-*, -O-ethylene-NH-*, -ethylene-NHC(O)-*, -NH-propylene-C(O)-*, and -O-propylene-C(O)-*, wherein the bond marked with "*" is attached to $R_{10}$;
more preferably, L is selected from -O-propylene-*, -O-cyclohexylidene-*, -NH-propylene-*, -NH-cyclohexylidene-*, -NH-piperidylidene-C(O)-*, wherein the bond marked with "*" is attached to $R_{10}$.

According to some embodiments of the present invention, the compound of the present invention is the compound of formula 1':

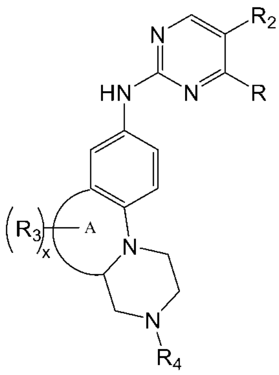

(1')

wherein R, $R_2$, $R_3$, $R_4$, ring A, and x are as defined hereinbefore for the compound of formula (1).

According to some embodiments of the present invention, the compound of the present invention is the compound of formula 1A, 1B, 1C, or 1D:

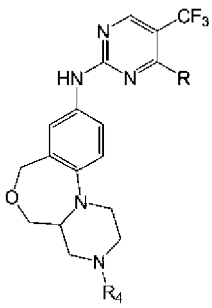

(1A),

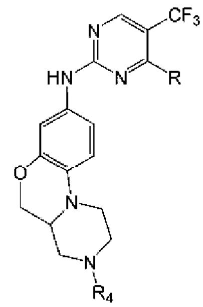

(1B),

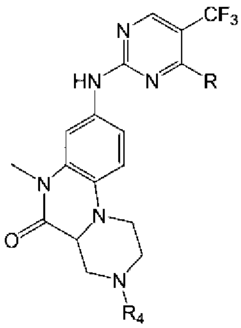

(1C),

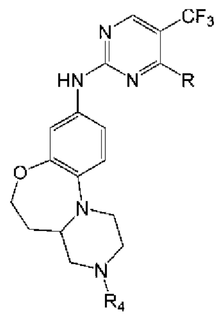

(1D);

wherein R, $R_4$ are as defined hereinbefore for the compound of formula (1).

According to some embodiments of the present invention, the compound of the present invention is the compound of formula 1A-1:

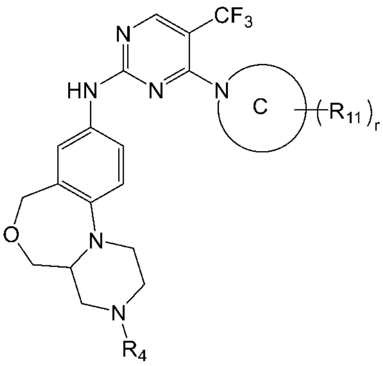

(1A-1)

wherein $R_{11}$, $R_4$ are as defined hereinbefore for the compound of formula (1); ring C is selected from 3-12-membered heterocyclyl and 5-10-membered heteroaryl; and r is selected from 0, 1, and 2.

In some embodiments of the present invention, in the compound of formula 1A-1, ring C is selected from 3-12-membered heterocyclyl.

In some embodiments of the present invention, in the compound of formula 1A-1:

ring C is 5-12-membered spiro heterocyclyl or 5-12-membered bridged heterocyclyl, preferably 7-12-membered spiro heterocyclyl or 7-12-membered bridged heterocyclyl, more preferably

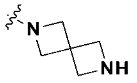, 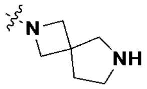, 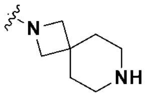, 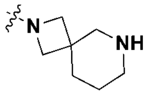, 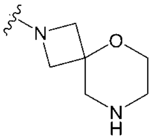, 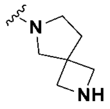,

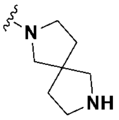, 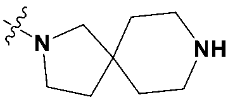, 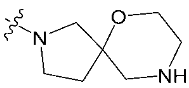, 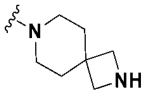, 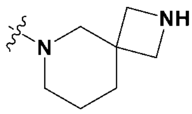, 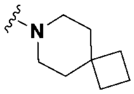,

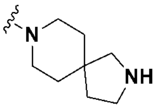, 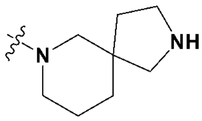, 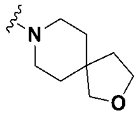, 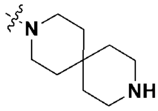, 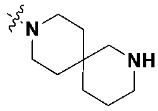, 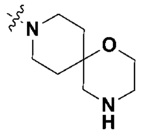,

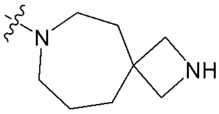, 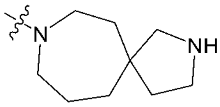, 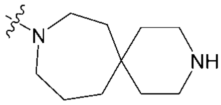, 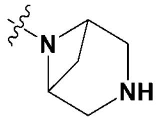, 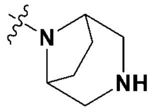,

or

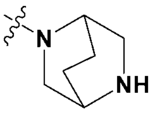, more preferably 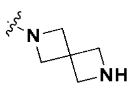, 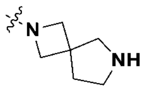, 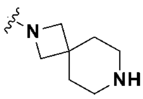, 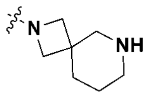, 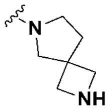,

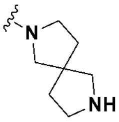, 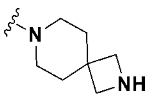, 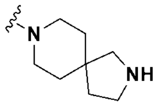, 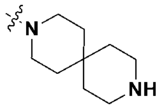, 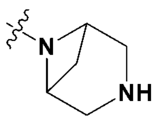, or 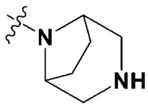;

and

each $R_{11}$ is independently selected from $C_1$-$C_6$ alkyl and oxo, preferably, each $R_{11}$ is independently selected from methyl and oxo,

wherein the wavy line ∿∿∿ represents the point of attachment of the group to the rest of the molecule.

In some embodiments of the present invention, ring C is

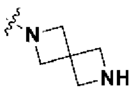, 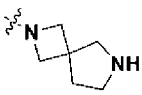,

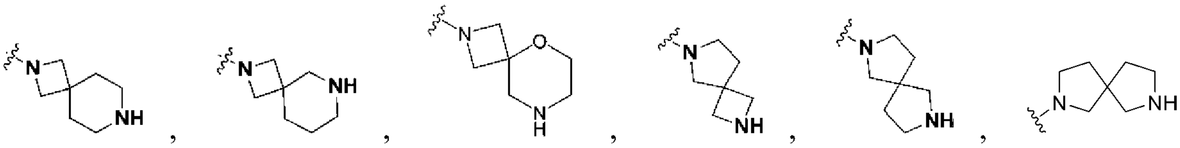, , , , , ,

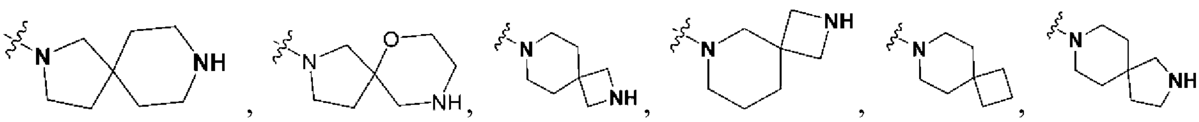, , , , , ,

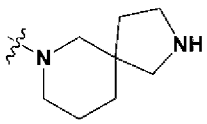, 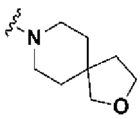, 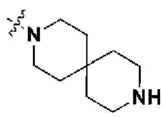, 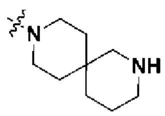, 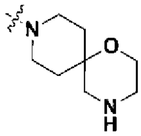, 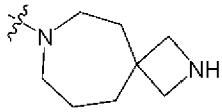,

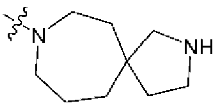, 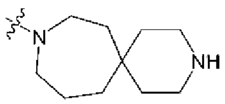, 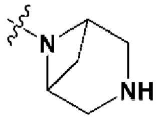, 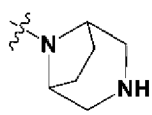, or 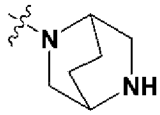,

more preferably

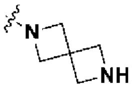, 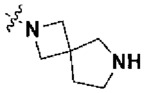, 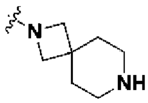, 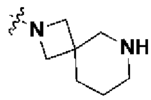, 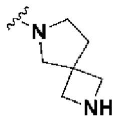, 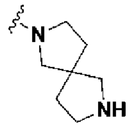,

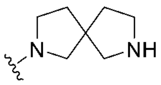, 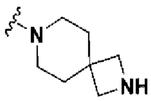, 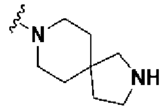, 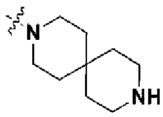, 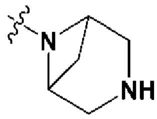, or 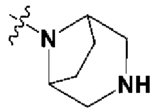;

and

each $R_{11}$ is independently selected from $C_1$-$C_6$ alkyl and oxo, preferably, each $R_{11}$ is independently selected from methyl and oxo,

wherein the wavy line 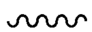 represents the point of attachment of the group to the rest of the molecule.

In some embodiments of the present invention, in the compound of formula 1A-1:

ring C is 3-10-membered monocyclic heterocyclyl or 5-10-membered heteroaryl, preferably 6-7-membered monocyclic heterocyclyl or 5-6-membered heteroaryl, more preferably piperidinyl, piperazinyl, azepanyl, 1,4-diazepanyl, or pyrazolyl;

each $R_{11}$ is independently selected from $C_1$-$C_6$ alkyl, oxo, 5-6-membered heteroaryl, 5-7-membered heterocyclyl, and $R_6C(O)$-, wherein each of the $C_1$-$C_6$ alkyl, 5-6-membered heteroaryl, or 5-7-membered heterocyclyl is optionally substituted by 1, 2, 3, or 4 $R_{15}$;

$R_6$ is $C_4$-$C_6$ cycloalkyl; and each $R_{15}$ is independently selected from oxo and $C_1$-$C_6$ alkyl,

preferably, ring C is 3-10-membered monocyclic heterocyclyl, preferably 6-7-membered monocyclic heterocyclyl, more preferably piperidinyl, piperazinyl, azepanyl, 1,4-diazepanyl;

preferably, each $R_{11}$ is independently selected from $C_1$-$C_6$ alkyl, oxo, pyrazolyl, piperidinyl, and $C_4$-$C_6$ cycloalkyl-C(O)-, wherein the piperidinyl is optionally substituted by 1 or 2 substituent selected from $C_1$-$C_6$ alkyl and oxo;

more preferably, each $R_{11}$ is independently selected from methyl, oxo, pyrazolyl, N-methylpiperidinyl, oxopiperidinyl, and cyclobutylformyl.

According to some embodiments of the present invention, the compound of the present invention is the compound of formula 1A-1a, 1A-1b, or 1A-1c:

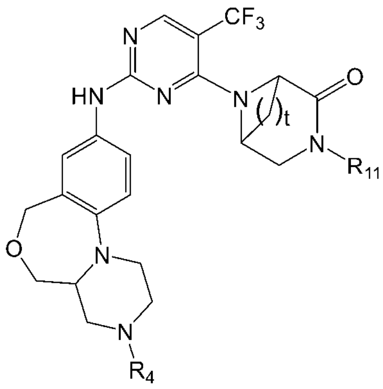
(1A-1a),

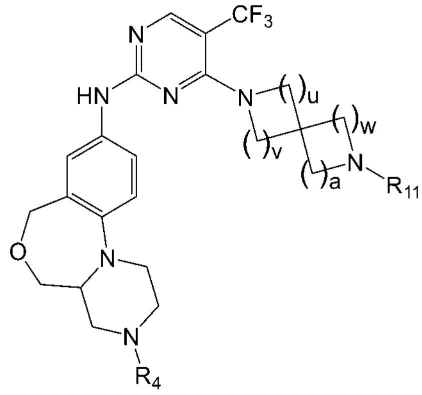
(1A-1b),

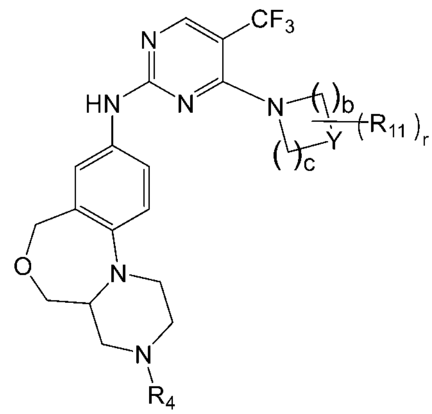
(1A-1c);

wherein $R_4$ is as defined hereinbefore for the compound of formula (1); t is selected from 0, 1, 2, and 3; u, v, w, a, b, and c are each independently selected from 1, 2, and 3; Y is selected from -CH- and -N-; and r is selected from 1 and 2.

According to some embodiments of the present invention, the compound of the present invention is the compound of formula 1A-2:

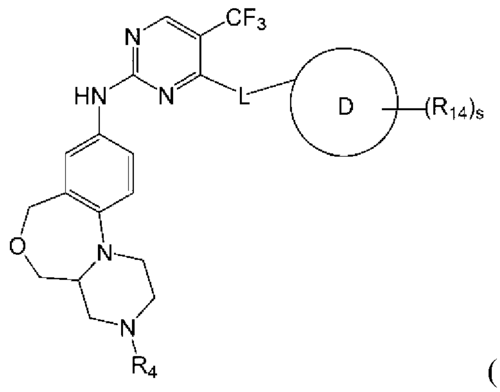
(1A-2)

wherein $R_4$ is as defined hereinbefore for the compound of formula (1);

L is $-(L_1)_m-(L_2)_n-(L_3)_p-$, preferably, L is $-(L_1)_m-(L_2)_n-(L_3)_p-*$, wherein the bond marked with "*" is attached to ring D;

$L_1$ is selected from -O-, -NH-, and $-CH_2-O-$, preferably, when $L_1$ is $-CH_2-O-$, the oxygen of the $-CH_2-O-$ is attached to $L_2$, preferably, $L_1$ is selected from -O-, -NH-;

$L_2$ is selected from $C_1$-$C_6$ alkylene, $C_3$-$C_{10}$ cycloalkylene, and 3-10-membered heterocyclylene, preferably selected from $C_1$-$C_6$ alkylene, $C_3$-$C_6$ cycloalkylene, and 3-6-membered heterocyclylene, preferably selected from $C_1$-$C_6$ alkylene and $C_3$-$C_6$ cycloalkylene, preferably selected from ethylene, propylene, cyclohexylidene, and piperidylidene, more preferably selected from ethylene, propylene, and cyclohexylidene;

$L_3$ is selected from -NH-, -NHC(O)-, and -C(O)-, preferably, when $L_3$ is -NHC(O)-, the amino moiety of the -NHC(O)- is attached to $L_2$, and the carbonyl moiety is attached to ring D;

m, n, and p are each independently selected from 0 and 1, and m, n, and p are not simultaneously 0;
preferably, L is selected from -O-, -O-cyclohexylidene-, -NH-cyclohexylidene-, -NH-piperidylidene-, -O-propylene-, -NH-propylene-, -ethylene-, -ethylene-NHC(O)-, and -O-propylene-C(O)-;
preferably, L is selected from -O-, -O-cyclohexylidene-, -NH-cyclohexylidene-, -NH-piperidylidene-C(O)-, -O-propylene-, -NH-propylene-, -O-ethylene-NH-, -ethylene-NHC(O)-, -NH-propylene-C(O)-, and -O-propylene-C(O)-;
preferably, L is selected from -O-, -O-cyclohexylidene-*, -NH-cyclohexylidene-*, -NH-piperidylidene-C(O)-*, -O-propylene-*, -NH-propylene-*, -O-ethylene-NH-*, -ethylene-NHC(O)-*, -NH-propylene-C(O)-*, and -O-propylene-C(O)-*, wherein the bond marked with "*" is attached to ring D;
more preferably, L is selected from -O-propylene-*, -O-cyclohexylidene-*, -NH-propylene-*, -NH-cyclohexylidene-*, -NH-piperidylidene-C(O)-*, wherein the bond marked with "*" is attached to ring D;
ring D is selected from $C_3$-$C_{10}$ cycloalkyl, $C_6$-$C_{10}$ aryl, 5-10-membered heteroaryl, and 3-10-membered heterocyclyl, preferably selected from $C_3$-$C_6$ cycloalkyl, $C_6$-$C_{10}$ aryl, 5-6-membered heteroaryl, and 5-7-membered heterocyclyl, preferably selected from cyclobutyl, phenyl, pyrazolyl, piperidinyl, piperazinyl, pyrrolidinyl, imidazolidinyl, morpholinyl, 1,3-oxazinanyl, azepanyl, and 1,4-oxazepanyl;
each $R_{14}$ is independently selected from H, halogen, cyano, oxo, and $C_1$-$C_6$ alkyl; and
s is selected from 0, 1, and 2.

According to some embodiments of the present invention, the compound of the present invention is the compound of formula 1A-2a, 1A-2b, 1A-2c, 1A-2d, 1A-2e, or 1A-2f:

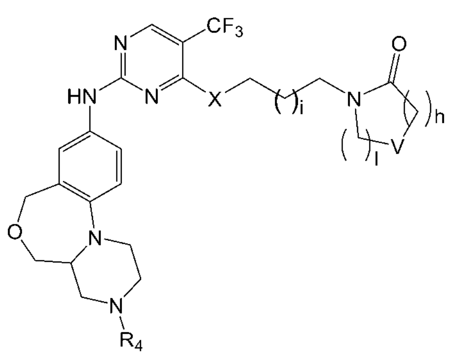

(1A-2a),

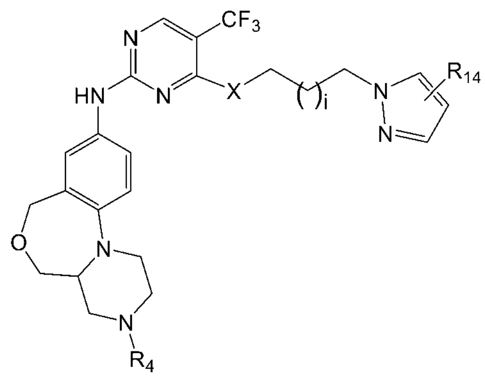

(1A-2b),

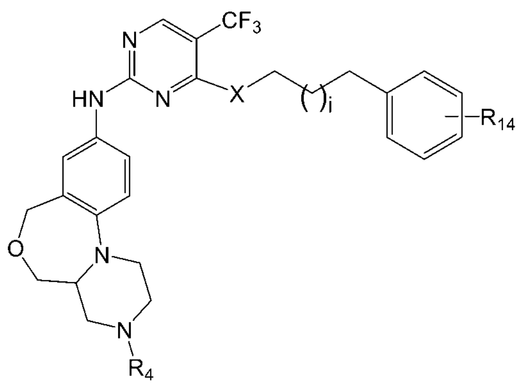

(1A-2c),

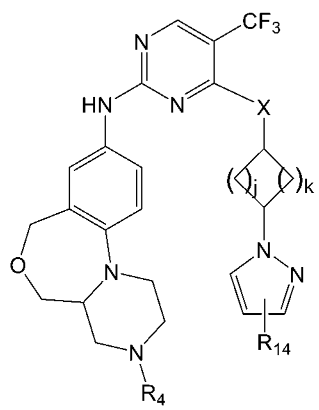

(1A-2d),

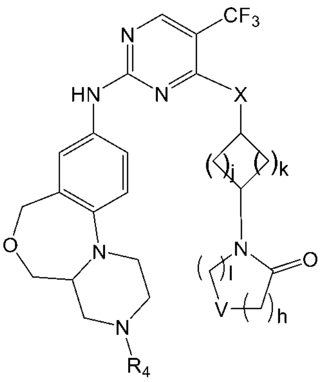
(1A-2e),
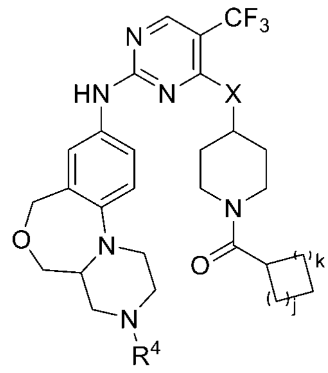
(1A-2f);

wherein $R_4$, $R_{14}$ are as defined hereinbefore for the compound of formula (1A-2); X is selected from -O-, -NH-, and $-CH_2-O-$, preferably, X is selected from -O- and-NH-; V is selected from -O-, -S-, $-NR_{12}-$, and $-CHR_{12}-$, preferably, V is selected from -O- and $-CHR_{12}-$; $R_{12}$ is selected from H and $C_1-C_6$ alkyl; h and 1 are each independently selected from 0, 1, 2, and 3; i is selected from 0, 1, and 2; and j and k are each independently selected from 1, 2, and 3.

According to some embodiments of the present invention, the compound of the present invention is the compound of formula 1B-2a, 1C-2a, or 1D-2a:

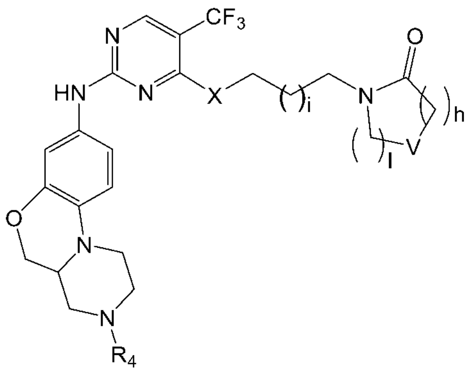
(1B-2a),
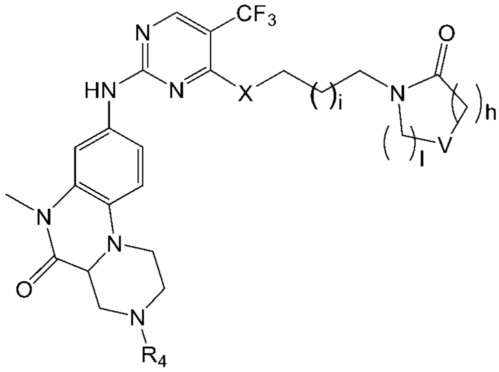
(1C-2a),

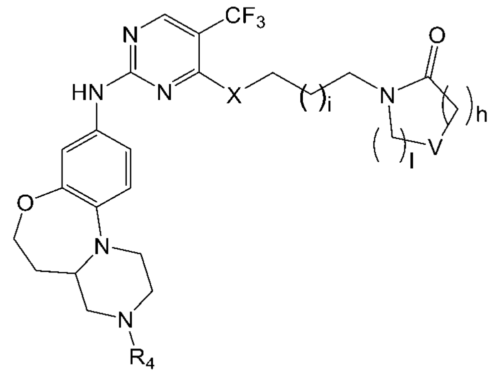
(1D-2a);

wherein $R_4$ is as defined hereinbefore for the compound of formula (1); X is selected from -O-, -NH-, and $-CH_2-O-$, preferably, X is selected from -O- and-NH-, more preferably, X is selected from -O-; V is selected from -O-, -S-, $-NR_{12}-$, and $-CHR_{12}-$, preferably, V is selected from $-CHR_{12}-$; $R_{12}$ is selected from H and $C_1-C_6$ alkyl; h and 1 are each independently selected from 0, 1, 2, and 3; and i is selected from 0, 1, and 2.

According to some embodiments of the present invention, the compound of the present invention is selected from:

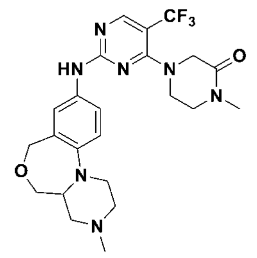 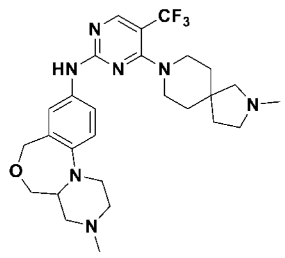 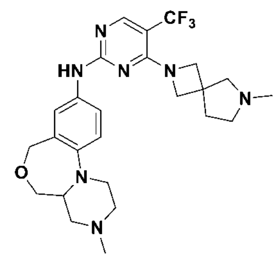 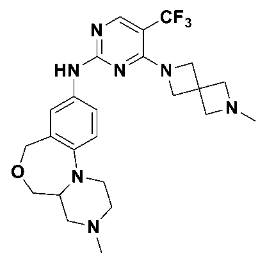

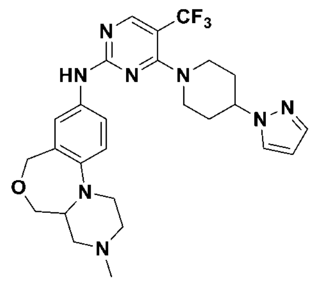 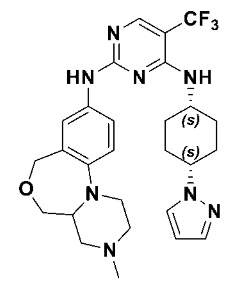 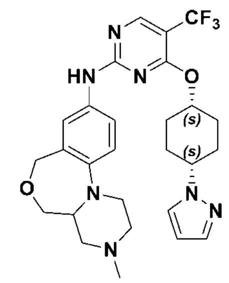 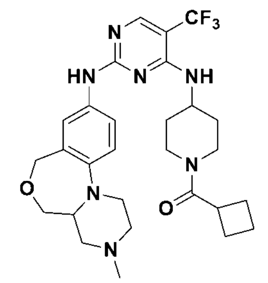

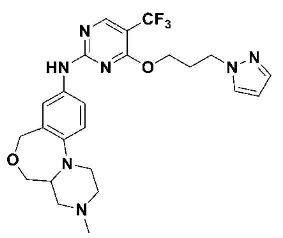 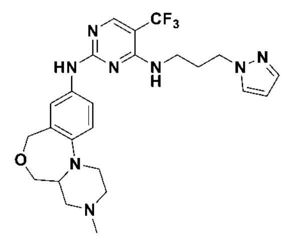 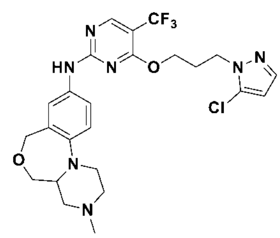 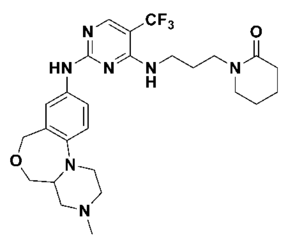

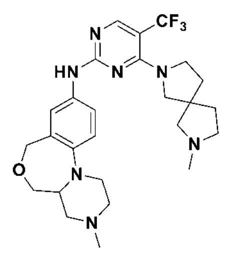 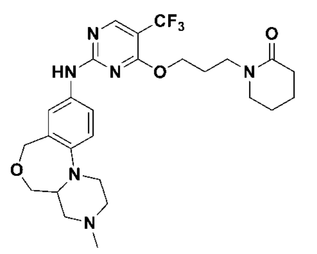 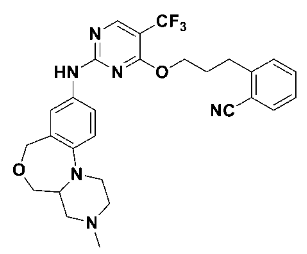 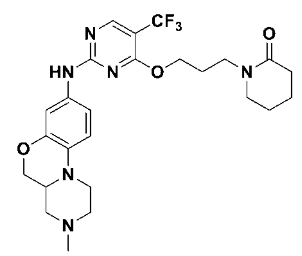

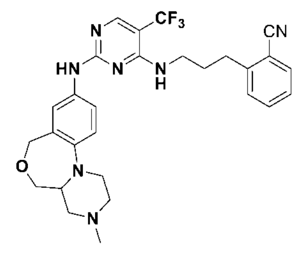 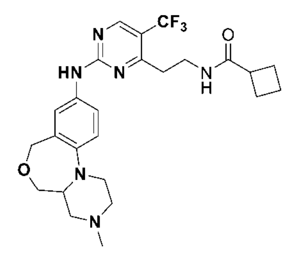 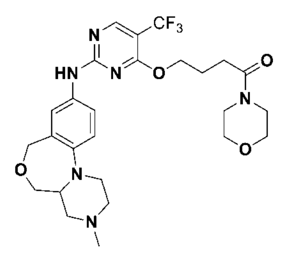 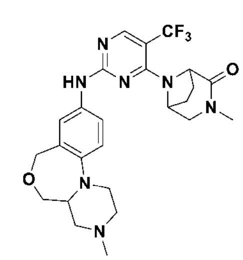

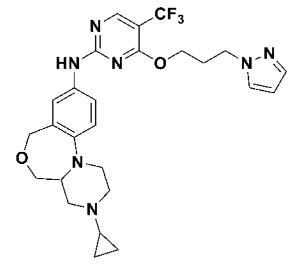 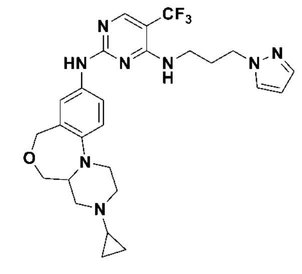 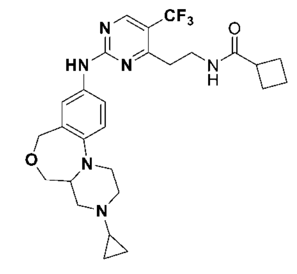 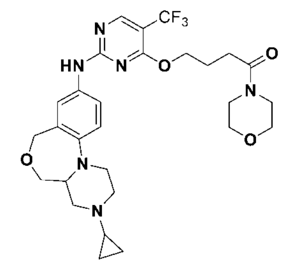

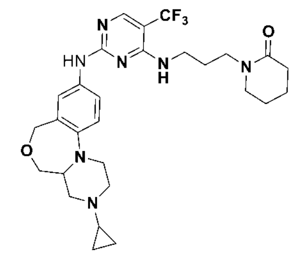 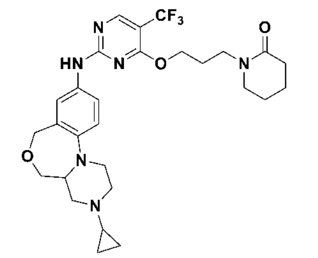 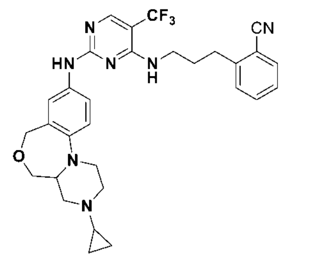 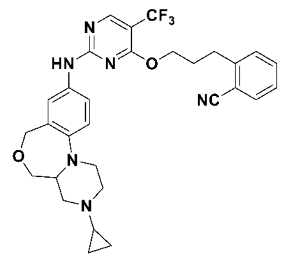

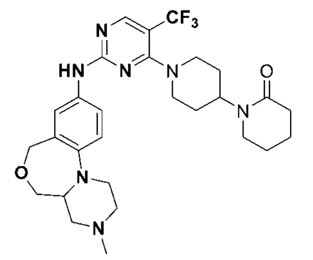 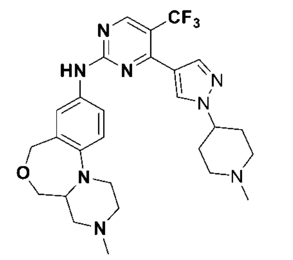 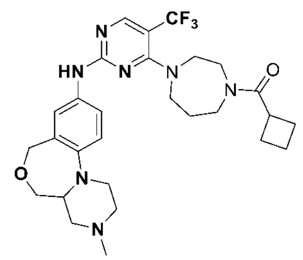 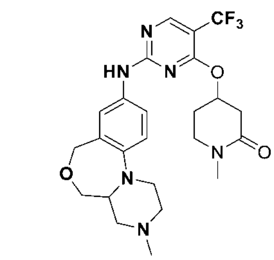

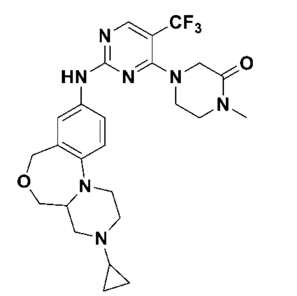 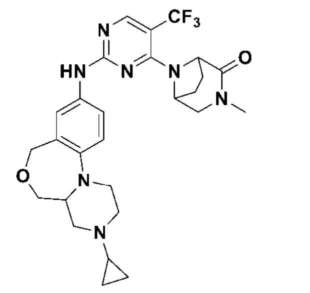 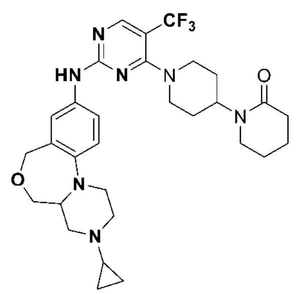 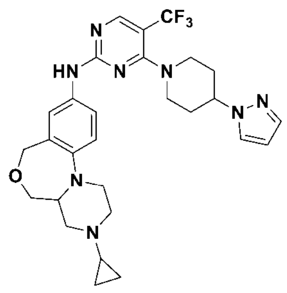

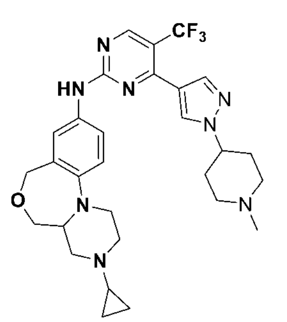 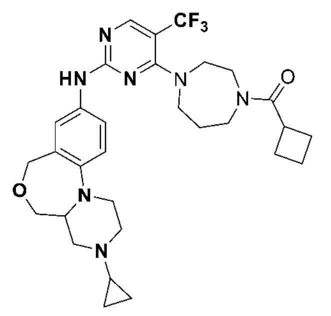 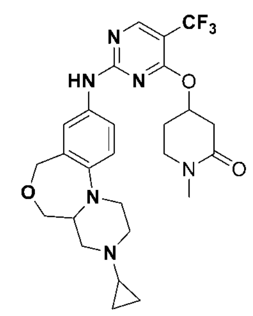 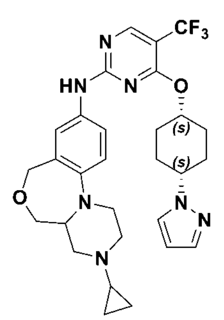

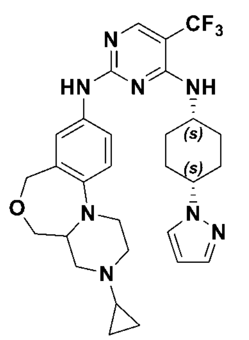 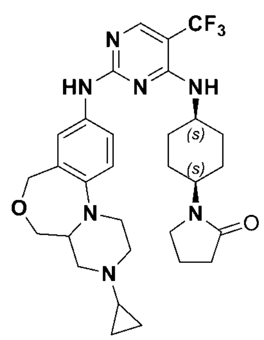 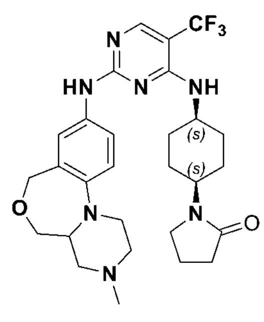 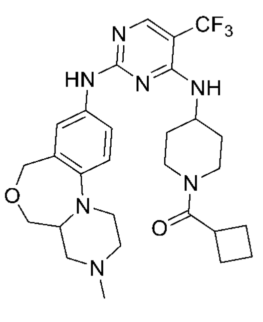

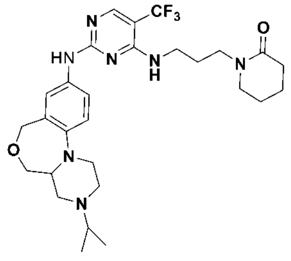
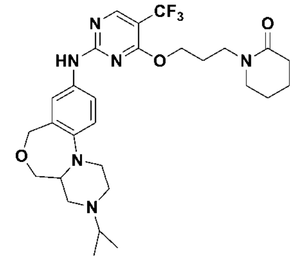
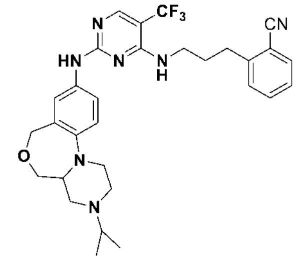
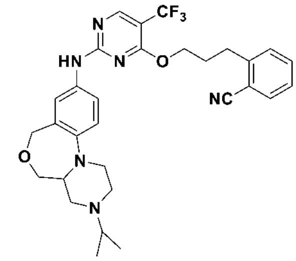

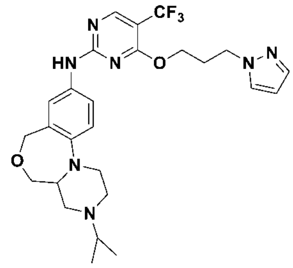
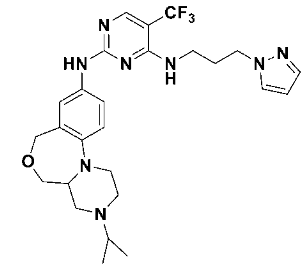
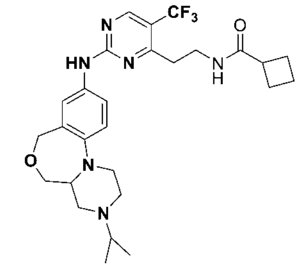
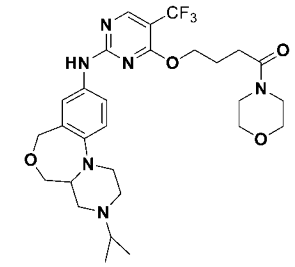

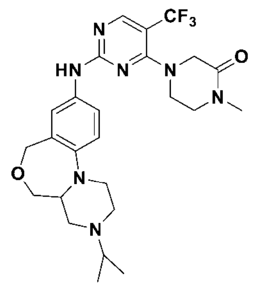
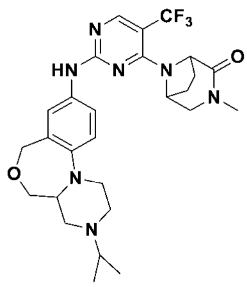
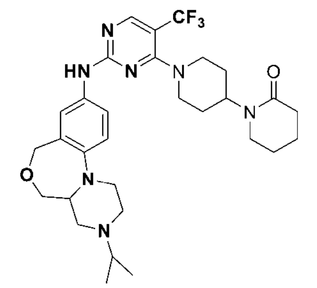
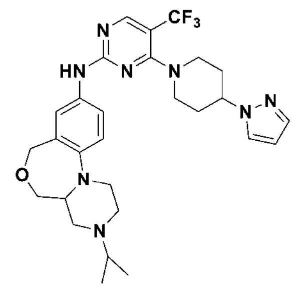

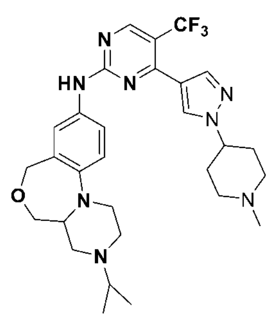
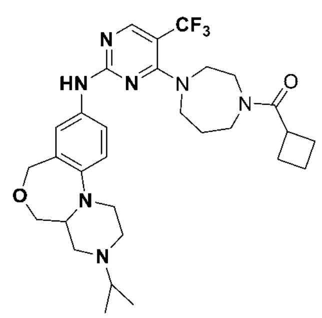
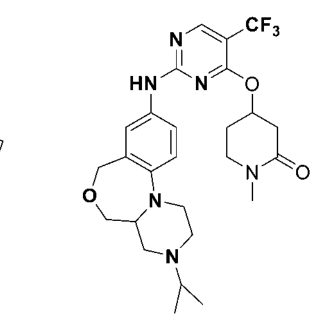
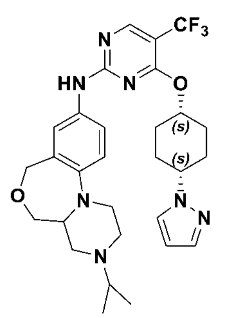

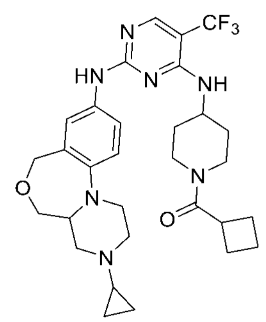
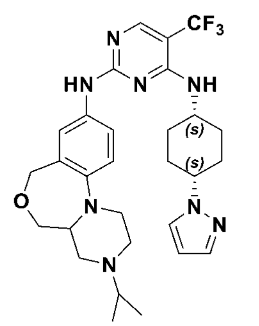
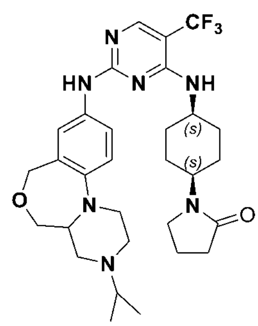
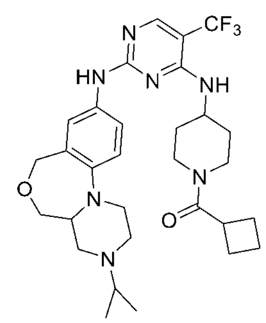

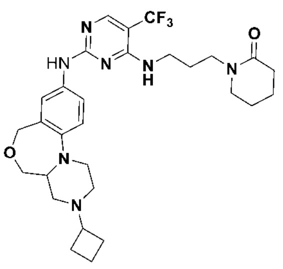
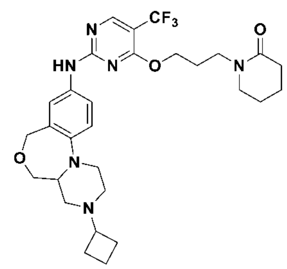
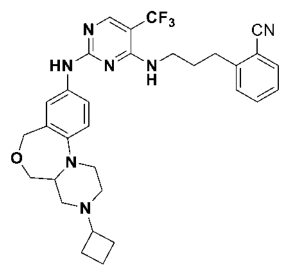
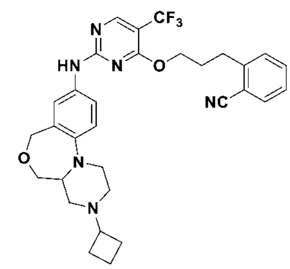

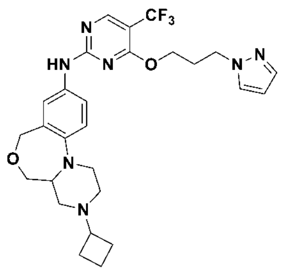
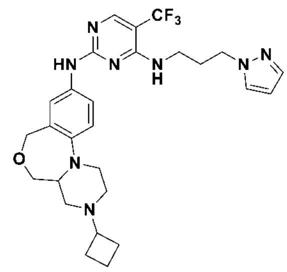
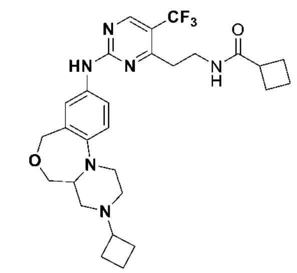
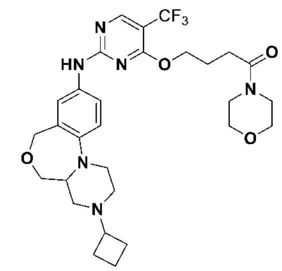

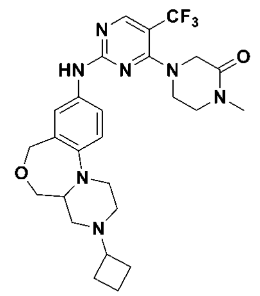
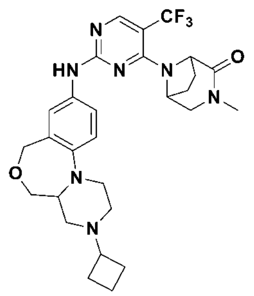
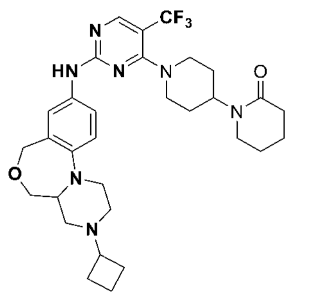
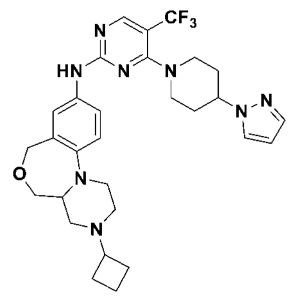

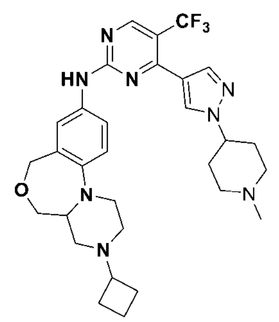
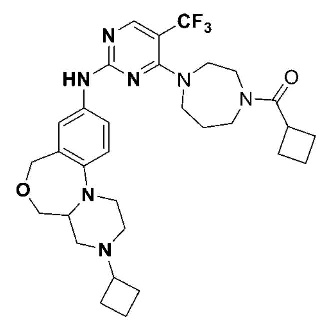
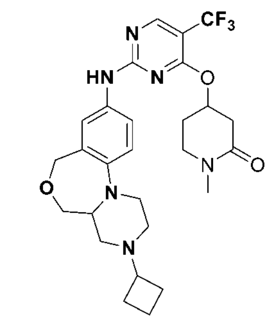
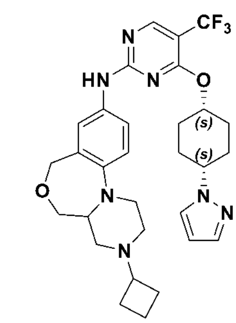

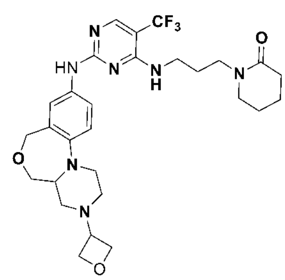
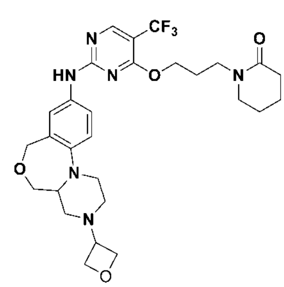
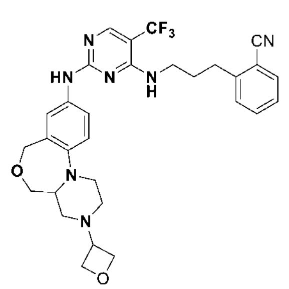
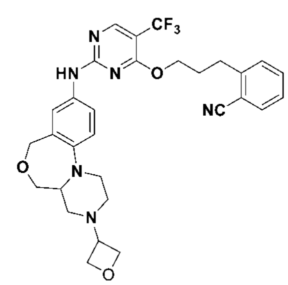

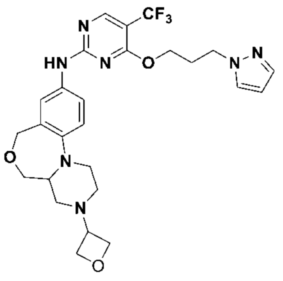
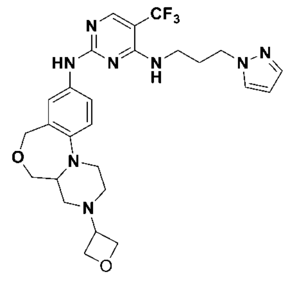
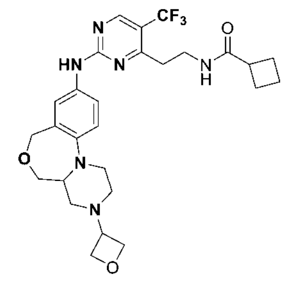
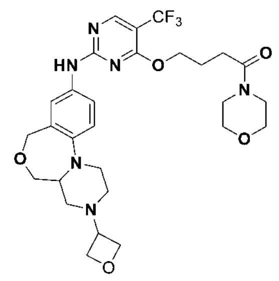
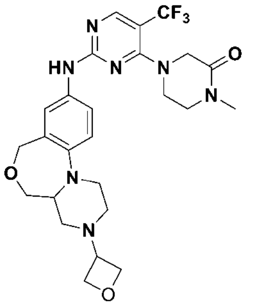
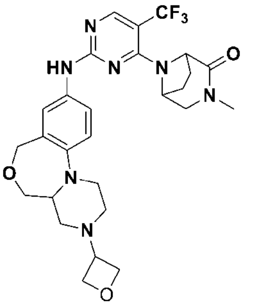
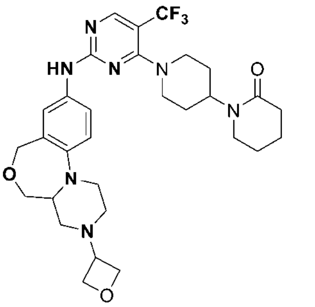
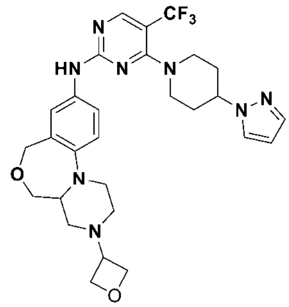
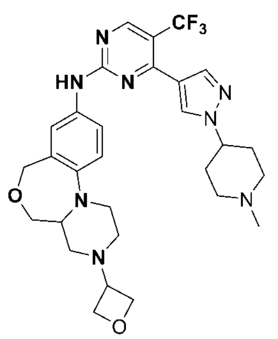
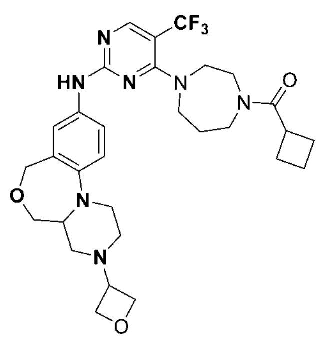
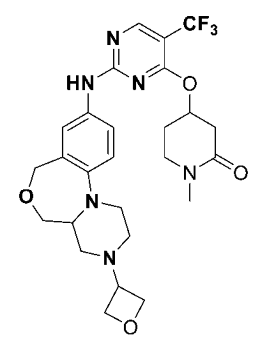
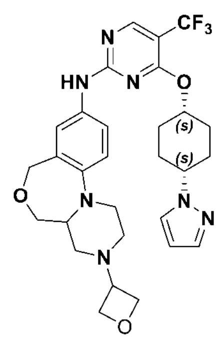
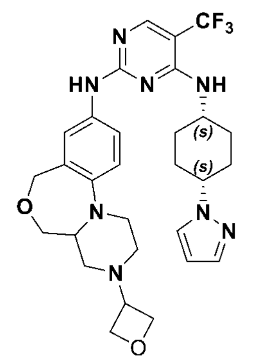
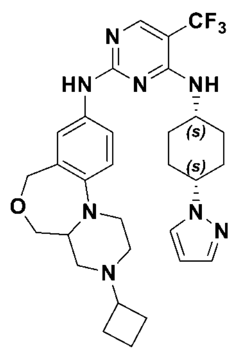
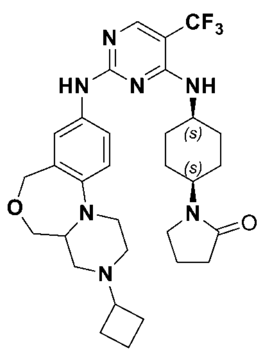
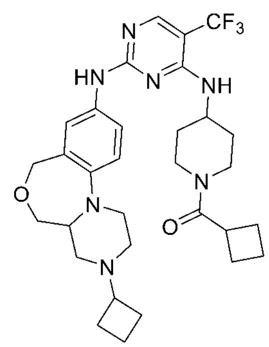
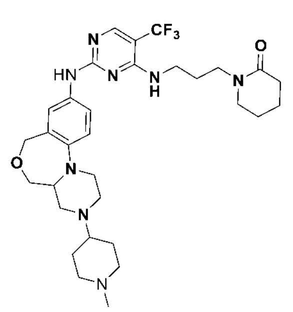
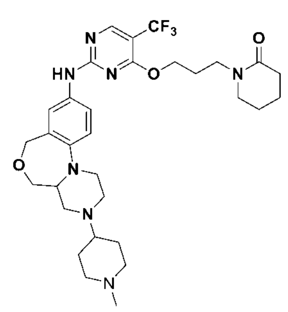
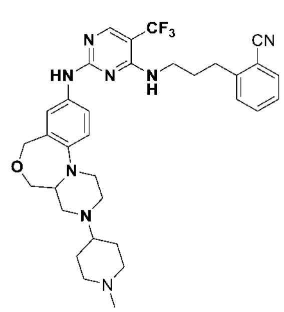
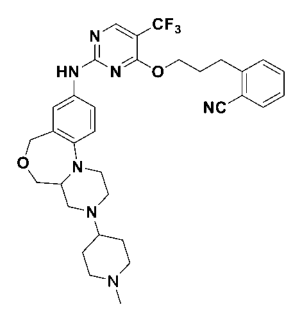

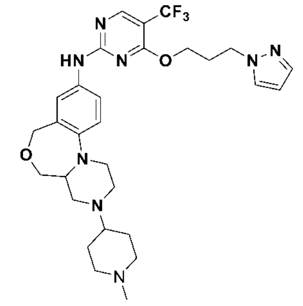
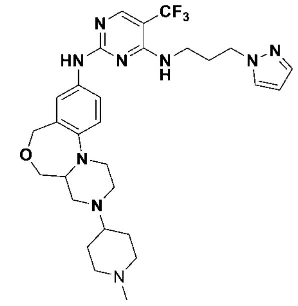
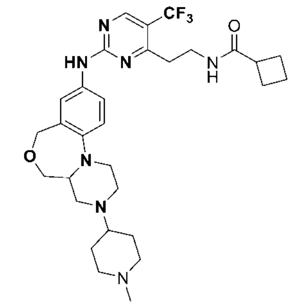
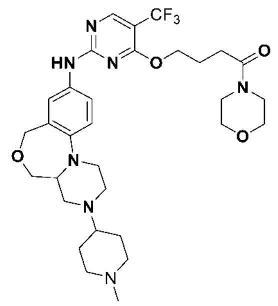

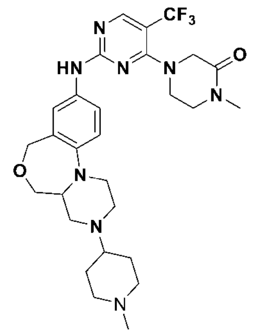
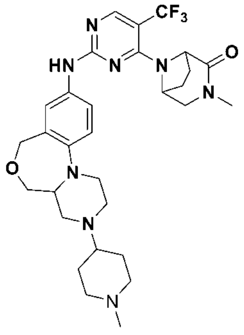
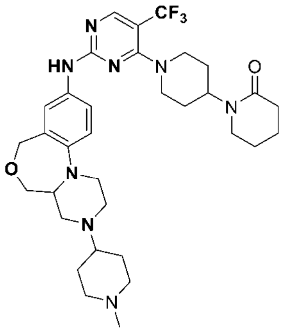
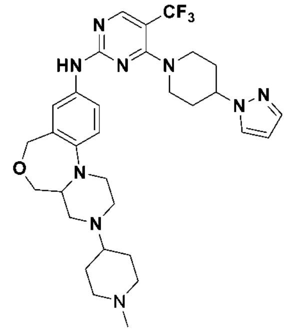

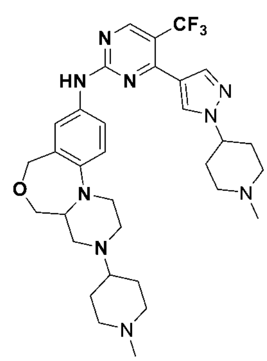
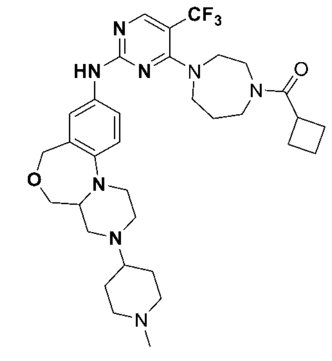
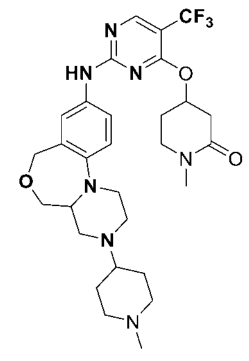
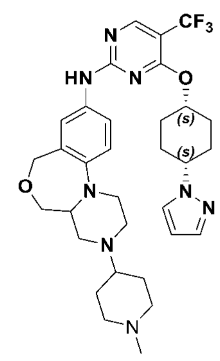

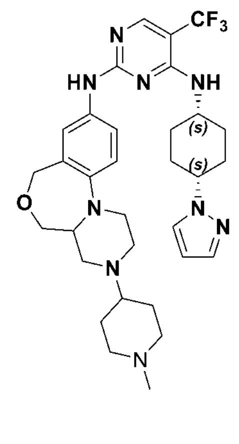
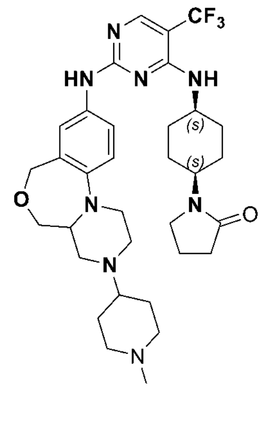
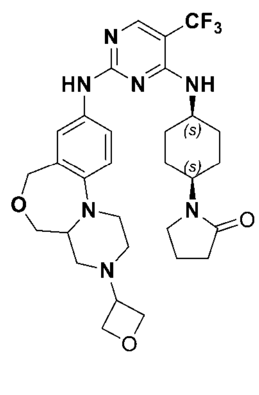
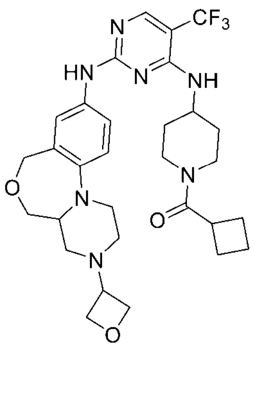

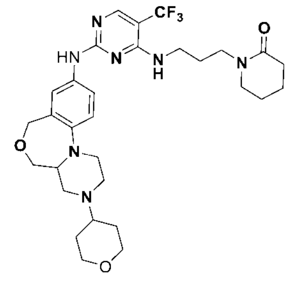
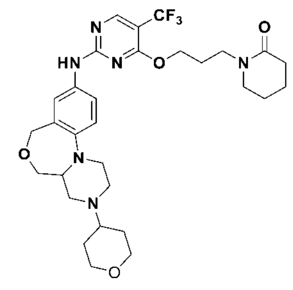
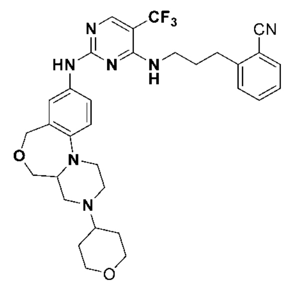
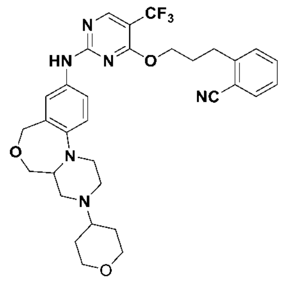
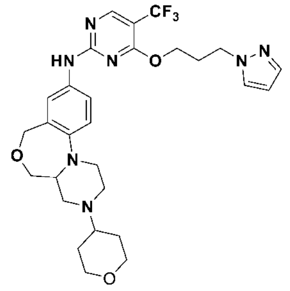
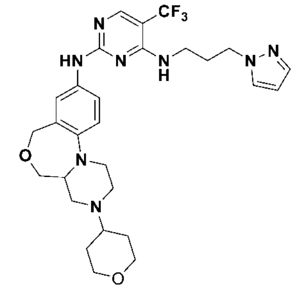
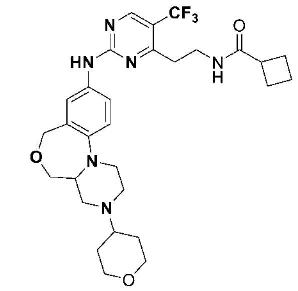
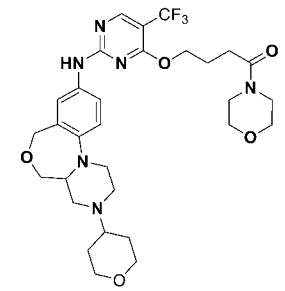
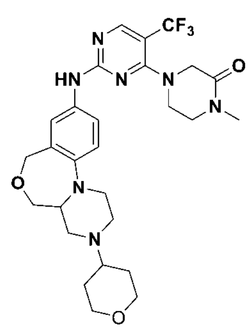
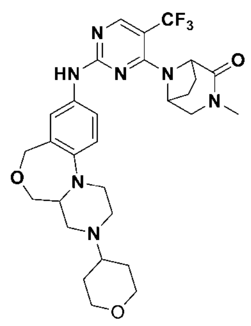
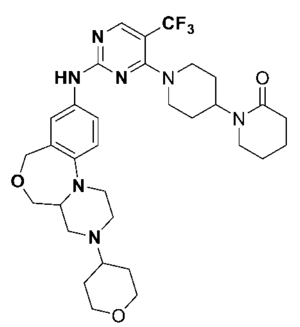
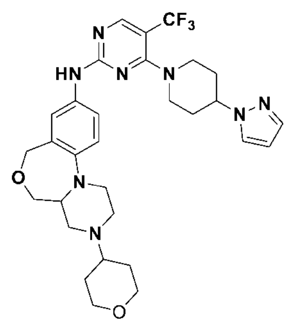
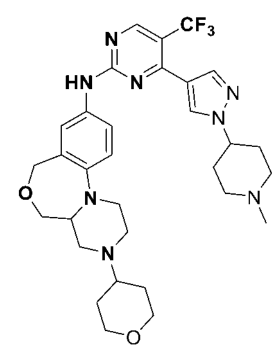
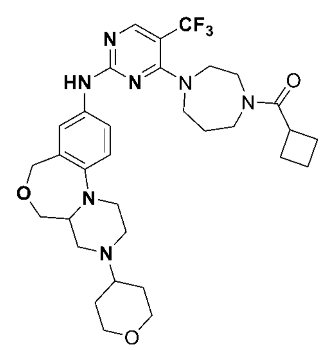
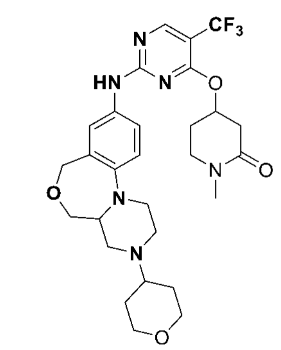
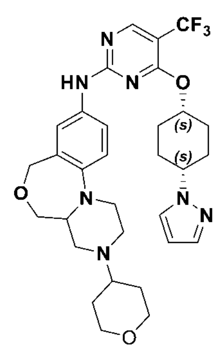
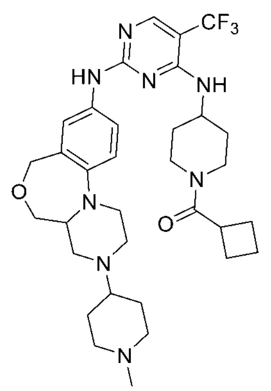
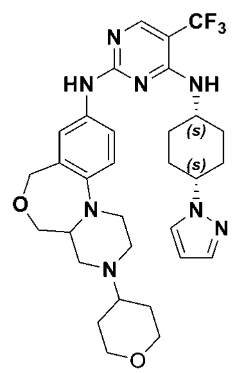
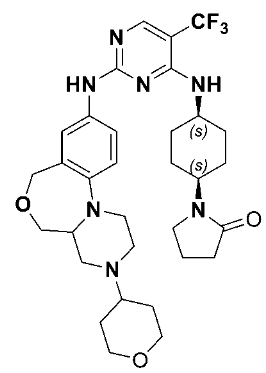
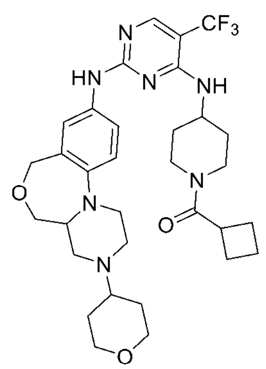

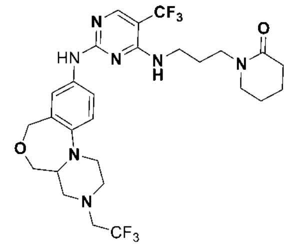
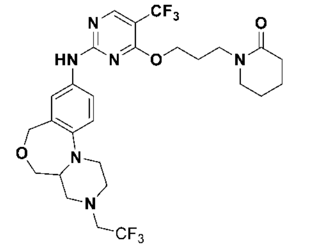
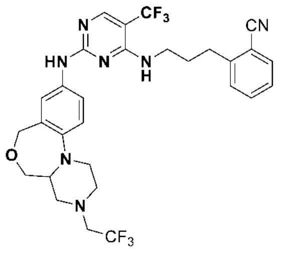
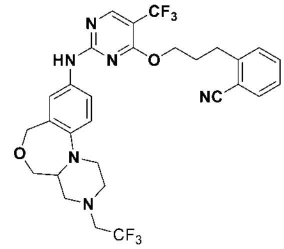

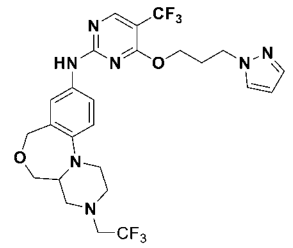
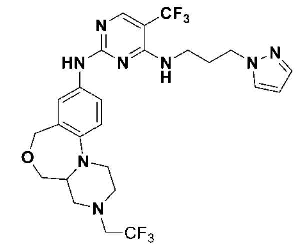
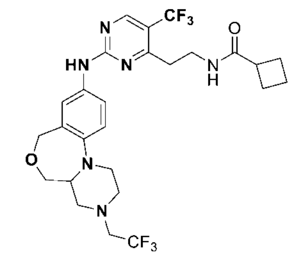
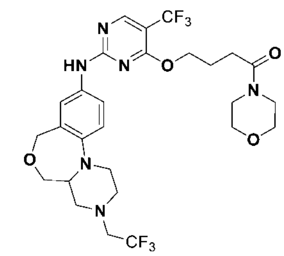

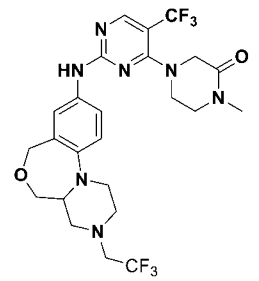
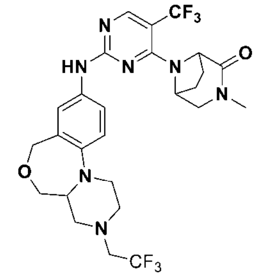
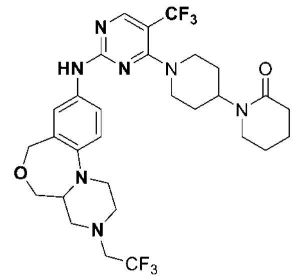
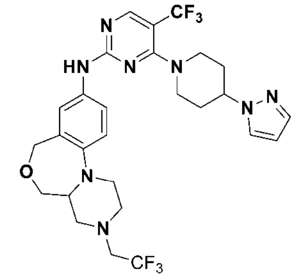

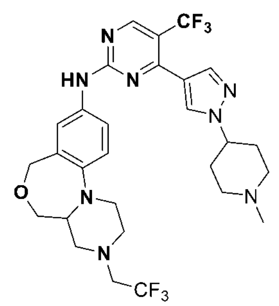
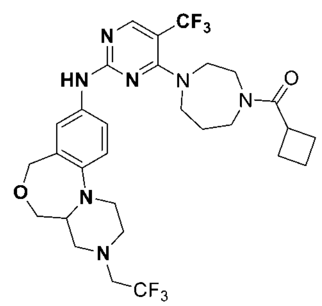
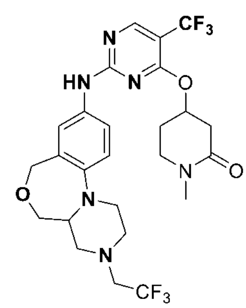
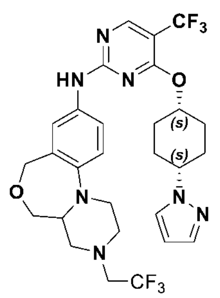

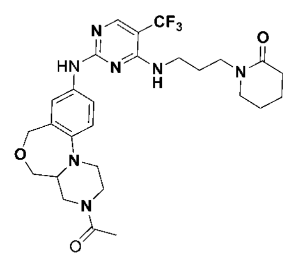
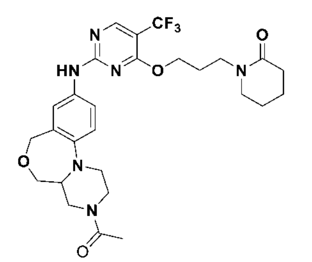
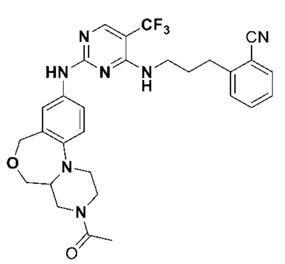
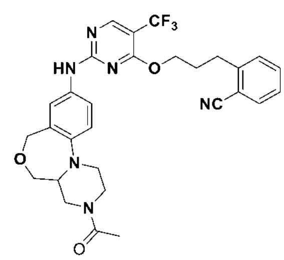

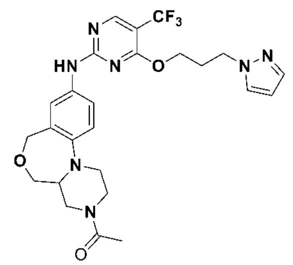
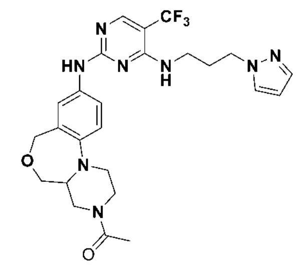
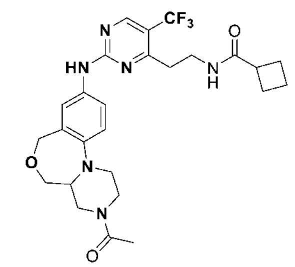
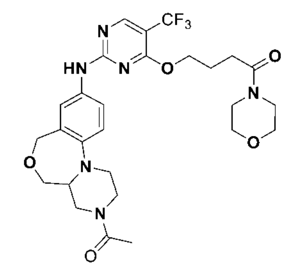

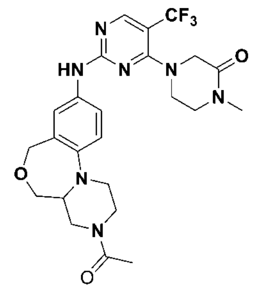
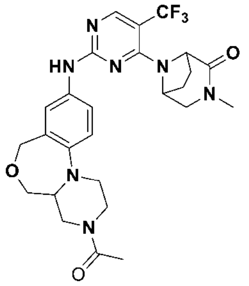
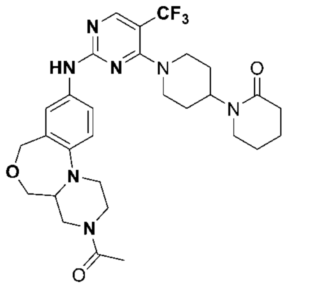
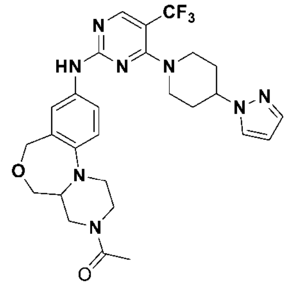

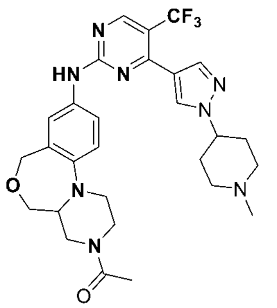
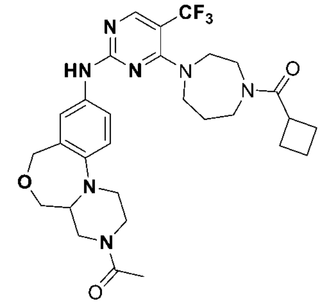
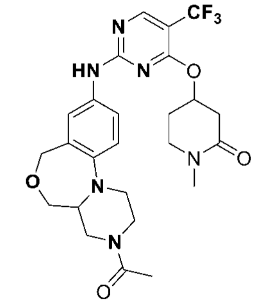
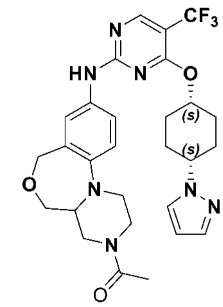

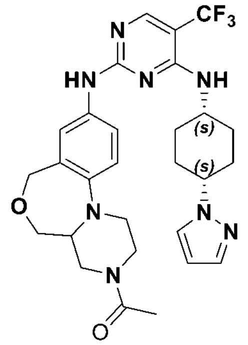
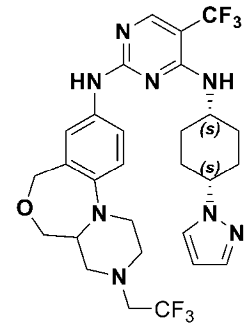
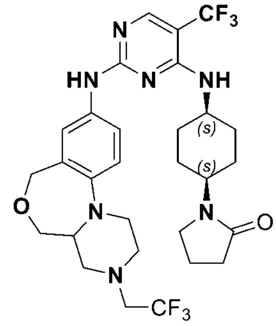
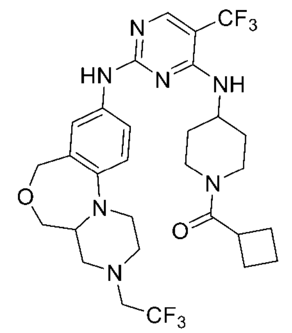

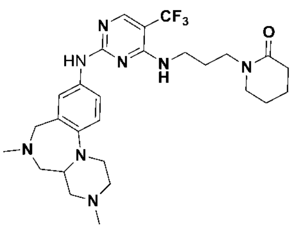
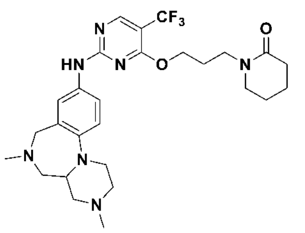
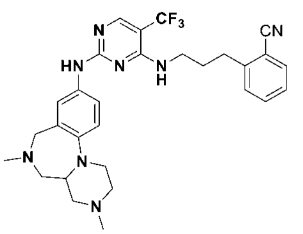
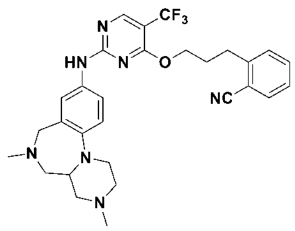

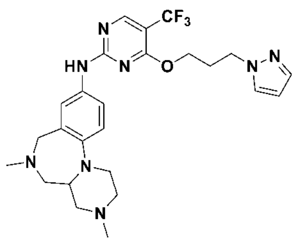
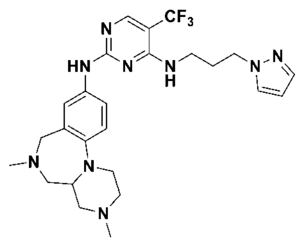
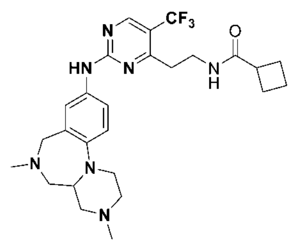
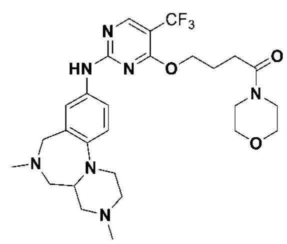

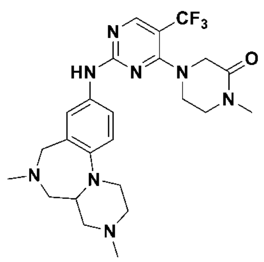
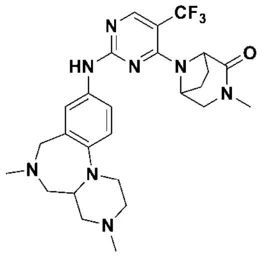
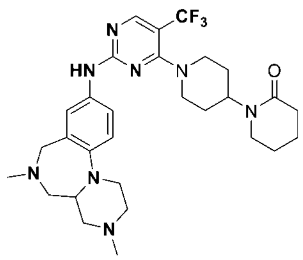
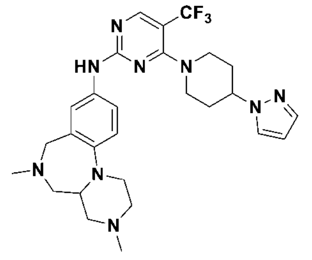
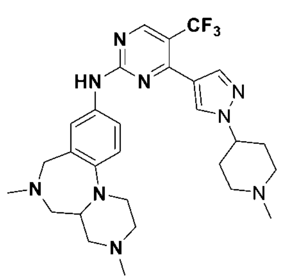
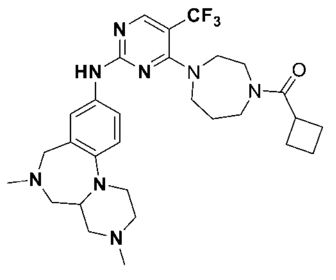
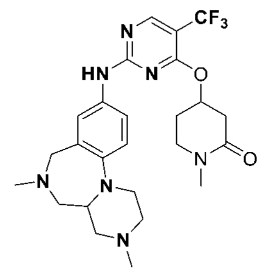
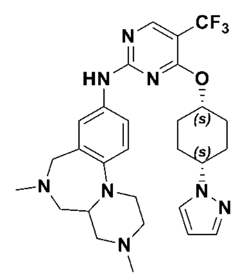
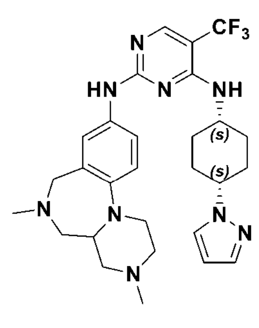
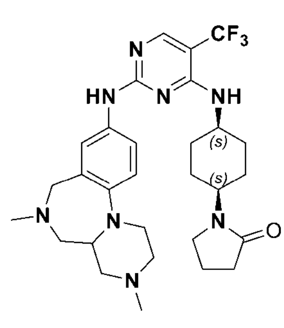
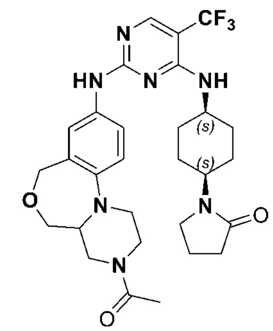
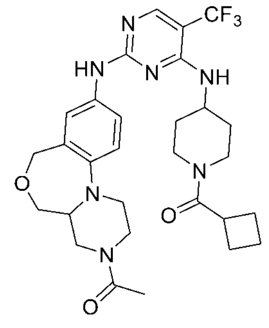
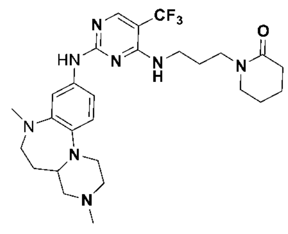
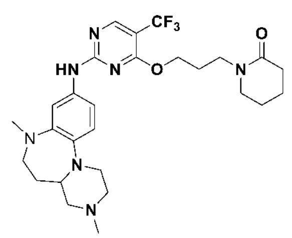
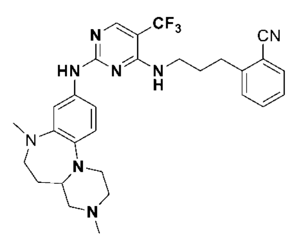
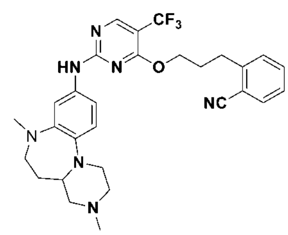
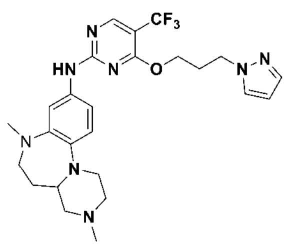
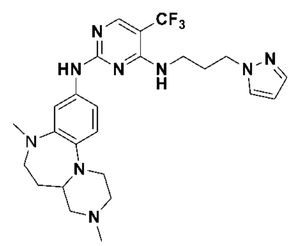
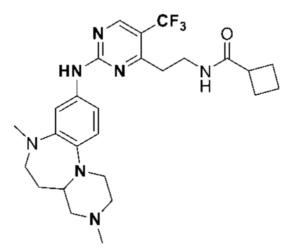
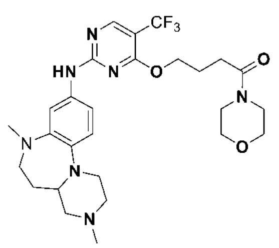

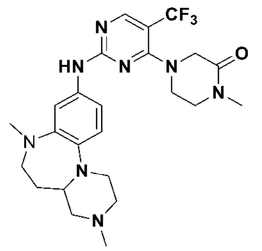
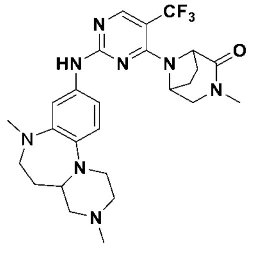
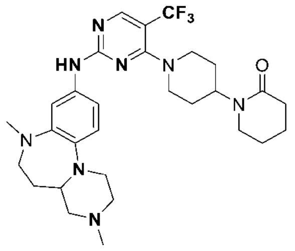
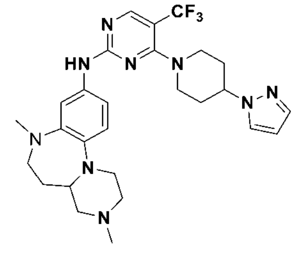

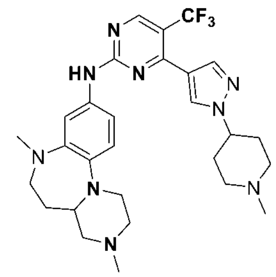
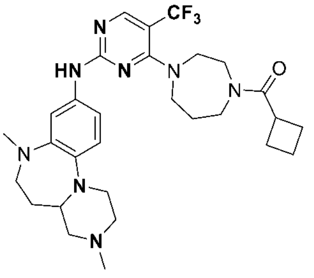
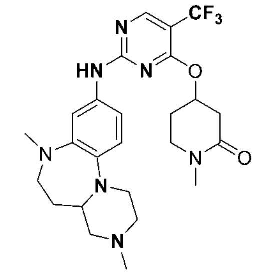
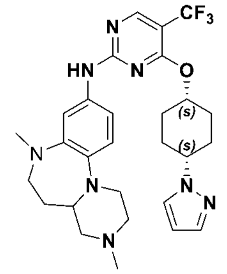

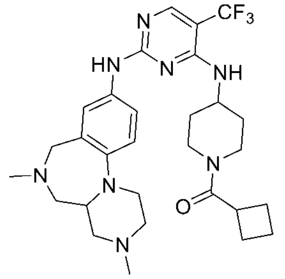
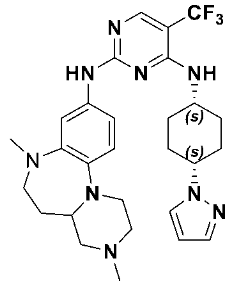
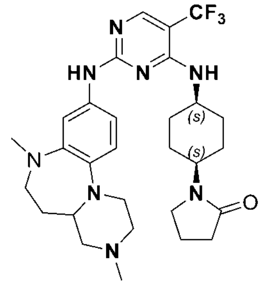
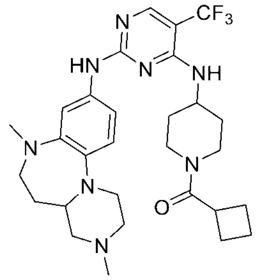

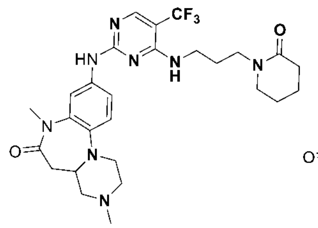
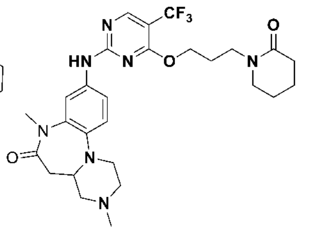
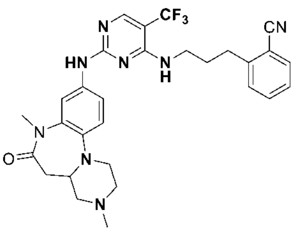
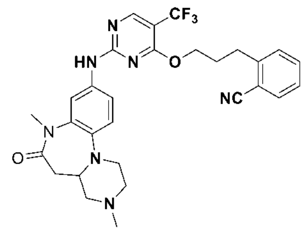

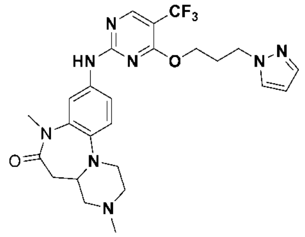
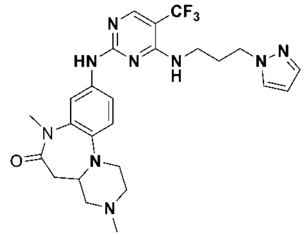
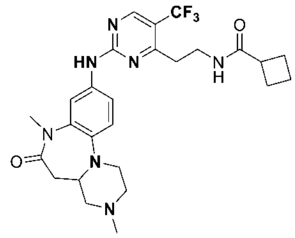
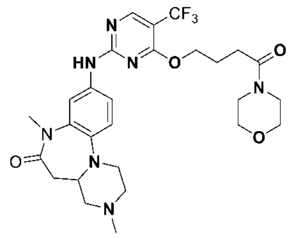

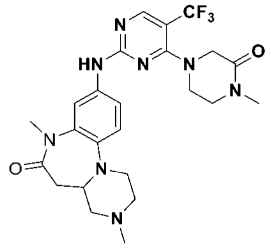
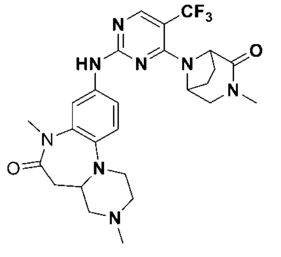
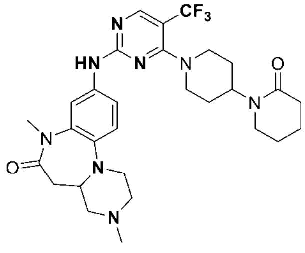
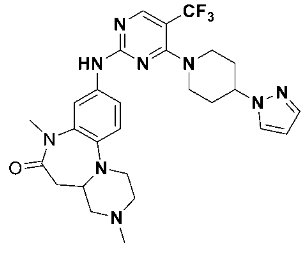

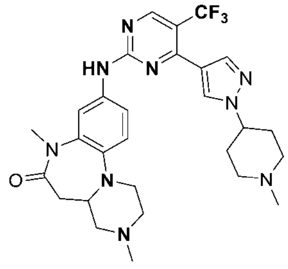
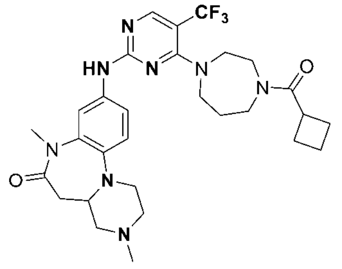
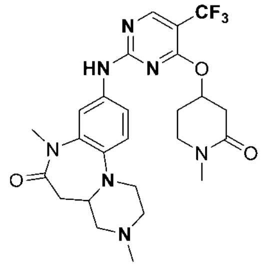
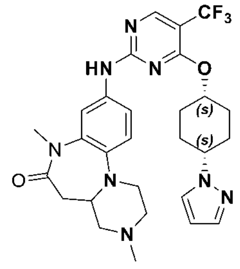

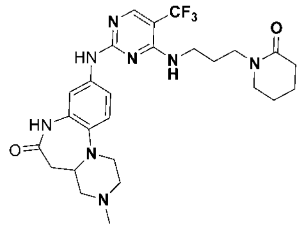
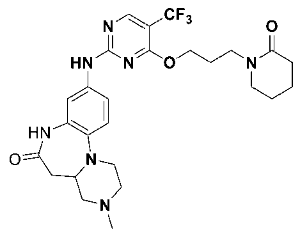
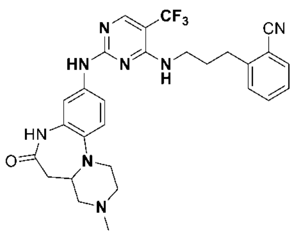
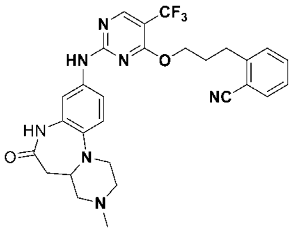

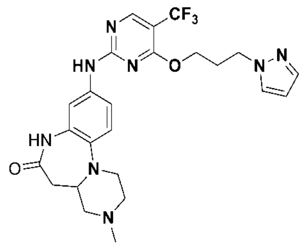
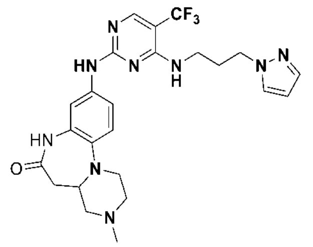
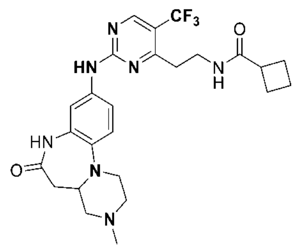
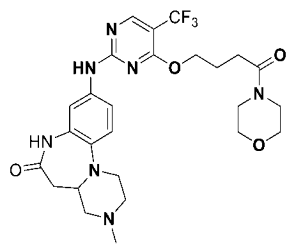

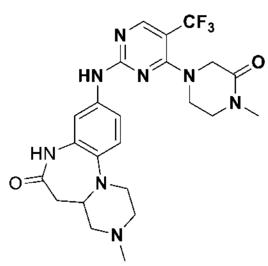
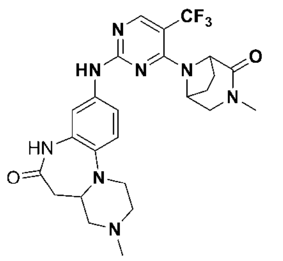
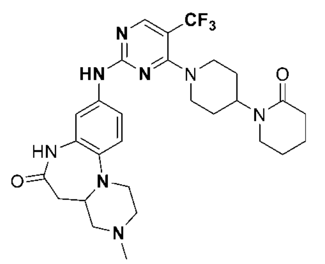
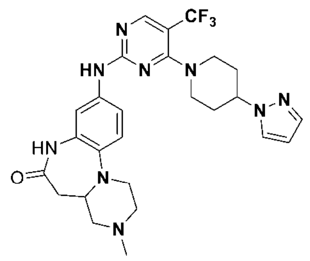

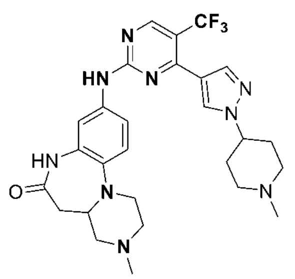
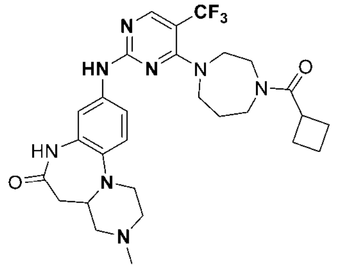
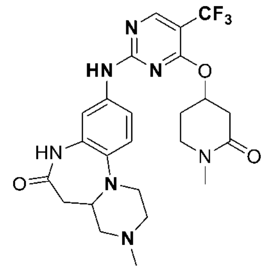
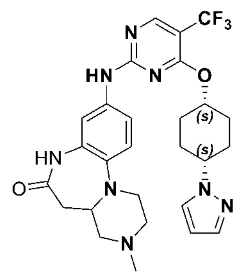

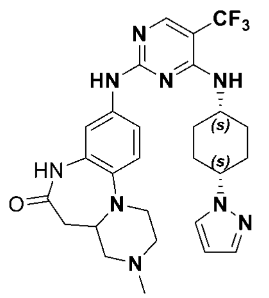
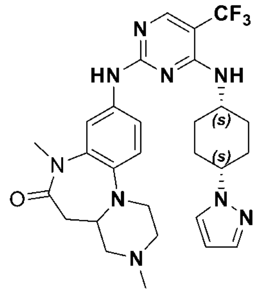
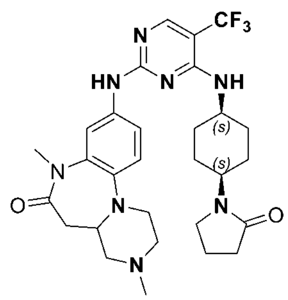
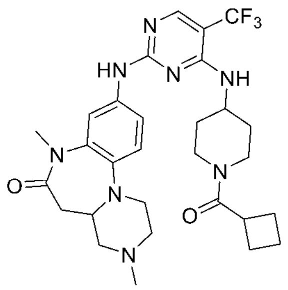

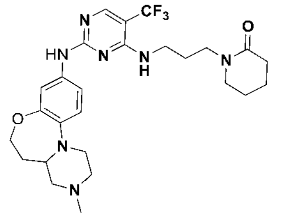
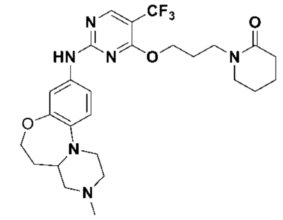
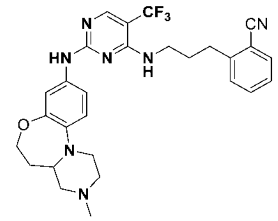
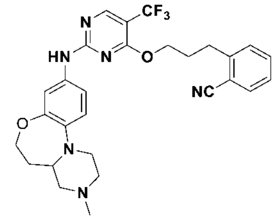

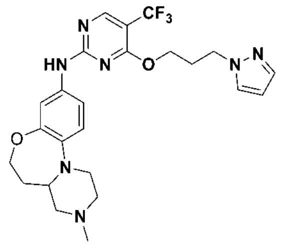
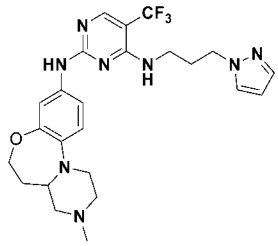
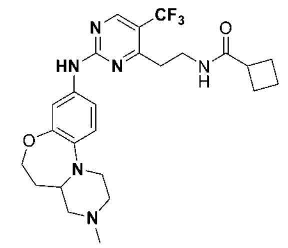
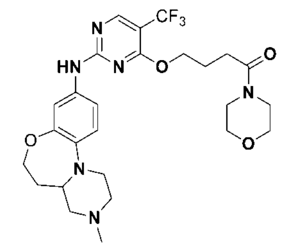

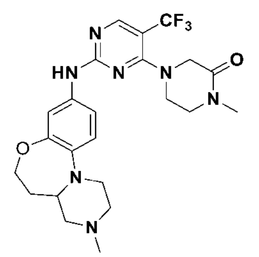
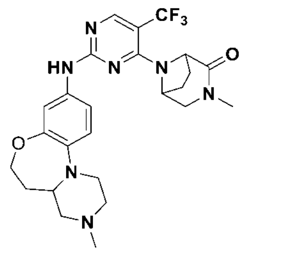
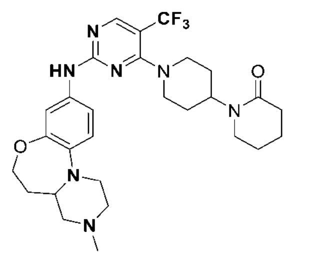
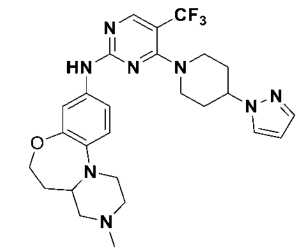

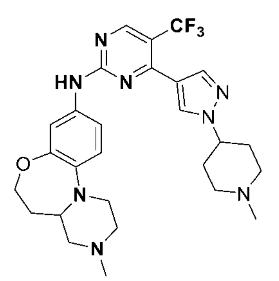
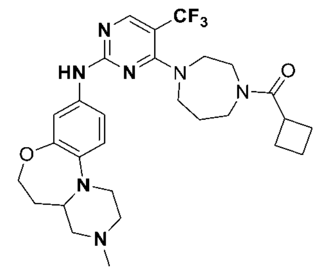
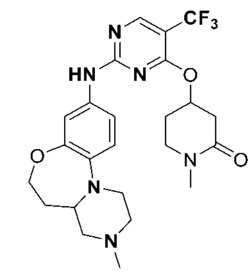
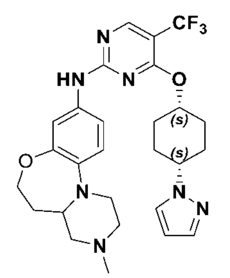

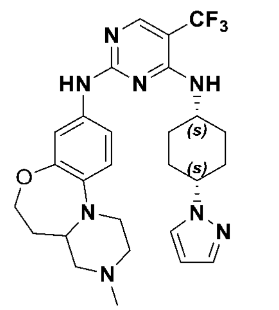
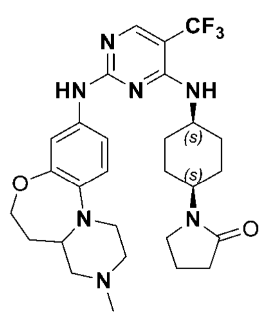
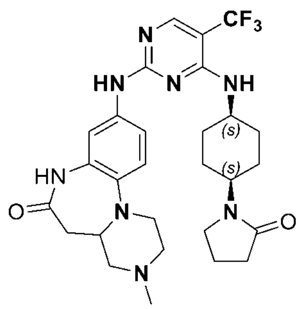
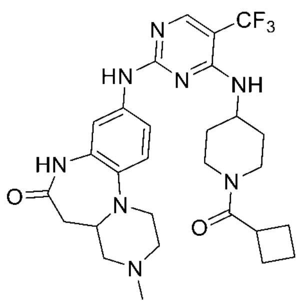

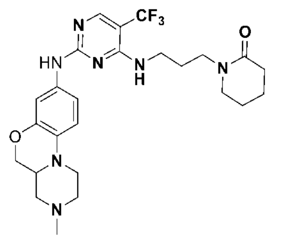
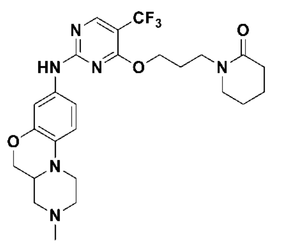
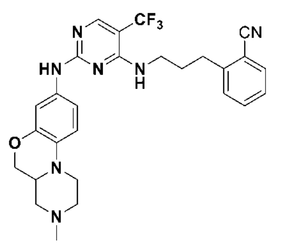
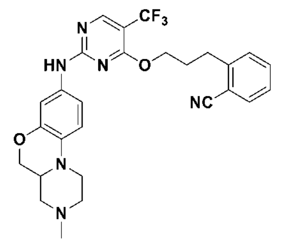

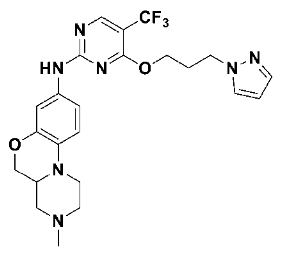
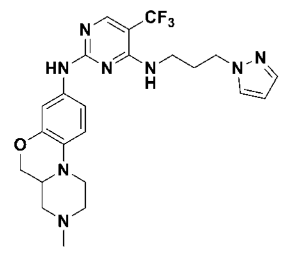
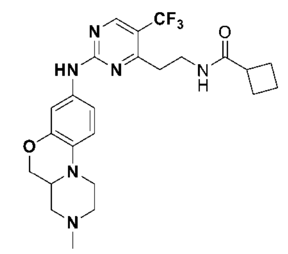
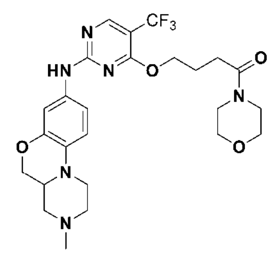

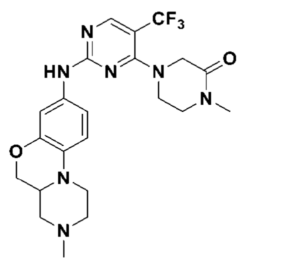
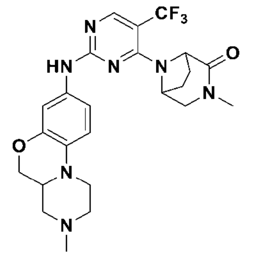
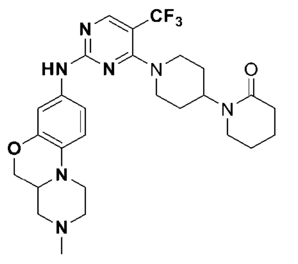
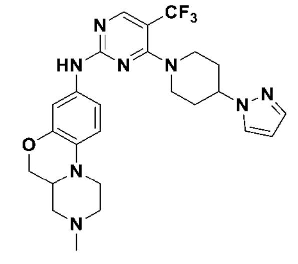

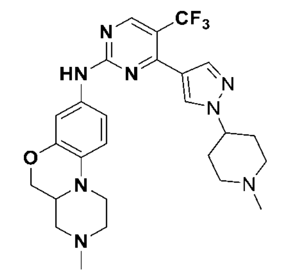
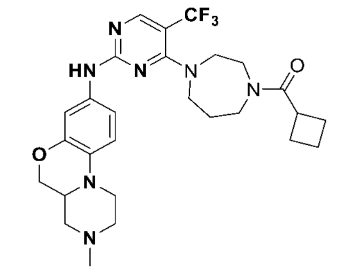
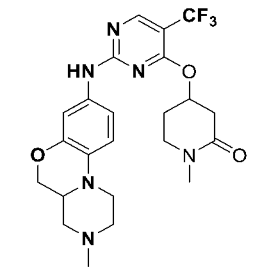
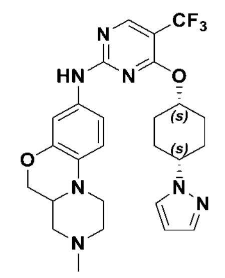

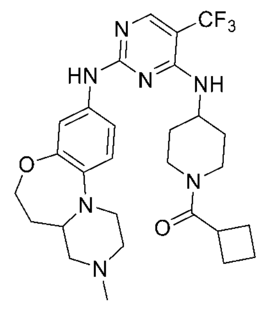
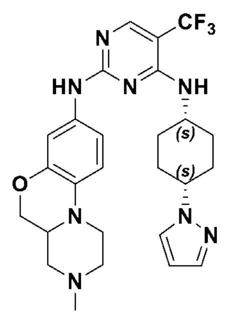
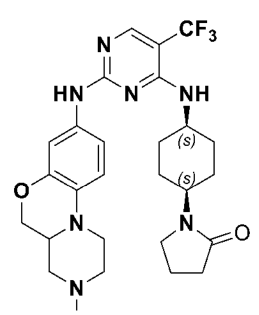
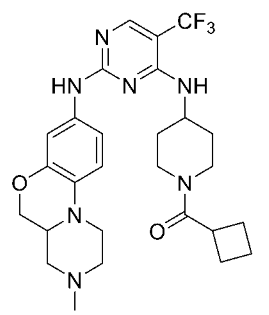

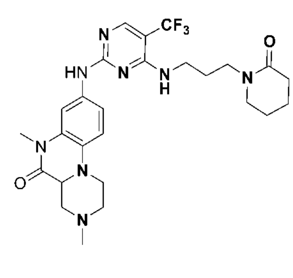
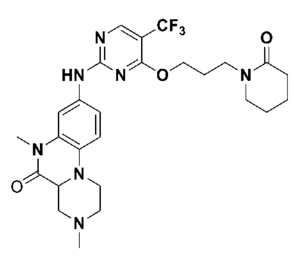
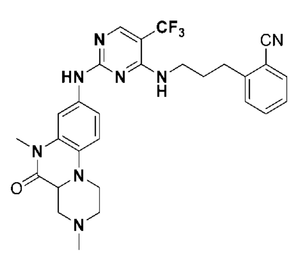
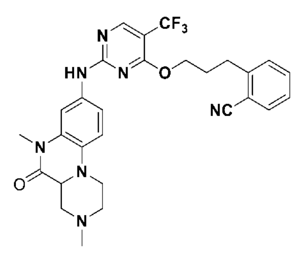

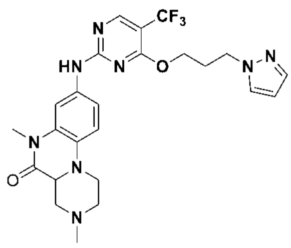
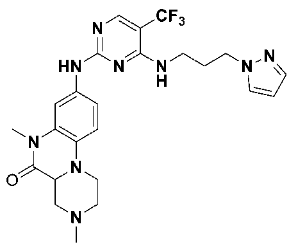
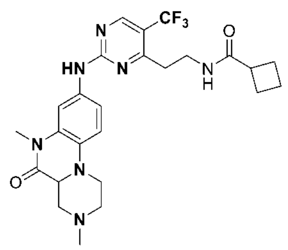
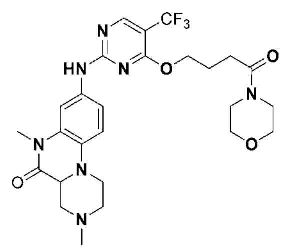

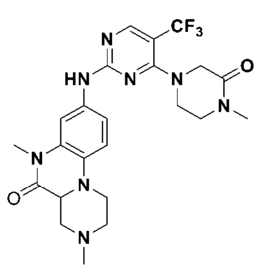
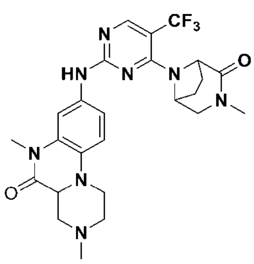
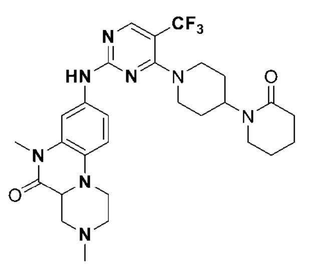
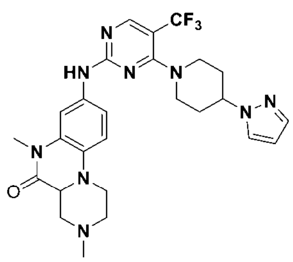

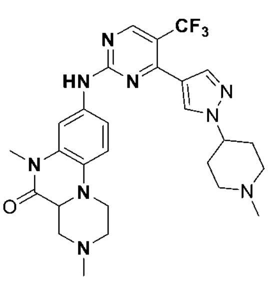
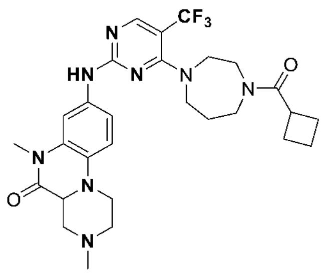
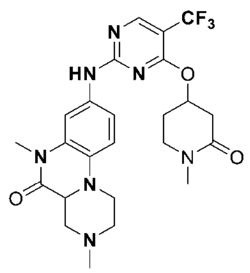
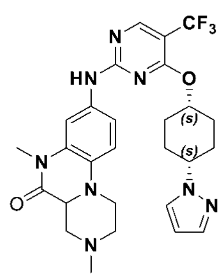

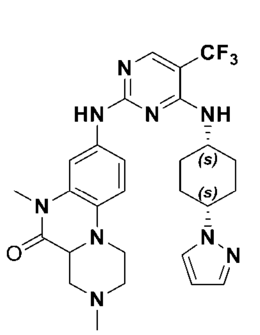
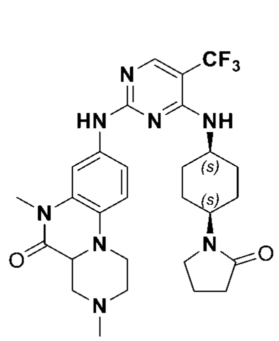
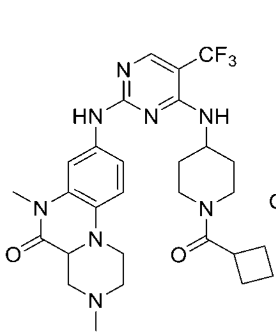
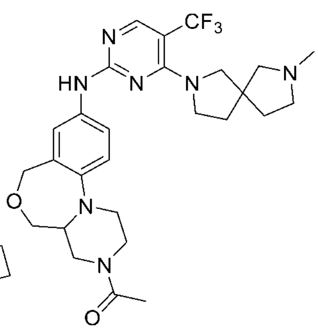

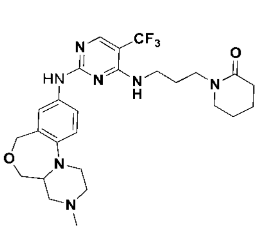
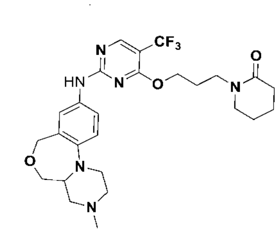
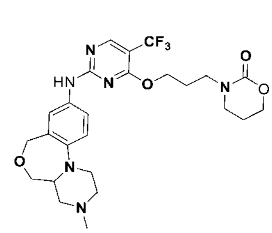
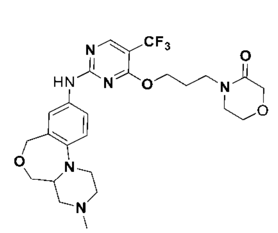

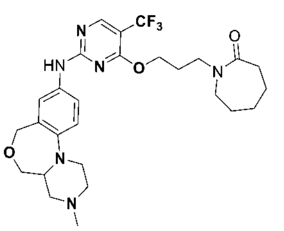
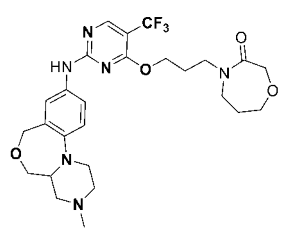
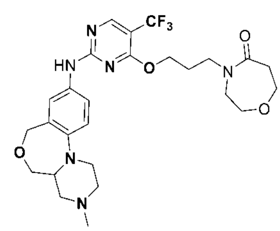
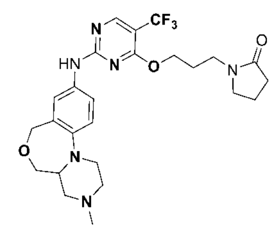

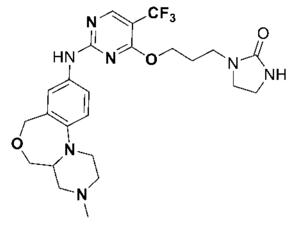
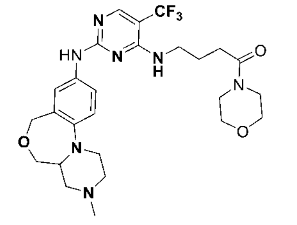
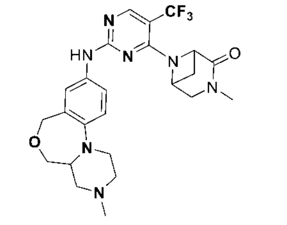
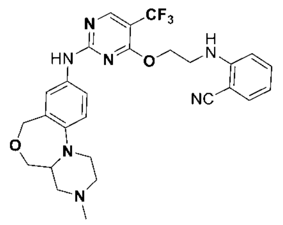

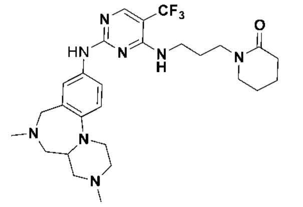
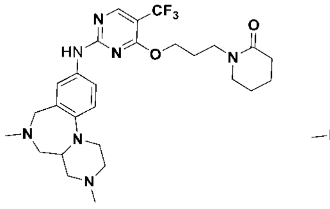
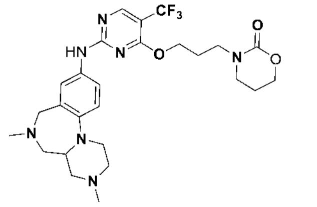
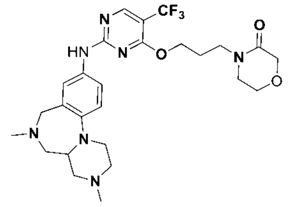

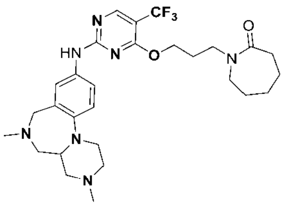
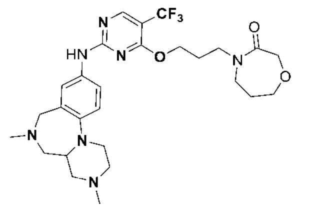
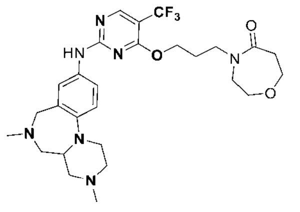
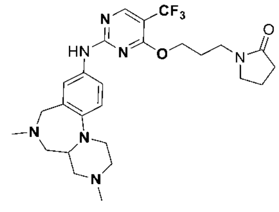

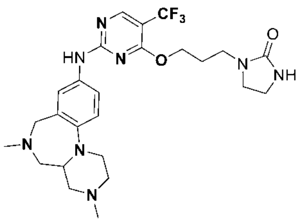
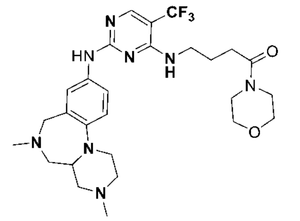
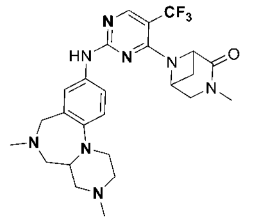
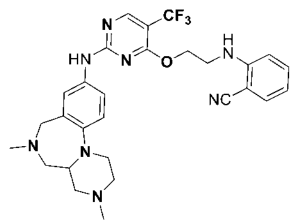

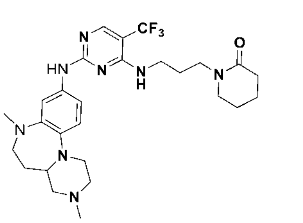
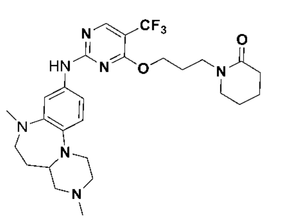
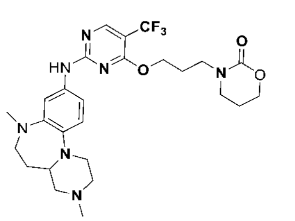
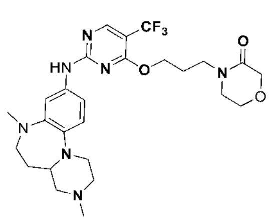

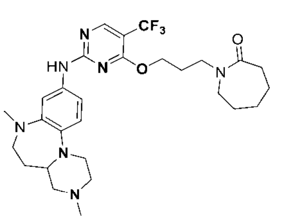
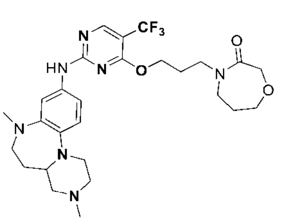
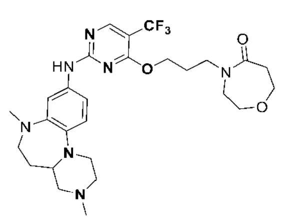
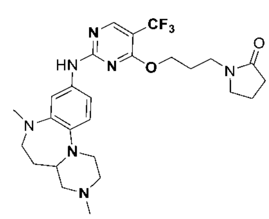

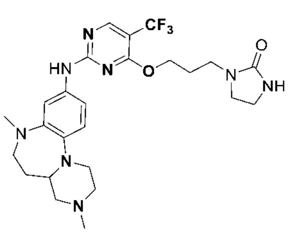
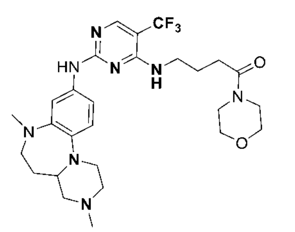
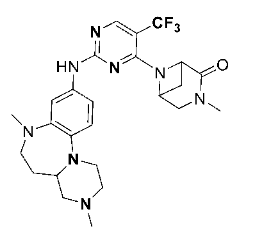
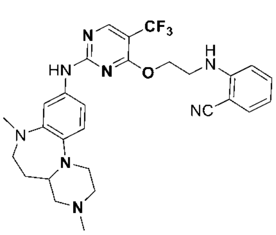

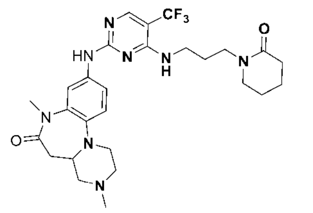
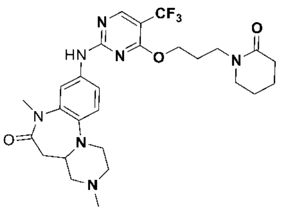
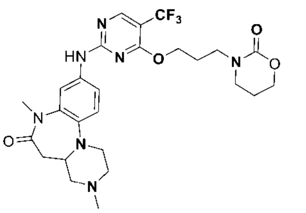
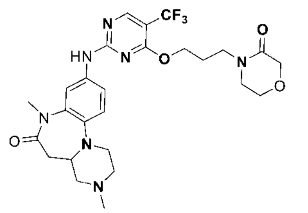
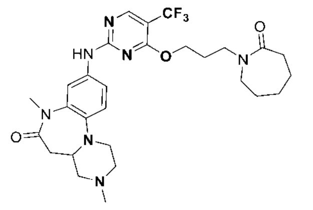
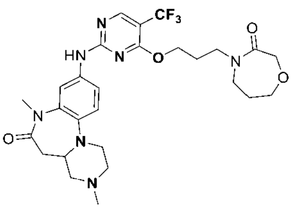
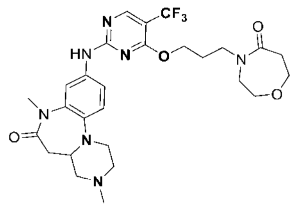
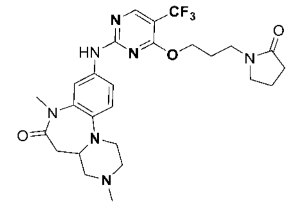
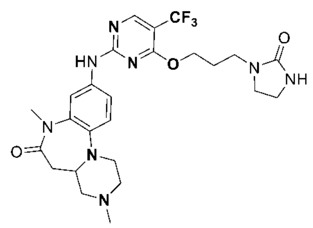
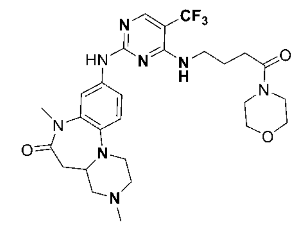
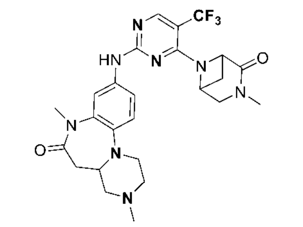
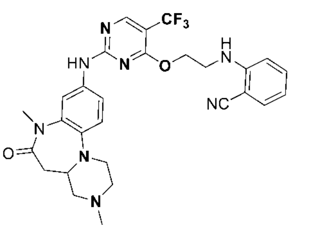
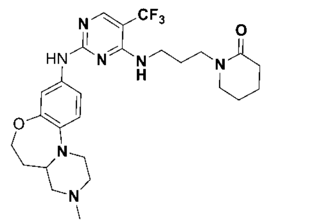
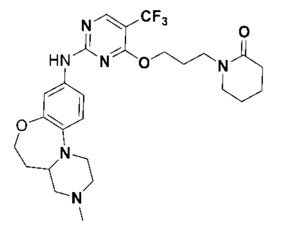
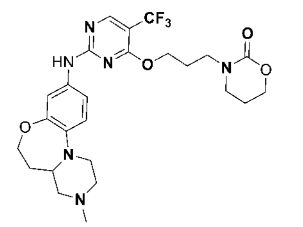
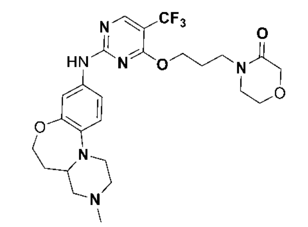
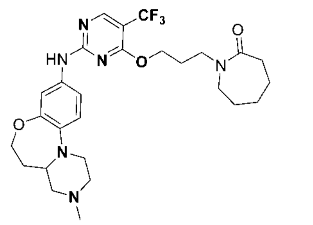
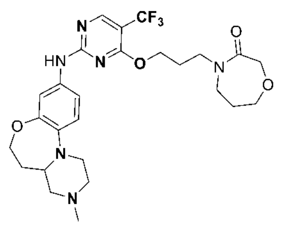
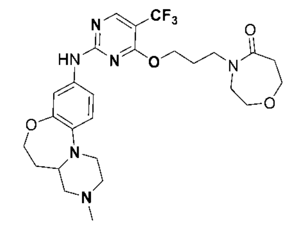
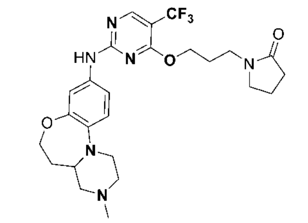
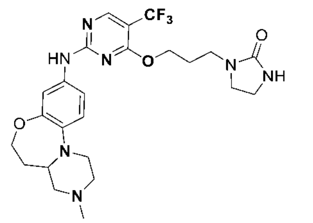
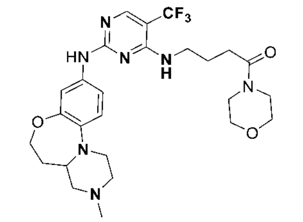
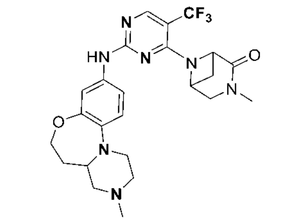
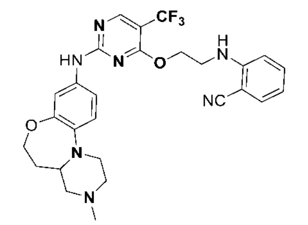
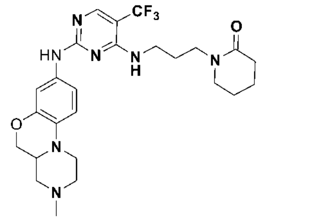
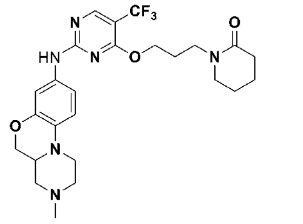
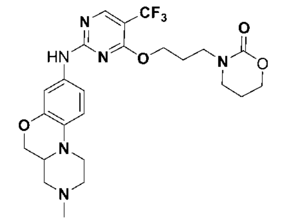
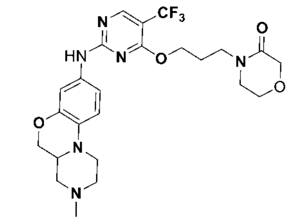

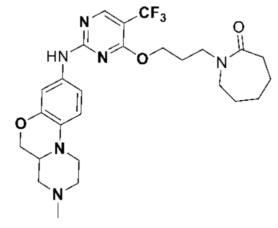
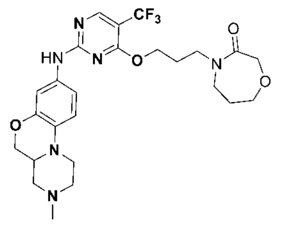
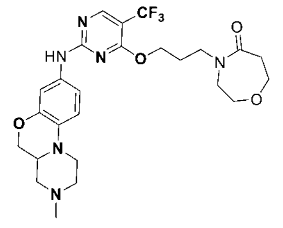
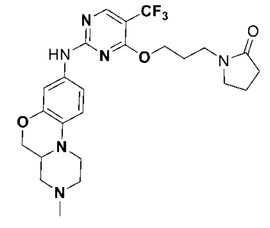
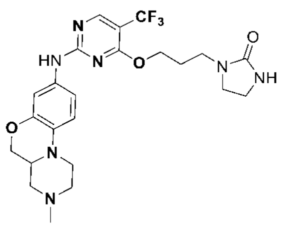
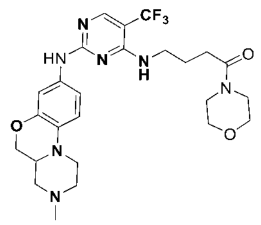
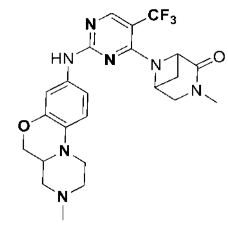
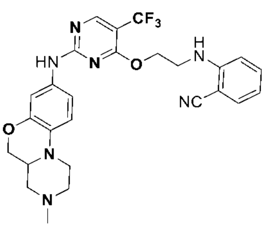
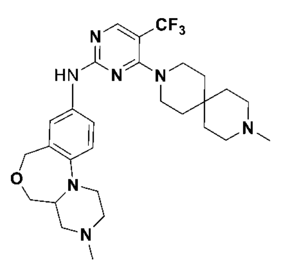
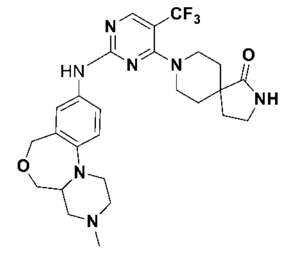
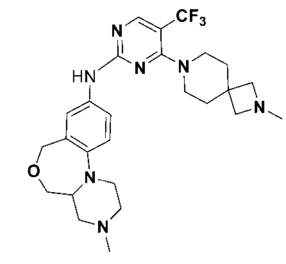
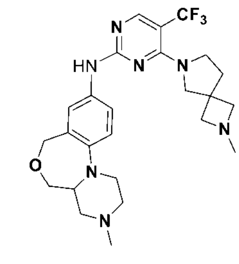
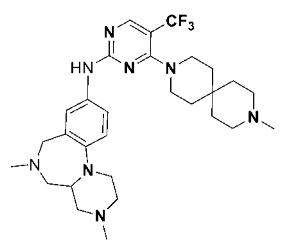
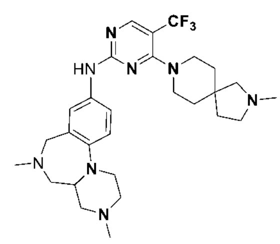
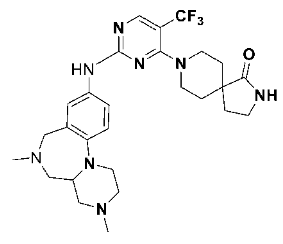
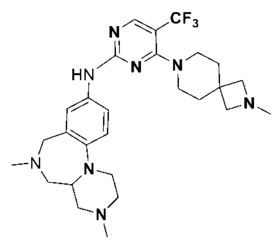
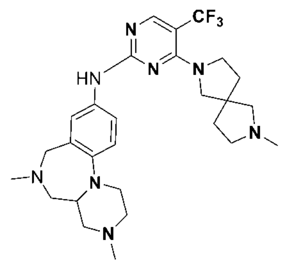
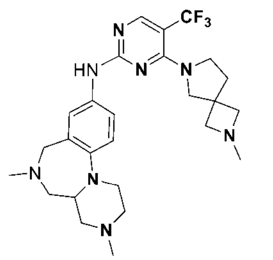
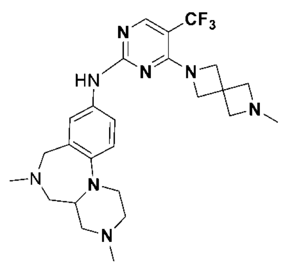
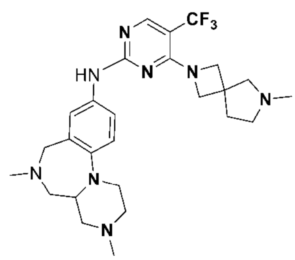
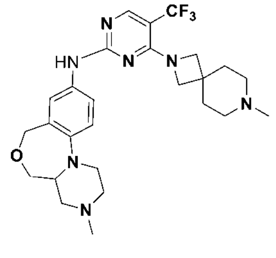
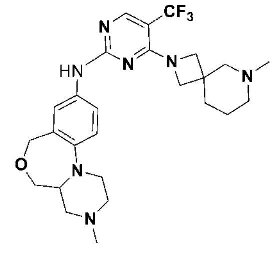
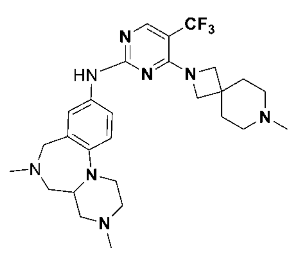
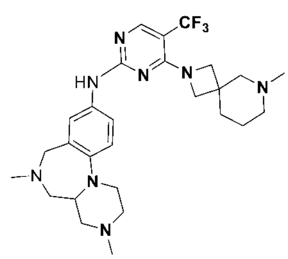

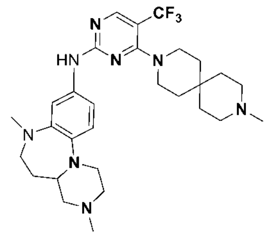
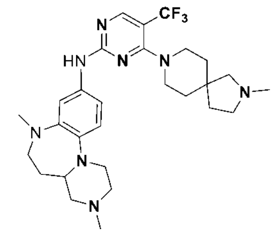
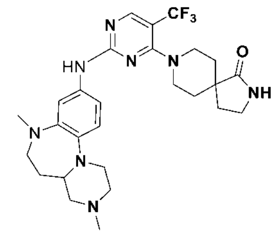
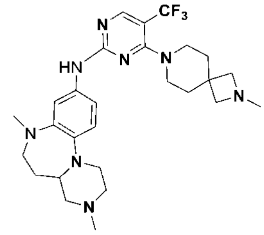

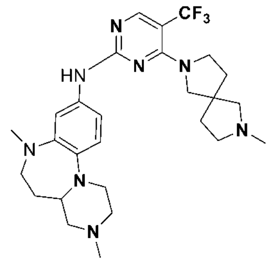
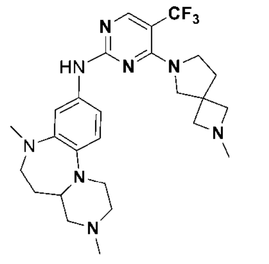
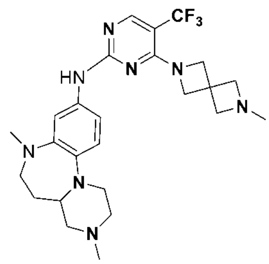
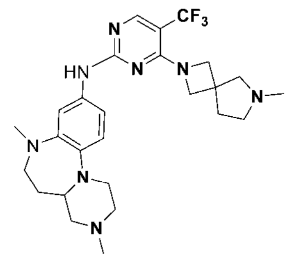

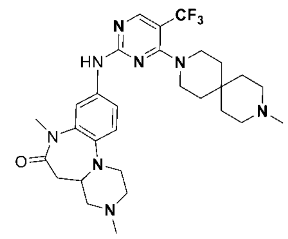
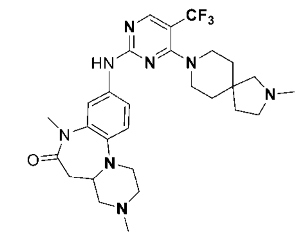
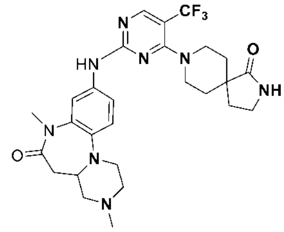
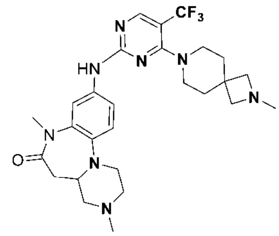

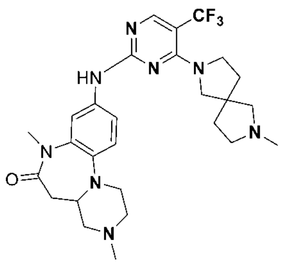
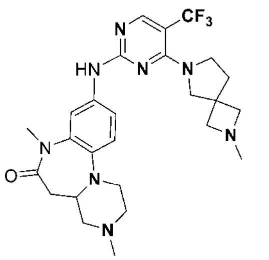
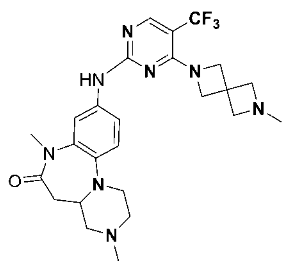
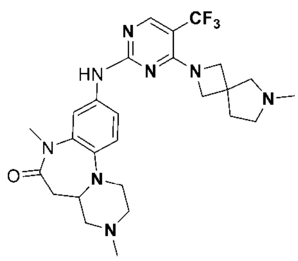

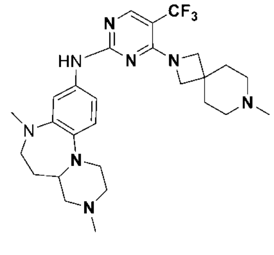
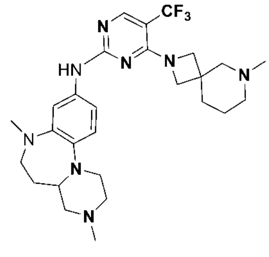
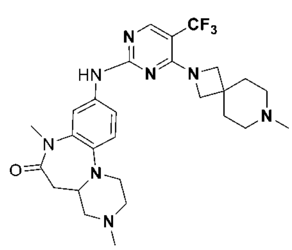
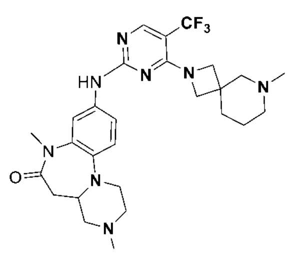

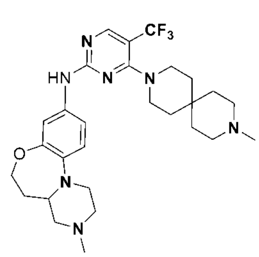
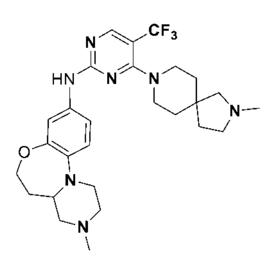
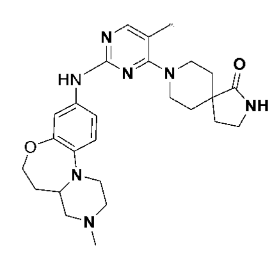
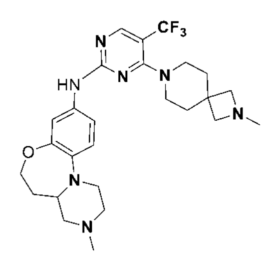

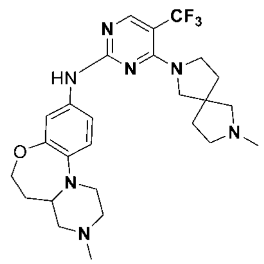
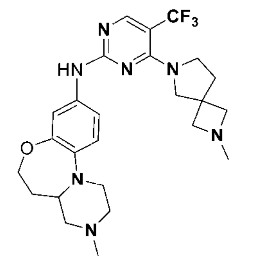
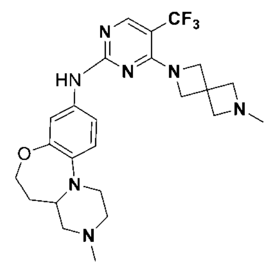
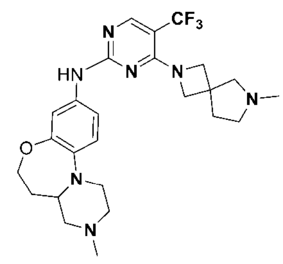

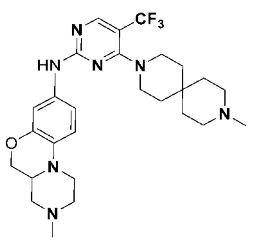
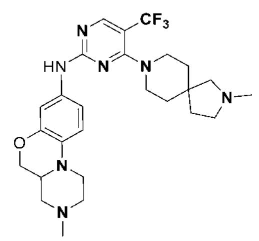
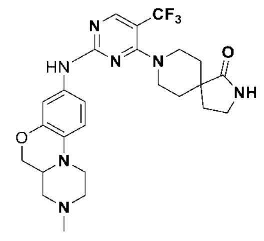
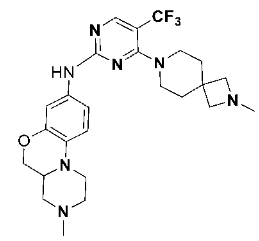

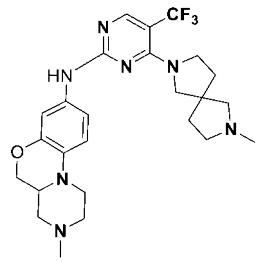
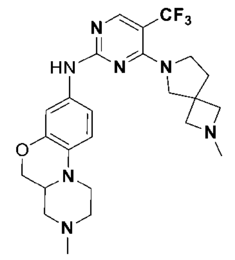
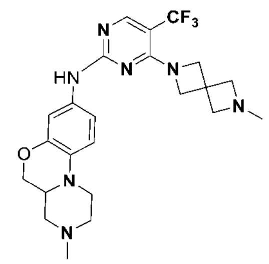
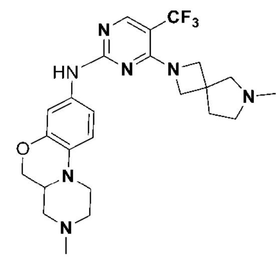

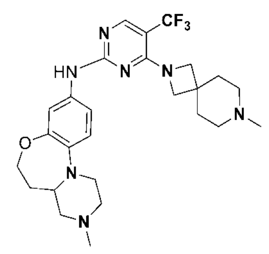
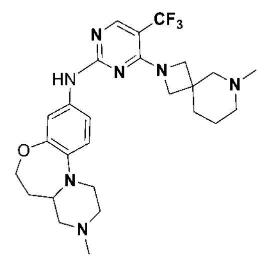
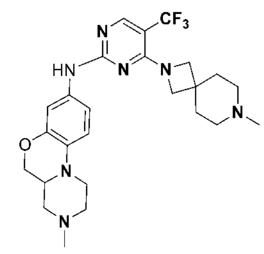
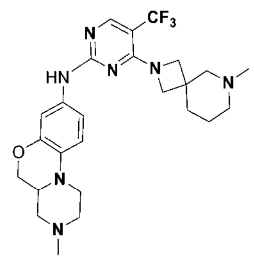

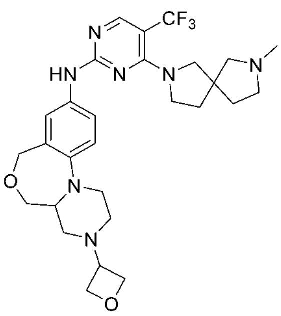
.

## Preparation methods

Another object of the present invention is to provide a method for preparing the compound of the present invention, comprising the route A or route B as follows:

Synthetic route A:

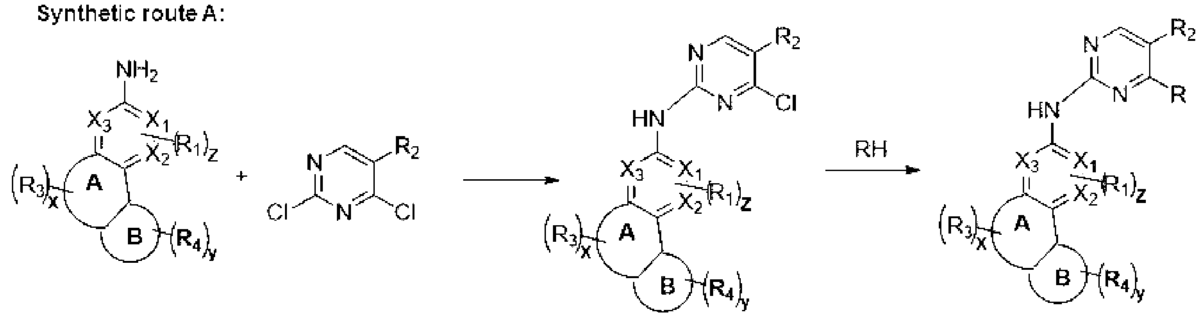


1

or,

Synthetic route B:

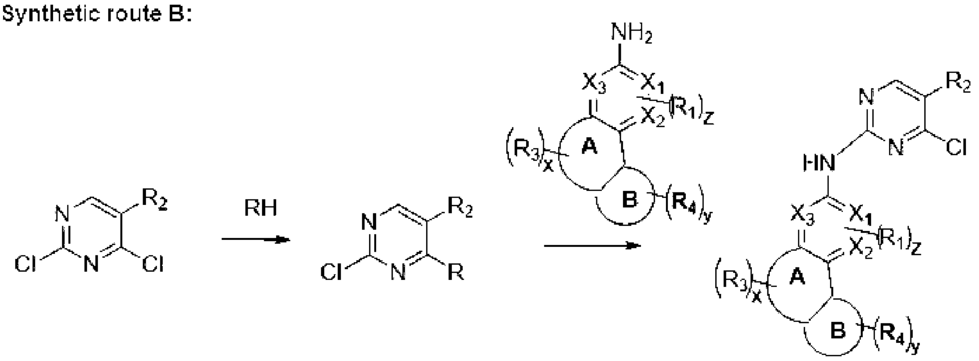


1

,

wherein $R_1$, $R_2$, $R_3$, $R_4$, x, y, z, $X_1$, $X_2$, and $X_3$ are as defined hereinbefore.

**Pharmaceutical compositions and kits**

Another object of the present invention is to provide a pharmaceutical composition, comprising an effective amount of the compound or pharmaceutically acceptable salt, stereoisomer, tautomer, polymorph, solvate, hydrate, N-oxide, isotope labeled compound, metabolite, ester, or prodrug thereof of the present invention, and one or more pharmaceutically acceptable carriers.

Another object of the present invention is to provide a kit, comprising an effective amount of the compound or pharmaceutically acceptable salt, stereoisomer, tautomer, polymorph, solvate, hydrate, N-oxide, isotope labeled compound, metabolite, ester, or prodrug thereof of the present invention, or the pharmaceutical composition of the present invention, and optionally present packaging instructions.

In the present invention, "pharmaceutically acceptable carrier" refers to a diluent, adjuvant, excipient or vehicle administered together with a therapeutic agent, which is suitable, to the extent of a reasonable medical judgment, for contacting the tissues of humans and/or other animals without excessive toxicity, irritation, allergic reactions, or other problems or complications commensurate with a reasonable benefit/risk ratio.

Pharmaceutically acceptable carriers that can be used in the pharmaceutical composition or pharmaceutical formulation of the present invention include, but are not limited to, sterile liquids, for example water and oils, including those of petroleum, animal, plant, or synthetic origin, for example peanut oil, soybean oil, mineral oil, sesame oil, etc.

The pharmaceutical composition may be in the form of, for example, solid formulations, semi-solid formulations, liquid formulations, or gaseous formulations. The solid formulations are, for example, tablets, capsules, powders, granules, or suppositories, etc.; the liquid formulations are, for example, solutions, suspensions, or injections. The composition may also be in dosage forms such as liposomes or microspheres. Particularly, the pharmaceutical composition is in a formulation form suitable for oral administration.

When the pharmaceutical composition is administered intravenously, water is an exemplary carrier. Physiological saline and glucose and glycerol aqueous solutions can also be used as liquid carriers, particularly for injections. Suitable pharmaceutical excipients include starch, glucose, lactose, sucrose, gelatin, maltose, chalk, silica gel, sodium stearate, glyceryl monostearate, talc, sodium chloride, skimmed milk powder, glycerol, propylene glycol, water, ethanol, etc. The composition may also contain small amounts of wetting agents, emulsifiers or pH buffers, as needed. Oral

formulations may contain standard carriers such as a pharmaceutical-grade mannitol, lactose, starch, magnesium stearate, sodium saccharin, cellulose, magnesium carbonate, etc. Examples of suitable pharmaceutically acceptable carriers are as described in Remington's Pharmaceutical Sciences (1990).

The pharmaceutical composition of the present invention can act systemically and/or locally. For this purpose, they can be administered by suitable routes, for example, by injection (such as intravenous, intraarterial, subcutaneous, intraperitoneal, intramuscular injection, including infusion) or transdermal administration; or by oral, sublingual, transnasal, transmucosal, topical administration, in the form of ophthalmic formulations, or by inhalation.

For these administration routes, the pharmaceutical composition of the present invention can be administered in suitable dosage forms. The dosage forms include, but are not limited to, tablets, capsules, lozenges, hard candies, powders, sprays, creams, ointments, suppositories, gels, pastes, lotions, ointments, aqueous suspensions, injectable solutions, elixirs, syrups, etc.

The content or usage amount of the compound of the present invention in the pharmaceutical composition can be about 0.001 mg to about 1000 mg, suitably 0.001-100 mg, preferably 0.001-10 mg, more preferably 0.05-5 mg, particularly preferably 0.01-1 mg.

In some embodiments, the present invention provides a method for preparing the pharmaceutical composition of the present invention, the method comprising combining the compound or pharmaceutically acceptable salt, stereoisomer, tautomer, polymorph, solvate, hydrate, N-oxide, isotope labeled compound, metabolite, ester, or prodrug thereof of the present invention, with one or more pharmaceutically acceptable carriers.

## Treatment methods and uses

Another object of the present invention is to provide a method for treating a ULK1-mediated disease in a subject in need thereof, the method comprising administering to the subject an effective amount of the compound or pharmaceutically acceptable salt, stereoisomer, tautomer, polymorph, solvate, hydrate, N-oxide, isotope labeled compound, metabolite, ester, or prodrug thereof of the present invention, or the pharmaceutical composition of the present invention.

Another object of the present invention is to provide use of the compound or pharmaceutically acceptable salt, stereoisomer, tautomer, polymorph, solvate, hydrate, N-oxide, isotope labeled compound, metabolite, ester, or prodrug thereof of the present invention, or the pharmaceutical composition of the present invention in the preparation of a medicament for treating a ULK1-mediated disease in a subject in need thereof.

Another object of the present invention is to provide the compound or pharmaceutically acceptable salt, stereoisomer, tautomer, polymorph, solvate, hydrate, N-oxide, isotope labeled compound, metabolite, ester, or prodrug thereof of the present invention, or the pharmaceutical composition of the present invention, for use in treating a ULK1-mediated disease in a subject in need thereof.

Another object of the present invention is to provide a method for preventing or treating a ULK1-mediated related disease, comprising administering to an individual in need thereof a preventive or therapeutically effective amount of the compound or pharmaceutically acceptable salt, stereoisomer, tautomer, polymorph, solvate, N-oxide, isotope labeled compound, metabolite or prodrug thereof of the present invention, or the pharmaceutical composition of the present invention.

According to some embodiments of the present invention, the ULK1-mediated disease is characterized by an abnormal autophagy.

According to some embodiments of the present invention, the abnormal autophagy is induced by treatment.

According to some embodiments of the present invention, the ULK1-mediated disease is a cancer, for example lung cancer, breast cancer or pancreatic cancer, particularly, the lung cancer is non-small cell lung cancer, the breast cancer is triple negative breast cancer, or the pancreatic cancer is pancreatic ductal adenocarcinoma.

According to some embodiments of the present invention, the ULK1-mediated disease is tuberous sclerosis (TSC) or lymphangioleiomyomatosis (LAM).

According to some embodiments of the present invention, the medicament is co-administered with an additional therapeutic agent.

According to some embodiments of the present invention, the additional therapeutic agent is a standard care therapy.

According to some embodiments of the present invention, the standard care therapy is an mTOR inhibitor, carboplatin, a MEK inhibitor or a PARP inhibitor.

According to some embodiments of the present invention, wherein the medicament is used to degrade ATG13 in the subject.

As used herein, the term "effective amount" refers to an amount sufficient to achieve the desired preventive or therapeutic effect, for example, an amount that achieves the alleviation of one or more symptoms associated with the disease to be treated.

The dosing regimen can be adjusted to provide the optimal desired response. For example, a single bolus

injection can be administered, several divided doses can be administered over time, or the dose can be proportionally reduced or increased as indicated by the urgency of the treatment situation. It should be noted that a dose value may vary depending on the type and severity of the condition to be alleviated, and may include a single or multiple doses. It is to be further understood that, for any specific individual, the particular dosing regimen should be adjusted over time according to the individual's needs and the professional judgment of the person administering the composition or supervising the administration of the composition.

The amount of the compound of the present invention administered will depend on the individual being treated, the severity of the disorder or condition, the rate of administration, the disposition of the compound, and the judgment of the prescribing physician. Generally, the effective dose is about 0.0001 to about 50 mg per kg of body weight per day, for example, about 0.01 to about 10 mg/kg/day (single or divided doses). For a 70 kg person, this would total about 0.007 mg/day to about 3500 mg/day, for example, about 0.7 mg/day to about 700 mg/day. In some cases, a dose level not exceeding the lower limit of the aforementioned range may be sufficient, while in other cases, a larger dose may still be used without causing any harmful side effects, with the proviso that the larger dose is first divided into several smaller doses for administration throughout the day.

Unless otherwise stated, as used herein, the term "treatment" means reversing, alleviating, inhibiting the disorder or condition to which such term applies or the progression of one or more symptoms of such disorder or condition, or preventing such disorder or condition or one or more symptoms of such disorder or condition.

As used herein, "individuals" include human beings or non-human animals. Exemplary human individuals include human individuals suffering from a disease (for example the diseases described herein) (referred to as patients) or normal individuals. In the present invention, "non-human animals" include all vertebrates, for example non-mammals (e.g., birds, amphibians, reptiles) and mammals, for example non-human primates, domestic animals and/or domesticated animals (e.g., sheep, dogs, cats, cows, pigs, etc.).

**Example**

To make the objectives and technical solutions of the present invention clearer, the embodiments of the present invention are described in detail below with reference to examples. However, those skilled in the art will understand that the following examples are only used to illustrate the present invention and should not be regarded as limiting the scope of the present invention. If a particular condition is not specified in the examples, it is carried out according to a conventional condition or the condition suggested by the manufacturer. The used reagents or instruments whose manufacturers are not specified are all commercially available conventional products.

The structure of a compound was determined by nuclear magnetic resonance (NMR) and/or mass spectrometry (MS). The NMR shift (δ) is given in unit of $10^{-6}$ (ppm). The determination of NMR was performed using a Bruker AVANCE NEO 500M nuclear magnetic instrument, the determination solvent was deuterated dimethyl sulfoxide (DMSO-$d_6$), deuterated chloroform ($CDCl_3$), deuterated methanol ($CD_3OD$), with tetramethylsilane (TMS) as the internal standard.

The determination of MS was performed using Agilent 1200/1290 DAD-6110/6120 Quadrupole MS liquid chromatograph-mass spectrometer (producer: Agilent, MS model: 6110/6120 Quadrupole MS), waters ACQuity UPLC-QD/SQD (producer: waters, MS model: waters ACQuity Qda Detector/waters SQ Detector), THERMO Ultimate 3000-Q Exactive (producer: THERMO, MS model: THERMO Q Exactive).

High performance liquid chromatography (HPLC) analysis was performed using Agilent HPLC 1200DAD, Agilent HPLC 1200VWD and Waters HPLC e2695-2489 liquid chromatograph.

Chiral HPLC analysis and determination were performed using Agilent 1260 DAD high performance liquid chromatograph.

Waters 2767, Waters 2767-SQ Detecor2, Shimadzu LC-20AP and Gilson-281 preparative chromatographs were used for high performance liquid preparation.

Shimadzu LC-20AP preparative chromatograph was used for chiral preparation.

Combiflash Rf200 (TELEDYNE ISCO) was used as the CombiFlash rapid preparation instrument.

Yantai Huanghai HSGF254 or Qingdao GF254 silica gel plate was used for a thin layer chromatography silica gel plate, and the specification of the silica gel plate employed for thin layer chromatography (TLC) was 0.15mm - 0.2mm.

Silica gel column chromatography typically used Yantai Huanghai silica gel with 200 - 300 mesh as the carrier.

The average inhibition ratio of a kinase and the IC50 value were determined using a NovoStar microplate reader (BMG company, Germany).

The known starting materials of the present disclosure can be synthesized using or according to methods known in the art, or can be purchased from ABCR GmbH&Co.KG, Acros Organics, Aldrich Chemical Company, Accela ChemBio Inc, Darui Chemicals Company, etc.

CEM Discover-S 908860 type microwave reactor was used for a microwave reaction.

Unless otherwise specified in the examples, "solution" refers to an aqueous solution.

Unless otherwise specified in the examples, the reaction temperature was room temperature, being 20°C - 30°C.

The reaction progress in the examples was monitored using thin layer chromatography (TLC), the developing agent used for the reaction, the eluant system for column chromatography used to purify a compound, and the developing agent system for thin layer chromatography included: A: dichloromethane/methanol system, B: n-hexane/ethyl acetate, the volume ratio of the solvents was adjusted according to the different polarities of the compounds, and a small amount of basic or acidic reagent such as triethylamine and acetic acid could also be added for adjustment.

**Preparation of intermediate Int-1**

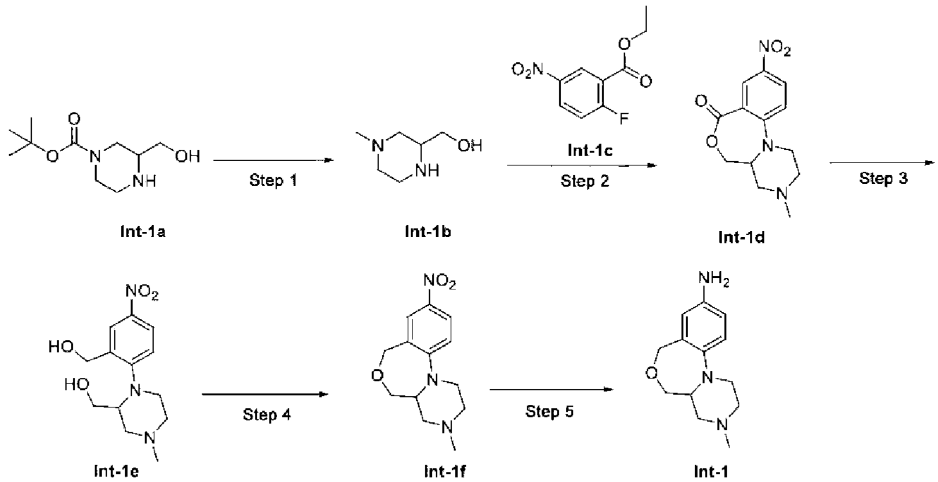


Step 1: At room temperature, compound **Int-1a** (8.00 g, 36.99 mmol, Bide) was dissolved in tetrahydrofuran (100 mL). Lithium aluminum hydride (48.09 mL, 48.09 mmol, Energy) was added dropwise under an ice bath, and the reaction was carried out at 65°C for 18 hours. The reaction solution was quenched with sodium sulfate decahydrate under an ice bath. The mixture was filtered, and the filtrate was concentrated under reduced pressure to afford compound Int-**1b.** MS m/z (ESI): 131.0 [M+1].

Step 2: At room temperature, triethylamine (7.00 g, 69.13 mmol) and compound **Int-1c** (4.59 g, 23.04 mmol, Bide) were added to a solution of compound **Int-1b** (3.00 g, 23.04 mmol) in dioxane (50 mL), respectively. After purging with nitrogen three times, the resulting mixture was stirred at 110°C for 24 hours. The reaction solution was concentrated under reduced pressure to afford the crude compound. The crude was slurried with petroleum ether: ethyl acetate (2:1, 90 mL). The solid was washed with petroleum ether: ethyl acetate (2:1, 20 mL) and then dried to afford compound **Int-1d.** MS m/z (ESI): 278.0 [M+1].

Step 3: Sodium borohydride (1.39 g, 36.79 mmol) and lithium borohydride (0.03 g, 1.23 mmol) were added to a solution of compound **Int-1d** (3.40 g, 12.26 mmol) in tetrahydrofuran (30 mL) under an ice bath, followed by stirring at room temperature for 18 hours. The reaction solution was poured into ice water (30 mL), extracted with ethyl acetate (50 mL×2). washed with saturated saline (20 mL), dried over anhydrous sodium sulfate, filtered, and concentrated under reduced pressure to afford the crude compound. The crude was purified by silica gel chromatography (eluant: MeOH:DCM= 0% - 8%) to afford compound **Int-1e.** MS m/z (ESI): 282.0 [M+1].

Step 4: At room temperature, compound **Int-1e** (2.10 g, 7.47 mmol) was dissolved in 50% sulfuric acid (20 mL), followed by stirring at 120°C for 1 hour to afford a black solution. The reaction solution was poured into ice water (50 mL), adjusted to pH=7-8 using saturated sodium bicarbonate solution, extracted with dichloromethane (100 mL×2), washed with saturated saline (50 mL), dried over anhydrous sodium sulfate, filtered, and concentrated under reduced pressure to afford the crude compound. The crude was purified by silica gel chromatography (eluant: DCM:MeOH = 0% - 8%) to afford compound **Int-1f.** MS m/z (ESI): 264.0 [M+1].

Step 5: At room temperature, palladium on carbon (60 mg, 0.57 mmol) was added to a solution of compound **Int-1f** (1.50 g, 5.70 mmol) in methanol (40 mL). After purging with hydrogen three times, the reaction solution was stirred at room temperature under a hydrogen balloon for 5 hours. The reaction solution was filtered. The filtrate was concentrated under reduced pressure to afford compound **Int-1.** MS m/z (ESI): 234.1 [M+1].

**Preparation of intermediate Int-2**

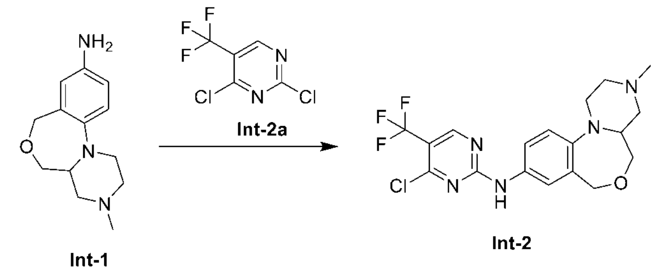


Int-1 Int-2

Compound **Int-1** (0.62 mL, 4.61 mmol, Bide) was added to a mixed solution of t-butyl alcohol (5 mL) and 1,2-dichloroethane (40 mL). Zinc dichloride (II) (10.14 mL, 10.14 mmol, 1 mol/L) was added at 0°C under nitrogen protection. The reaction was carried out under stirring at 0°C for 1h, and then triethylamine (0.70 mL, 5.07 mmol) and compound **Int-2a** (1.08 g, 4.61 mmol) were added, respectively. The reaction was then carried out under stirring at 0°C for 2 hours. The reaction was concentrated, and the crude product was prepared by thin layer chromatography (developing agent: DCM:MeOH = 100:1-10:1) to afford compound **Int-2.** MS m/z (ESI): 414.9 [M+1].

**Example 1. Preparation of compound 1**

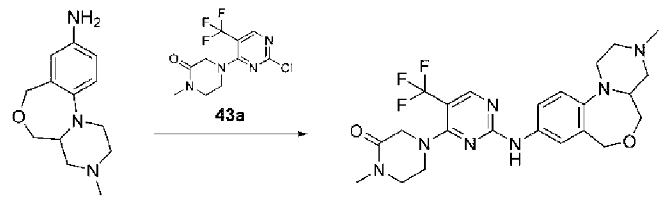


Int-1 Compound 1

At room temperature, compound **Int-1** (120 mg, 0.51 mmol) was dissolved in dioxane (2 mL). Cesium carbonate (503 mg, 1.54 mmol), **43a** (152 mg, 0.51 mmol), 1,1'-binaphthyl-2,2'-diphenylphosphine (64 mg, 0.10 mmol, Bide), and palladium acetate (12 mg, 0.05 mol, Energy) were added to the above reaction solution, respectively, followed by stirring at 100°C for 18 hours under nitrogen protection, to afford a suspension. The reaction solution was filtered. The filtrate was concentrated under reduced pressure to afford the crude compound. The crude product was purified by preparative HPLC (chromatographic column: Waters-Xbridge-C18-10μm-19*250mm; mobile phase: A: 10 mM ammonium bicarbonate/-water; B: acetonitrile, gradient ratio: acetonitrile 95%, flow rate: 20 mL/min) to afford compound **1.** MS m/z (ESI): 492.2 [M+1]. $^1$H NMR (400 MHz, DMSO-$d_6$): δ 9.75 (s, 1H), 8.42 (s, 1H), 7.52 (d, *J* = 6.4 Hz, 2H), 6.91 (d, *J* = 9.0 Hz, 1H), 4.78 (d, *J* = 11.8 Hz, 1H), 4.56 (d, *J* = 11.8 Hz, 1H), 4.11 (s, 2H), 3.83 (t, *J* = 5.2 Hz, 2H), 3.70 - 3.55 (m, 2H), 3.44 (t, *J* = 5.2 Hz, 2H), 3.22 - 3.08 (m, 2H), 2.89 (d, *J* = 8.8 Hz, 4H), 2.74 (d, *J* = 11.0 Hz, 1H), 2.64 (d, *J* = 9.4 Hz, 1H), 2.36 - 2.19 (m, 5H).

**Example 2. Preparation of compound 2**

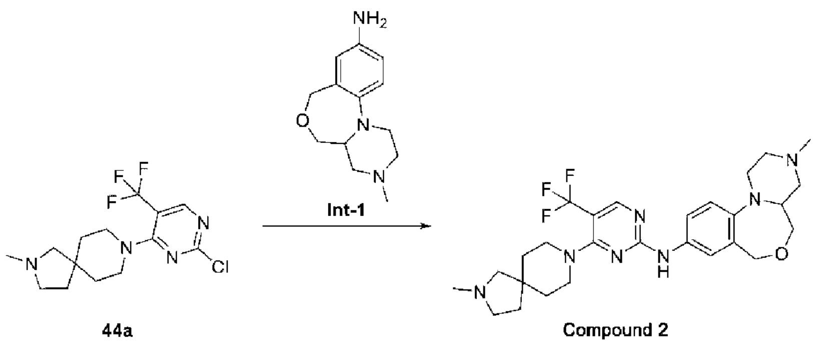


44a Compound 2

At 25°C, hydrochloric acid (1.5 mL, 6.00 mmol, C=4 mol/L) was added to a solution of compound **44a** (160 mg, 0.48 mmol) and compound **Int-1** (123 mg, 0.53 mmol) in N,N-dimethylformamide (4 mL). The resulting mixture was heated

to 100 °C and stirred for 2 hours. The mixture was concentrated under reduced pressure to afford the crude product. The crude product was purified by preparative HPLC (chromatographic column: Waters-Xbridge-C18-10 μm-19*250 mm; mobile phase: A: 10 mM ammonium bicarbonate/water; B: acetonitrile, gradient ratio: acetonitrile 40 - 60%, flow rate: 25 mL/min) to afford compound **2.** MS m/z (ESI): 532.2 [M+1]. $^1$HNMR(400 MHz, DMSO-$d_6$): δ 9.58 (s, 1H), 8.34 (s, 1H), 7.52 (d, *J* = 2.2 Hz, 2H), 6.90 (d, *J* = 9.0 Hz, 1H), 4.78-4.72 (m, 1H), 4.52 (d, *J* = 11.8 Hz, 1H), 3.67-3.45 (m, 6H), 3.20-3.09 (m, 2H), 2.88 (dt, *J* = 10.6, 6.0 Hz, 1H), 2.69 (dd, *J* = 10.2, 3.0 Hz, 1H), 2.60 (s, 1H), 2.46 (d, *J* = 6.8 Hz, 2H), 2.32 (s, 2H), 2.25 (d, *J* = 10.2 Hz, 8H), 1.68-1.52 (m, 6H).

**Example 3. Preparation of compound 3**

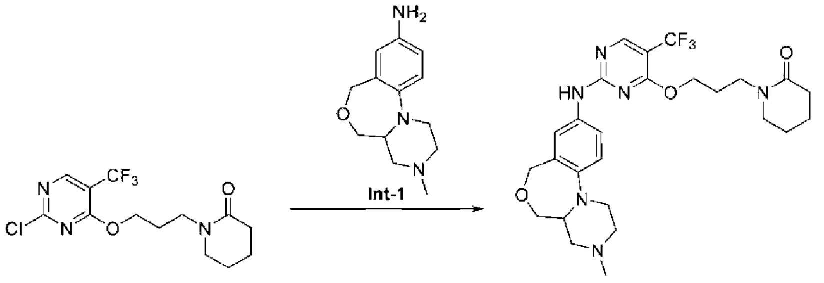


47a

Compound 3

At room temperature, trifluoroacetic acid (300 μL, 4.00 mmol) was added to a solution of compound **47a** (135 mg, 0.40 mmol) and compound **Int-1** (112 mg, 0.48 mmol) in isopropanol (5 mL), followed by heating to 40°C and stirring for 16 h. The crude product was afforded by concentration under reduced pressure. The crude product was purified by preparative HPLC (chromatographic column: Waters-SunFire-C18-10 μm-19*250 mm; mobile phase: A: 0.1% formic acid/water; B: acetonitrile, gradient ratio: acetonitrile 20-50%, flow rate: 25 mL/min) to afford compound **3.** MS m/z (ESI): 535.3 [M+1]. $^1$H NMR (400 MHz, DMSO-$d_6$): δ 10.00 (s, 1H), 8.47 (s, 1H), 7.75 - 7.59 (m, 1H), 7.48 (d, *J* = 2.0 Hz, 1H), 6.95 (d, *J* = 8.6 Hz, 1H), 4.77 (d, *J* = 11.8 Hz, 1H), 4.56 (d, *J* = 11.8 Hz, 1H), 4.42 (t, *J* = 6.4 Hz, 2H), 3.68 - 3.55 (m, 2H), 3.37 (t, *J* = 7.0 Hz, 2H), 3.25 - 3.09 (m, 4H), 2.94 - 2.85 (m, 1H), 2.81 - 2.72 (m, 1H), 2.69 - 2.62 (m, 1H), 2.36 - 2.24 (m, 5H), 2.22 - 2.15 (m, 2H), 2.00 - 1.88 (m, 2H), 1.77 - 1.61 (m, 4H).

**Example 4. Preparation of compound 4**

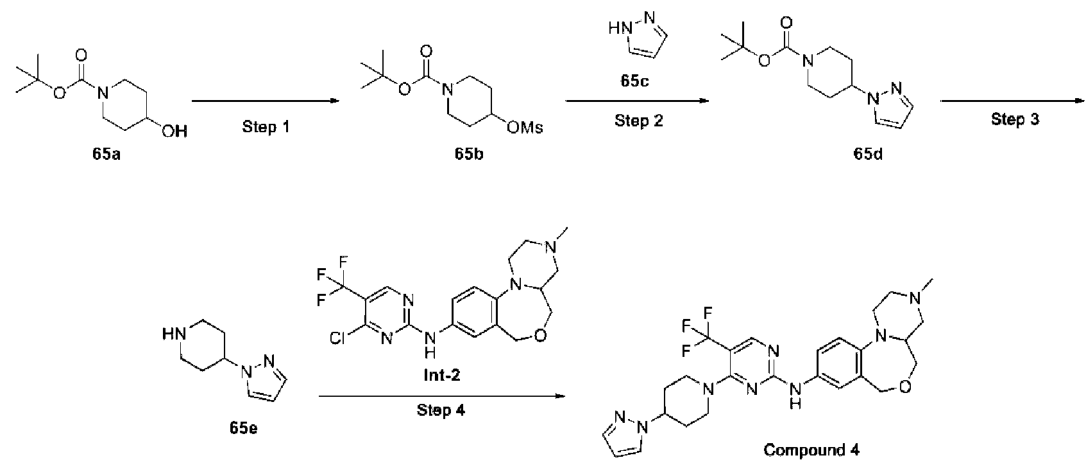


65a 65b 65d 65e Compound 4

Step 1: At 0°C, methylsulfonyl chloride (1.73 mL, 22.36 mmol) was added to a solution of compound **65a** (3.00 g, 14.91 mmol, Bide) and triethylamine (6.2 mL, 44.72 mmol) in dichloromethane (30 mL), followed by stirring at 0°C for 3 hours. The mixture was poured into water (20 mL) and extracted with dichloromethane (30 mL×3). The organic phases were combined, washed with saline (30 mL), dried over anhydrous sodium sulfate, filtered, and concentrated under reduced pressure, to afford compound **65b.** MS m/z (ESI): 280.11 [M+1].

Step 2: At 0°C, sodium hydride (1.32 g, 33.05 mmol, 60% purity) was added to a solution of compound **65c** (1.50 g, 22.03 mmol, Bide) in N,N-dimethylformamide (30 mL), followed by stirring at 0°C for 20 minutes. Then, compound **65b** (7.00 g, 13.78 mmol) was added, and the resulting mixture was warmed to 25°C and stirred for 18 hours. The mixture was poured into water (50 mL) for quenching, followed by extraction with ethyl acetate (50 mL×3). The organic phases were combined, washed with saline (30 mL×4), dried over anhydrous sodium sulfate, and concentrated under reduced pressure to afford the crude product. The crude product was purified by silica gel chromatography (elution solution: petroleum ether - tetrahydrofuran = 5 - 45%) to afford compound **65d.** MS m/z (ESI): 252.16 [M+1].

Step 3: At 25°C, hydrochloric acid (10 mL, 40.00 mmol, 4 mol/L) was added to a solution of compound **65d** (889 mg, 3.54 mmol) in 1,4-dioxane (15 mL), followed by stirring at 25°C for 3 hours. Compound **65e** was afforded by concentration under reduced pressure. MS m/z (ESI): 152.16 [M+1].

Step 4: At 25°C, compound **65e** (27.40 mg, 0.18 mmol) and triethylamine (83.74 μL, 0.60 mmol) were added to a solution of compound **Int-2** (110 mg, 0.12 mmol) in N,N-dimethylformamide (3 mL), followed by stirring at 100°C for 2 hours. The crude product was afforded by concentration under reduced pressure. The crude was purified by preparative HPLC (chromatographic column: Waters-Xbridge-C18-10μm-19*250mm; mobile phase: A: 10 mM ammonium bicarbonate/water; B: acetonitrile, gradient ratio: acetonitrile 40 - 60%, flow rate: 25 mL/min) to afford compound **4.** MS m/z (ESI): 529.26 [M+1]. $^1$HNMR(400 MHz, DMSO-$d_6$): δ 9.65 (s, 1H), 8.39 (s, 1H), 7.79 (s, 1H), 7.55 (s, 2H), 7.44 (d, *J* = 5.0 Hz, 1H), 6.90 (d, *J* = 9.0 Hz, 1H), 6.23 (d, *J* = 1.4 Hz, 1H), 4.74 (d, *J* = 11.8 Hz, 1H), 4.56-4.46 (m, 2H), 4.16 (dd, *J* = 13.2, 0.6 Hz, 2H), 3.66-3.53 (m, 2H), 3.26-3.06 (m, 4H), 2.86 (dd, *J* = 9.2, 6.6 Hz, 1H), 2.69 (dd, *J* = 9.2, 4.6 Hz, 1H), 2.57 (dd, *J* = 10.0, 0.6 Hz, 1H), 2.23 (d, *J*= 14.8 Hz, 5H), 2.10 (dd, *J* = 11.8, 1.4 Hz, 2H), 1.99-1.94 (m, 2H).

**Example 5. Preparation of compound 5**

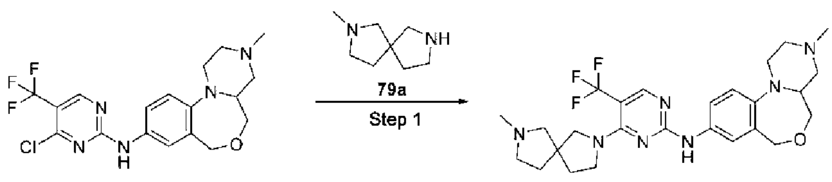


Int-2

Compound 5

Compound **Int-2** (50 mg, 0.30 mmol) and **79a** (16.94 mg, 0.12 mmol, Bide) were dissolved in N,N-dimethylformamide (0.5 mL). N,N-diisopropylethylamine (39.96 μL, 0.24 mmol) was added at room temperature, and the resulting mixture was stirred at room temperature for 1 hour. The reaction solution was purified by high performance liquid chromatography Gilson_306_1741, chromatographic column: Waters-SunFire-C18-10μm-19*250mm; mobile phase: water (containing 10 mM ammonium bicarbonate) and acetonitrile, gradient ratio: acetonitrile 30%-68%, flow rate: 20 mL/min) to afford compound 5. MS m/z (ESI): 518.4 [M+1]. $^1$H NMR (400 MHz, DMSO-$d_6$): δ 9.53 (s, 1H), 8.29 (s, 1H), 7.64 - 7.54 (m, 2H), 6.90 (d, *J* = 8.6 Hz, 1H), 4.76 (d, *J* = 11.6 Hz, 1H), 4.52 (d, *J* = 11.8 Hz, 1H), 3.68 - 3.52 (m, 5H), 3.47 (s, 1H), 3.20 - 3.06 (m, 2H), 2.86 (s, 1H), 2.70 - 2.65 (m, 1H), 2.58 (d, *J* = 10.6 Hz, 2H), 2.45 (d, *J* = 8.6 Hz, 2H), 2.38 - 2.31 (m, 2H), 2.22 (d, *J* = 4.6 Hz, 7H), 1.93 (dt, *J* = 13.8, 6.6 Hz, 2H), 1.76 (d, *J* = 7.6 Hz, 2H).

**Example 6. Preparation of compound 6**

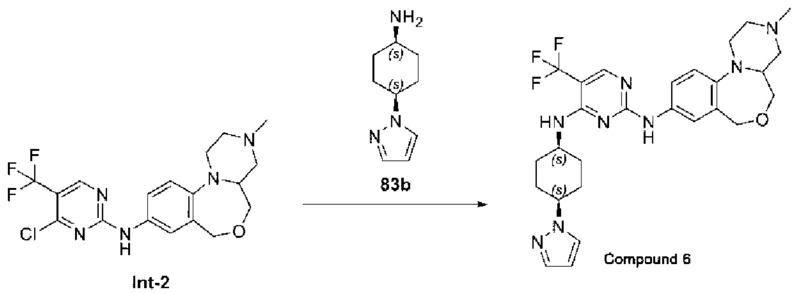


Int-2

Compound 6

Compound **Int-2** (50 mg, 0.12 mmol) and **83b** (24.16 mg, 0.12 mmol) were dissolved in N,N-dimethylformamide (1 mL). N,N-diisopropylethylamine (0.10 mL, 0.60 mmol) was added at room temperature, and the resulting mixture was

stirred at 100°C for 1 hour. The reaction solution was purified by high performance liquid chromatography Gilson_306_1741, chromatographic column: Waters-SunFire-C18-10μm-19*250mm; mobile phase: water (containing 0.1% formic acid) and acetonitrile, gradient ratio: acetonitrile 14%-95%, flow rate: 20 mL/min) to afford compound **6.** MS m/z (ESI): 543.2 [M+1]. $^1$H NMR (400 MHz, DMSO-*d*$_6$): δ 9.55 (s, 1H), 8.17 (s, 1H), 7.84 (d, *J* = 2.2 Hz, 1H), 7.56 (dd, *J* = 6.4, 2.8 Hz, 2H), 7.47 (d, *J* = 1.8 Hz, 1H), 6.91 (d, *J* = 9.2 Hz, 1H), 6.27 (t, *J* = 2.2 Hz, 2H), 4.79 (d, *J* = 11.8 Hz, 1H), 4.55 (d, *J* = 11.8 Hz, 1H), 4.29 (d, *J* = 19.2 Hz, 2H), 3.71 - 3.55 (m, 2H), 3.21 - 3.09 (m, 2H), 2.87 (d, *J* = 8.4 Hz, 1H), 2.72 (d, *J* = 11.0 Hz, 1H), 2.61 (d, *J* = 10.6 Hz, 1H), 2.25 (d, *J* = 11.6 Hz, 7H), 1.93 - 1.69 (m, 6H).

**Example** 7. **Preparation of compound 7**

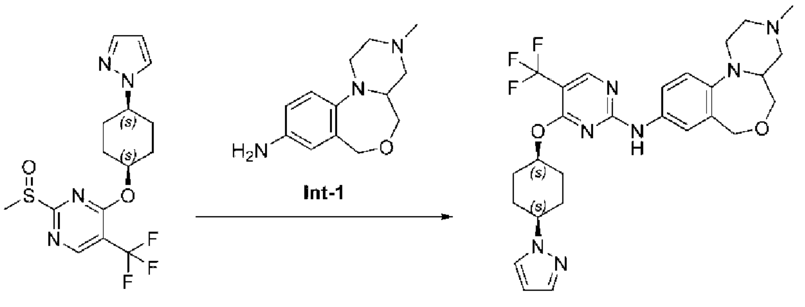


84a

Compound 7

At 25°C, trifluoroacetic acid (0.12 mL, 1.60 mmol) was added to a solution of compound **84a** (100 mg, 0.16 mmol) and compound **Int-1** (37.33 mg, 0.16 mmol) in isopropanol (3 mL). The resulting mixture was heated to 40°C and stirred for 18 hours. After cooling to room temperature, the reaction solution was purified by high performance liquid chromatography Gilson_306_1741, chromatographic column: Waters-SunFire-C18-10μm-19*250mm; mobile phase: water (containing 0.1% formic acid) and acetonitrile, gradient ratio: acetonitrile 0%-95%, flow rate: 20 mL/min) to afford compound **7.** MS m/z (ESI): 544.4 [M+1]. $^1$H NMR (400 MHz, DMSO-*d*$_6$): δ 10.01 (s, 1H), 8.53-8.44 (m, 1H), 7.67 (d, *J* = 2.2 Hz, 1H), 7.59 -7.49 (m, 2H), 7.45 (d, *J* = 1.8 Hz, 1H), 6.94 (d, *J* = 8.6 Hz, 1H), 6.24 (t, *J* = 2.0 Hz, 1H), 5.43 (s, 1H), 4.78 (d, *J* = 11.8 Hz, 1H), 4.56 (d, *J* = 11.8 Hz, 1H), 4.34-4.28 (m, 1H), 3.69-3.57 (m, 2H), 3.23-3.11 (m, 3H), 2.92-2.90 (m, 1H), 2.78-2.65 (m, 2H), 2.32-2.28 (m, 4H), 2.14-1.82 (m, 8H).

**Example 8. Preparation of compound 8**

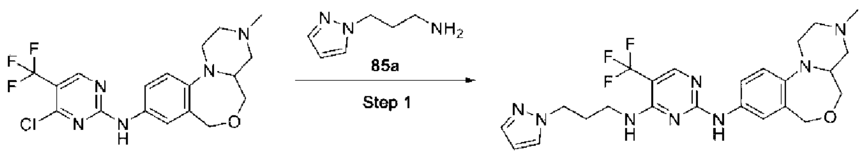


Int-2

Compound 8

Compound **Int-2** (40 mg, 0.10 mmol) and **85a** (12 mg, 0.10 mmol) were dissolved in N,N-dimethylformamide (1.5 mL). N,N-diisopropylethylamine (0.03 mL, 0.19 mmol) was added at room temperature, and the resulting mixture was stirred at room temperature for 1 hour. The reaction solution was purified by high performance liquid chromatography Gilson_306_1741, chromatographic column: Waters-SunFire-C18-10μm-19*250mm; mobile phase: water (containing 0.1% formic acid) and acetonitrile, gradient ratio: acetonitrile 14%-95%, flow rate: 20 mL/min) to afford compound 8. MS m/z (ESI): 503.4 [M+1]. $^1$H NMR (400 MHz, DMSO-*d*$_6$): δ 9.49 (s, 1H), 8.15 (s, 1H), 7.71 (d, *J* = 2.2 Hz, 1H), 7.59 (dd, *J* = 8.6, 2.4 Hz, 1H), 7.51 (d, *J* = 2.4 Hz, 1H), 7.42 (d, *J* = 1.8 Hz, 1H), 7.17 (s, 1H), 6.90 (d, *J* = 8.6 Hz, 1H), 6.21 (t, *J* = 2.0 Hz, 1H), 4.76 (d, *J* = 11.8 Hz, 1H), 4.52 (d, *J* = 11.8 Hz, 1H), 4.13 (t, *J* = 7.0 Hz, 2H), 3.69 - 3.53 (m, 2H), 3.43 (q, *J* = 6.4 Hz, 2H), 3.14 (dd, *J* = 26.0, 10.6 Hz, 2H), 2.85 (s, 1H), 2.77 - 2.56 (m, 2H), 2.23 (s, 5H), 2.11-2.04 (m, 2H).

**Example 9. Preparation of compound 9**

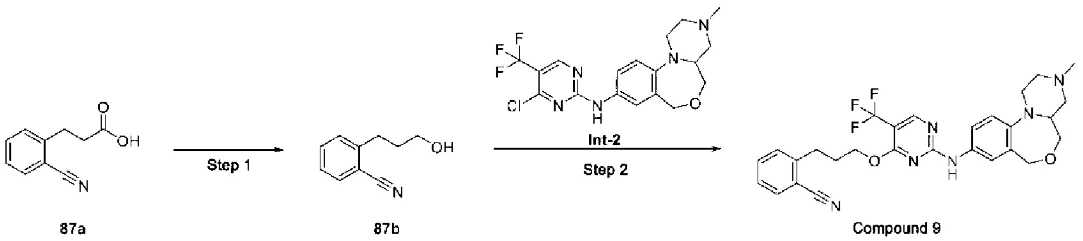


Step 1: At 0°C, 2-chloro-2-oxoacetyl chloride (0.49 mL, 5.71 mmol) was added to a solution of **87a** (400 mg, 2.28 mmol, Bide) in dichloromethane (3 mL), followed by stirring at 25°C for 1 hour under $N_2$ atmosphere. The mixed solution was concentrated to dryness. Then, the solid was dissolved in tetrahydrofuran (3 mL), and sodium borohydride (0.84 mL, 22.80 mmol) was added. The resulting mixture was stirred at 25°C for 16 hours. The mixture was poured into water (10 mL) for quenching, followed by extraction with ethyl acetate (20 mL×3). The organic phases were combined, washed with saline (20 mL), dried over anhydrous sodium sulfate, filtered, and concentrated under reduced pressure to dryness, to afford the crude product. The crude product was purified by silica gel chromatography (eluant: petroleum ether: tetrahydrofuran =1:1) to afford compound **87b.** MS m/z (ESI): 162.2 [M+1].

Step 2: At 0°C, sodium hydride (38.66 mg, 60% purity, 0.97 mmol) was added to a solution of compound **87b** (101 mg, 0.63 mmol) in tetrahydrofuran (5 mL) with stirring. Then, **Int-2** (200 mg, 0.48 mmol) was added. The mixture was stirred at 60°C for 18 hours under $N_2$ atmosphere. It was quenched by adding water (1 mL). The mixture was concentrated under reduced pressure to afford the crude product. The crude product was purified by preparative HPLC (chromatographic column: Waters-Xbridge-C18-10μm-19*250mm; mobile phase: A: 10 mM formic acid/water; B: acetonitrile, gradient ratio: acetonitrile 40-60%, flow rate: 25 mL/min) to afford compound **9.** MS m/z (ESI): 539.2 [M+1]. $^1$H NMR (400 MHz, DMSO-$d_6$): δ 10.01 (s, 1H), 8.48 (s, 1H), 7.78 (dd, *J* = 7.8, 1.4 Hz, 1H), 7.67 - 7.62 (m, 1H), 7.57 (dd, *J* = 8.6, 2.4 Hz, 1H), 7.48 - 7.39 (m, 3H), 6.91 (d, *J* = 8.6 Hz, 1H), 4.74 (d, *J* = 11.8 Hz, 1H), 4.54 - 4.44 (m, 3H), 3.66 - 3.56 (m, 2H), 3.18 - 3.09 (m, 2H), 2.96 - 2.86 (m, 3H), 2.72 - 2.57 (m, 2H), 2.22 (s, 5H), 2.15 - 2.08 (m, 2H).

**Example 10. Preparation of compound 10**

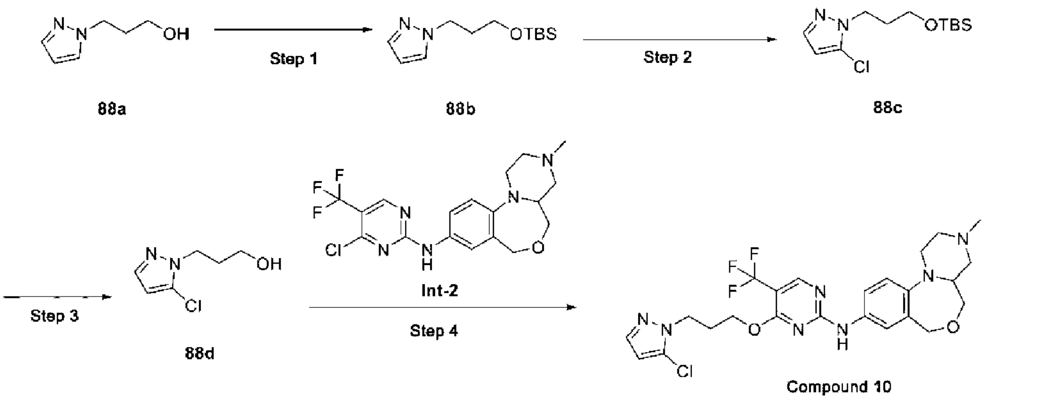


Step 1: At 0°C, tert-butyldimethylsilyl chloride (7.7 g, 51.19 mmol) and imidazole (4.60 g, 68.22 mmol) were added to a solution of compound **88a** (4.30 g, 34.12 mmol, Bide) in dichloromethane (40 mL), respectively, followed by stirring at room temperature for 18 hours. The crude compound was afforded by concentration under reduced pressure, which was purified by silica gel chromatography (eluant: petroleum ether: ethyl acetate = 50:1-10:1) to afford compound **88b.** MS m/z (ESI): 241.1 [M+1].

Step 2: At -78°C, n-butyl lithium (15.0 mL, 24.0 mmol) was added to a solution of compound **88b** (4.80 g, 20.0 mmol) in tetrahydrofuran (60 mL), followed by stirring at -78°C for 1 hour. Then, hexachloroethane (7.10 g, 30.0 mmol) was added. The resulting mixture was allowed to warm to 25°C gradually and then stirred for 18 hours. The mixture was poured into water (100 mL), followed by extraction with ethyl acetate (50 mL x 3). The organic phases were combined, washed with saturated saline (100 mL), dried over anhydrous sodium sulfate, filtered, and concentrated under reduced pressure to afford the crude compound. The crude was purified by silica gel chromatography (eluant: petroleum ether : ethyl acetate = 50:1-10:1) to afford compound **88c.** MS m/z (ESI): 275.1 [M+1].

Step 3: At 0°C, hydrochloric acid/dioxane (2.00 mL, 8.00 mmol) was added to a solution of compound **88c** (1.00 g,

3.63 mmol) in 1,4-dioxane (8 mL), followed by stirring at room temperature for 2 hours. The mixture was adjusted to pH=7 using saturated sodium bicarbonate solution, followed by extraction with dichloromethane (20 mL× 3). The organic phases were combined, washed with saturated saline (10 mL), dried over anhydrous sodium sulfate, filtered, and concentrated under reduced pressure to afford compound **88d.** MS m/z (ESI): 161.0 [M+1].

Step 4: At 0°C, sodium hydride (12 mg, 0.300 mmol, 60% purity) was added to a solution of compound **88d** (60 mg, 0.375 mmol) in tetrahydrofuran (5 mL). After stirring for 30 minutes, compound **Int-2** (100 mg, 0.250 mmol) was added. The resulting mixture was heated to 60°C and stirred for 18 hours. The crude product was afforded by concentration under reduced pressure. The crude product was purified by preparative HPLC (chromatographic column: Waters-Xbridge-C18-10μm-19*250 mm; mobile phase: A: 10 mM ammonium bicarbonate/water; B: acetonitrile, gradient ratio: acetonitrile 35-95%, flow rate: 25 mL/min) to afford compound 10. MS m/z (ESI): 537.9 [M+1]. $^1$H NMR (400 MHz, DMSO-$d_6$): δ 10.02 (s, 1H), 8.49 (s, 1H), 7.61 - 7.52 (m, 2H), 7.45 (s, 1H), 6.92 (d, *J* = 8.6 Hz, 1H), 6.39 (d, *J* = 1.8 Hz, 1H), 4.75 (d, *J* = 11.8 Hz, 1H), 4.54 (d, *J* = 11.8 Hz, 1H), 4.39 (t, *J* = 6.0 Hz, 2H), 4.26 (t, *J* = 6.8 Hz, 2H), 3.69 - 3.53 (m, 2H), 3.22 - 3.07 (m, 2H), 2.93 - 2.85 (m, 1H), 2.76 - 2.65 (m, 1H), 2.63 - 2.56 (m, 1H), 2.29 - 2.15 (m, 7H).

**Example 11. Preparation of compound 11**

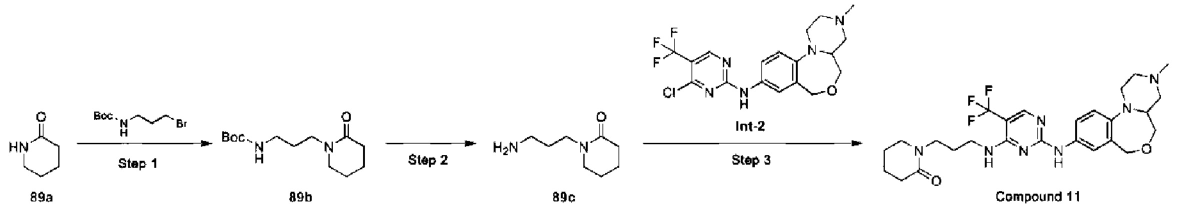


89a 89b 89c Compound 11

Step 1: At 25°C, sodium hydride (0.61 g, 15.13 mmol, 60% purity) was added to a solution of compound **89a** (3.00 g, 14.91 mmol, Bide) in tetrahydrofuran (20 mL). After reacting under stirring at 25°C for 20 minutes, N-tert-butoxycarbonyl-3-aminopropyl bromide (2643.00 mg, 11.10 mmol, Bide) was added. The resulting mixture was heated to 60°C and stirred for 18 hours. After cooling to room temperature, the mixture was poured into water (30 mL) for quenching, followed by extraction with ethyl acetate (50 mL x3). The organic phases were combined, washed with saturated saline (50 mL), dried over anhydrous sodium sulfate, filtered, and concentrated under reduced pressure to afford the crude product. The crude was purified by silica gel chromatography (elution solution: petroleum ether-tetrahydrofuran=50-60%) to afford compound **89b.** MS m/z (ESI): 257.1 [M+1].

Step 2: At 25°C, hydrochloric acid (3 mL, 12.00 mmol, 4 mol/L) was added to a solution of compound **89b** (300 mg, 1.17 mmol) in dichloromethane (3 mL), followed by stirring at 25°C for 3 hours. Compound **89c** was afforded by concentration under reduced pressure. MS m/z (ESI): 157.1 [M+1].

Step 3: At 25°C, compound **89c** (24mg, 0.16 mmol) and triethylamine (90.44 μL, 0.65 mmol) were added to a solution of compound **Int-2** (108 mg, 0.13 mmol) in acetonitrile (3 mL), respectively, followed by stirring at 90 °C for 18 hours. The crude product was afforded by concentration under reduced pressure. The crude was purified by preparative HPLC (chromatographic column: Waters-Xbridge-C18-10μm-19*250mm; mobile phase: A: 10 mM ammonium bicarbonate/water; B: acetonitrile, gradient ratio: acetonitrile 40 - 60%, flow rate: 25 mL/min) to afford compound **11.** MS m/z (ESI): 534.4 [M+1]. $^1$HNMR(400 MHz, DMSO-$d_6$): δ 9.49 (s, 1H), 8.18 (s, 1H), 7.62-7.53 (m, 2H), 7.19 (t, *J* = 5.8 Hz, 1H), 6.91 (d, *J* = 8.6 Hz, 1H), 4.77 (d, *J* = 11.8 Hz, 1H), 4.52 (d, *J* = 11.8 Hz, 1H), 3.39 (d, *J* = 6.2 Hz, 2H), 3.31 (t, *J* = 6.6 Hz, 2H), 3.27-3.01 (m, 6H), 2.85 (dt, *J* = 7.2, 3.6 Hz, 1H), 2.72 (dd, *J* = 9.0, 5.6 Hz, 1H), 2.62 (dd, *J* = 10.8, 2.6 Hz, 1H), 2.32-2.17 (m, 7H), 1.79-1.63 (m, 6H).

**Example 12. Preparation of compound 12**

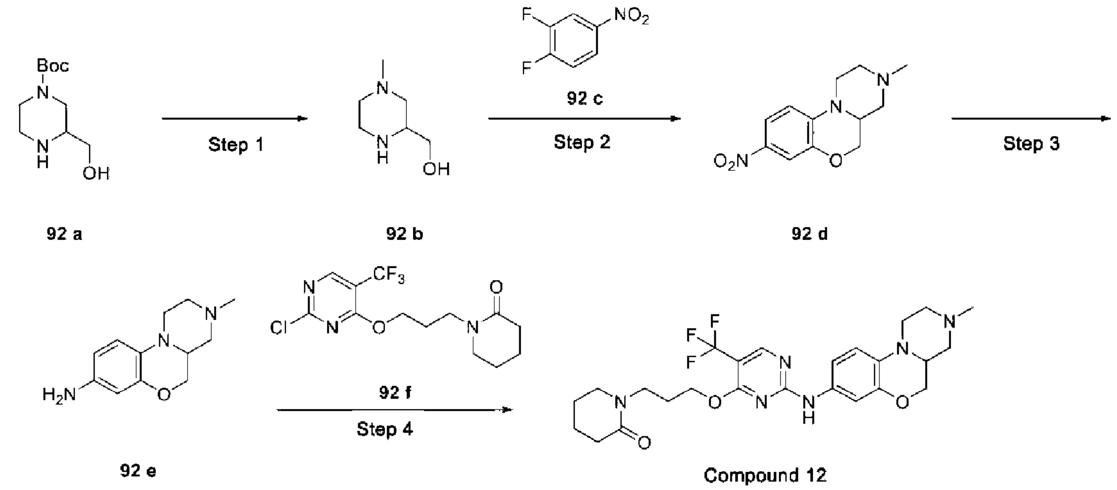


92 a

92 b

92 d

92 e

Compound 12

Step 1: Under nitrogen protection, compound **92a** (5.00 g, 23.12 mmol, Bide) was dissolved in tetrahydrofuran (50 mL). Then, lithium aluminium hydride (36.99 mL, 92.47 mmol, Energy) was added under an ice bath. The resulting mixture was slowly warmed to room temperature, followed by reacting under stirring for 18 hours. After the reaction was completed, water (37 mL) and sodium hydroxide aqueous solution (37 mL, 15% wt) were added to the reaction solution in sequence, followed by stirring at room temperature for 15 minutes. Then, anhydrous sodium sulfate was added, followed by filtration, The filter residue was washed with ethyl acetate (100 mL), and the layers were separated. The organic layers were combined and concentrated under reduced pressure to dryness to afford compound **92b.** MS m/z (ESI): 131.1 [M+1].

Step 2: Compound **92b** (2.00 g, 15.36 mmol) was dissolved in dimethyl sulfoxide (30 mL). Then, compound **92c** (1.96 g, 12.29 mmol) and potassium hydroxide (2.15 g, 38.41 mmol, Energy) were added, respectively, followed by reacting under stirring at 60°C for 2 hours. After the reaction was completed, water (30 mL) was added to the reaction solution, followed by extraction with ethyl acetate (50 mL×3). The organic phases were combined, washed with saturated saline (30 mL), dried over anhydrous sodium sulfate, filtered, and concentrated under reduced pressure to dryness. The residue was purified by silica gel column chromatography (methanol: dichloromethane =2% - 3%) to afford compound **92d.** MS m/z (ESI): 250.1 [M+1].

Step 3: Compound **92d** (300 mg, 1.2 mmol) was dissolved in methanol (5 mL). Then, wet palladium on carbon (128 mg, 0.12 mmol, 10%, Bide) was added, and the resulting mixture was stirred at room temperature for 2 hours under hydrogen protection. After the reaction was completed, the reaction solution was filtered and washed with methanol. The filtrate was concentrated under reduced pressure to dryness to afford compound **92e.** MS m/z (ESI): 220.0 [M+1].

Step 4: Compound **92e** (150 mg, 0.68 mmol) was dissolved in isopropanol (1.5 mL). Then, compound **92f** (184 mg, 0.54 mmol) and trifluoroacetic acid (254.88 μL, 3.42 mmol) were added, respectively, and the resulting mixture was stirred at 50°C for 18 hours. After the reaction was completed, the reaction solution was concentrated under reduced pressure to dryness. The residue was purified by high performance liquid chromatography (chromatographic column: Waters-Xbridge-C18-10μm-19*250mm; mobile phase: water (containing 10 mmol $NH_4HCO_3$) and acetonitrile, gradient ratio: acetonitrile 39%-69%, flow rate: 25 mL/min) to afford compound **12.** MS m/z (ESI): 521.0 [M+1]. $^1$H NMR (400 MHz, DMSO-d6): δ 9.90 (s, 1H), 8.44 (s, 1H), 7.13 (d, J = 7.2 Hz, 2H), 6.82 (d, J = 8.8 Hz, 1H), 4.41 (t, J = 6.4 Hz, 2H), 4.21 (dd, J = 10.4, 2.8 Hz,1H), 3.94 - 3.81 (m, 1H), 3.70 - 3.58 (m, 1H), 3.39 - 3.34 (m, 2H), 3.24 (t, J = 5.6 Hz, 2H), 3.02 - 2.94 (m, 1H), 2.85 (d, J = 11.2 Hz, 1H), 2.78 (d, J = 10.4 Hz, 1H),2.58 (td, J = 11.6, 3.2 Hz, 1H), 2.21 (s, 3H), 2.18 (t, J = 6.4 Hz, 2H), 2.11 - 2.03 (m, 1H), 1.97-1.92 (m, 2H), 1.73 - 1.63 (m, 5H).

**Example 13. Preparation of compound 13**

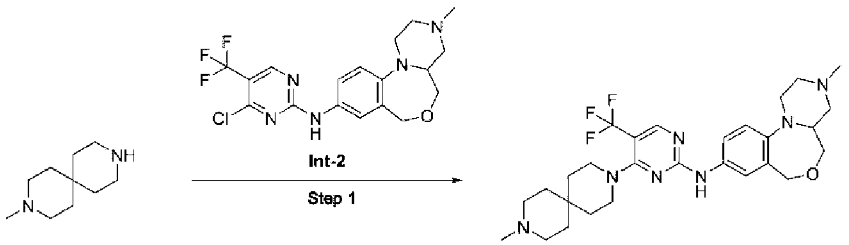


95a Compound 13

Triethylamine (0.20 mL, 1.45 mmol) and **Int-2** (200 mg, 0.48 mmol) were added to a solution of compound **95a** (89 mg, 0.53 mmol, Bide) in acetonitrile (4 mL), respectively, followed by stirring at 25°C for 1 hour. The mixture was concentrated under reduced pressure to afford the crude product. The crude product was purified by preparative HPLC (chromatographic column: Waters-Xbridge-C18-10 μm-19*250 mm; mobile phase: A: 10 mM ammonium bicarbonate/-water; B: acetonitrile, gradient ratio: acetonitrile 40 - 60%, flow rate: 25 mL/min) to afford compound **13.** MS m/z (ESI): 546.4 [M+1]. $^1$H NMR (400 MHz, DMSO-$d_6$): δ 9.59 (s, 1H), 8.33 (s, 1H), 7.54 (d, *J* = 8.2 Hz, 2H), 6.91 (d, *J* = 8.4 Hz, 1H), 4.76 (d, *J* = 11.8 Hz, 1H), 4.52 (d, J = 11.8 Hz, 1H), 3.67 - 3.45 (m, 7H), 3.19 - 3.09 (m, 2H), 2.74 - 2.65 (m, 1H), 2.62 - 2.56 (m, 1H), 2.33 - 2.16 (m, 12H), 1.51-1.47 (m, 8H).

**Example 14. Preparation of compound 14**

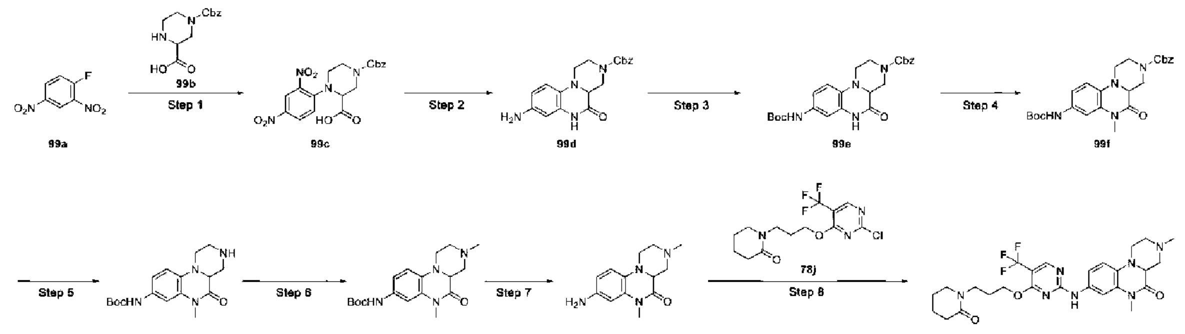


99a 99c 99d 99e 99f 99g 99h 99i Compound 14

Step 1: Compound **99a** (3.38 mL, 26.87 mmol, Bide) and **99b** (7.81 g, 29.55 mmol, Bide) were dissolved in dimethyl sulfoxide (50 mL). Triethylamine (11.17 mL, 80.60 mmol) was added at room temperature, followed by heating to 60°C. The reaction was carried out for 18 hours. The reaction solution was diluted with water (50 mL) and adjusted to pH=5 using hydrochloric acid aqueous solution (1 M), followed by extraction with ethyl acetate (30 mL×3). The organic phases were combined, washed with saturated saline (200 mL), dried over anhydrous sodium sulfate, filtered, and concentrated under reduced pressure to afford compound **99c.** MS m/z(ESI):431.3 [M+1].

Step 2: Compound **99c** (1.20 g, 2.79 mmol) and iron powder (1.25 g, 22.31 mmol) were dissolved in acetic acid (30 mL). The reaction was carried out at 60°C for 3 hours. The reaction solution was diluted with water (50 mL) and adjusted to pH=7 using sodium hydroxide aqueous solution (2 M). After filtration, the solid was washed with dichloromethane/-methanol (5:1, 30 mL). The resulting filtrate was concentrated to afford compound **99d.** MS m/z (ESI): 353.4 [M+1].

Step 3: Compound **99d** (666 mg, 1.89 mmol) and triethylamine (0.52 mL, 3.78 mmol) were dissolved in tetrahydrofuran (5 mL), and di-tert-butyl dicarbonate (0.49 mL, 2.27 mmol) was added. The reaction was carried out at room temperature for 18 hours. The reaction solution was concentrated to dryness. The crude was purified by silica gel column chromatography (eluant system: ethyl acetate: petroleum ether = 10:1 to 5:1) to afford compound **99e.** MS m/z (ESI): 453.6 [M+1].

Step 4: Compound **99e** (230 mg, 0.51 mmol) was dissolved in tetrahydrofuran (5 mL), and sodium hydride (24 mg, 60% purity, 0.61 mmol) was added at 0 °C. The reaction was carried out at 0°C for 0.5 hours. Iodomethane (30.04 μL, 0.48 mmol) was added to the reaction solution, followed by warming to room temperature to react for 1 hour. The reaction solution was diluted with water (20 mL), followed by extraction with ethyl acetate (30 mL x 3). The organic phases were combined, washed with saturated saline (20 mL), dried over anhydrous sodium sulfate, filtered, and concentrated to dryness. The crude was purified by silica gel column chromatography (eluant system: ethyl acetate: petroleum ether = 10:1 to 1:1) to afford compound **99f.** MS m/z (ESI): 467.5 [M+1].

Step 5: Compound **99f** (220 mg, 0.33 mmol) was dissolved in methanol (5 mL), and 10% palladium on carbon (85

mg, 0.08 mmol) was added. The reaction was carried out at room temperature for 18 hours under a hydrogen atmosphere. The reaction solution was filtered, and the filtrate was concentrated to dryness to afford compound **99g.** MS m/z (ESI): 333.4 [M+1].

Step 6: Compound **99g** (170 mg, 0.51 mmol), and formaldehyde (0.38 mL, 5.11 mmol) were dissolved in methanol (5 mL), and sodium triacetoxyborohydride (539 mg, 2.56 mmol) was added. The reaction was carried out at room temperature for 4 hours. It was quenched with a saturated sodium carbonate solution (20 mL), followed by extraction with ethyl acetate (30 mL×3). The organic phases were combined, washed with saturated saline (20 mL), dried over anhydrous sodium sulfate, filtered, and concentrated to afford compound **99h.** MS m/z (ESI): 347.4 [M+1].

Step 7: Compound **99h** (150 mg, 0.43 mmol) and trifluoroacetic acid (0.65 mL, 8.66 mmol) were dissolved in dichloromethane (5 mL). The reaction was carried out at room temperature for 1 hour. The reaction solution was concentrated to dryness to afford compound **99i.** MS m/z (ESI): 247.4 [M+1].

Step 8: Compound **99i** (50 mg, 0.20 mmol) and **78j** (206 mg, 0.61 mmol) were dissolved in isopropanol (1 mL). The reaction was carried out at 40°C for 2 hours. The reaction solution was purified by high performance liquid chromatography (Gilson_306_1741, chromatographic column: Shimadzu-Shim-pack-C18-5μm-20*250mm; mobile phase: water (containing 0.1% formic acid) and acetonitrile, gradient ratio: acetonitrile 14%-95%, flow rate: 20 mL/min) to afford compound **14.** MS m/z (ESI): 548.4 [M+1]. $^1$H NMR (400 MHz, DMSO-$d_6$): δ 10.03 (s, 1H), 8.48 (s, 1H), 7.51 (d, $J$ = 2.4 Hz, 1H), 7.34 (s, 1H), 6.87 (d, $J$ = 8.6 Hz, 1H), 4.44 (t, $J$ = 6.4 Hz, 2H), 3.51 (s, 1H), 3.25 (d, $J$ = 10.4 Hz, 8H), 2.89 (d, $J$ = 11.2 Hz, 1H), 2.68 (d, $J$ = 11.2 Hz, 1H), 2.28 (s, 3H), 2.17 (t, $J$ = 6.0 Hz, 2H), 2.09 - 1.92 (m, 5H), 1.68 (s, 4H).

## Example 15. Preparation of compound 15

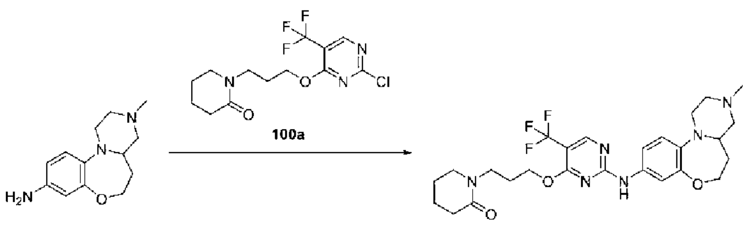


Int-1

Compound 15

Compound **Int-1** (40 mg, 0.14 mmol) was dissolved in isopropanol (1 mL). Then, compound **100a** (46 mg, 0.14 mmol) and trifluoroacetic acid (0.10 mL, 1.37 mmol) were added, respectively, and the resulting mixture was stirred at 40°C for 18 hours. After the reaction was completed, the reaction solution was purified by high performance liquid chromatography (chromatographic column: Waters-SunFire-C18-10μm-19*250mm; mobile phase: water (containing 0.1% formic acid) and acetonitrile, gradient ratio: acetonitrile 12%-52%, flow rate: 25 mL/min) to afford compound **15.** MS m/z (ESI): 535.0 [M+1]. $^1$H NMR (400 MHz, DMSO-$d_6$): δ 9.96 (s, 1H), 8.47 (s, 1H), 7.30 (d, $J$ = 8.8 Hz, 1H), 7.20 - 7.09 (m, 1H), 6.91 (d, $J$ = 8.8 Hz, 1H), 4.46 - 4.37 (m, 3H), 4.11 - 4.06 (m, 1H),3.40 - 3.36 (s, 2H), 3.24 (t, $J$ = 5.6 Hz, 3H), 3.12 - 2.97 (m, 4H), 2.65 - 2.60 (m, 1H), 2.26 - 2.20 (m, 4H), 2.19 - 2.15 (m, 2H), 2.13 - 2.07 (m, 1H), 2.02 - 1.91 (m, 3H), 1.90 -1.83 (m, 1H), 1.71 - 1.66 (m, 3H).

## Example 16. Preparation of compound 16

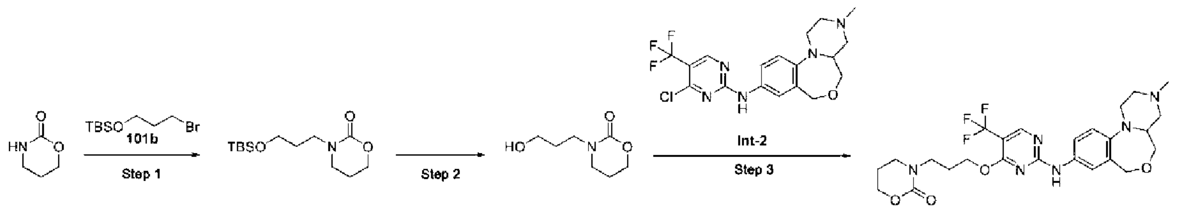


101a 101c 101d Compound 16

Step 1: At 0°C, sodium hydride (396 mg, 9.89 mmol, 60% purity) was added to a solution of compound **101a** (500 mg, 4.95 mmol, Bide) in N,N-dimethylformamide (10 mL), followed by stirring at 0°C for 20 minutes. Then, compound **101b** (1.73 mL, 7.42 mmol, Bide) was added, and the resulting mixture was stirred at 25°C for 18 hours. The mixture was poured

into water (20 mL), followed by extraction with ethyl acetate (20 mL x 3). The organic phases were combined, washed with saturated saline (20 mL x 4), dried over anhydrous sodium sulfate, filtered, and concentrated under reduced pressure to afford the crude product. The crude product was purified by silica gel chromatography (elution solution: petroleum ether - tetrahydrofuran =20 - 40%) to afford compound **101c.** MS m/z (ESI): 274.2 [M+1].

Step 2: At 25°C, tetrabutylammonium fluoride (0.81 ml, 2.74 mmol) was added to a solution of compound **101c** (0.50 g, 1.83 mmol) in tetrahydrofuran(5 mL), followed by stirring at 25°C for 18 hours. The mixture was concentrated under reduced pressure to afford the crude product. The crude product was purified by silica gel chromatography (elution solution: dichloromethane-methanol = 7-10%) to afford compound **101d.** MS m/z (ESI): 160.0 [M+1].

Step 3: At 0°C, sodium hydride (396 mg, 9.89 mmol, 60% purity) was added to a solution of compound **101d** (96 mg, 0.48 mmol) in tetrahydrofuran (4 mL), followed by reacting under stirring at 0°C for 20 minutes. Then, compound **Int-2** (19 mg, 0.48 mmol) was added, and the resulting mixture was stirred at 25°C for 18 hours. The mixture was poured into water (10 mL), followed by extraction with ethyl acetate (10 mL x 3). The organic phases were combined, washed with saturated saline (10 mL), dried over anhydrous sodium sulfate, filtered, and concentrated under reduced pressure to afford the crude product. The crude was purified by high performance liquid chromatography (Gilson_306_1741, chromatographic column: Waters-Xbridge-C18-10μm-19*250mm; mobile phase: water (containing 0.1% ammonium bicarbonate) and acetonitrile, gradient ratio: acetonitrile 0%-95%, flow rate: 20 mL/min) to afford compound **16.** MS m/z (ESI): 537.0 [M+1]. $^1H$ NMR (400 MHz, DMSO-$d_6$): δ 10.01 (s, 1H), 8.47 (d, *J* = 0.8 Hz, 1H), 7.60 (dd, *J* = 8.6, 2.6 Hz, 1H), 7.47 (s, 1H), 6.95 (d, *J* = 8.2 Hz, 1H), 4.76 (d, *J* = 11.8 Hz, 1H), 4.56 (d, *J* = 11.8 Hz, 1H), 4.45 (t, *J* = 6.4 Hz, 2H), 4.14 (t, *J* = 5.4 Hz, 2H), 3.69-3.54 (m, 2H), 3.36-3.32 (m, 2H), 3.29-3.26 (m, 2H), 3.21-3.08 (m, 2H), 2.92-2.85 (m, 1H), 2.71-2.67 (m, 1H), 2.61-2.58 (m,1H), 2.26-2.22 (m, 5H), 2.02-1.89 (m, 4H).

**Example 17. Preparation of compound 17**

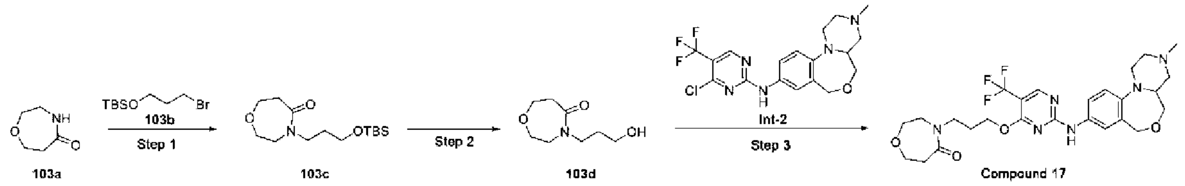


Step 1: At 0°C under $N_2$ protection, compound **103a** (1.00 g, 8.69 mmol, Bide) was dissolved in N,N-dimethylformamide (20 mL) solution, and the resulting solution was then slowly added to a reaction flask containing sodium hydride (0.42 g, 60% purity, 10.42 mmol). After stirring at 0°C for 10 minutes, **103b** (3.30 g, 13.03 mmol, Bide) was added, followed by warming to 25°C and stirring for 18 hours. The reaction solution was poured into water (20 mL) for dilution, followed by extraction with ethyl acetate (30 mL × 3). The organic phases were combined, washed with saturated saline (20 mL), dried over anhydrous sodium sulfate, filtered, and concentrated under reduced pressure to afford the crude product. The crude product was purified by silica gel column chromatography (eluant system: dichloromethane : methanol = 100 : 1 to 10 : 1) to afford compound **103c.** MS m/z (ESI): 288.1 [M+1].

Step 2: compound **103c** (1.00 g, 3.48 mmol) was dissolved in tetrahydrofuran (15 mL). Tetrabutylammonium fluoride (5.22 mL, 5.22 mmol) was added, and the resulting mixture was stirred at 25°C for 2 hours. The reaction solution was poured into water (10 mL) for dilution, followed by extraction with ethyl acetate (20 mL × 3). The organic phases were combined, washed with saturated saline (10 mL), dried over anhydrous sodium sulfate, filtered, and concentrated under reduced pressure to afford the crude product. The crude product was purified by silica gel column chromatography (eluant system: dichloromethane : methanol = 100 : 1 to 10 : 1) to afford compound **103d.** MS m/z (ESI): 174.0 [M+1].

Step 3: At 0°C under $N_2$ protection, compound **103d** (50 mg, 0.29 mmol) was dissolved in tetrahydrofuran (2 mL) solution, and the resulting solution was then slowly added to a reaction flask filled with sodium hydride (11.60 mg, 60% purity, 0.48 mmol). After stirring at 0°C for 10 minutes, **Int-2** (100 mg, 0.24 mmol) was added, followed by warming to 25°C and stirring for 18 hours. It was quenched by adding water (0.5 mL). The mixed solution was purified by high performance liquid chromatography Gilson_306_1741, chromatographic column: Waters-SunFire-C18-10μm-19*250mm; mobile phase: water (containing 0.1% ammonium bicarbonate) and acetonitrile, gradient ratio: acetonitrile 0%-95%, flow rate: 20 mL/min) to afford compound **17.** MS m/z (ESI): 551.3 [M+1]. $^1H$ NMR (400 MHz, DMSO-$d_6$): δ 10.01 (s, 1H), 8.47 (s, 1H), 7.60 (dd, *J* = 8.6, 2.6 Hz, 1H), 7.47 (d, *J* = 2.6 Hz, 1H), 6.95 (d, *J* = 8.8 Hz, 1H), 4.76 (d, *J* = 11.8 Hz, 1H), 4.56 (d, *J* = 11.8Hz, 1H), 4.42 (t, *J* = 6.6 Hz, 2H), 3.68 - 3.56 (m, 6H), 3.49 - 3.39 (m, 4H), 3.29 (s, 1H), 3.20-3.09 (m, 2H), 2.89 (s, 1H), 2.73 - 2.66 (m, 1H), 2.61 - 2.58 (m, 2H), 2.23 (s, 5H), 1.94 -1.88 (m , 2H).

**Example 18. Preparation of compound 18**

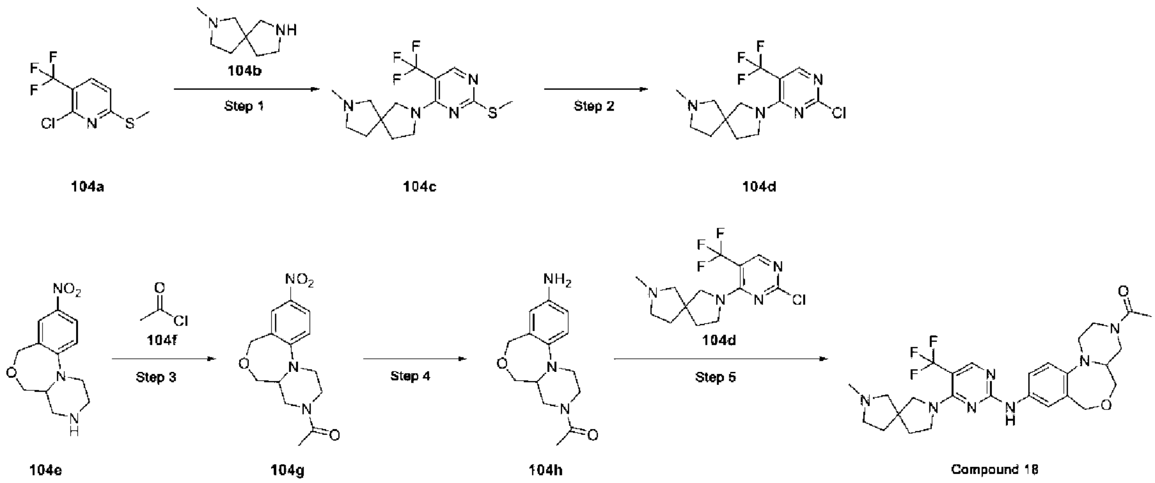


Step 1: Compound **104a** (195 mg, 0.86 mmol) was dissolved in acetonitrile (3 mL). Compound **104b** (80 mg, 0.57 mmol, Bide) and N,N-diisopropylethylamine (0.19 mL, 1.14 mmol, Bide) were added, respectively, and the resulting mixture was stirred at 40°C for 1 hour. After the reaction was completed, the reaction solution was concentrated under reduced pressure to dryness. The residue was purified by silica gel column chromatography (methanol: dichloromethane =10% - 18%) to afford compound **104c.** MS m/z (ESI): 333.2 [M+1].

Step 2: Compound **104c** (150 mg, 0.45 mmol) was dissolved in a mixed solution of dichloromethane : acetonitrile (1:1, 4 mL) under an ice bath. Sulfonyl chloride (364.70 μL, 4.51 mmol, Bide) was added, and the resulting mixture was stirred at 0°C for 3 hours. After the reaction was completed, the reaction solution was concentrated under reduced pressure to dryness. The residue was purified by silica gel chromatography (methanol : dichloromethane = 30% - 50%) to afford compound **104d.** MS m/z (ESI): 320.9 [M+1].

Step 3: At room temperature, compound **104e** (500 mg, 2.01 mmol) was dissolved in dichloromethane (10 mL). Then, compound **104f** (25.24 μL, 0.44 mmol, Bide) was added, and the resulting mixture was stirred at 25°C for 1 hour. After the reaction was completed, methanol (10 mL) was added to the reaction solution. Then, the reaction solution was concentrated under reduced pressure to dryness. The residue was purified by silica gel column chromatography (methanol : dichloromethane =1% - 3%) to afford compound **104g.** MS m/z (ESI): 292.0 [M+1].

Step 4: Compound **104g** (200 mg, 0.69 mmol) was dissolved in tetrahydrofuran (5 mL), and then wet palladium on carbon (73 mg, 0.07 mmol, 10%) was added. The reaction solution was purged with hydrogen 3 times, and then stirred at room temperature for 3 hours. After the reaction was completed, the reaction solution was filtered and washed with ethyl acetate (30 mL). The filtrate was concentrated to dryness to afford compound **104h.** MS m/z (ESI): 262.1 [M+1].

Step 5: Compound **104h** (100 mg, 0.38 mmol) was dissolved in isopropanol (2 mL). Then, compound **104d** (98 mg, 0.30 mmol) and trifluoroacetic acid (436.32 mg, 3.83 mmol) were added, respectively, and the resulting mixture was stirred at 40°C for 18 hours. After the reaction was completed, the reaction solution was purified by high performance liquid chromatography (chromatographic column: Waters-Xbridge-C18-10μm-19*250 mm; mobile phase: water (containing 10 mM ammonium bicarbonate) and acetonitrile, gradient ratio: acetonitrile 30%-60%, flow rate: 25 mL/min) to afford compound **18.** MS m/z (ESI): 546.3 [M+1]. $^1$H NMR (400 MHz, DMSO-$d_6$): δ 9.52 (s, 1H), 8.29 (s, 1H), 7.68 - 7.60 (m, 1H), 7.58 - 7.52 (m, 1H), 6.93 (d, *J* = 8.4 Hz, 1H), 4.80 - 4.65 (m, 1H), 4.59 - 4.47 (m, 1H), 4.02 - 3.41 (m, 9H), 3.30 - 3.19 (m, 2H), 3.15 (t, *J* = 8.0 Hz, 1H), 2.91 - 2.77 (m, 1H), 2.57 (q, *J* = 7.6 Hz, 1H), 2.44 (m, 2H), 2.35 (d, *J* = 9.2 Hz, 1H), 2.23 (s, 3H), 2.03 (d, *J* = 8.4 Hz, 3H), 1.98 - 1.86 (m, 2H), 1.81 - 1.69 (m, 2H).

**Example 19. Preparation of compound 19**

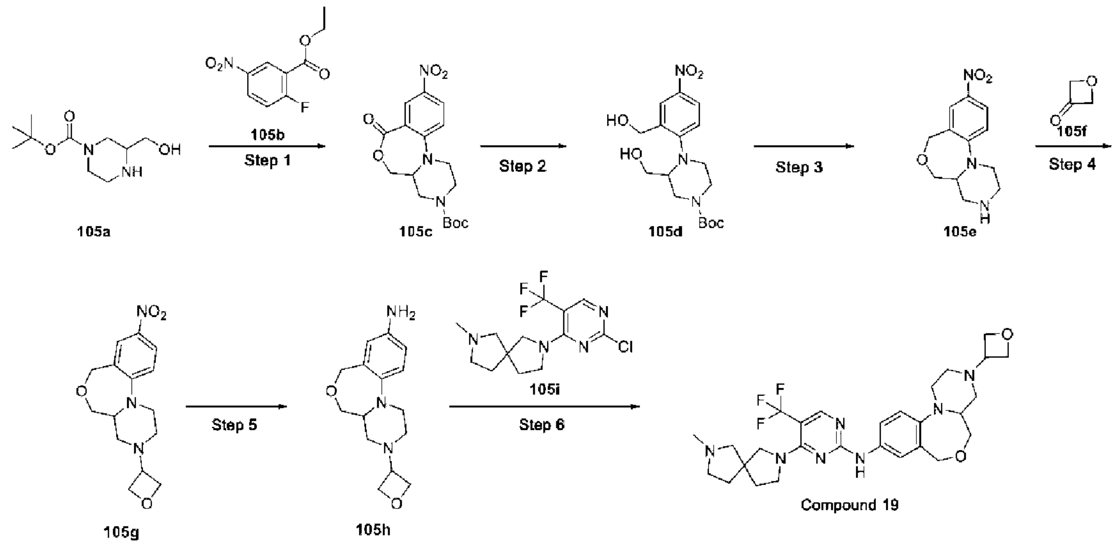


Step 1: Compound **105a** (5.00 g, 23.12 mmol, Bide) and **105b** (4.93 g, 23.12 mmol, Bide) were added to 1,4-dioxane (80 mL). N,N-diisopropylethylamine (11.49 mL, 4.01 mmol) was added at room temperature, and the resulting mixture was stirred at 100 °C for 18 hours. The reaction solution was concentrated to dryness. The crude was purified by silica gel column chromatography (eluant system: ethyl acetate : petroleum ether = 10:1 to 1:1) to afford compound **105c.** MS m/z (ESI): 364.4 [M+1].

Step 2: Compound **105c** (2.40 g, 6.60 mmol) was added to tetrahydrofuran (30 mL). Lithium borohydride (0.33 mL, 0.66 mmol) and sodium borohydride (0.75 g, 19.81 mmol) were added at 0°C, respectively. The reaction was carried out at room temperature for 18 hours. The reaction solution was quenched with water (100 mL), followed by extraction with ethyl acetate (20 mL×3). The organic phases were combined, washed with saturated saline (100 mL), dried over anhydrous sodium sulfate, filtered, and concentrated to dryness. The crude was purified by silica gel column chromatography (eluant system : dichloromethane : methanol = 20:1 to 10:1) to afford compound **105d.** MS m/z (ESI): 368.4 [M+1].

Step 3: Compound **105d** (4.00 g, 10.89 mmol) was added to 50% of concentrated sulfuric acid (20 mL). The reaction was carried out at 120 °C for 1 hour. The reaction solution was quenched with saturated sodium bicarbonate (1200 mL) and concentrated to dryness. The solid was washed with dichloromethane (50 mL × 3) and filtered. The filtrate was concentrated to dryness to afford compound **105e.** MS m/z (ESI): 250.2 [M+1].

Step 4: Compound **105e** (0.50 g, 2.01 mmol) and **105f** (216.81 mg, 3.01 mmol, Bide) was added to 1,2-dichloroethane (10 mL). Sodium triacetoxyborohydride (0.85 g, 4.01 mmol) was added at room temperature. The reaction was carried out at room temperature for 18 hours. The reaction solution was quenched with saturated sodium bicarbonate (10 mL) solution, followed by extraction with ethyl acetate (20 mL×3). The organic phases were combined, washed with saturated saline (10 mL), dried over anhydrous sodium sulfate, filtered, and concentrated to afford compound **105g.** MS m/z (ESI): 306.4 [M+1].

Step 5: Compound **105g** (0.45 g, 1.47 mmol) was dissolved in tetrahydrofuran (5 mL), and 10% palladium on carbon (0.16 g, 0.15 mmol) was added. The reaction solution was stirred at room temperature for 18 hours under a hydrogen atmosphere. The reaction solution was filtered. The filter cake was washed with tetrahydrofuran (5 mL × 3), and the filtrate was concentrated to dryness to afford compound **105h.** MS m/z (ESI): 276.4 [M+1].

Step 6: Compound **105h** (0.15 g, 0.54 mmol) and **105i** (0.21 g, 0.65 mmol) were dissolved in isopropanol (5 mL). Trifluoroacetic acid (0.20 mL, 2.72 mmol) was added, and the reaction was carried out at 40°C for 1 hour. The reaction solution was purified by high performance liquid chromatography (Gilson_306_1741, chromatographic column: Shimadzu-Shim-pack-C18-5um-20*250mm; mobile phase: water (containing 10 mM ammonium bicarbonate) and acetonitrile, gradient ratio: acetonitrile 30%-95%, flow rate: 20 mL/min) to afford compound **19.** MS m/z (ESI): 560.0 [M+1]. $^1$H NMR (400 MHz, DMSO-$d_6$): δ 9.51 (s, 1H), 8.29 (s, 1H), 7.62 (s, 1H), 7.57 (dd, *J* = 8.8, 2.6 Hz, 1H), 6.91 (d, *J* = 8.6 Hz, 1H), 4.78 (d, *J* = 11.8 Hz, 1H), 4.56 (t, *J* = 6.8 Hz, 3H), 4.48 - 4.45 (m, 2H), 3.63 (dd, *J* = 12.8, 5.4 Hz, 3H), 3.56 (d, *J* = 11.4 Hz, 2H), 3.45 (d, *J* = 11.6 Hz, 2H), 2.87 (s, 1H), 2.72 - 2.63 (m, 2H), 2.57 (d, *J* = 8.8 Hz, 2H), 2.44 (d, *J* = 8.8 Hz, 2H), 2.38 - 2.29 (m, 2H), 2.23 (s, 3H), 2.19 - 2.12 (m, 2H), 1.93 (dt, *J* = 13.8, 6.6 Hz, 2H), 1.75 (s, 2H).

**Example 20. Preparation of compound 20**

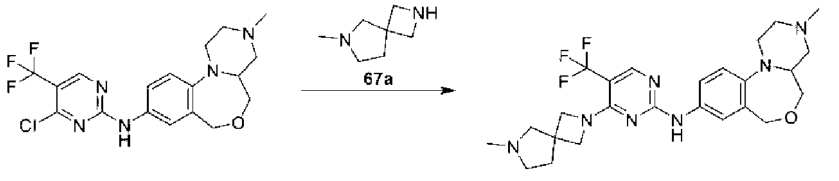


Int-2 Compound 20

Compound **Int-2** (0.10 g, 0.24 mmol) and **67a** (30.50 mg, 0.24 mmol) were added to N,N-dimethylformamide (1 mL) solution. Then, N,N-diisopropylethylamine (80.08 μL, 0.48 mmol) was added, and the resulting mixture was stirred at 25°C for 1 hour. The reaction solution was filtered, and the filtrate was purified by high performance liquid chromatography (chromatographic column: Waters-Xbridge-C18-10μm-19*250mm; mobile phase: A: 10 mM ammonium bicarbonate/-water; B: acetonitrile, gradient ratio: acetonitrile 95%, flow rate: 20 mL/min) to afford compound **20.** MS m/z (ESI): 504.4 [M+1]. $^1$H NMR (400 MHz, DMSO-$d_6$): δ 9.54 (s, 1H), 8.23 (s, 1H), 7.72 - 7.65 (m, 1H), 7.56 - 7.51 (m, 1H), 6.94 - 6.88 (m, 1H), 4.76 (d, 1H), 4.52 (d, 1H), 4.12 (s, 4H), 3.68 - 3.54 (m, 2H), 3.21 - 3.06 (m, 2H), 2.90 - 2.82 (m, 1H), 2.72 - 2.64 (m, 3H), 2.61 - 2.55 (m, 1H), 2.49 - 2.44 (m, 3H), 2.23 (d, $J$ = 7.4 Hz, 7H), 2.12 - 2.04 (m, 2H).

**Example 21. Preparation of compound 21**

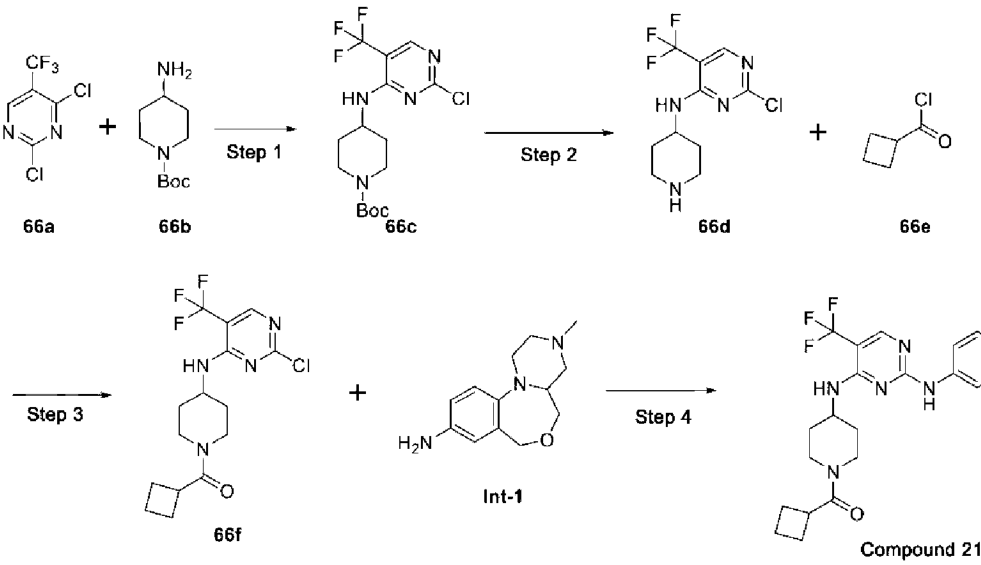


66a 66b 66c 66d 66e

66f Int-1 Compound 21

Step 1: Compound **66b** (0.83 g, 4.15 mmol, Bide) and triethylamine (0.70 mL, 5.07 mmol) were dissolved in N,N-dimethylformamide (7 mL), followed by stirring at -10°C for 20 minutes. Then, **66a** (0.62 mL, 4.61 mmol, Bide) was added, and the resulting mixture was stirred at -10°C for 3 hours. The reaction solution was diluted by adding water (30 mL), followed by extraction with ethyl acetate (20 mL×3). The organic phases were combined, dried over anhydrous sodium sulfate, filtered, and concentrated. The residue was purified by silica gel column chromatography (eluant system: petroleum ether : ethyl acetate = 10:1 to 1:1) to afford compound **66c.** MS m/z (ESI): 381.2 [M+1].

Step 2: Compound **66c** (0.35 g, 0.92 mmol) was dissolved in hydrochloric acid/ethyl acetate (7 mL, 4M) solution, followed by stirring at room temperature for 2 hour. The reaction solution was concentrated to dryness to afford compound **66d.** MS m/z (ESI): 281.2 [M+1].

Step 3: Compound **66d** (0.32 g, 0.80 mmol) and N,N-diisopropylethylamine (0.66 mL, 3.99 mmol) were dissolved in N,N-dimethylformamide (5 mL). **66e** (66.23 mg, 0.56 mmol) was added dropwise at 0°C, and the resulting mixture was stirred at 0°C for 30 minutes. The reaction solution was diluted by adding water (20 mL), followed by extraction with ethyl acetate (20 mL×3). The organic phases were combined, dried over anhydrous sodium sulfate, filtered, and concentrated. The residue was purified by silica gel column chromatography (eluant system: petroleum ether : tetrahydrofuran = 10:1 to 1:1) to afford compound **66f.** MS m/z (ESI): 363.2 [M+1].

Step 4: Compound **66f** (86 mg, 0.24 mmol) and **Int-1** (50 mg, 0.21 mmol) were dissolved in isopropanol (1 mL). Trifluoroacetic acid (0.08 mL, 1.07 mmol) was added, and the resulting mixture was stirred at 40°C for 18 hours. The reaction solution was purified by high performance liquid chromatography (Gilson_306_1741, chromatographic column:

Waters-Xbridge-C18-10μm-19*250mm; mobile phase: water (containing 10 mmol $NaHCO_3$) and acetonitrile, gradient ratio: acetonitrile 19%-49%, flow rate: 25 mL/min) to afford compound **21.** MS m/z (ESI): 560.4 [M+1]. $^1$H NMR (400 MHz, DMSO-$d_6$): δ 9.48 (s, 1H), 8.16 (s, 1H), 7.52 (d, *J* = 8.4 Hz, 2H), 6.90 (d, *J* = 8.4 Hz, 1H), 6.53 (d, *J* = 7.6 Hz, 1H), 4.77 (d, *J* = 11.6 Hz, 1H), 4.53 (d, *J* = 11.8 Hz, 1H), 4.47-4.25 (m, 2H), 3.73 (s, 1H), 3.68 - 3.53 (m, 2H), 3.35 (dt, *J* = 9.2, 4.8 Hz, 2H), 3.20 - 3.06 (m,2H), 2.91 (dd, J = 33.6, 10.0 Hz, 2H), 2.70 (d, *J* = 11.0 Hz, 1H), 2.63 - 2.54 (m, 2H), 2.27 - 2.23(m, 1H), 2.22 (s, 3H), 2.20 - 2.06 (m, 4H), 1.92 - 1.72 (m, 4H), 1.61 - 1.43 (m, 2H).

**Example 22. Preparation of compound 22**

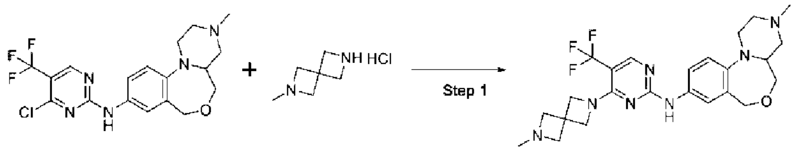


Int-2 68a Compound 22

Compound **Int-2** (0.13 g, 0.31 mmol) and **68a** (56 mg, 0.38 mmol, Bide) were dissolved in N,N-dimethylformamide (2 mL). N,N-diisopropylethylamine (0.26 mL, 1.57 mmol) was added, and the resulting mixture was stirred at 100°C for 1 hour. The reaction solution was purified by high performance liquid chromatography (Gilson_306_1741, chromatographic column: Waters-Xbridge-C18-10μm-19*250mm; mobile phase: water (containing 10 mM $NH_4HCO_3$) and acetonitrile, gradient ratio: acetonitrile 29%-59%, flow rate: 25 mL/min) to afford compound **22.** MS m/z (ESI): 490.2 [M+1]. $^1$H NMR (400 MHz, DMSO-$d_6$): δ 9.69 - 9.37 (m, 1H), 8.22 (s, 1H), 7.67 (d, *J* = 8.6 Hz, 1H), 7.55 (d, *J* = 2.6 Hz, 1H), 6.91 (d, *J* = 8.6 Hz, 1H), 4.76 (d, *J* = 11.8 Hz, 1H), 4.53 (d, *J* = 11.8 Hz, 1H), 4.25 (s, 4H), 3.68 - 3.55 (m, 2H), 3.29 (s, 4H), 3.20 - 3.09 (m, 2H), 2.86 (dd, *J* = 8.2, 4.0 Hz, 1H), 2.70 (d, *J* =11.0 Hz, 1H), 2.62 - 2.56 (m, 1H), 2.25 (dd, *J* = 7.6, 3.4 Hz, 2H), 2.22 (s, 3H), 2.18 (s, 3H).

**Example 23. Preparation of compound 23**

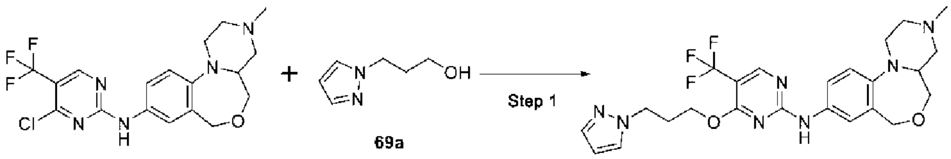


Int-2 69a Compound 23

Compound **69a** (36.6 mg, 0.29 mmol, Bide) was dissolved in tetrahydrofuran (2 mL), and sodium hydride (19.3 mg, 60% purity, 0.48 mmol) was added. After stirring at 0°C for 20 minutes, compound **Int-2** (100 mg, 0.24 mmol) was added, and the resulting mixture was stirred at 60°C for 18 hours. The reaction solution was purified by high performance liquid chromatography (Gilson_306_1741, chromatographic column: Waters-Xbridge-C18-10μm-19*250mm; mobile phase: water (containing 10 mM $NH_4HCO_3$) and acetonitrile, gradient ratio: acetonitrile 38%-68%, flow rate: 25 mL/min) to afford compound **23.** MS m/z (ESI): 504.4 [M+1]. $^1$H NMR (400 MHz, DMSO-$d_6$): δ 10.04 (s, 1H), 8.49 (s, 1H), 7.68 (d, *J* = 2.2 Hz, 1H), 7.57-7.54 (m, 1H), 7.46 (s, 1H), 7.44 (d, *J* = 1.8 Hz, 1H), 6.92 (d, *J* = 8.6 Hz, 1H), 6.24-6.23 (m, 1H), 4.75 (d, *J* =11.8 Hz, 1H), 4.54 (d, *J* = 11.8 Hz, 1H), 4.36 (t, *J* = 6.2 Hz, 2H), 4.24 (t, *J* = 6.8 Hz, 2H), 3.68 - 3.56 (m, 2H),3.21 - 3.08 (m, 2H), 2.91 - 2.86 (m, 1H), 2.70 (d, *J* = 11.2 Hz, 1H), 2.62 - 2.56 (m, 1H), 2.28 - 2.24 (m, 2H), 2.23 (d, *J* = 5.4 Hz, 5H).

**Biological evaluation**

Test example 1: Experiment on the inhibition of the compound on ULK1 protein activity

1. Test purpose

In this experiment, the inhibitory effect of a compound on the phosphorylation function of ULK1 was detected by ADP-Glo method. The inhibitory effect of the compound of the present disclosure on the ULK1 target was evaluated based on $IC_{50}$.

2. Experimental method

Compound formulation: Using an ECHO (Beckman, ECHO650), the compound was serially diluted 3-fold in DMSO in a 384-well plate (PE, 6007290). A total of 10 concentration points were set, with the maximum concentration of the compound being 10 μM, and 50 nL per well. Positive control wells and negative control wells were added with 50 nL DMSO, respectively.

Reaction process: A 32 nM of ULK1 (Promega, V3521) solution was formulated, and 2.5 μL/well was transferred to the compound test wells and the negative control wells (NC). 2.5 μL/well of 1× Assay buffer was added to the positive control wells (PC). A mixed solution of 100 μM of ATP and 1.86 μM of MBP was formulated, and 2.5 μL/well was transferred to the reaction plate. After centrifugation, the reaction was initiated and incubated at room temperature for 60 min. After the reaction was completed, 5 μL/well of ADP-Glo Reagent (Promega, V9102) was added to the reaction plate. After centrifugation, the reaction was incubated at room temperature for 40 minutes. Finally, 10 μL/well of Kinase Detection Buffer was added, followed by centrifugation and incubation at room temperature for 30 min. The chemiluminescence value was read using Envision (PE, Envision2105).

Assay buffer: a reaction buffer; and Kinase Detection buffer: a detection buffer, both were taken from the ADP-Glodetection kit, purchased from Promega, model V9102.

3. Data analysis

The inhibition ratio of a compound on ULK1 activity was calculated using the following formula:

Inhibition ratio = (sample signal value - negative control signal value)/(positive control signal value - negative control signal value) x 100%.

The dose-effect curve was fitted using the log(inhibitor) vs. response -Variable slope of the analysis software GraphPad Prism 8, thereby affording the $IC_{50}$ value of each compound for enzyme activity.

The experimental results of this test example are shown in Table 1 below:

Table 1: $IC_{50}$ of the compounds of the present invention for inhibiting ULK1

| Compounds | ULK1 $IC_{50}$ (nM) | Compounds | ULK1 $IC_{50}$ (nM) | Compounds | ULK1 $IC_{50}$ (nM) |
|---|---|---|---|---|---|
| 1 | B | 5 | A | 13 | B |
| 2 | A | 6 | A | 14 | A |
| 3 | A | 7 | A | 15 | A |
| 4 | A | 8 | A | 16 | A |
| 21 | A | 9 | A | 18 | A |
| 20 | A | 10 | A | 19 | A |
| 22 | A | 11 | A | | |
| 23 | A | 12 | A | | |

Upon testing, the compounds of the present invention have a better inhibitory activity on ULK1. The activities of some compounds of the present invention, measured using the above method, are as shown in Table 1, wherein A represents $IC_{50}$ < 100 nM; B represents 100 nM < $IC_{50}$ < 1 μM; and C represents $IC_{50}$ > 1 μM.

Test example 2: Experiment on the inhibition of the compound on ATG13 phosphorylation level in MIA PaCa-2 cells

1. Test purpose

The inhibitory effect of the compound of the present disclosure on ULK1 was evaluated by testing the inhibitory effect of the compound of the present disclosure on ATG13 phosphorylation level in MIA PaCa-2 cells.

2. Experimental method

MIA PaCa-2 cells (ATCC, CRM-CRL-1420) were cultured in a complete medium, namely, a DMEM medium

(Gibco, 11965-092) containing 10% fetal bovine serum (Corning, 35-081-CV), 2.5% horse serum (Gibco, 16050-122) and 1% double antibody (Gibco, 15140-122). The experiments were performed according to the requirements of the kit (Cell Signaling Technology, 17208C).

Cell plating: MIA PaCa-2 at 80% growth confluence were digested, diluted with the complete medium, and added to a 96-well cell culture plate (Costar, 3596) at 20,000 cells per well, with a volume of 90 μL.

Compound incubation: 5 μL of Torin1 and 5 μL of a serially diluted compound were added to each well so that the highest concentration of the compound in the reaction system was 10 μM. 5-fold serial dilution with 8 concentrations was performed, and the final concentration of Torin1 was 100 nM. The DMSO content in the system was 0.1%. The reaction plate was placed in an incubator at 37°C in 5% $CO_2$, and was incubated for 24 h. 10 μL of Torin1 was added to the negative control wells (NC) to a final concentration of 100 nM, with a DMSO content of 0.1%.

Reaction detection: After the incubation was completed, the cell plate was washed with a pre-cooled DPBS (Corning, 21-031-CVC) to wash away the compound and medium. 50 μL of pre-cooled cell lysis solution was added to each well, incubated on ice for 10 min. The cell plate was centrifuged, and the supernatant was transfered to an ELISA reaction plate. Simultaneously, 50 μL of Phospho-Atg13 (ser355) rabbit detection antibody was added, followed by incubation at room temperature under shaking for 60 min. After the incubation was completed, the reaction plate was washed with a washing solution for 4 times. 100 μL of TMB substrate was added for color development reaction, followed by incubation under shaded conditions with shaking for 15 min. Finally, 100 μL of stop solution was added to each well. The reaction was terminated by shaking for 10 s, and the absorbance value at 450 nm and 560 nm were collected within 10 min using Envision (Perkin Elmer, EnVision 2105).

3. Data analysis

The inhibition ratio was calculated by subtracting the absorbance value at 560 nM from the absorbance value at 450 nm for each well. The inhibition ratio formula is as follows:

Inhibition ratio = (sample signal value - negative control signal value)/(positive control signal value - negative control signal value) x 100%.

The dose-effect curve was fitted using the log (inhibitor) vs. response -Variable slope of Graphpad Prism8, thereby affording the $IC_{50}$ value of each compound for inhibiting ATG13 phosphorylation level.

The experimental results of this test example are shown in Table 2 below:

Table 2: $IC_{50}$ of the compounds of the present invention for inhibiting ATG13 phosphorylation level

| Compounds | pATG13 ELISA $IC_{50}$ (nM) | Compounds | pATG13 ELISA $IC_{50}$ (nM) | Compounds | pATG13 ELISA $IC_{50}$ (nM) |
|---|---|---|---|---|---|
| 1 | c | 20 | B | 8 | C |
| 2 | c | 22 | B | 9 | B |
| 3 | B | 23 | C | 11 | A |
| 4 | c | 5 | B | 12 | B |
| 21 | c | 6 | B | | |

Upon testing, the compounds of the present invention have a better inhibitory activity on ATG13 phosphorylation level. The activities of some compounds of the present invention, measured using the above method, are as shown in Table 2, wherein A represents $IC_{50}$ < 100 nM; B represents 100 nM <$IC_{50}$ < 1 μM; and C represents $IC_{50}$ > 1 μM.

Test example 3: Experiment on the inhibition of the compound on autophagy level in HEK293 GFP-LC3 stably transfected cells

1. Test purpose

The inhibitory effect of the compound of the present disclosure on intracellular autophagy was evaluated by testing the impact of the compound of the present disclosure on the fluorescence intensity of HEK293 GFP-LC3 stably transfected cells.

2. Experimental method

HEK293 GFP-LC3 stably transfected cells (HEK293, ATCC, CRL-1573) were cultured in a complete medium, namely, a DMEM medium (Gibco, 11965-092) containing 10% fetal bovine serum (Corning, 35-081-CV), 1% double antibody (Gibco, 15140-122) and 1 μg/mL of Puromycin (Invitrogen- A1113803).

Cell plating: HEK293 GFP-LC3 stably transfected cells at 80% growth confluence were digested, diluted with a medium free of Puromycin, and added to a 96-well cell culture plate (Geriner, 655090) at 12,000 cells per well, with a volume of 90 μL.

Compound incubation: 5 μL of Torin1 and 5 μL of a serially diluted compound were added to each well so that the highest concentration of the compound in the reaction system was 10 μM. 3-fold serial dilution with 8 concentrations was performed, and the final concentration of Torin1 was 100 nM. The DMSO content in the system was 0.1%. The positive control wells (PC) contain DMSO at a final concentration of 0.1%, and the negative control wells (NC) contain 100 nM of Torin1 with 0.1% of DMSO. The reaction plate was placed in an incubator at 37°C in 5% $CO_2$, and was incubated for 24 h.

Cell staining and fixation: The cell plate was taken out, and 10 μL of Hochest33342 nuclear staining reagent (Thermo, H1399) was added to each well, followed by incubation at 37°C in 5% $CO_2$ for 30 min. After the incubation was completed, the cell supernatant was discarded, and 100 μL of pre-cooled 4% paraformaldehyde was added to each well to fix the cells for 15 min. Then, the fixative was discarded, and 100 μL of PBS was added to each well. Images were captured for analysis using a high-content cell imaging analyzer (PE, Operetta CLS), and GFP fluorescence signals were collected for data processing.

3. Data analysis

The average GFP fluorescence signal intensity in the reaction wells was collected for the calculation of the inhibition ratio:

Inhibition ratio = (sample signal value - negative control signal value)/(positive control signal value - negative control signal value) x 100%.

The dose-effect curve was fitted using the log(inhibitor) vs. response -Variable slope of Graphpad Prism8, thereby affording the $IC_{50}$ value of each compound for inhibiting autophagy level in HEK293 GFP-LC3 stably transfected cells.

The experimental results of this test example are shown in Table 3 below:

Table 3: $IC_{50}$ of the compounds of the present invention for inhibiting autophagy level in HEK293 GFP-LC3 stably transfected cells

| Compounds | Autophagic Flux $IC_{50}$ (nM) | Compounds | Autophagic Flux $IC_{50}$ (nM) | Compounds | Autophagic Flux $IC_{50}$ (nM) |
|---|---|---|---|---|---|
| 1 | C | 20 | C | 8 | B |
| 2 | B | 22 | C | 9 | B |
| 3 | B | 23 | B | 11 | C |
| 4 | B | 5 | B | 12 | B |
| 21 | B | 6 | B | | |

Upon testing, the compounds of the present invention have a better inhibitory activity on autophagy level in HEK293 GFP-LC3 stably transfected cells. The activities of some compounds of the present invention, measured using the above method, are as shown in Table 3, wherein A represents $IC_{50}$ < 100 nM; B represents 100 nM <$IC_{50}$ < 1 μM; and C represents $IC_{50}$ > 1 μM.

## Claims

**1.** A compound of formula 1 or a pharmaceutically acceptable salt, a stereoisomer, a tautomer, a polymorph, a solvate, a hydrate, an N-oxide, an isotope labeled compound, a metabolite, an ester, or a prodrug thereof:

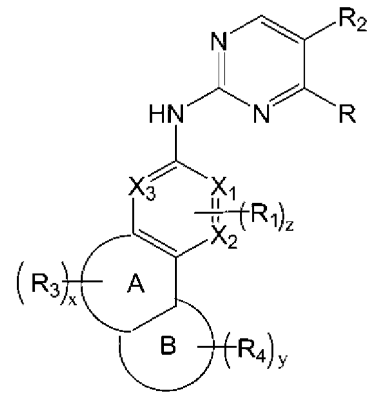

(1)

wherein $X_1$, $X_2$, and $X_3$ are independently CH or N;
ring A is selected from 3-10-membered heterocyclyl, $C_3$-$C_{10}$ cycloalkyl, $C_6$-$C_{10}$ aryl, and 5-10-membered heteroaryl;
ring B is selected from 3-10-membered heterocyclyl, $C_3$-$C_{10}$ cycloalkyl, $C_6$-$C_{10}$ aryl, and 5-10-membered heteroaryl;
each $R_1$ is independently selected from H, halogen, cyano, nitro, $C_1$-$C_6$ alkyl, $C_2$-$C_6$ alkenyl, $C_2$-$C_6$ alkynyl, $C_1$-$C_6$ alkoxy, $C_3$-$C_{10}$ cycloalkyl, 3-10-membered heterocyclyl, $C_6$-$C_{10}$ aryl, and 5-10-membered heteroaryl;
$R_2$ is selected from H, cyano, halogen, $C_1$-$C_6$ alkyl, $C_1$-$C_6$ hydroxyalkyl, $C_1$-$C_6$ aminoalkyl, $C_1$-$C_6$ haloalkyl, $C_1$-$C_6$ heteroalkyl, $C_2$-$C_6$ alkenyl, $C_2$-$C_6$ alkynyl, and $C_3$-$C_{10}$ cycloalkyl;
$R_3$ and $R_4$ are each independently selected from H, oxo, halogen, cyano, nitro, $C_1$-$C_6$ alkyl, $C_2$-$C_6$ alkenyl, $C_2$-$C_6$ alkynyl, $C_3$-$C_{10}$ cycloalkyl, 3-10-membered heterocyclyl, $C_6$-$C_{10}$ aryl, 5-10-membered heteroaryl, $R_5O$-, $R_6C(O)$-, $-NR_7R_8$ and $-NHCONR_7R_8$, wherein each of the $C_1$-$C_6$ alkyl, $C_2$-$C_6$ alkenyl, $C_2$-$C_6$ alkynyl, $C_3$-$C_{10}$ cycloalkyl, 3-10-membered heterocyclyl, $C_6$-$C_{10}$ aryl, or 5-10-membered heteroaryl is optionally substituted by one or more $R_9$;
$R_5$ and $R_6$ are each independently selected from H, $C_1$-$C_6$ alkyl, $C_3$-$C_{10}$ cycloalkyl, $C_6$-$C_{10}$ aryl, and 5-10-membered heteroaryl;
$R_7$ and $R_8$ are each independently selected from H, $C_1$-$C_6$ alkyl, $C_3$-$C_{10}$ cycloalkyl, 3-10-membered heterocyclyl, and $C_6$-$C_{10}$ aryl; or $R_7$ and $R_8$, together with N atom that they are attached to, form 5-6-membered heterocycle or 5-6-membered heteroaromatic ring;
each $R_9$ is independently selected from halogen, cyano, nitro, amino, $C_1$-$C_6$ alkyl, $C_2$-$C_6$ alkenyl, $C_2$-$C_6$ alkynyl, $C_3$-$C_{10}$ cycloalkyl, 3-10-membered heterocyclyl, $C_6$-$C_{10}$ aryl, and 5-10-membered heteroaryl;
R is selected from $-NR_7R_8$, $R_5O$-, $R_5S$-, halogen, $C_1$-$C_6$ alkyl, 3-12-membered heterocyclyl, 5-10-membered heteroaryl, and $-L-R_{10}$, wherein each of the 3-12-membered heterocyclyl or 5-10-membered heteroaryl is optionally substituted by one or more $R_{11}$;
L is $-(L_1)_m-(L_2)_n-(L_3)_p-$;
$L_1$ is selected from -O-, $-N(R_{12})$-, $-C(R_{12})(R_{13})-O$-, $-N(R_{12})CO$-, and -S-;
$L_2$ is selected from $C_1$-$C_6$ alkylene, $C_1$-$C_6$ heteroalkylene, $C_3$-$C_{10}$ cycloalkylene, and 3-10-membered heterocyclylene;
$L_3$ is selected from $-N(R_{12})$-, $-N(R_{12})C(O)$-, and -C(O)-;
m, n, and p are each independently selected from 0, 1 and 2;
$R_{10}$ is selected from $C_3$-$C_{10}$ cycloalkyl, $C_6$-$C_{10}$ aryl, 5-10-membered heteroaryl, and 3-10-membered heterocyclyl, each of the $C_3$-$C_{10}$ cycloalkyl, $C_6$-$C_{10}$ aryl, 5-10-membered heteroaryl, or 3-10-membered heterocyclyl is optionally substituted by one or more $R_{14}$;
each $R_{11}$ is independently selected from $C_1$-$C_6$ alkyl, oxo, 5-10-membered heteroaryl, 3-10-membered heterocyclyl, $R_6C(O)$-, $R_6C(O)N(R_{12})$-, and $-NR_7R_8$, wherein each of the $C_1$-$C_6$ alkyl, 5-10-membered heteroaryl, or 3-10-membered heterocyclyl is optionally substituted by one or more $R_{15}$;
$R_{12}$ and $R_{13}$ are each independently selected from H, $C_1$-$C_6$ alkyl, and $C_3$-$C_{10}$ cycloalkyl;
each $R_{14}$ is independently selected from H, halogen, cyano, oxo, $C_1$-$C_6$ alkyl, $C_1$-$C_6$ alkoxy, $-CONR_7R_8$, and $R_6C(O)$-; and
each $R_{15}$ is independently selected from H, halogen, cyano, oxo, $C_1$-$C_6$ alkyl, $C_3$-$C_{10}$ cycloalkyl, and 3-10-membered heterocyclyl, wherein each of the $C_1$-$C_6$ alkyl, $C_3$-$C_{10}$ cycloalkyl, or 3-10-membered heterocyclyl is optionally substituted by one or more substituent selected from $C_1$-$C_6$ alkyl and oxo; and
x, y, and z are each independently selected from 1, 2 and 3.

2. The compound or pharmaceutically acceptable salt, stereoisomer, tautomer, polymorph, solvate, hydrate, N-oxide, isotope labeled compound, metabolite, ester, or prodrug thereof of claim 1, wherein, at most one of $X_1$, $X_2$, and $X_3$ is N.

3. The compound or pharmaceutically acceptable salt, stereoisomer, tautomer, polymorph, solvate, hydrate, N-oxide, isotope labeled compound, metabolite, ester, or prodrug thereof of claim 1 or 2, wherein,

ring A is 5-8-membered heterocyclyl, preferably 6-7-membered heterocyclyl, more preferably 6-7-membered nitrogen-containing heterocyclyl;
preferably, ring A is

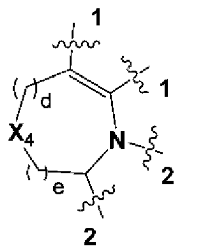
,

wherein
$X_4$ is selected from O and NH;
d is 0 or 1;
e is 1 or 2;
the wavy line 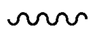 represents the point of attachment of the group to the rest of the molecule;
symbol 1 represents the point of attachment of ring A to

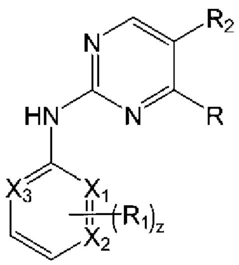

moiety; and
symbol 2 represents the point of attachment of ring A to ring B;
preferably, ring A is selected from

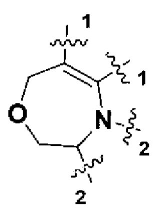 , 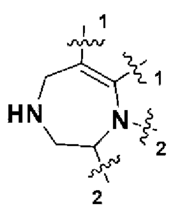 , 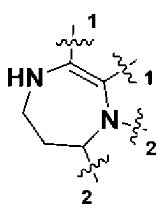 , 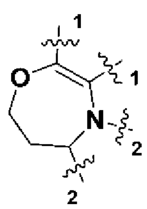 ,

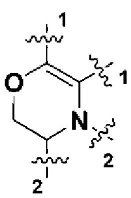 , and 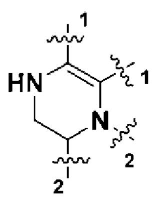 .

4. The compound or pharmaceutically acceptable salt, stereoisomer, tautomer, polymorph, solvate, hydrate, N-oxide, isotope labeled compound, metabolite, ester, or prodrug thereof of anyone of claims 1 - 3, wherein,

ring B is 5-6-membered heterocycle, more preferably 5-6-membered nitrogen-containing heterocycle; preferably, ring B is

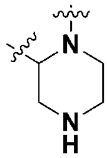 ;

wherein the wavy line 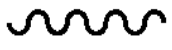 represents the point of attachment of ring B to ring A.

5. The compound or pharmaceutically acceptable salt, stereoisomer, tautomer, polymorph, solvate, hydrate, N-oxide, isotope labeled compound, metabolite, ester, or prodrug thereof of anyone of claims 1 - 4, wherein, $R_1$ is H.

6. The compound or pharmaceutically acceptable salt, stereoisomer, tautomer, polymorph, solvate, hydrate, N-oxide, isotope labeled compound, metabolite, ester, or prodrug thereof of anyone of claims 1 - 5, wherein, $R_2$ is selected from H and $C_1$-$C_6$ haloalkyl, preferably $C_1$-$C_6$ haloalkyl, further preferably $C_1$-$C_3$ haloalkyl, more preferably trifluoromethyl.

7. The compound or pharmaceutically acceptable salt, stereoisomer, tautomer, polymorph, solvate, hydrate, N-oxide, isotope labeled compound, metabolite, ester, or prodrug thereof of anyone of claims 1 - 6, wherein,

$R_3$ and $R_4$ are each independently selected from H, oxo, $C_1$-$C_6$ alkyl, $C_3$-$C_{10}$ cycloalkyl, 3-10-membered heterocyclyl, and $R_6C(O)$-, wherein each of the $C_1$-$C_6$ alkyl, $C_3$-$C_{10}$ cycloalkyl, or 3-10-membered heterocyclyl is optionally substituted by one or more $R_9$;
$R_6$ is selected from $C_1$-$C_6$ alkyl and $C_3$-$C_{10}$ cycloalkyl; and
each $R_9$ is independently selected from halogen and $C_1$-$C_6$ alkyl;
preferably, $R_3$ and $R_4$ are each independently selected from H, oxo, $C_1$-$C_6$ alkyl, $C_3$-$C_6$ cycloalkyl, 4-6-membered heterocyclyl, and $R_6C(O)$-, wherein the $C_1$-$C_6$ alkyl is optionally substituted by 1, 2, 3, 4, or 5 of halogen, and the 4-6-membered heterocyclyl is optionally substituted by 1, 2, or 3 $C_1$-$C_6$ alkyl; and
$R_6$ is $C_1$-$C_6$ alkyl;
more preferably, $R_3$ and $R_4$ are each independently selected from H, oxo, $C_1$-$C_6$ alkyl, $C_3$-$C_6$ cycloalkyl, oxetanyl, piperidinyl, tetrahydropyranyl, and $R_6C(O)$-, wherein the $C_1$-$C_6$ alkyl is optionally substituted by 1, 2, 3, 4, or 5 of halogen, and each of the piperidinyl or tetrahydropyranyl is optionally substituted by 1 or 2 $C_1$-$C_6$ alkyl; and
$R_6$ is $C_1$-$C_6$ alkyl;
more preferably, $R_3$ and $R_4$ are each independently selected from H, oxo, methyl, isopropyl, cyclopropyl, cyclobutyl, $CF_3CH_2$-, $CH_3CO$-,

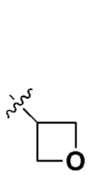 , 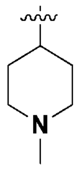 , and 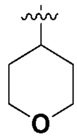 ,

wherein the wavy line 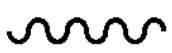 represents the point of attachment of the group to the rest of the molecule.

8. The compound or pharmaceutically acceptable salt, stereoisomer, tautomer, polymorph, solvate, hydrate, *N*-oxide, isotope labeled compound, metabolite, ester, or prodrug thereof of claim 7, wherein, $R_3$ is selected from H, oxo, and $C_1$-$C_6$ alkyl, preferably selected from H, oxo, and $C_1$-$C_3$ alkyl, more preferably selected from H, oxo, and methyl.

9. The compound or pharmaceutically acceptable salt, stereoisomer, tautomer, polymorph, solvate, hydrate, *N*-oxide,

isotope labeled compound, metabolite, ester, or prodrug thereof of claim 7, wherein,

$R_4$ is selected from $C_1$-$C_6$ alkyl, $C_3$-$C_6$ cycloalkyl, 4-6-membered heterocyclyl, and $R_6C(O)$-, wherein the $C_1$-$C_6$ alkyl is optionally substituted by 1, 2, 3, 4, or 5 of halogen, and the 4-6-membered heterocyclyl is optionally substituted by 1, 2, or 3 $C_1$-$C_6$ alkyl; and
$R_6$ is $C_1$-$C_6$ alkyl;
preferably, $R_4$ is selected from $C_1$-$C_6$ alkyl, $C_3$-$C_6$ cycloalkyl, oxetanyl, piperidinyl, tetrahydropyranyl, and $R_6C(O)$-, wherein the $C_1$-$C_6$ alkyl is optionally substituted by 1, 2, 3, 4, or 5 halogen, and each of the piperidinyl or tetrahydropyranyl is optionally substituted by 1 or 2 $C_1$-$C_6$ alkyl; and
$R_6$ is $C_1$-$C_6$ alkyl;
more preferably, $R_4$ is selected from methyl, isopropyl, cyclopropyl, cyclobutyl, $CF_3CH_2$-, $CH_3CO$-,

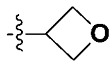, 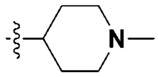, and 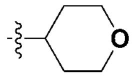,

wherein the wavy line 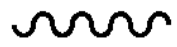 represents the point of attachment of the group to the rest of the molecule.

**10.** The compound or pharmaceutically acceptable salt, stereoisomer, tautomer, polymorph, solvate, hydrate, N-oxide, isotope labeled compound, metabolite, ester, or prodrug thereof of anyone of claims 1 - 9, wherein,
x, y, and z are each independently selected from 1 and 2.

**11.** The compound or pharmaceutically acceptable salt, stereoisomer, tautomer, polymorph, solvate, hydrate, N-oxide, isotope labeled compound, metabolite, ester, or prodrug thereof of anyone of claims 1 - 10, wherein,

R is selected from 3-12-membered heterocyclyl, 5-10-membered heteroaryl, and -L-$R_{10}$, wherein each of the 3-12-membered heterocyclyl or 5-10-membered heteroaryl is optionally substituted by one or more $R_{11}$;
L is $-(L_1)_m-(L_2)_n-(L_3)_p-$;
$L_1$ is selected from -O-, -NH-, and $-CH_2-O-$;
$L_2$ is selected from $C_1$-$C_6$ alkylene, $C_3$-$C_{10}$ cycloalkylene, and 3-10-membered heterocyclylene;
$L_3$ is selected from -NH-, -NHC(O)-, and -C(O)-;
m, n, and p are each independently selected from 0 and 1, and m, n, and p are not simultaneously 0;
$R_{10}$ is selected from $C_3$-$C_{10}$ cycloalkyl, $C_6$-$C_{10}$ aryl, 5-10-membered heteroaryl, and 3-10-membered heterocyclyl, each of the $C_3$-$C_{10}$ cycloalkyl, $C_6$-$C_{10}$ aryl, 5-10-membered heteroaryl, or 3-10-membered heterocyclyl is optionally substituted by one or more $R_{14}$;
each $R_{11}$ is independently selected from $C_1$-$C_6$ alkyl, oxo, 5-10-membered heteroaryl, 3-10-membered heterocyclyl, and $R_6C(O)$-, wherein each of the 3-10-membered heterocyclyl is optionally substituted by one or more $R_{15}$;
each $R_{14}$ is independently selected from H, halogen, cyano, oxo, and $C_1$-$C_6$ alkyl; and
each $R_{15}$ is independently selected from oxo and $C_1$-$C_6$ alkyl.

**12.** The compound or pharmaceutically acceptable salt, stereoisomer, tautomer, polymorph, solvate, hydrate, *N*-oxide, isotope labeled compound, metabolite, ester, or prodrug thereof of anyone of claims 1 - 11, wherein,

L is $-(L_1)_m-(L_2)_n-(L_3)_p-*$, wherein the bond marked with "*" is attached to $R_{10}$;
$L_1$ is selected from -O-, $-N(R_{12})-$, $-C(R_{12})(R_{13})-O-@$, $-N(R_{12})CO-@$, and -S-, wherein the bond marked with "@" is attached to $L_2$;
$L_2$ is selected from $C_1$-$C_6$ alkylene, $C_1$-$C_6$ heteroalkylene, $C_3$-$C_{10}$ cycloalkylene, and 3-10-membered heterocyclylene;
$L_3$ is selected from $-N(R_{12})-$, $-N(R_{12})C(O)-\$$, and -C(O)-, wherein the bond marked with "$" is attached to $R_{10}$;
m, n, and p are each independently selected from 0 and 1, and m, n, and p are not simultaneously 0;
preferably, $L_1$ is selected from -O-, $-N(R_{12})-$;
$L_2$ is selected from $C_1$-$C_6$ alkylene, $C_3$-$C_6$ cycloalkylene, and 3-6-membered heterocyclylene;
$L_3$ is selected from $-N(R_{12})-$, $-N(R_{12})C(O)-\$$, and -C(O)-, more preferably, -C(O)-, wherein the bond marked with "$" is attached to $R_{10}$;
preferably, $L_1$ is selected from -O-, -NH-;
$L_2$ is selected from ethylene, propylene, cyclohexylidene, piperidylidene;
$L_3$ is selected from -NH-, -NH-C(O)-$, -C(O)-, wherein the bond marked with "$" is attached to $R_{10}$;

preferably, L is -O-, -O-cyclohexylidene-*, -NH-cyclohexylidene-*, -NH-piperidylidene-C(O)-*, -O-propylene-*, -NH-propylene-*, -O-ethylene-NH-*, -ethylene-NHC(O)-*, -NH-propylene-C(O)-*, and -O-propylene-C(O)-*, wherein the bond marked with "*" is attached to $R_{10}$;
more preferably, L is selected from -O-propylene-*, -O-cyclohexylidene-*, -NH-propylene-*, -NH-cyclohexylidene-*, -NH-piperidylidene-C(O)-*, wherein the bond marked with "*" is attached to $R_{10}$.

**13.** The compound or pharmaceutically acceptable salt, stereoisomer, tautomer, polymorph, solvate, hydrate, *N*-oxide, isotope labeled compound, metabolite, ester, or prodrug thereof of anyone of claims 1 - 12, wherein the compound is the compound of formula 1':

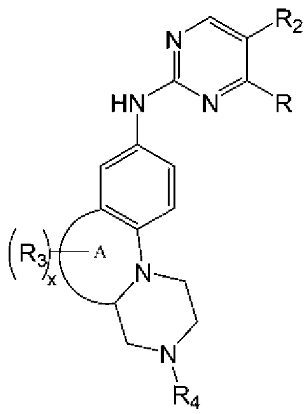

(1').

**14.** The compound or pharmaceutically acceptable salt, stereoisomer, tautomer, polymorph, solvate, hydrate, *N*-oxide, isotope labeled compound, metabolite, ester, or prodrug thereof of anyone of claims 1 - 13, wherein the compound is the compound of formula 1A, 1B, 1C, or 1D:

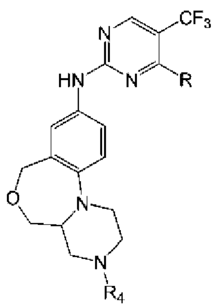

(1A),

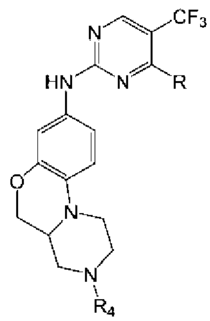

(1B),

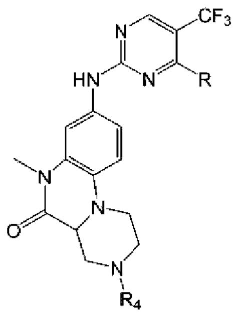

(1C),

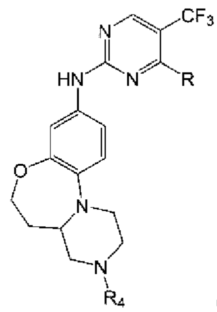

(1D).

**15.** The compound or pharmaceutically acceptable salt, stereoisomer, tautomer, polymorph, solvate, hydrate, *N*-oxide, isotope labeled compound, metabolite, ester, or prodrug thereof of claim 14, wherein the compound is the compound of formula 1A-1:

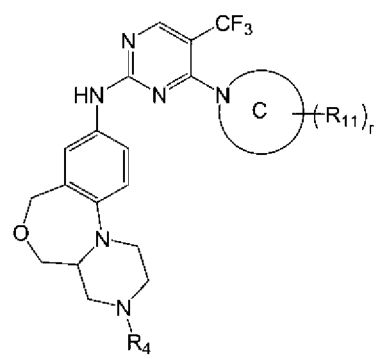

(1A-1)

wherein ring C is selected from 3-12-membered heterocyclyl and 5-10-membered heteroaryl; preferably, ring C is selected from 3-12-membered heterocyclyl; and r is selected from 0, 1, and 2.

**16.** The compound or pharmaceutically acceptable salt, stereoisomer, tautomer, polymorph, solvate, hydrate, *N*-oxide, isotope labeled compound, metabolite, ester, or prodrug thereof of claim 15, wherein, ring C is 5-12-membered spiro heterocyclyl or 5-12-membered bridged heterocyclyl, preferably 7-12-membered spiro heterocyclyl or 7-12-membered bridged heterocyclyl, more preferably

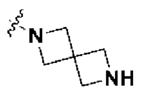, 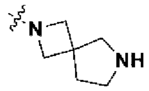, 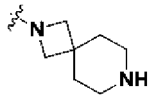, 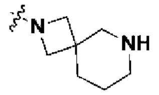,

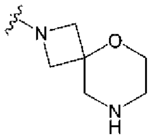, 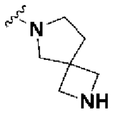, 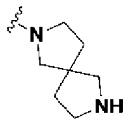, 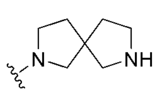, 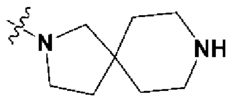, 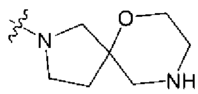,

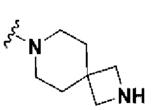, 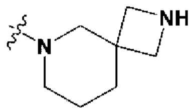, 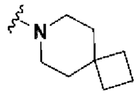, 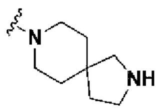, 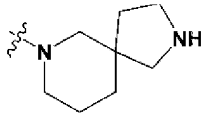, 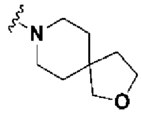,

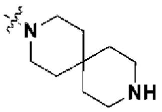, 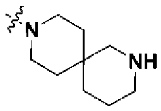, 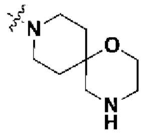, 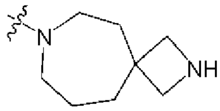, 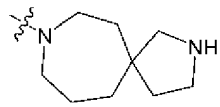,

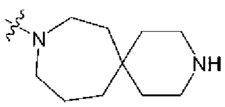, 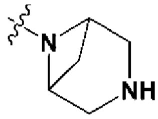, 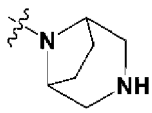, or 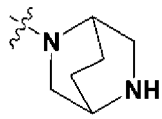,

more preferably

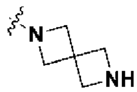,

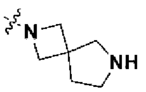, 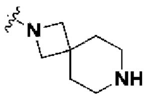, 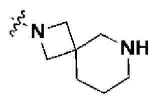, 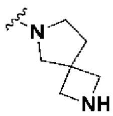, 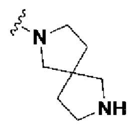, 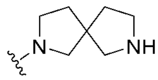, 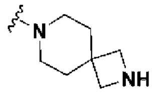,

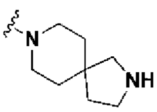, 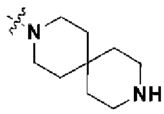, 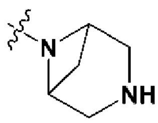, or 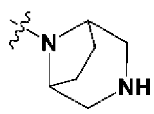;

and

each $R_{11}$ is independently selected from $C_1$-$C_6$ alkyl and oxo, preferably, each $R_{11}$ is independently selected from methyl and oxo,

wherein the wavy line represents the point of attachment of the group to the rest of the molecule.

**17.** The compound or pharmaceutically acceptable salt, stereoisomer, tautomer, polymorph, solvate, hydrate, *N*-oxide, isotope labeled compound, metabolite, ester, or prodrug thereof of claim 15, wherein,

ring C is 3-10-membered monocyclic heterocyclyl or 5-10-membered heteroaryl, preferably 6-7-membered monocyclic heterocyclyl or 5-6-membered heteroaryl, more preferably piperidinyl, piperazinyl, azepanyl, 1,4-diazepanyl, or pyrazolyl; each $R_{11}$ is independently selected from $C_1$-$C_6$ alkyl, oxo, 5-6-membered heteroaryl, 5-7-membered heterocyclyl, and $R_6C(O)$-, wherein each of the $C_1$-$C_6$ alkyl, 5-6-membered heteroaryl, or 5-7-membered heterocyclyl is optionally substituted by 1, 2, 3, or 4 $R_{15}$;
$R_6$ is $C_4$-$C_6$ cycloalkyl; and
each $R_{15}$ is independently selected from oxo and $C_1$-$C_6$ alkyl,
preferably, ring C is 3-10-membered monocyclic heterocyclyl, preferably 6-7-membered monocyclic heterocyclyl, more preferably piperidinyl, piperazinyl, azepanyl, 1,4-diazepanyl;
preferably, each $R_{11}$ is independently selected from $C_1$-$C_6$ alkyl, oxo, pyrazolyl, piperidinyl, and $C_4$-$C_6$ cycloalkyl-C(O)-, wherein the piperidinyl is optionally substituted by 1 or 2 substituent selected from $C_1$-$C_6$ alkyl and oxo;
more preferably, each $R_{11}$ is independently selected from methyl, oxo, pyrazolyl, N-methylpiperidinyl, oxopiperidinyl, and cyclobutylformyl.

**18.** The compound or pharmaceutically acceptable salt, stereoisomer, tautomer, polymorph, solvate, hydrate, N-oxide, isotope labeled compound, metabolite, ester, or prodrug thereof of anyone of claims 15 - 17, wherein the compound is the compound of formula 1A-1a, 1A-1b, or 1A-1c:

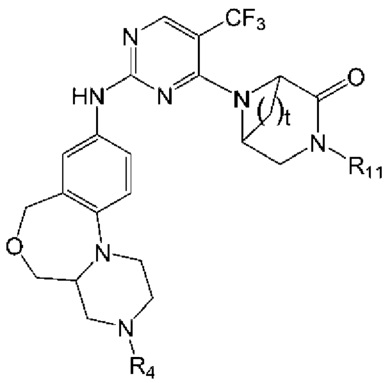

(1A-1a),

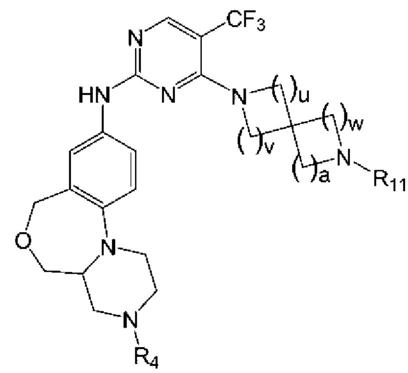

(1A-1b),

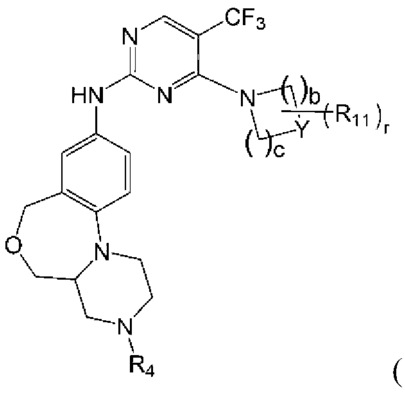

(1A-1c);

wherein t is selected from 0, 1, 2, and 3;
u, v, w, a, b, and c are each independently selected from 1, 2, and 3;
Y is selected from -CH- and-N-; and
r is selected from 1 and 2.

**19.** The compound or pharmaceutically acceptable salt, stereoisomer, tautomer, polymorph, solvate, hydrate, N-oxide, isotope labeled compound, metabolite, ester, or prodrug thereof of anyone of claims 1 - 14, wherein the compound is

the compound of formula 1A-2:

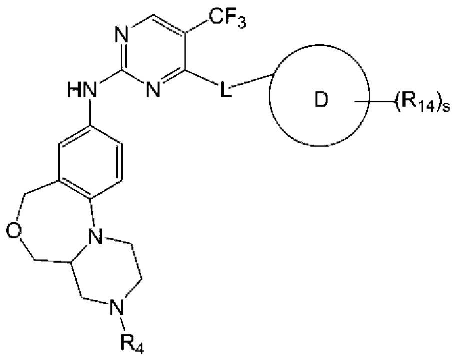

(1A-2)

wherein L is $-(L_1)_m-(L_2)_n-(L_3)_p-$, preferably, L is $-(L_1)_m-(L_2)_n-(L_3)_p-*$, wherein the bond marked with "*" is attached to ring D;

$L_1$ is selected from -O-, -NH-, and $-CH_2-O-$, preferably, when $L_1$ is $-CH_2-O-$, the oxygen of the $-CH_2-O-$ is attached to $L_2$, preferably, $L_1$ is selected from -O-, -NH-;

$L_2$ is selected from $C_1$-$C_6$ alkylene, $C_3$-$C_{10}$ cycloalkylene, and 3-10-membered heterocyclylene, preferably selected from $C_1$-$C_6$ alkylene, $C_3$-$C_6$ cycloalkylene, and 3-6-membered heterocyclylene, preferably selected from $C_1$-$C_6$ alkylene and $C_3$-$C_6$ cycloalkylene, preferably selected from ethylene, propylene, cyclohexylidene, and piperidylidene, more preferably selected from ethylene, propylene, and cyclohexylidene;

$L_3$ is selected from -NH-, -NHC(O)-, and -C(O)-, preferably, when $L_3$ is -NHC(O)-, the amino moiety of the -NHC(O)- is attached to $L_2$, and the carbonyl moiety is attached to ring D;

m, n, and p are each independently selected from 0 and 1, and m, n, and p are not simultaneously 0;

preferably, L is selected from -O-, -O-cyclohexylidene-, -NH-cyclohexylidene-, -NH-piperidylidene-, -O-propylene-, -NH-propylene-, -ethylene-, -ethylene-NHC(O)-, and -O-propylene-C(O)-;

preferably, L is selected from -O-, -O-cyclohexylidene-, -NH-cyclohexylidene-, -NH-piperidylidene-C(O)-, -O-propylene-, -NH-propylene-, -O-ethylene-NH-, -ethylene-NHC(O)-, -NH-propylene-C(O)-, and -O-propylene-C(O)-;

preferably, L is selected from -O-, -O-cyclohexylidene-*, -NH-cyclohexylidene-*, -NH-piperidylidene-C(O)-*, -O-propylene-*, -NH-propylene-*, -O-ethylene-NH-*, -ethylene-NHC(O)-*, -NH-propylene-C(O)-*, and -O-propylene-C(O)-*, wherein the bond marked with "*" is attached to ring D;

more preferably, L is selected from -O-propylene-*, -O-cyclohexylidene-*, -NH-propylene-*, -NH-cyclohexylidene-*, and -NH-piperidylidene-C(O)-*, wherein the bond marked with "*" is attached to ring D;

ring D is selected from $C_3$-$C_{10}$ cycloalkyl, $C_6$-$C_{10}$ aryl, 5-10-membered heteroaryl, and 3-10-membered heterocyclyl, preferably selected from $C_3$-$C_6$ cycloalkyl, $C_6$-$C_{10}$ aryl, 5-6-membered heteroaryl, and 5-7-membered heterocyclyl, preferably selected from cyclobutyl, phenyl, pyrazolyl, piperidinyl, piperazinyl, pyrrolidinyl, imidazolidinyl, morpholinyl, 1,3-oxazinanyl, azepanyl, and 1,4-oxazepanyl;

each $R_{14}$ is independently selected from H, halogen, cyano, oxo, and $C_1$-$C_6$ alkyl; and

s is selected from 0, 1, and 2.

**20.** The compound or pharmaceutically acceptable salt, stereoisomer, tautomer, polymorph, solvate, hydrate, N-oxide, isotope labeled compound, metabolite, ester, or prodrug thereof of claims 19, wherein the compound is the compound of formula 1A-2a, 1A-2b, 1A-2c, 1A-2d, 1A-2e, or 1A-2f:

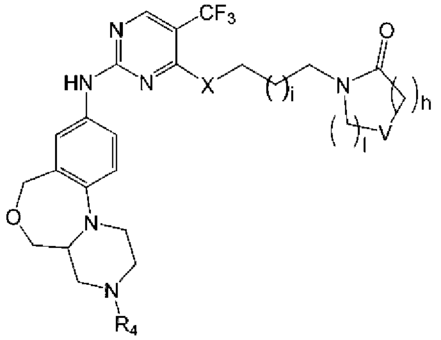 (1A-2a), 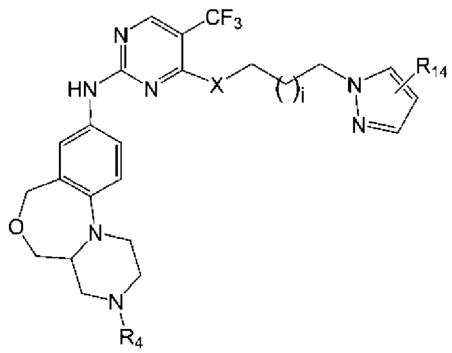 (1A-2b),

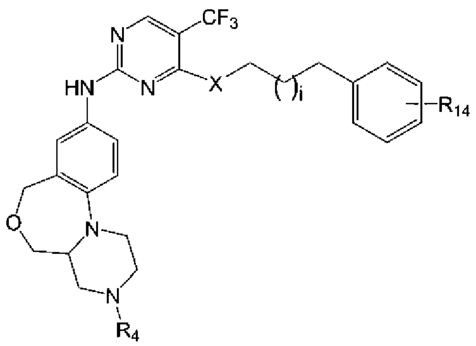 (1A-2c), 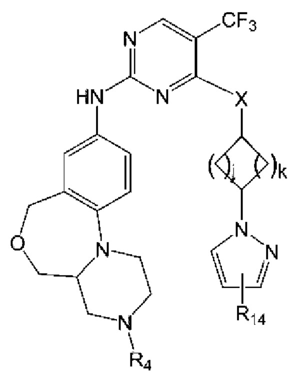 (1A-2d),

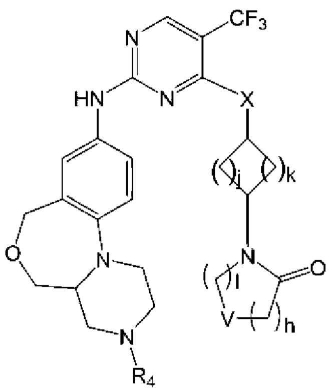 (1A-2e), 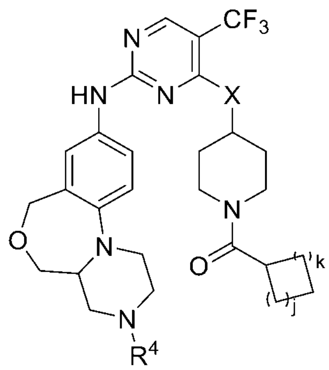 (1A-2f);

wherein X is selected from -O-, -NH-, and $-CH_2-O-$, preferably, X is selected from -O- and-NH-;
V is selected from -O-, -S-, $-NR_{12}-$, and $-CHR_{12}-$, preferably, V is selected from -O- and$-CHR_{12}-$;
$R_{12}$ is selected from H and $C_1$-$C_6$ alkyl;
h and l are each independently selected from 0, 1, 2, and 3;
i is selected from 0, 1, and 2; and
j and k are each independently selected from 1, 2, and 3.

**21.** The compound or pharmaceutically acceptable salt, stereoisomer, tautomer, polymorph, solvate, hydrate, N-oxide, isotope labeled compound, metabolite, ester, or prodrug thereof of claims 14, wherein the compound is the compound of formula 1B-2a, 1C-2a, or 1D-2a:

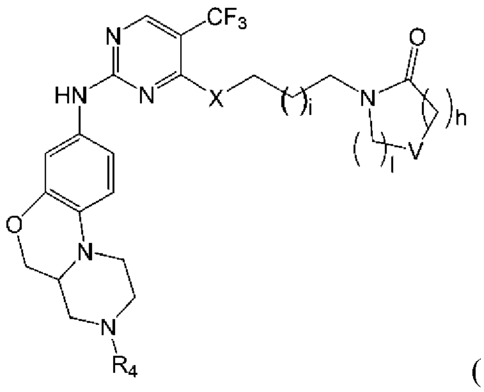 (1B-2a), 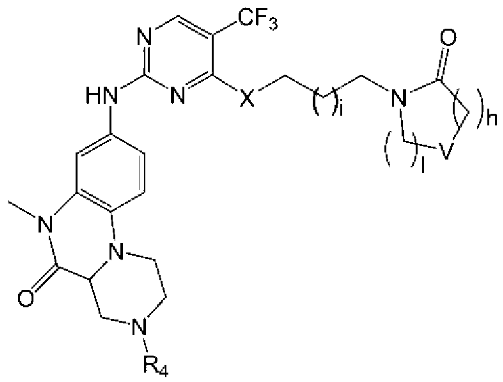 (1C-2a),

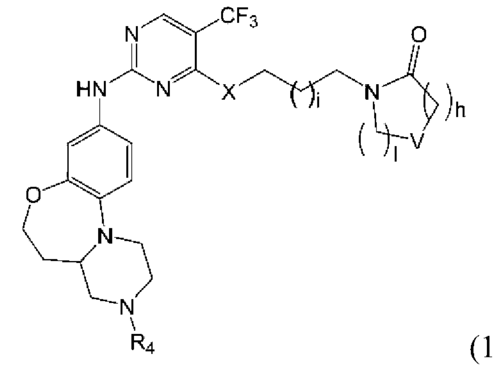 (1D-2a);

wherein X is selected from -O-, -NH-, and $-CH_2-O-$, preferably, X is selected from -O- and-NH-, more preferably, X is selected from -O-;
V is selected from -O-, -S-, $-NR_{12}-$, and $-CHR_{12}-$, preferably, V is selected from $-CHR_{12}-$;
$R_{12}$ is selected from H and $C_1$-$C_6$ alkyl;
h and 1 are each independently selected from 0, 1, 2, and 3; and
i is selected from 0, 1, and 2.

**22.** The compound or pharmaceutically acceptable salt, stereoisomer, tautomer, polymorph, solvate, hydrate, *N*-oxide, isotope labeled compound, metabolite, ester, or prodrug thereof of claim 1, wherein the compound is selected from:

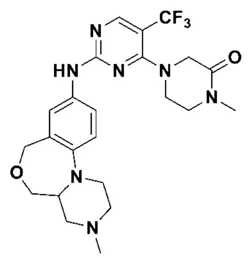 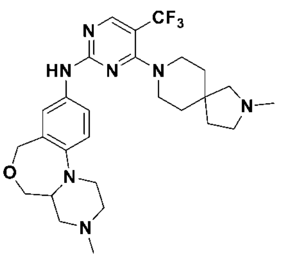 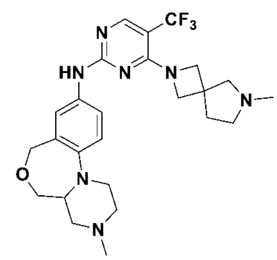 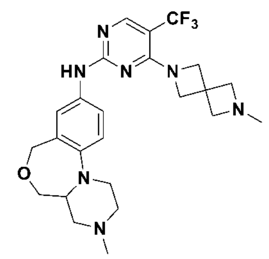

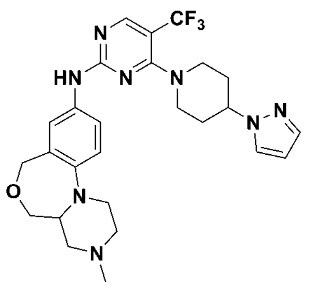 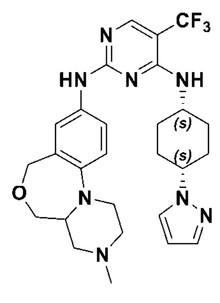 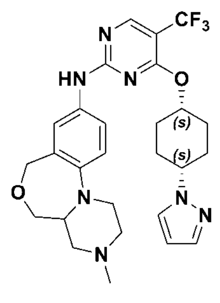 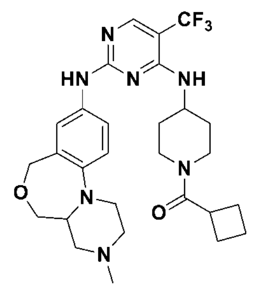

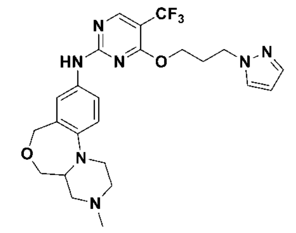
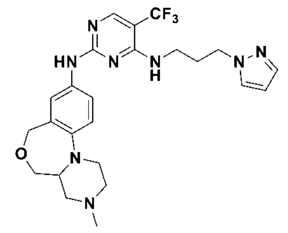
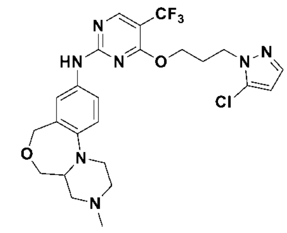
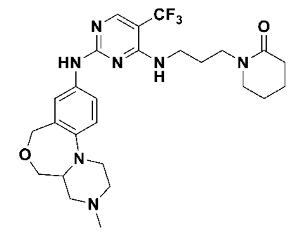
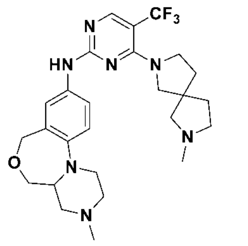
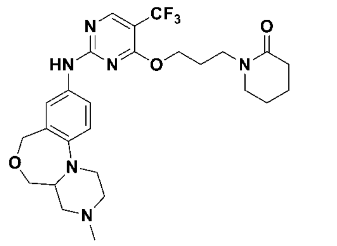
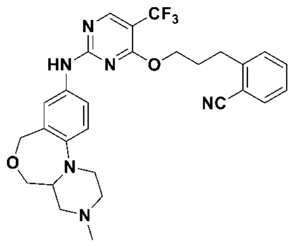
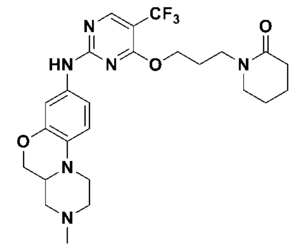
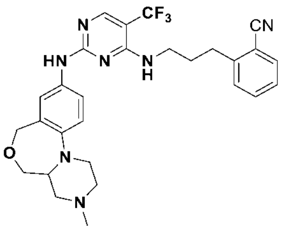
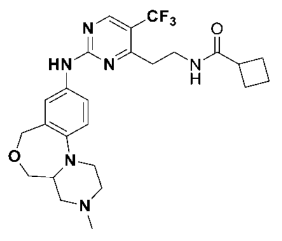
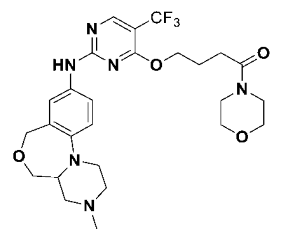
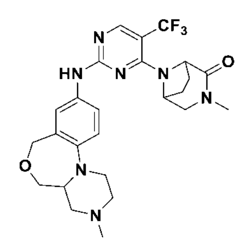
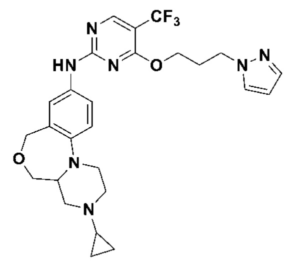
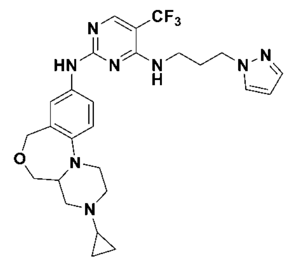
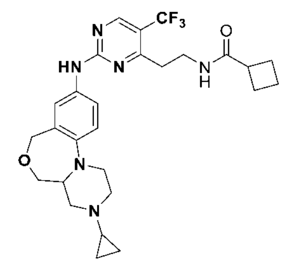
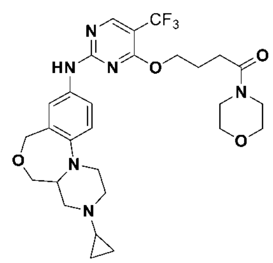
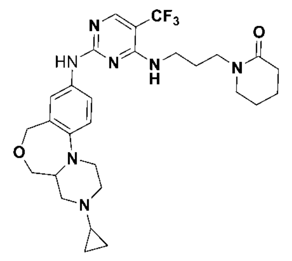
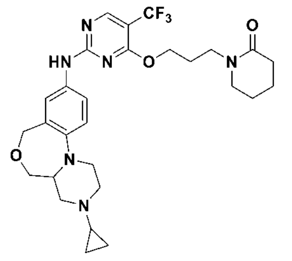
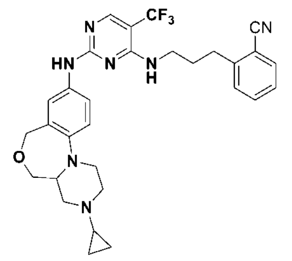
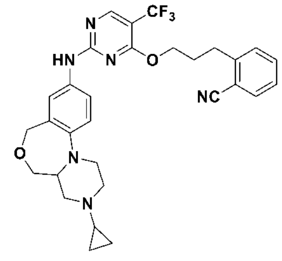
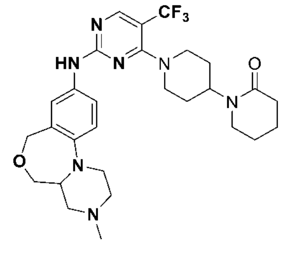
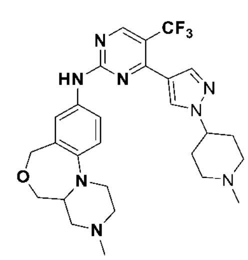
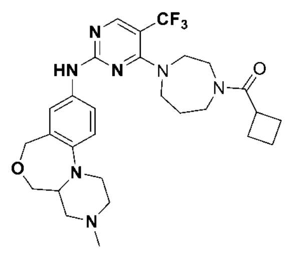
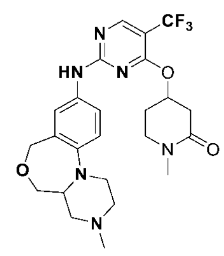

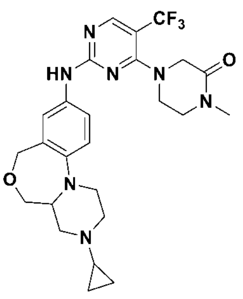
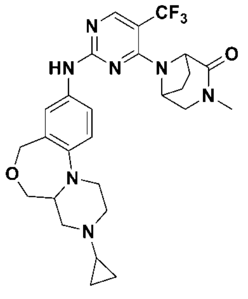
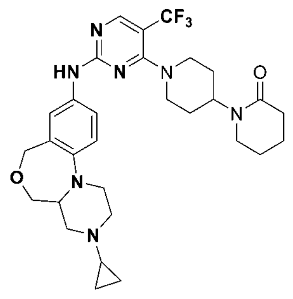
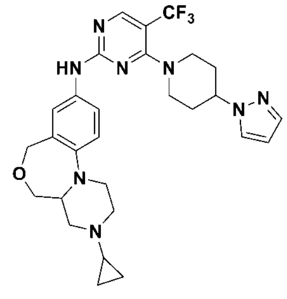

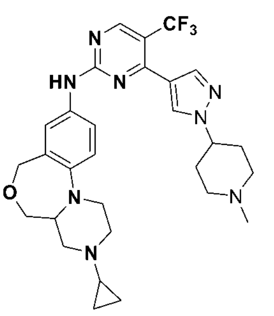
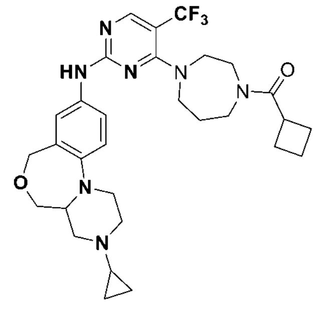
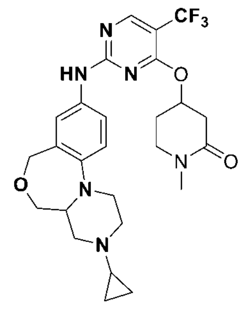
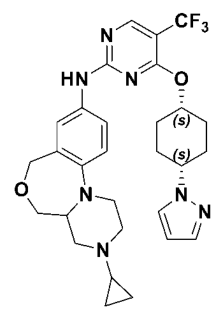

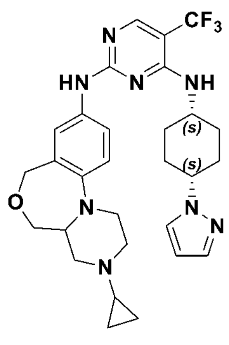
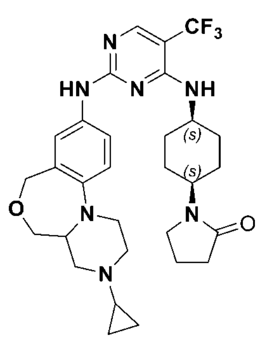
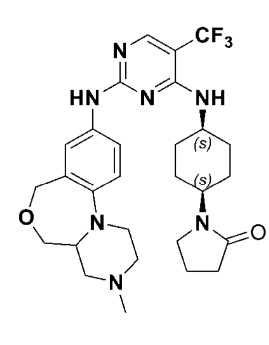
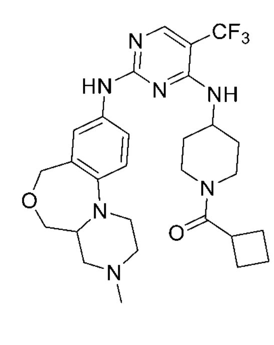

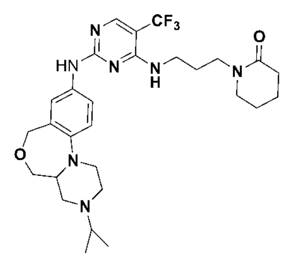
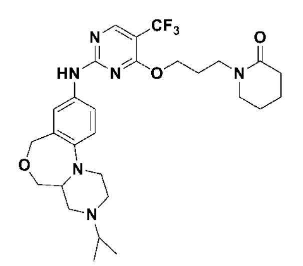
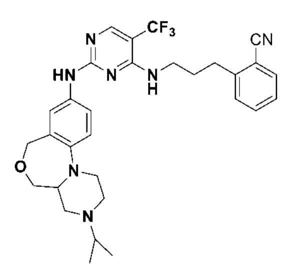
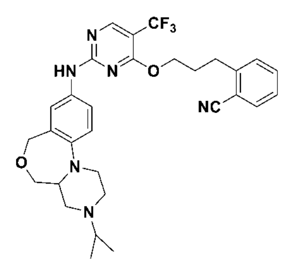

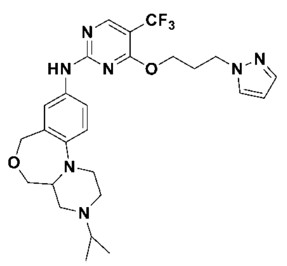
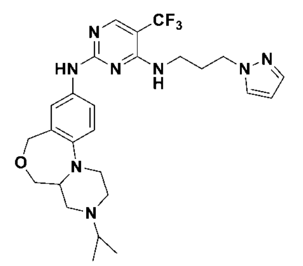
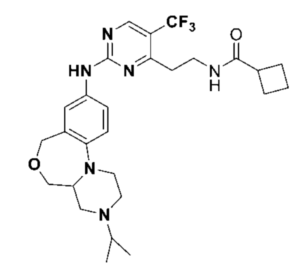
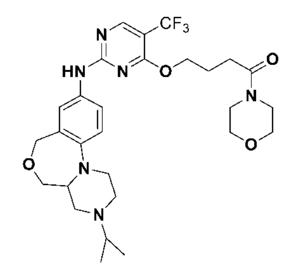

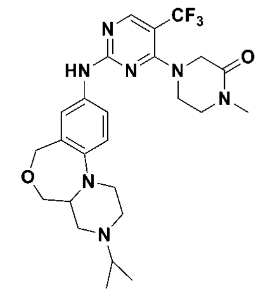
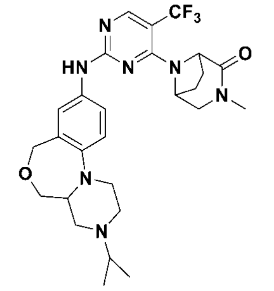
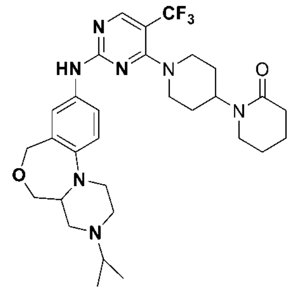
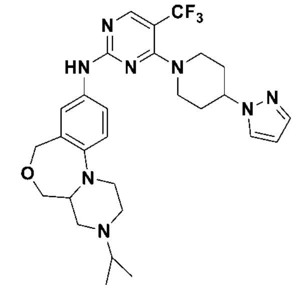

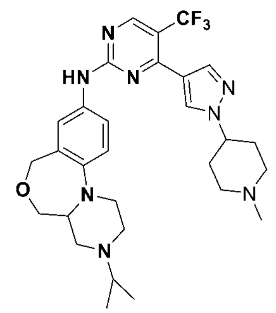
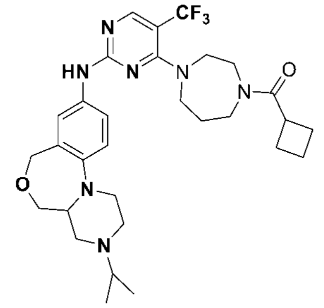
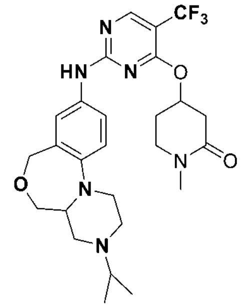
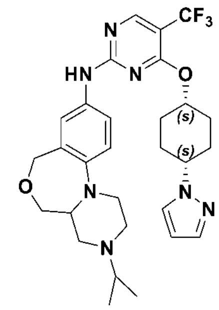

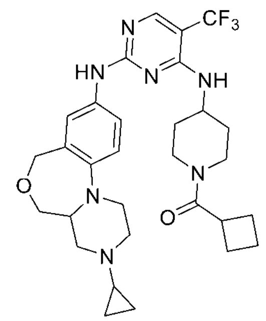
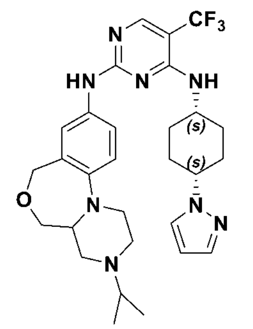
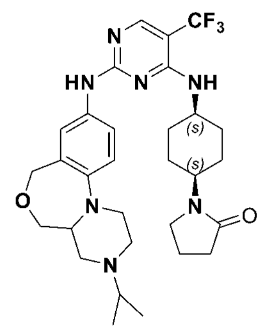
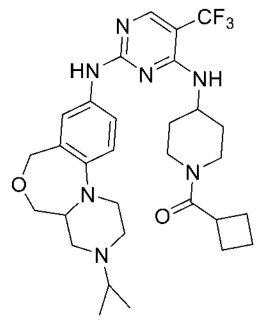

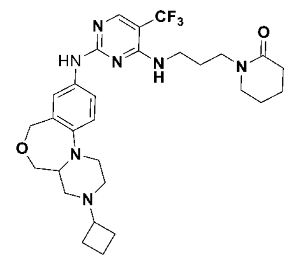
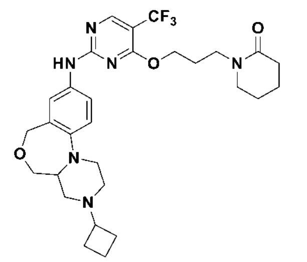
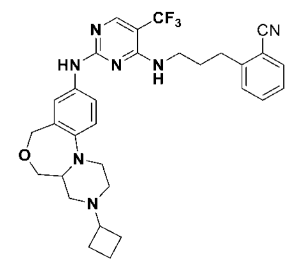
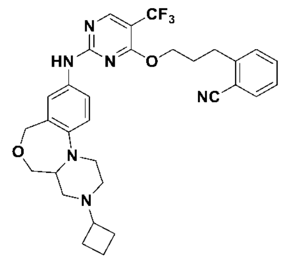

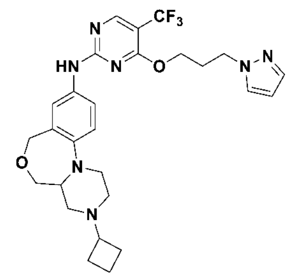
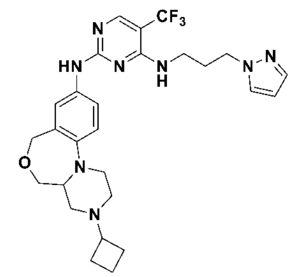
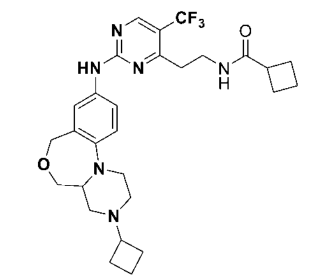
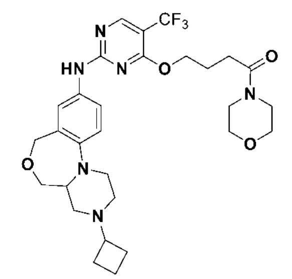

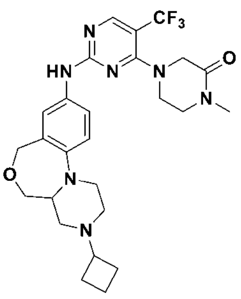
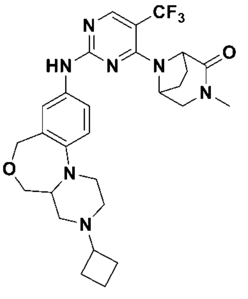
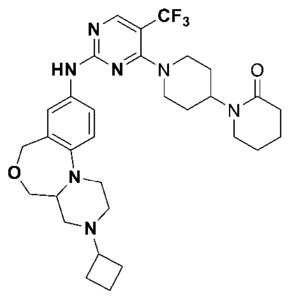
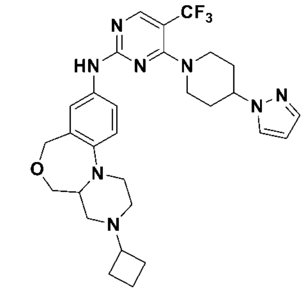

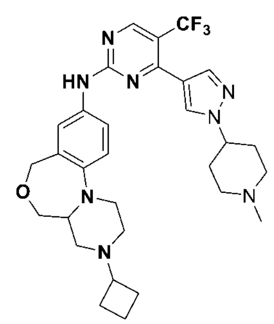
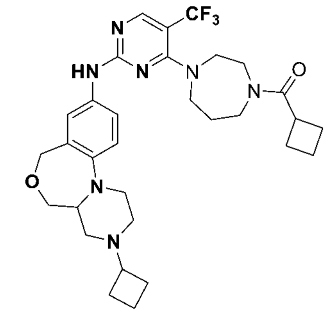
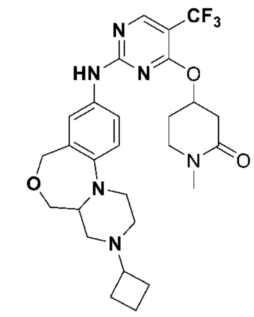
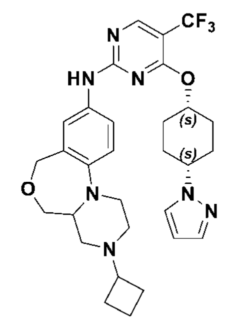

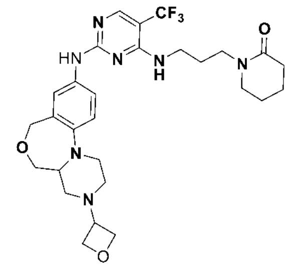
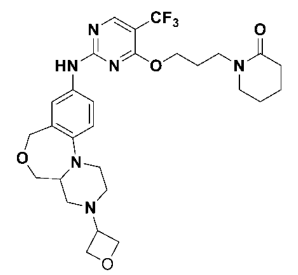
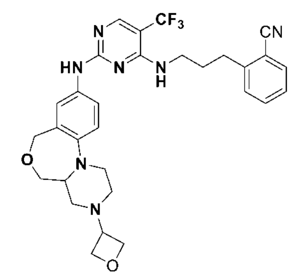
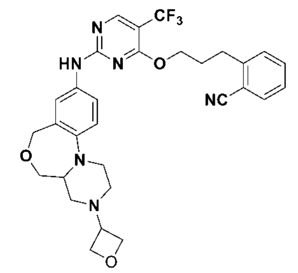

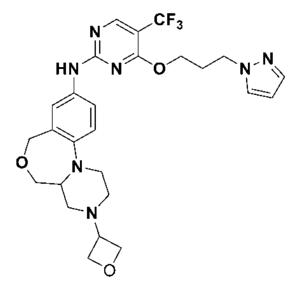
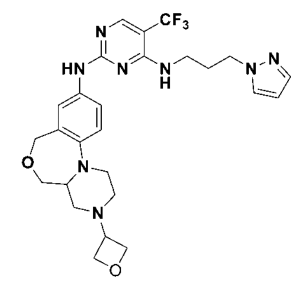
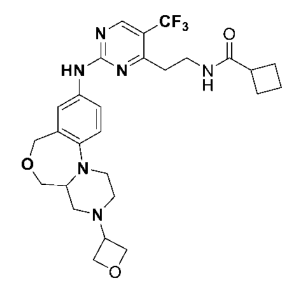
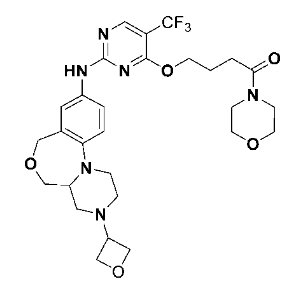

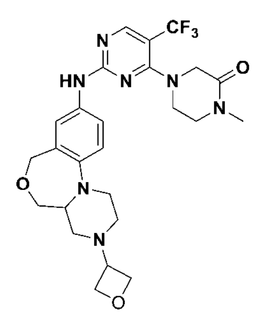
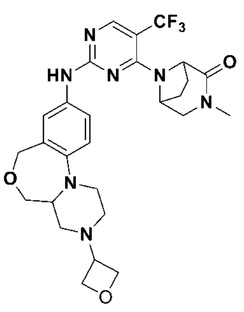
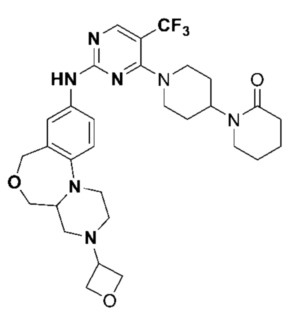
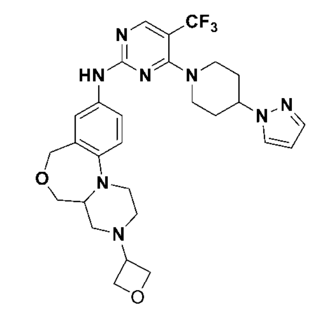

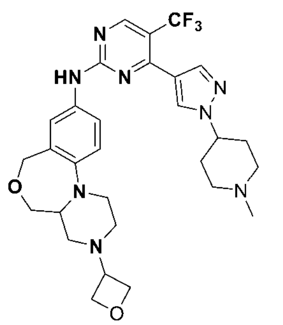
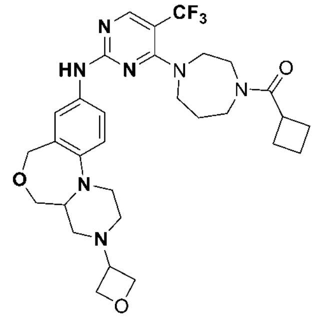
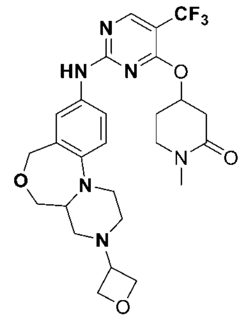
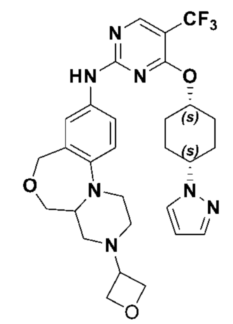

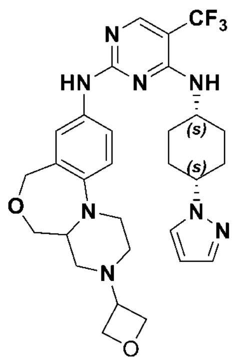
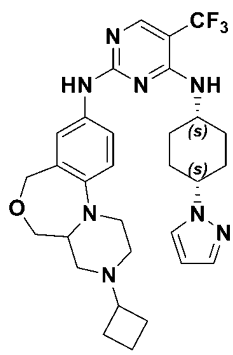
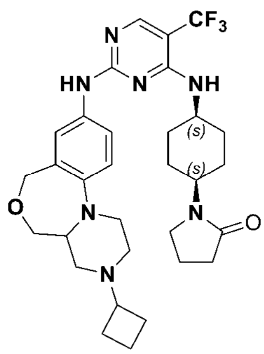
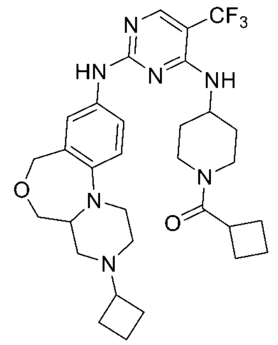

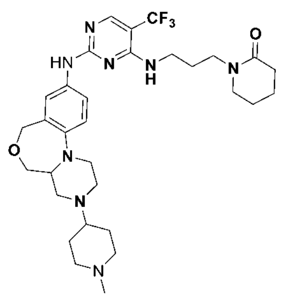
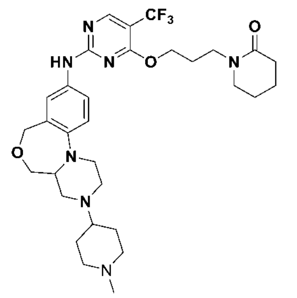
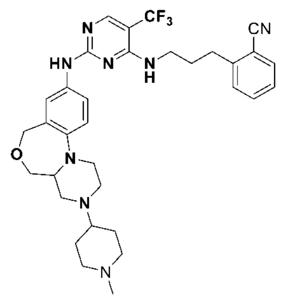
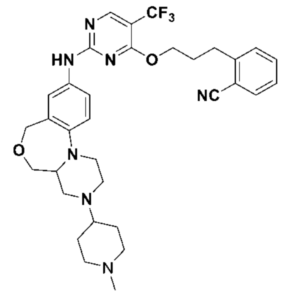

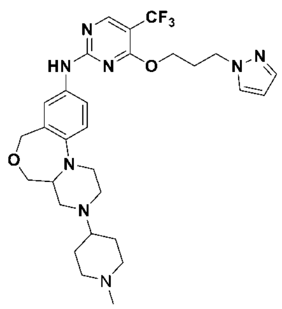
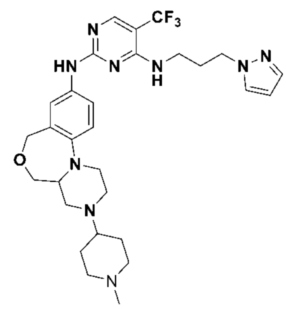
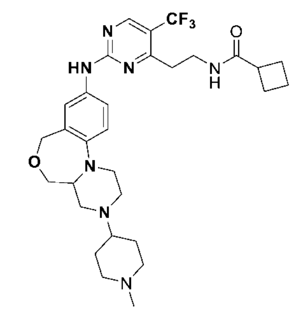
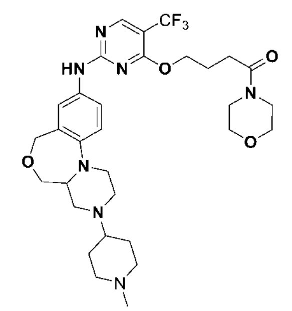

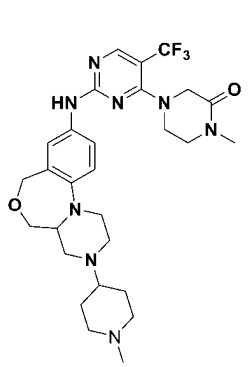
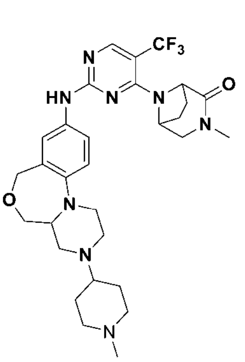
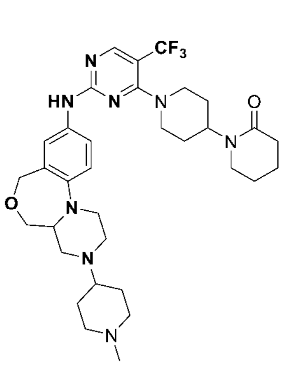
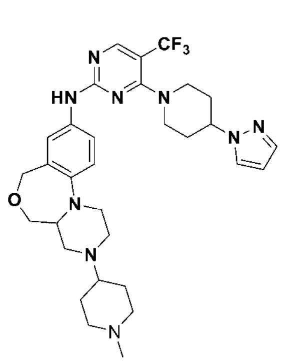

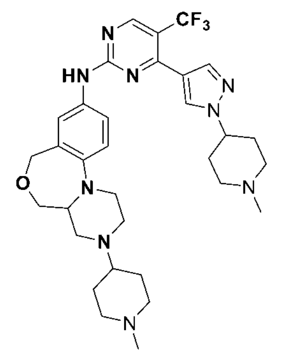
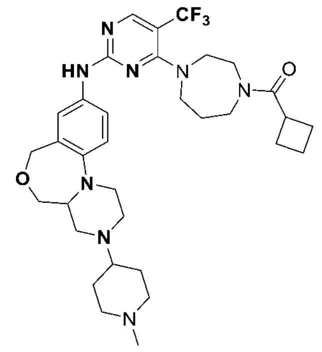
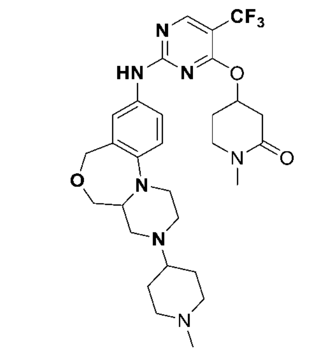
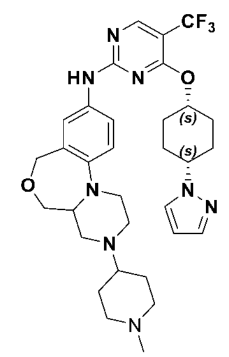

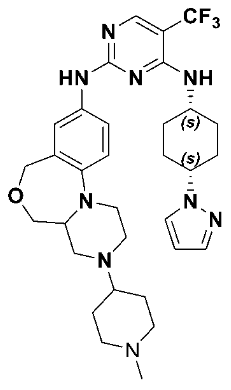
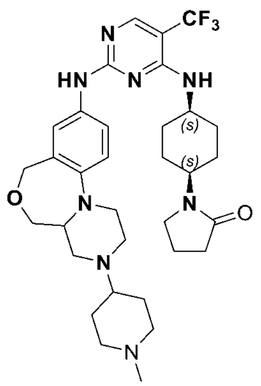
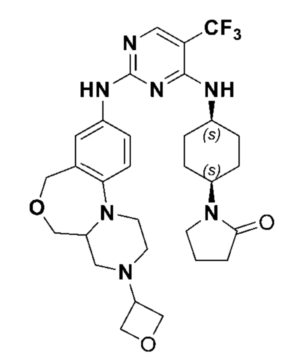
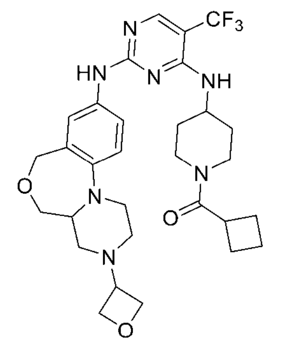

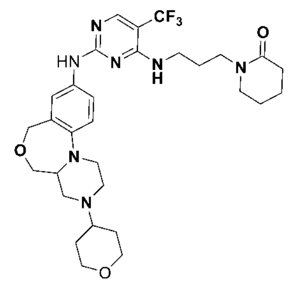
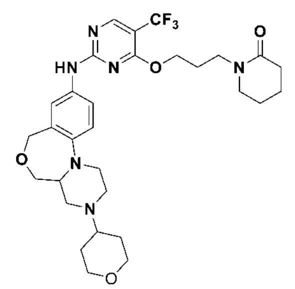
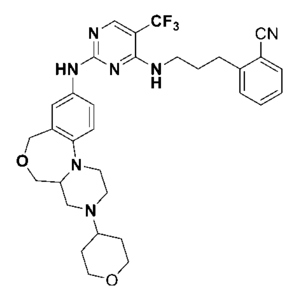
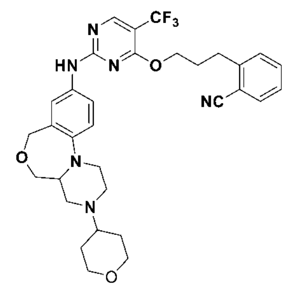

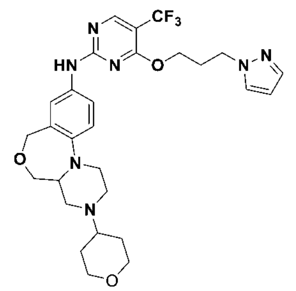
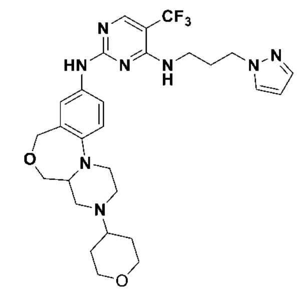
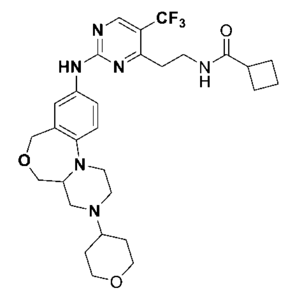
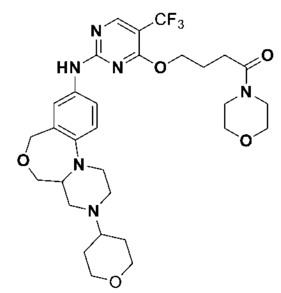

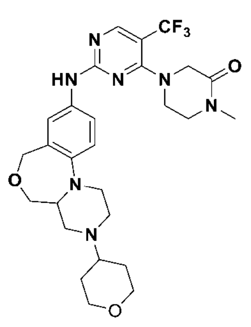
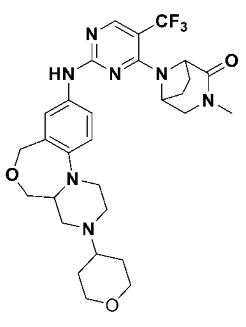
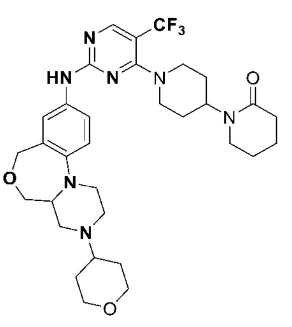
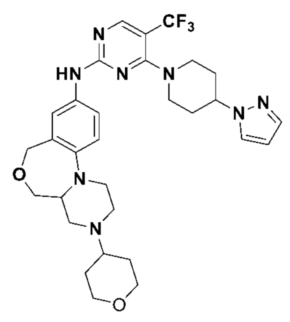

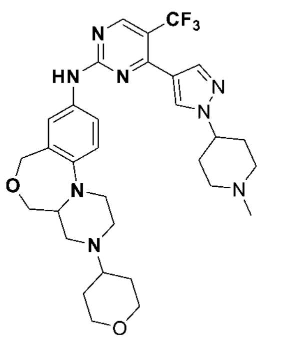
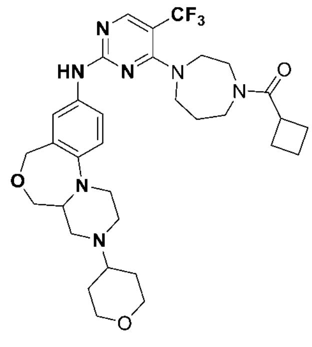
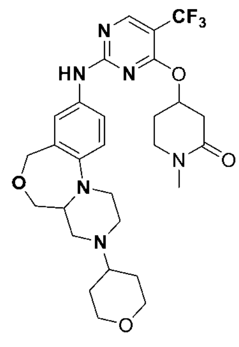
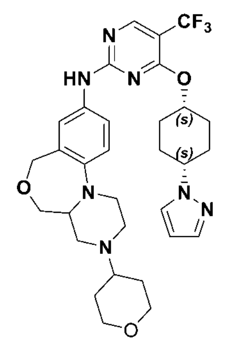

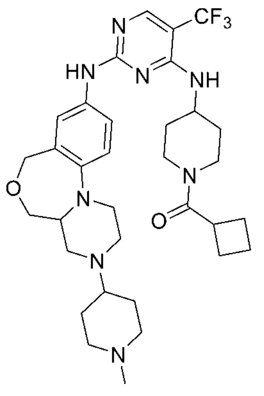
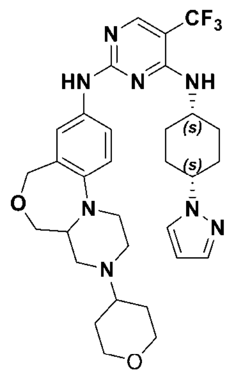
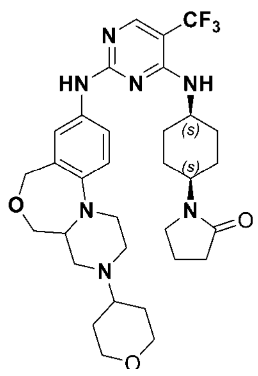
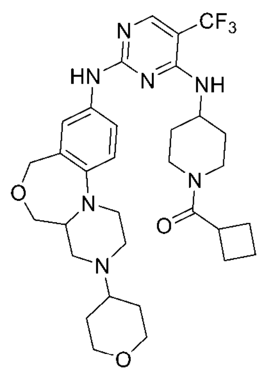

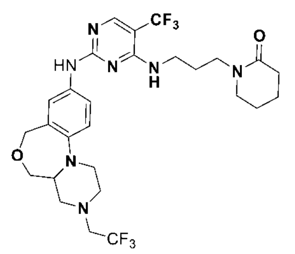
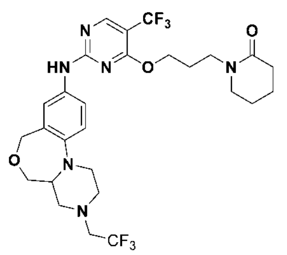
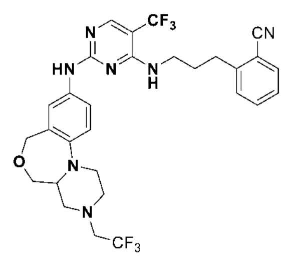
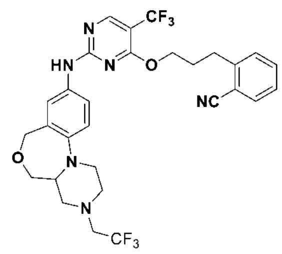

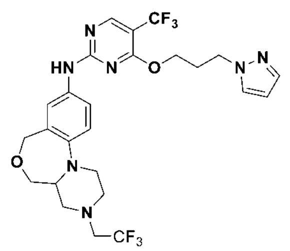
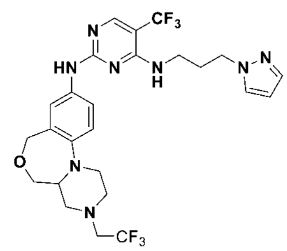
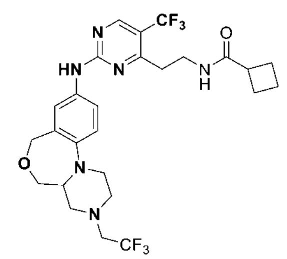
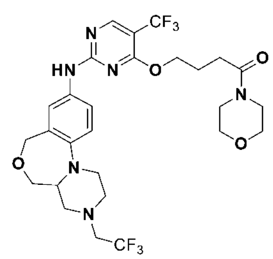

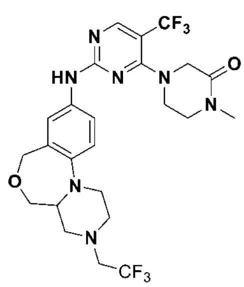
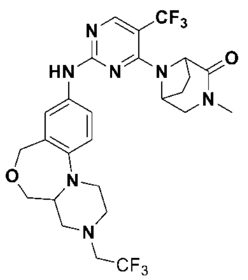
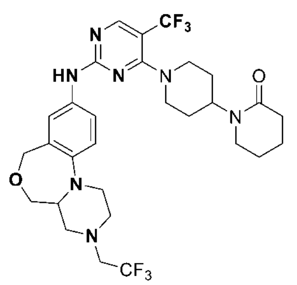
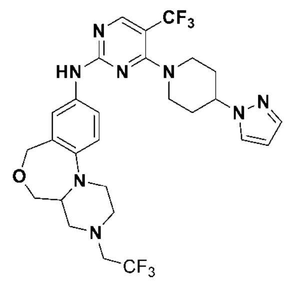

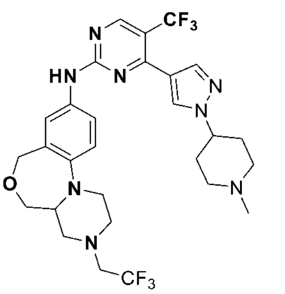
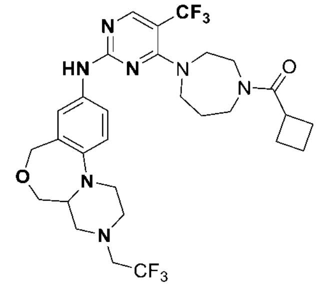
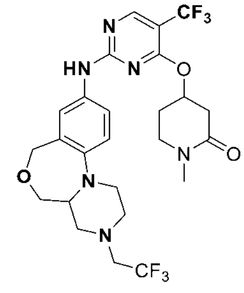
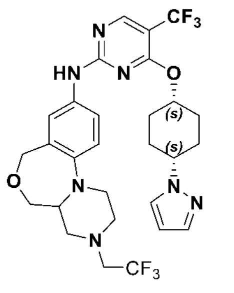
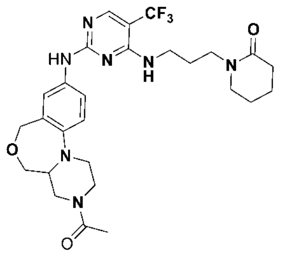
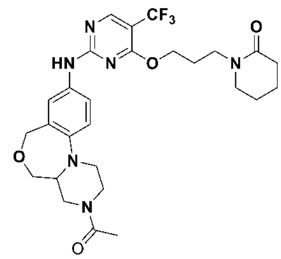
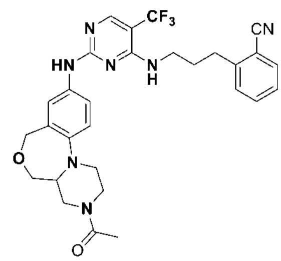
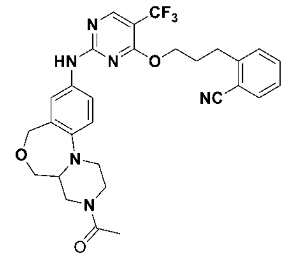
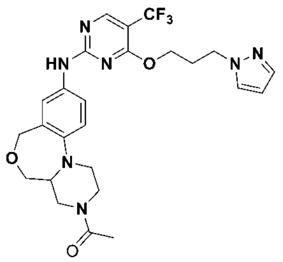
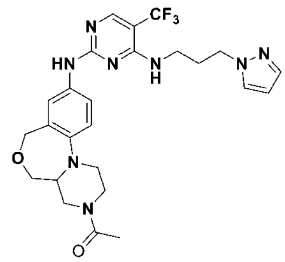
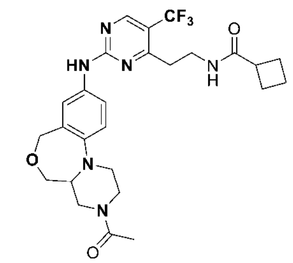
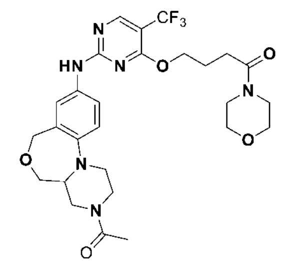
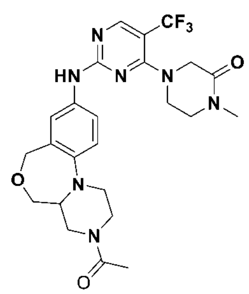
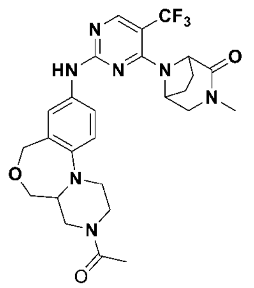
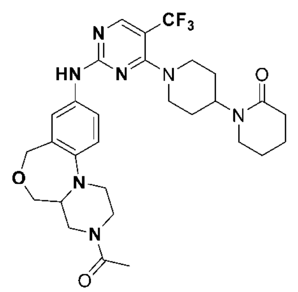
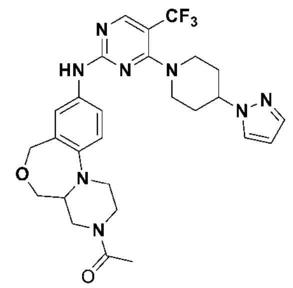
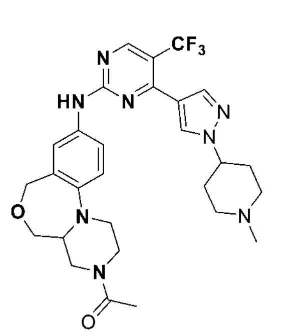
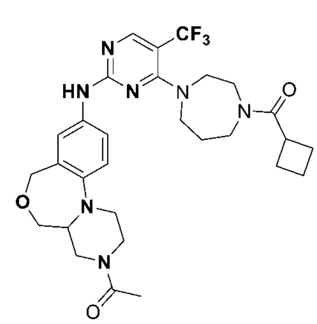
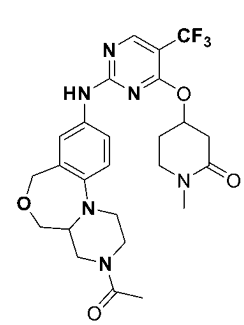
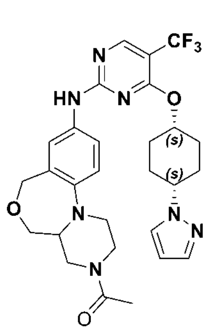

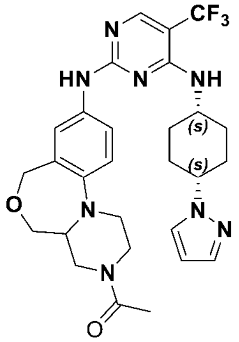
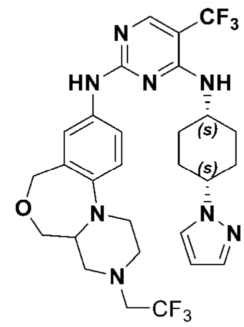
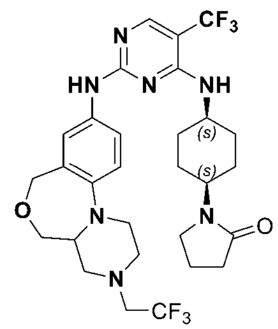
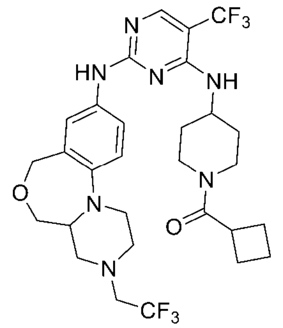

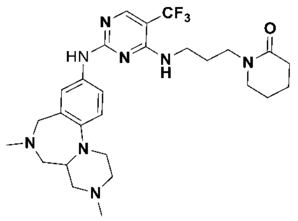
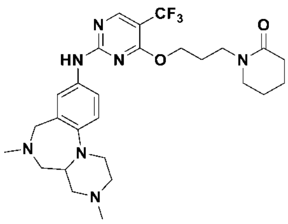
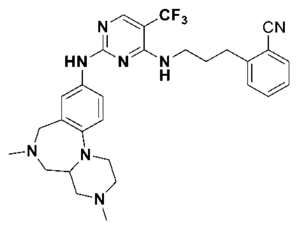
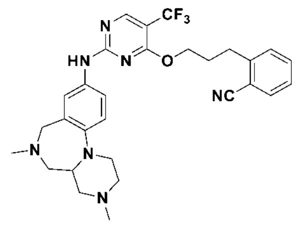

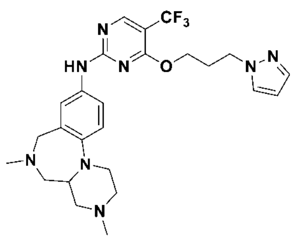
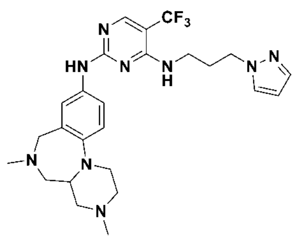
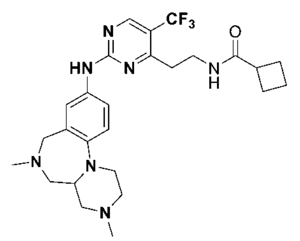
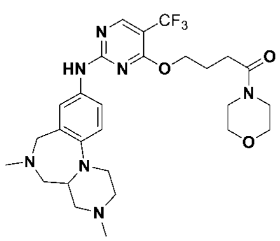

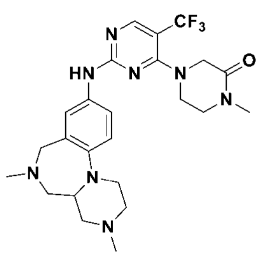
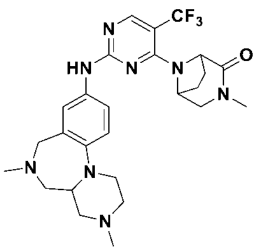
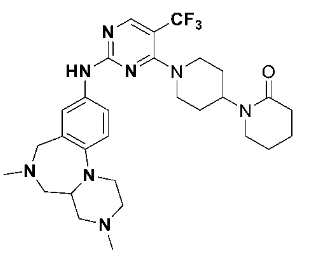
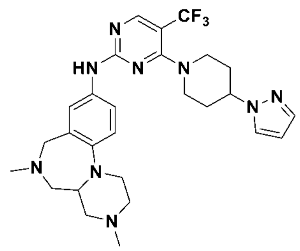

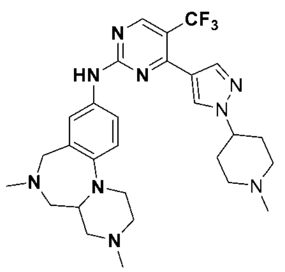
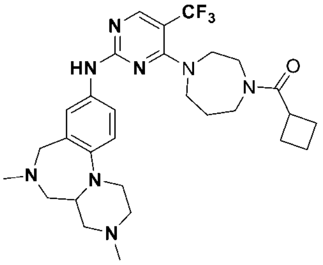
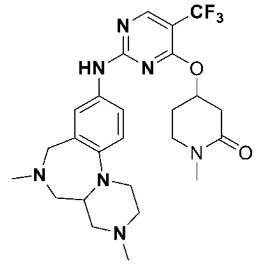
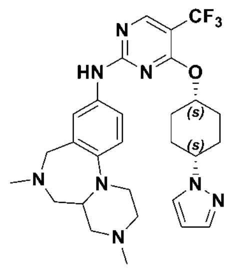

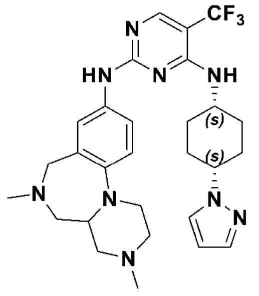
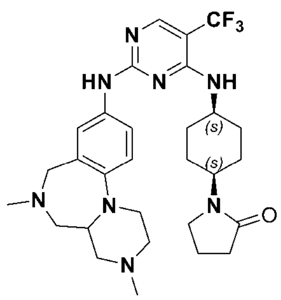
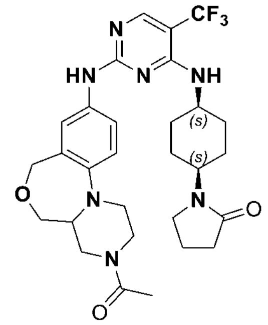
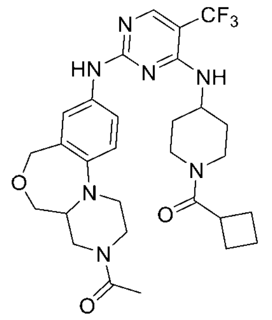
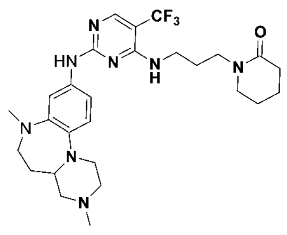
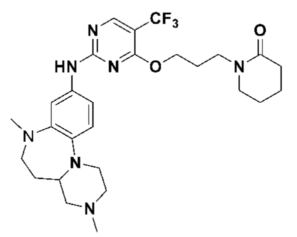
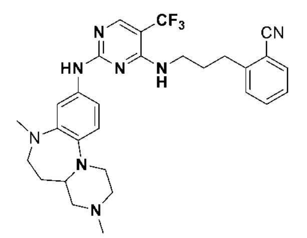
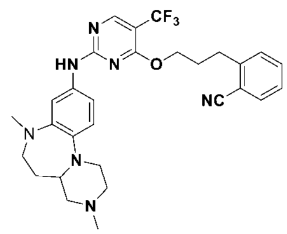
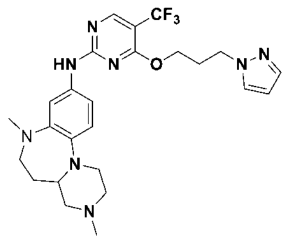
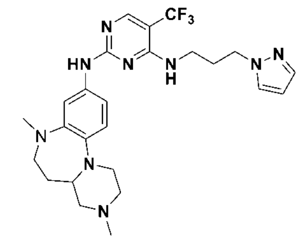
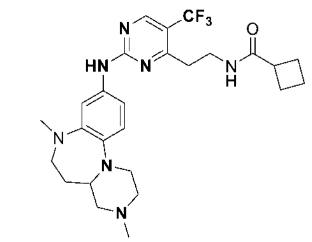
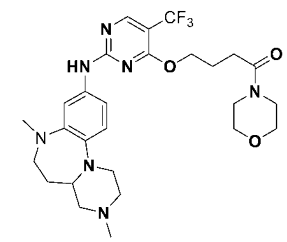
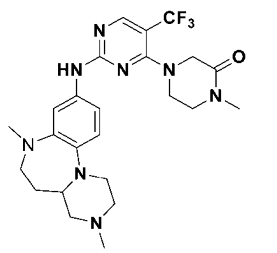
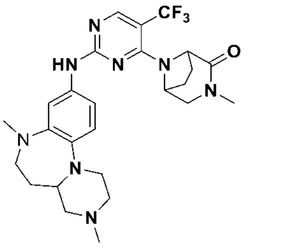
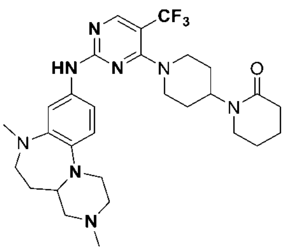
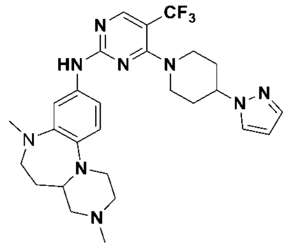
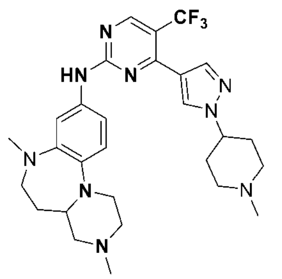
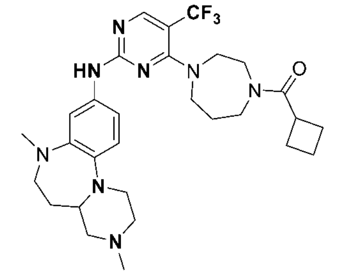
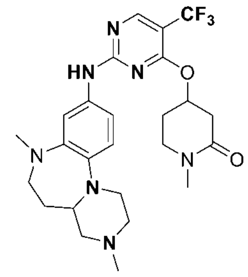
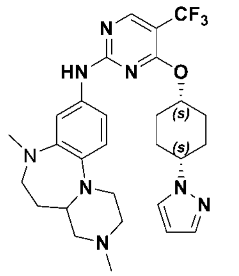

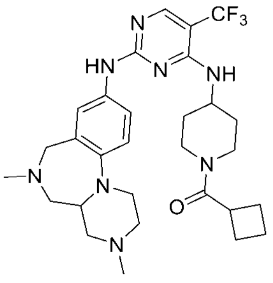
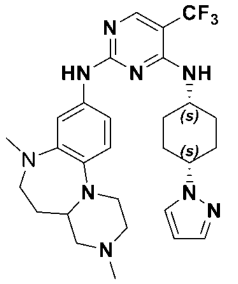
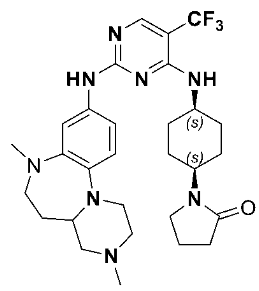
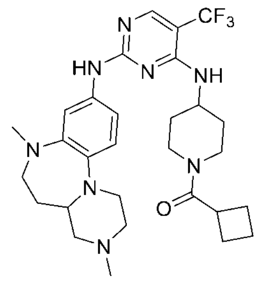

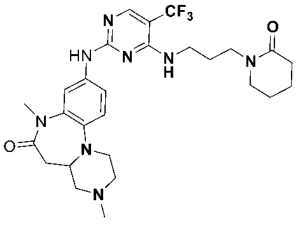
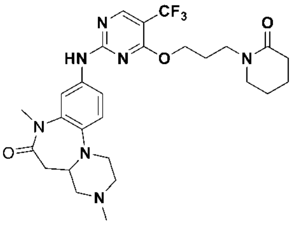
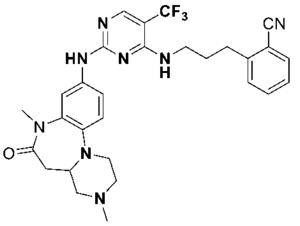
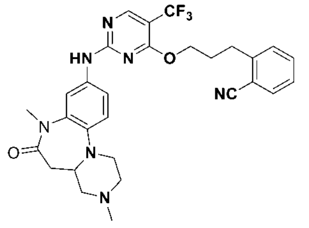

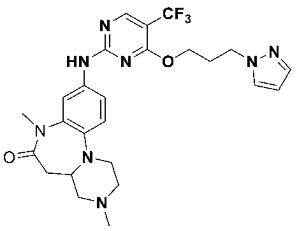
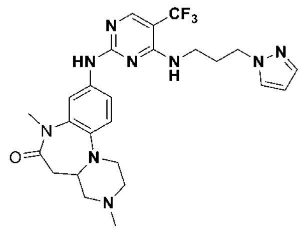
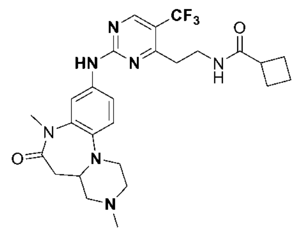
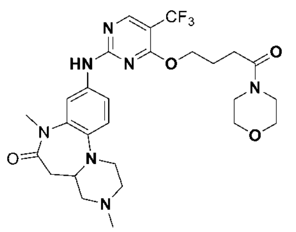

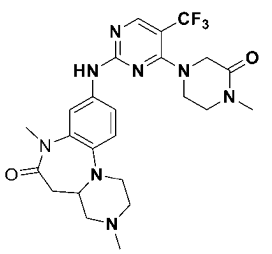
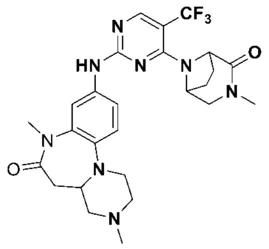
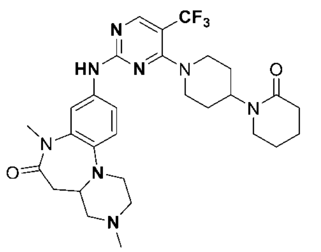
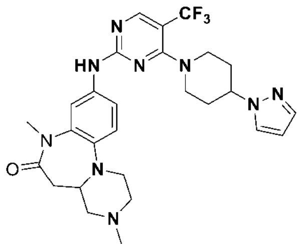

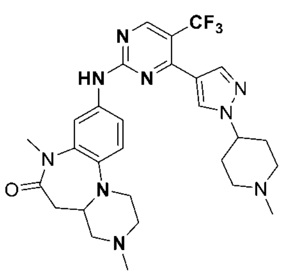
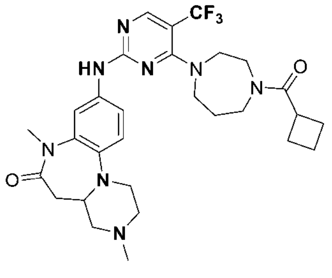
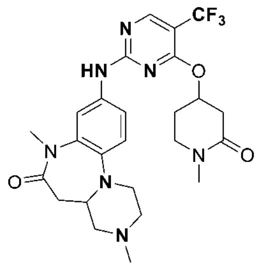
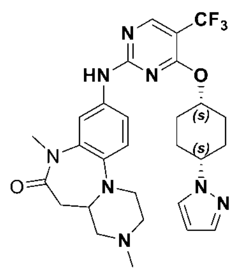

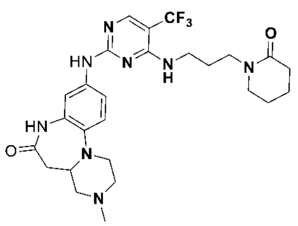
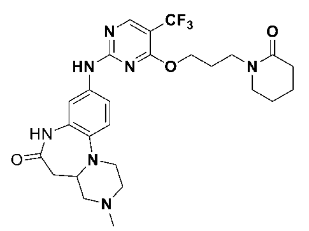
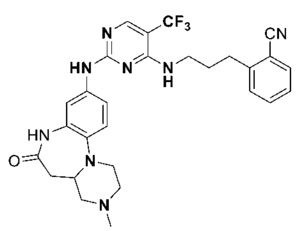
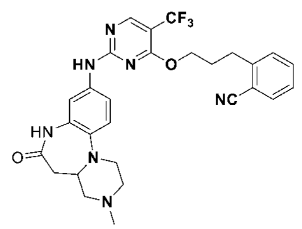

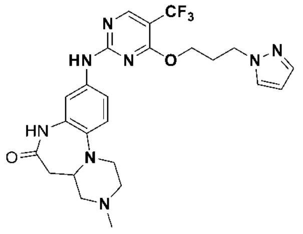
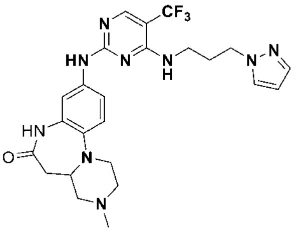
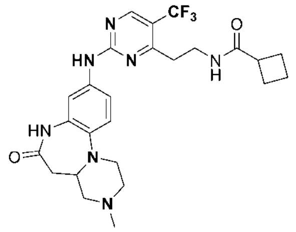
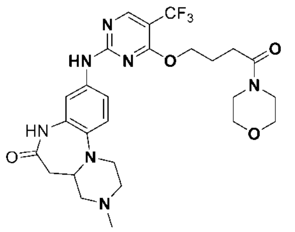

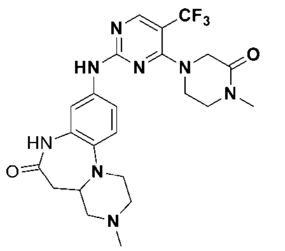
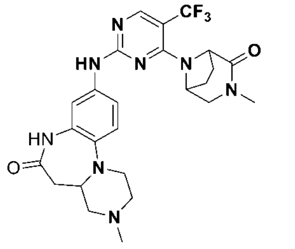
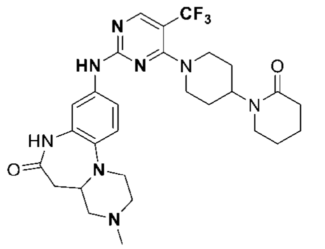
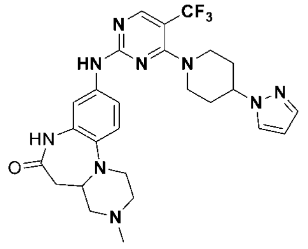

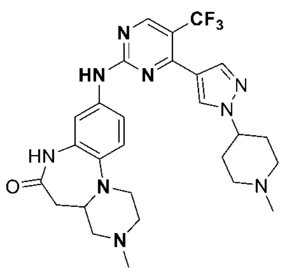
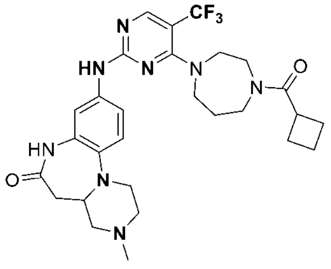
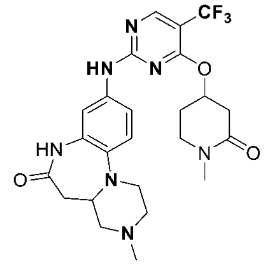
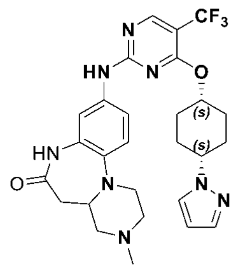

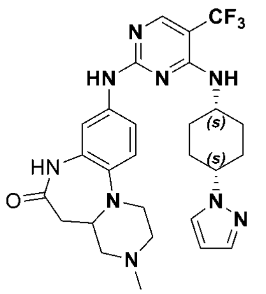
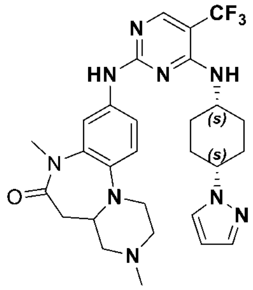
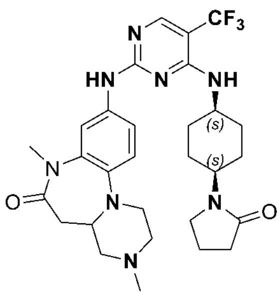
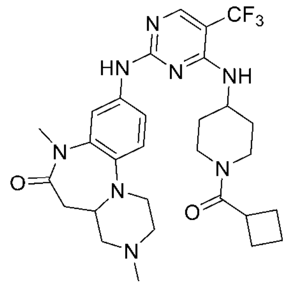

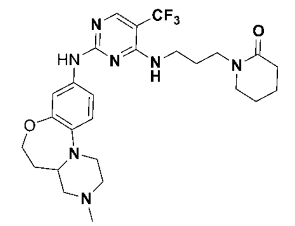
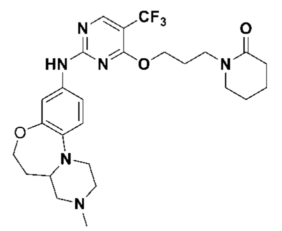
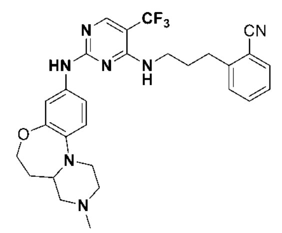
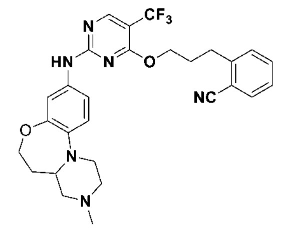

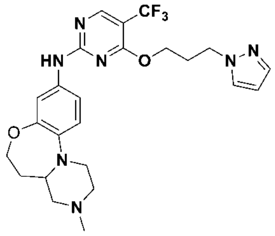
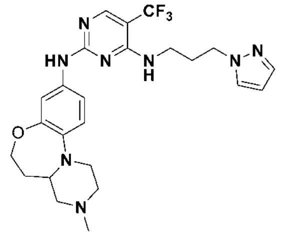
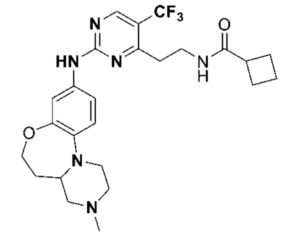
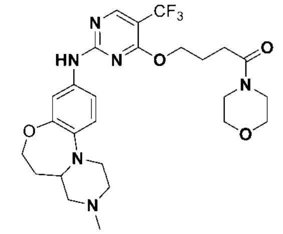

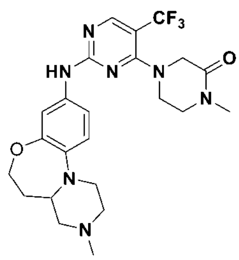
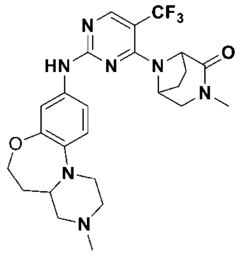
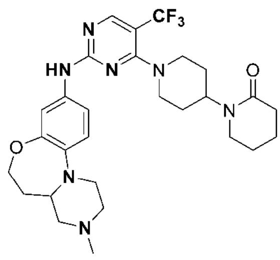
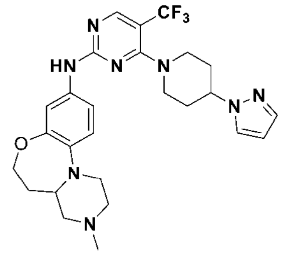
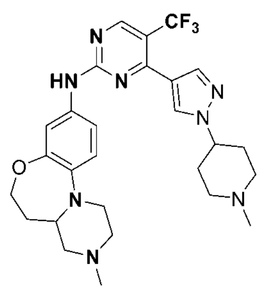
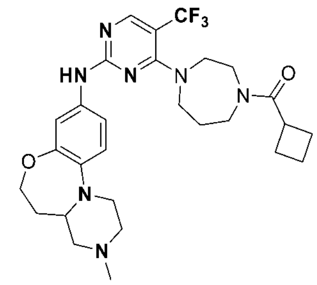
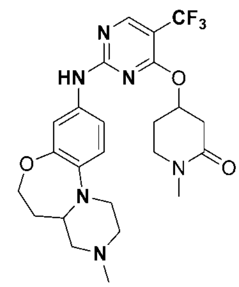
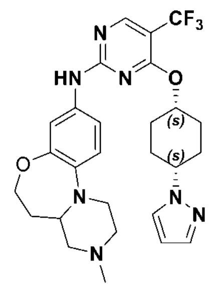
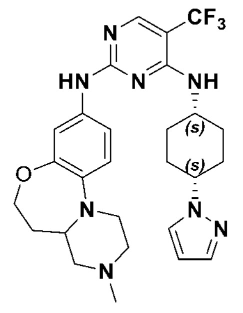
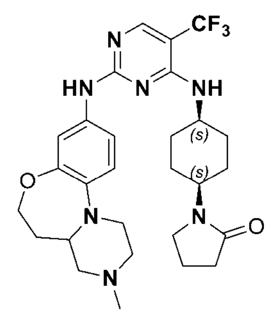
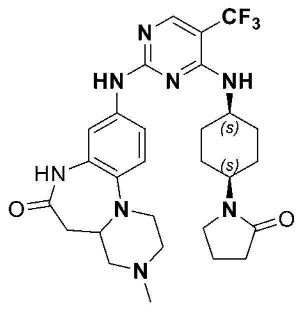
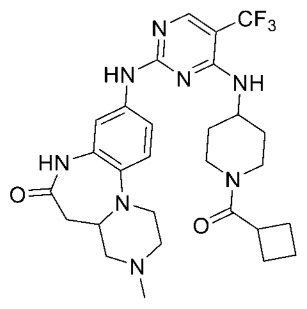
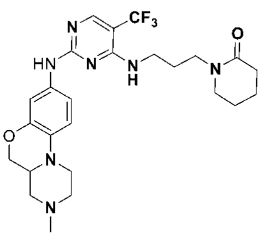
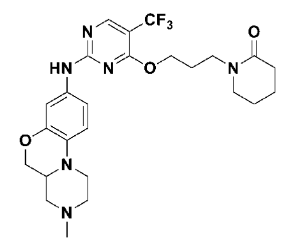
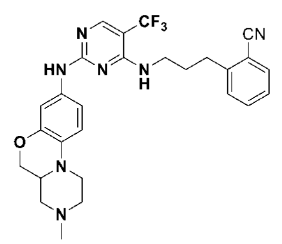
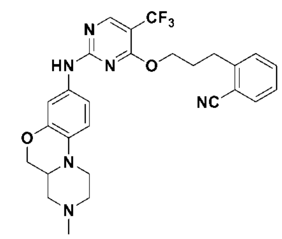
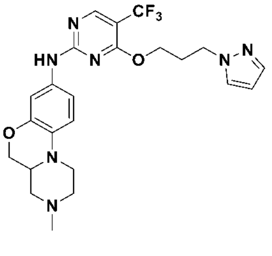
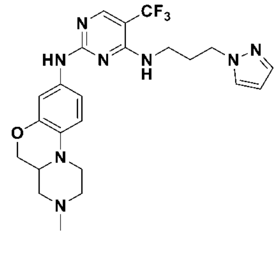
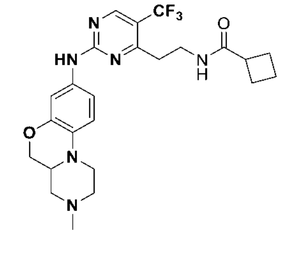
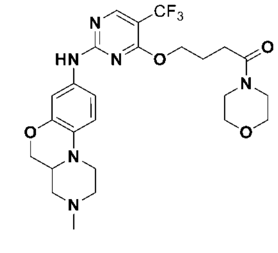
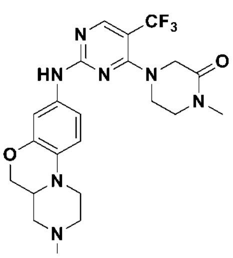
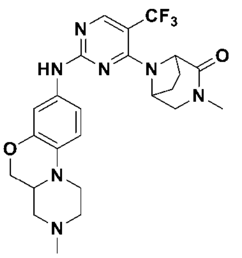
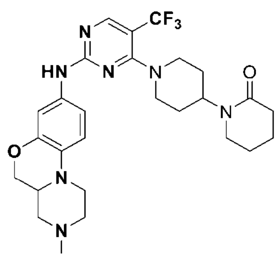
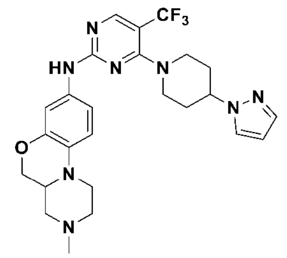

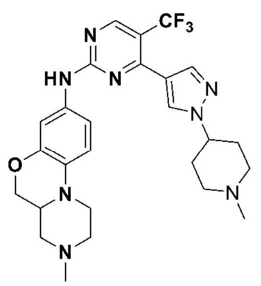
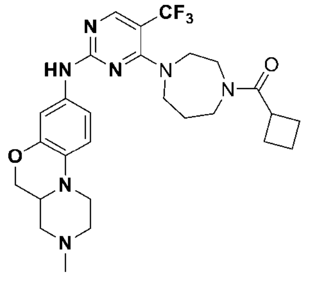
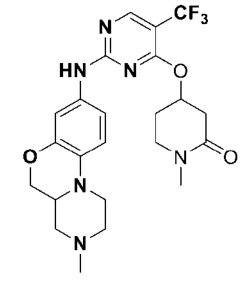
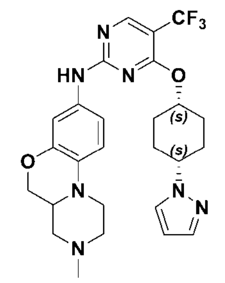

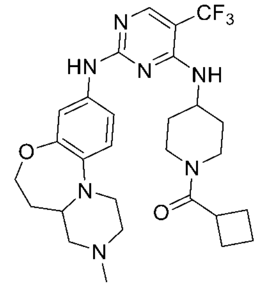
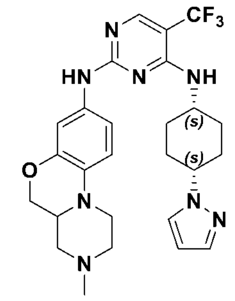
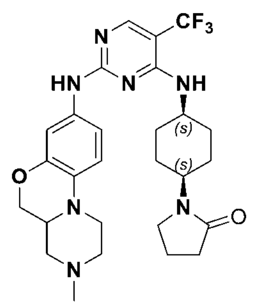
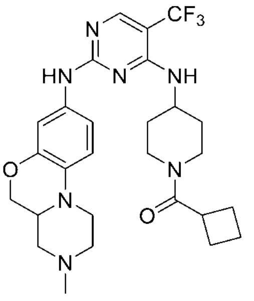

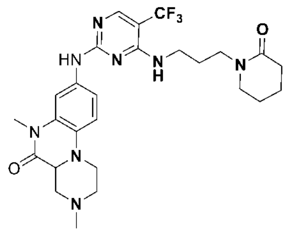
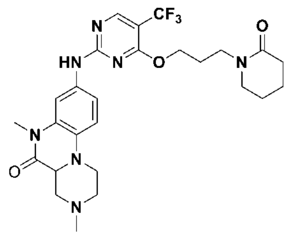
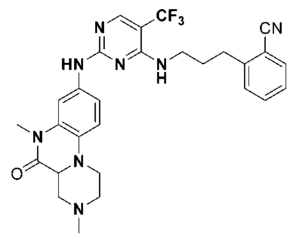
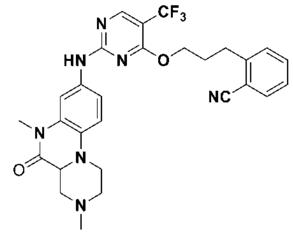

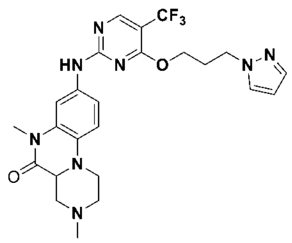
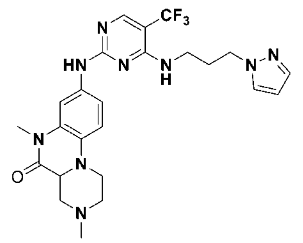
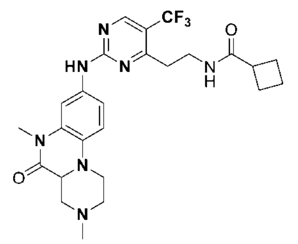
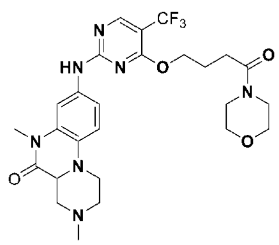

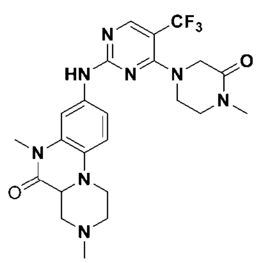
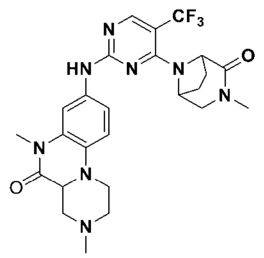
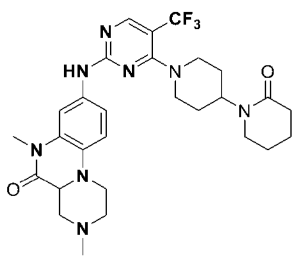
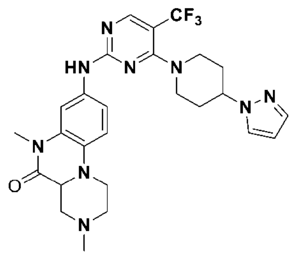

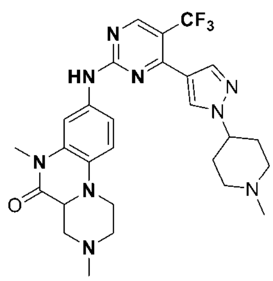
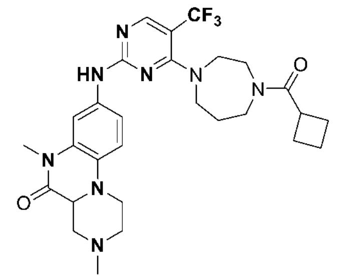
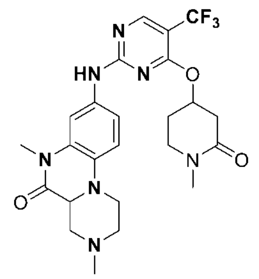
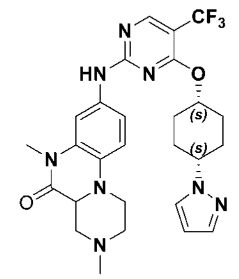

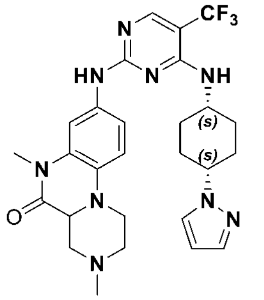
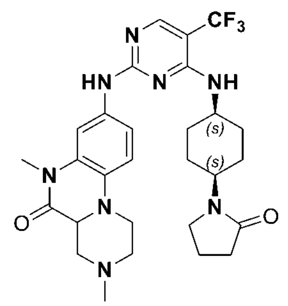
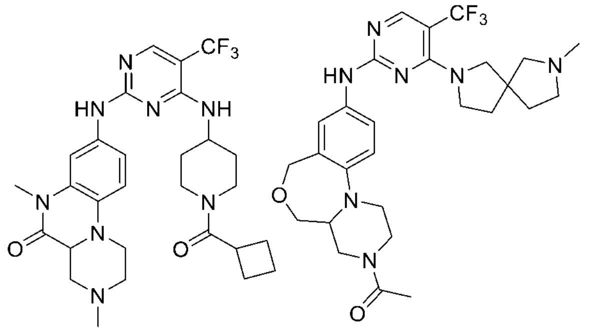

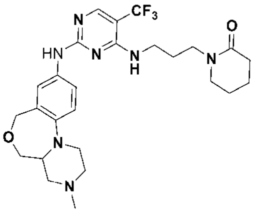
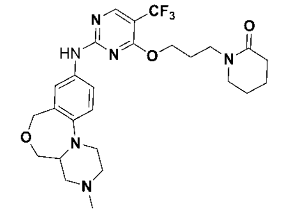
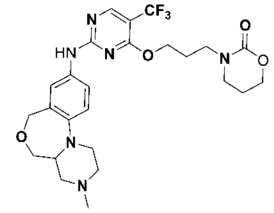
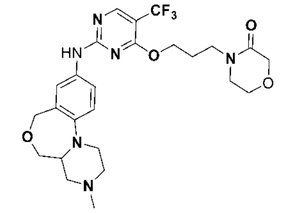

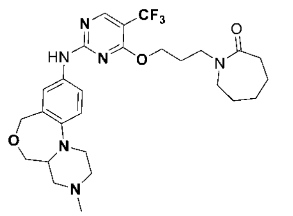
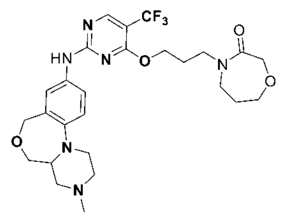
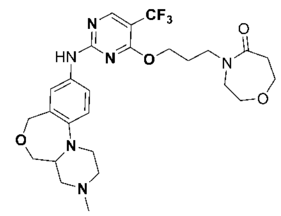
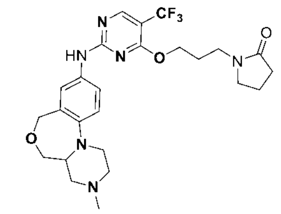

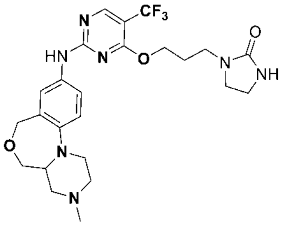
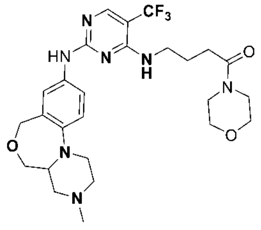
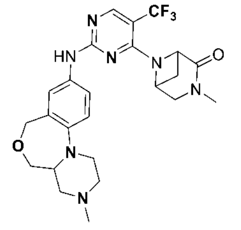
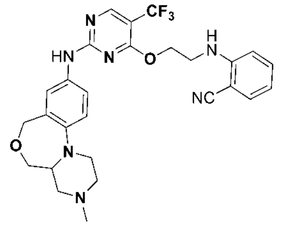

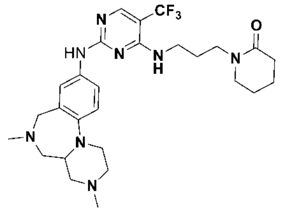
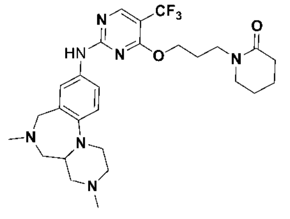
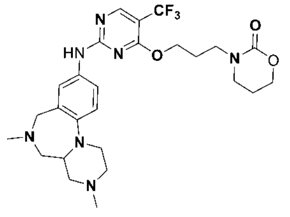
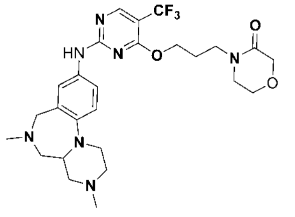

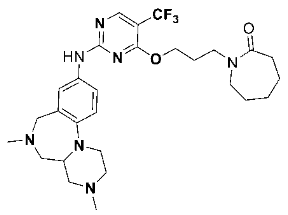
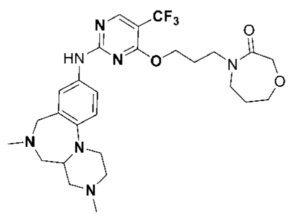
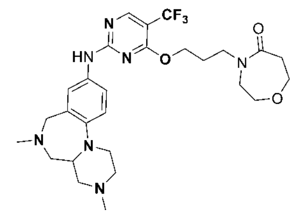
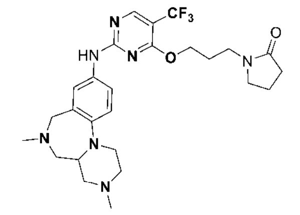

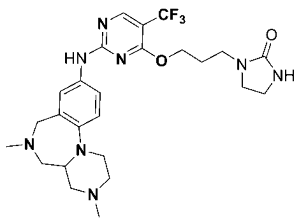
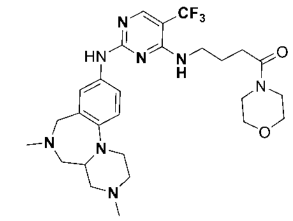
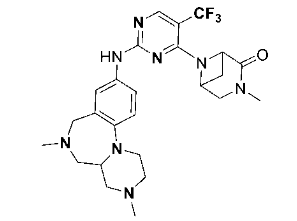
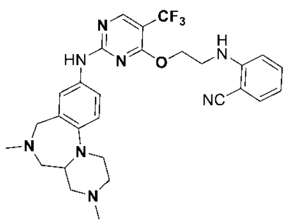

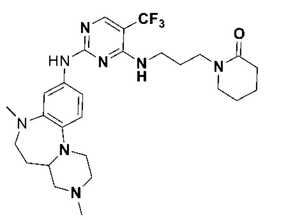
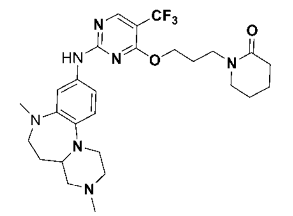
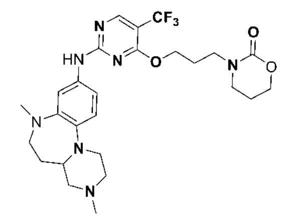
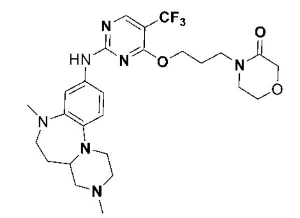

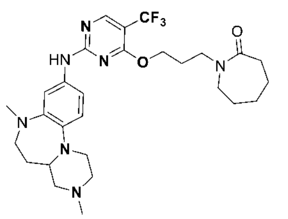
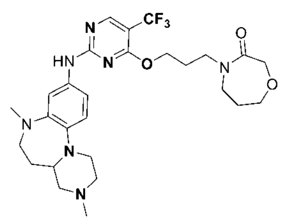
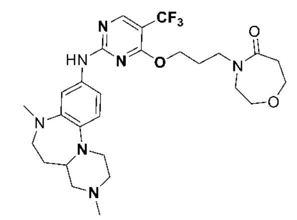
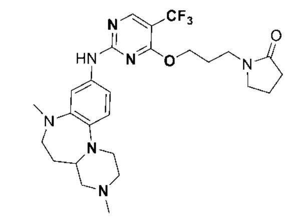

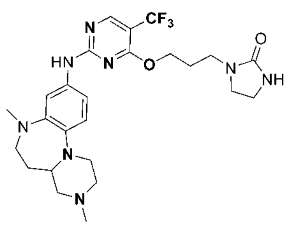
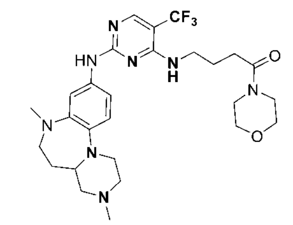
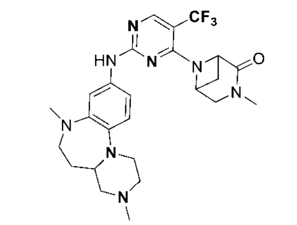
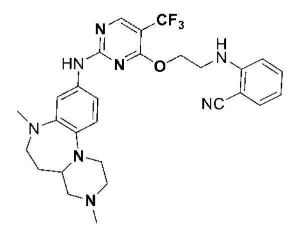

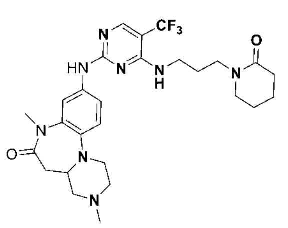
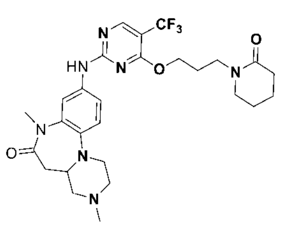
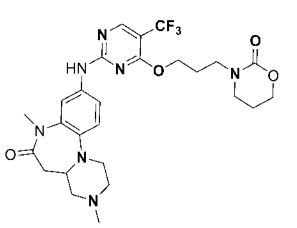
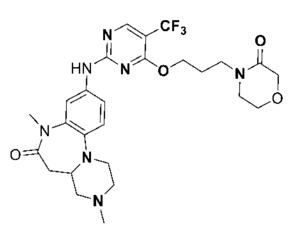

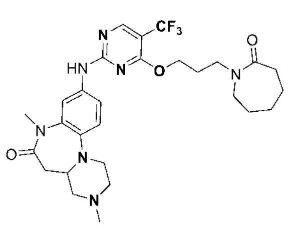
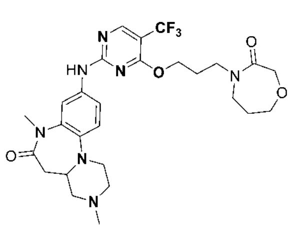
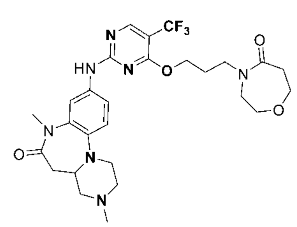
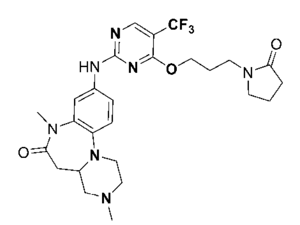

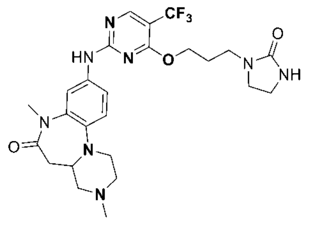 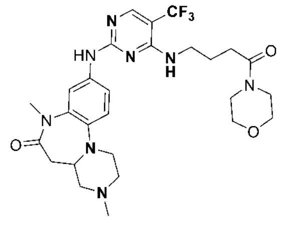 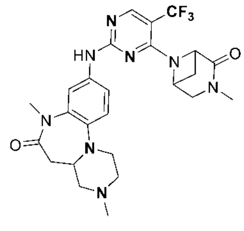 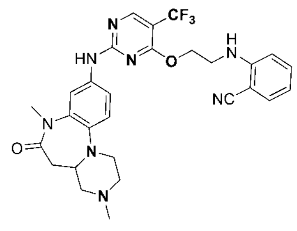

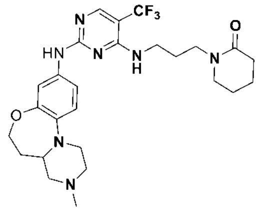 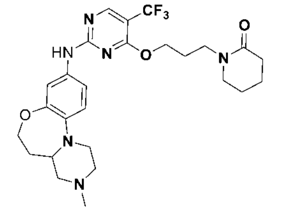 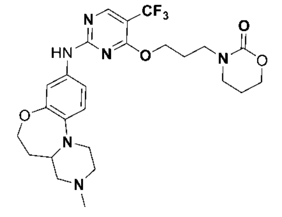 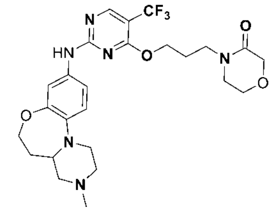

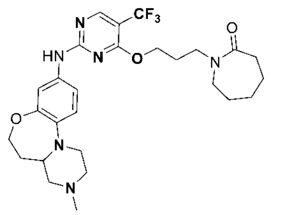 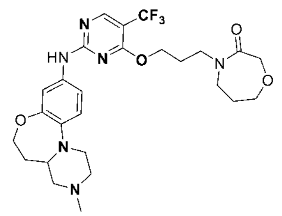 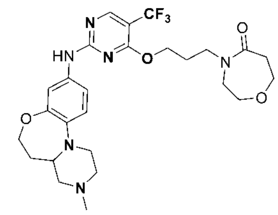 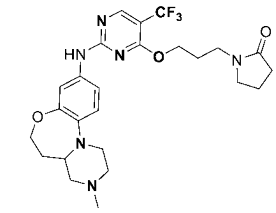

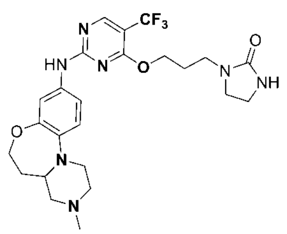 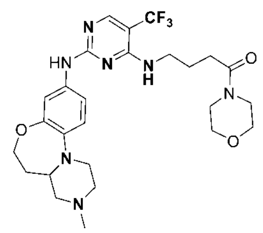 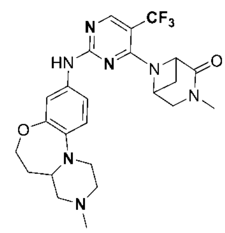 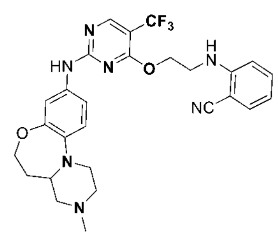

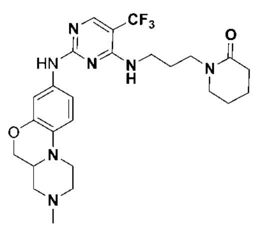 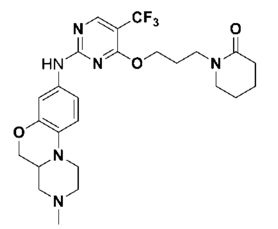 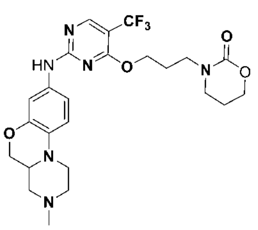 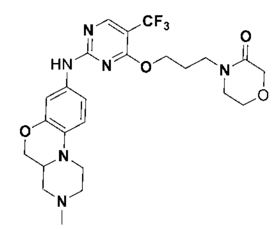

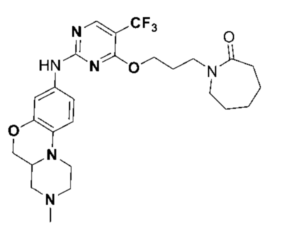 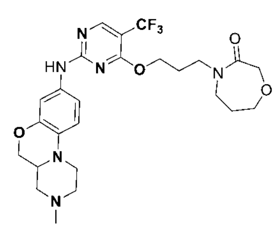 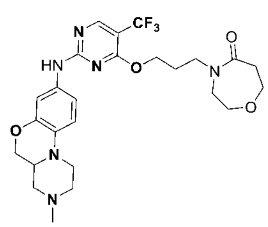 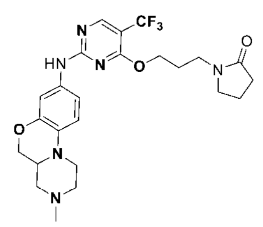

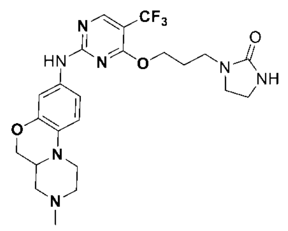
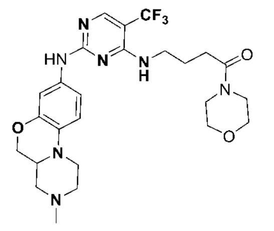
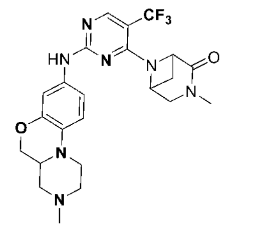
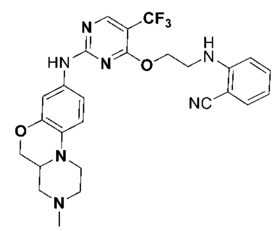
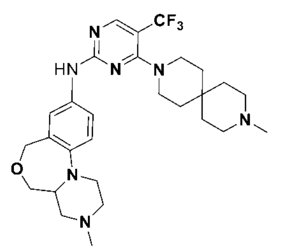
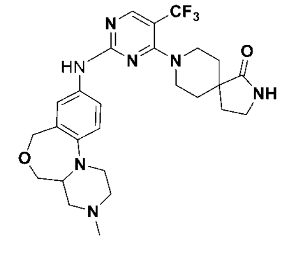
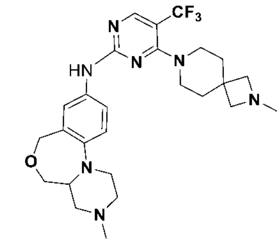
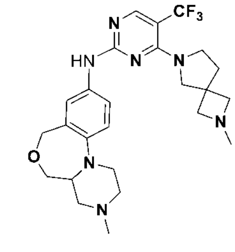
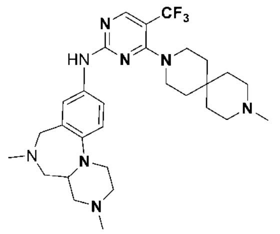
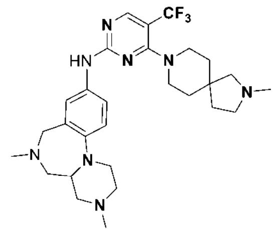
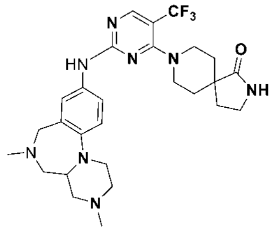
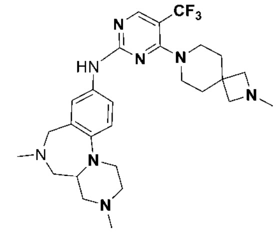
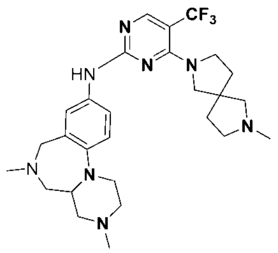
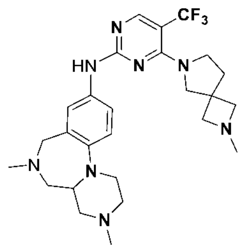
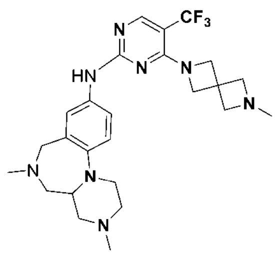
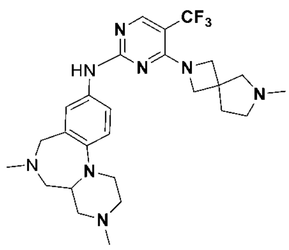
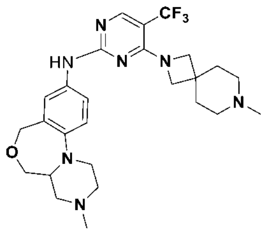
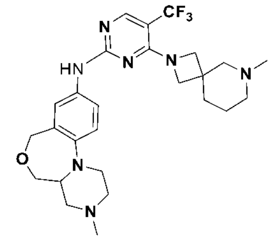
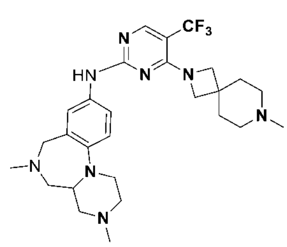
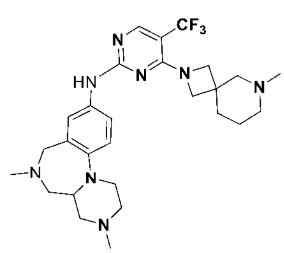
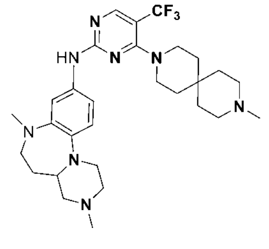
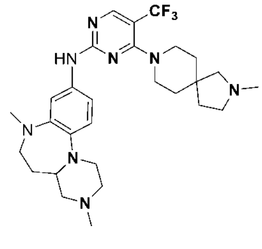
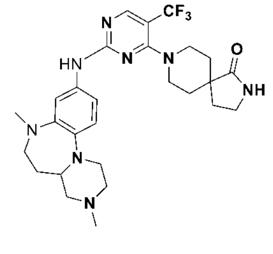
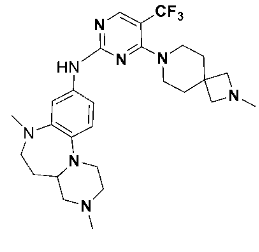

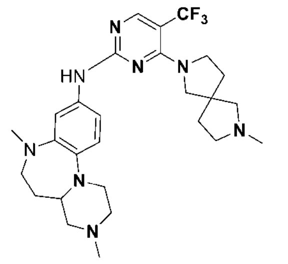
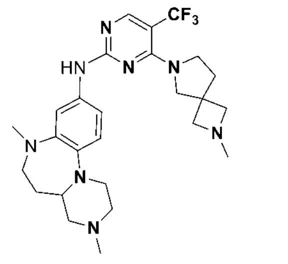
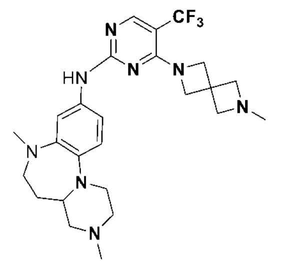
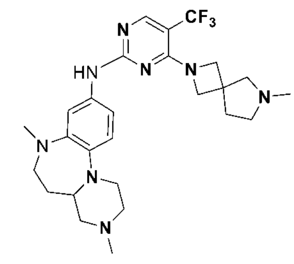
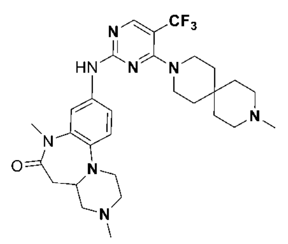
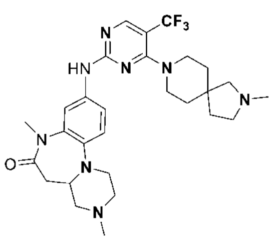
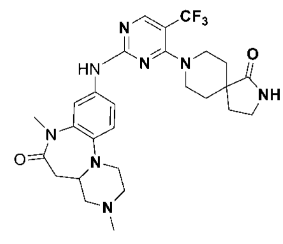
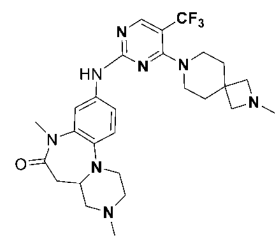
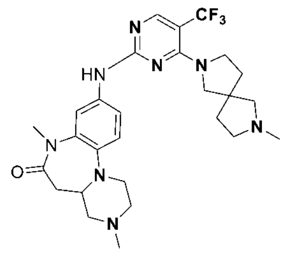
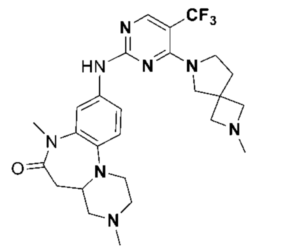
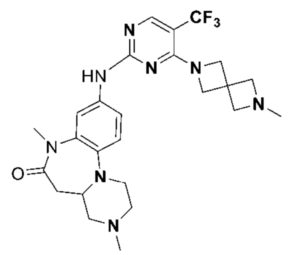
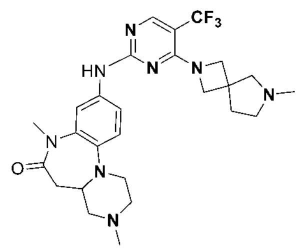
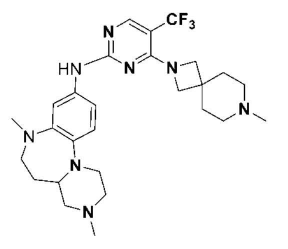
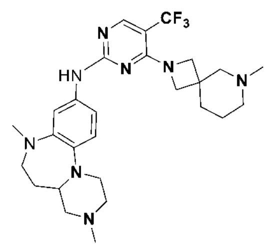
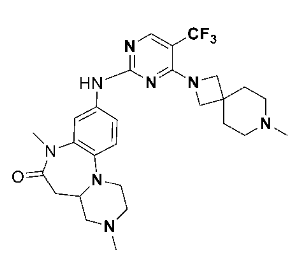
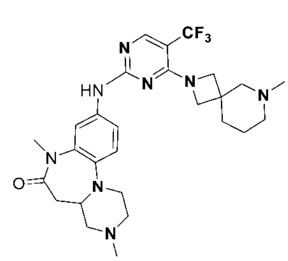
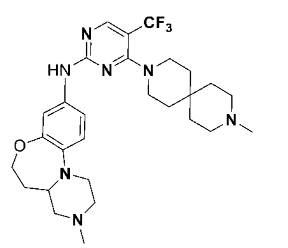
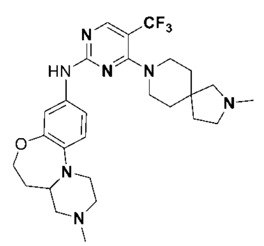
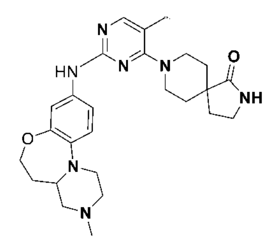
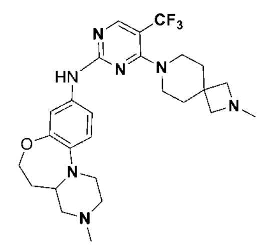
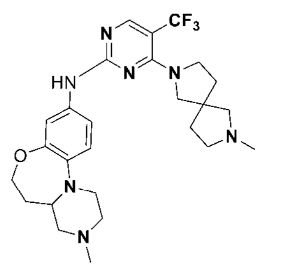
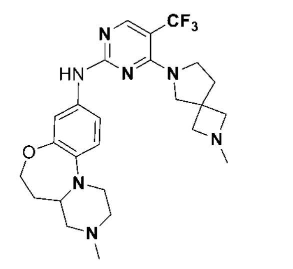
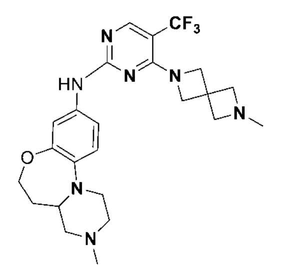
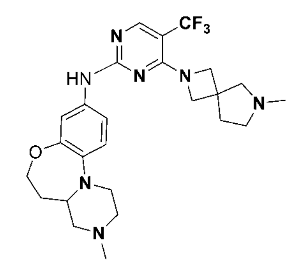

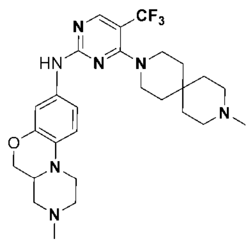

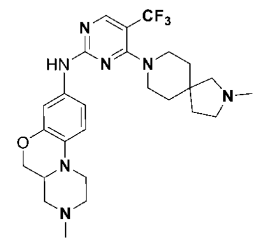

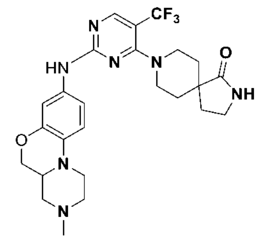

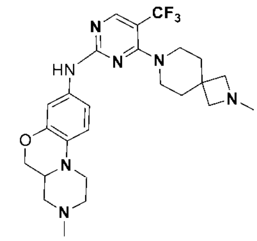

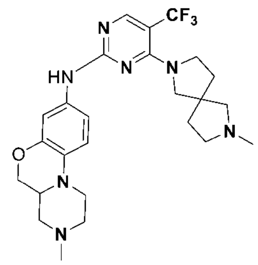

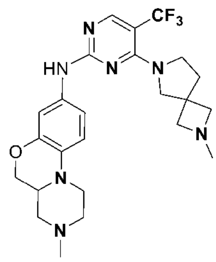

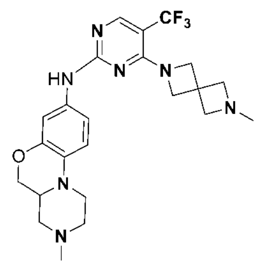

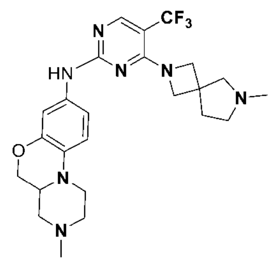

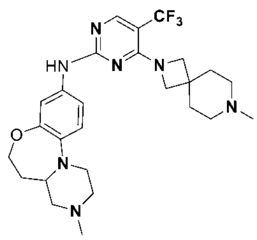

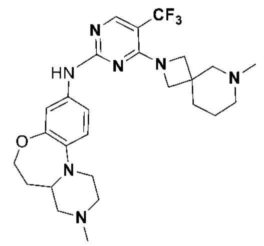

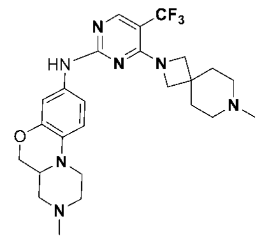

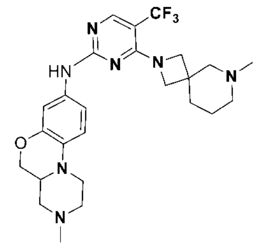

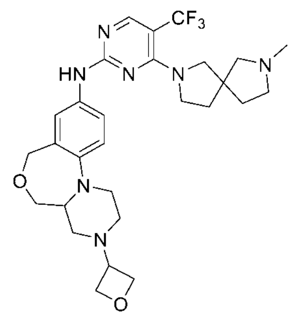

.

**23.** A pharmaceutical composition, comprising an effective amount of the compound or pharmaceutically acceptable salt, stereoisomer, tautomer, polymorph, solvate, hydrate, N-oxide, isotope labeled compound, metabolite, ester, or prodrug thereof of anyone of claims 1 - 22, and one or more pharmaceutically acceptable carrier.

**24.** Use of the compound or pharmaceutically acceptable salt, stereoisomer, tautomer, polymorph, solvate, hydrate, N-oxide, isotope labeled compound, metabolite, ester, or prodrug thereof of anyone of claims 1 - 22, or the pharmaceutical composition of claim 23 in the preparation of a medicament for treating a ULK1-mediated disease in a subject in need thereof; preferably, wherein the ULK1-mediated disease is **characterized by** an abnormal autophagy; preferably, wherein the abnormal autophagy is induced by treatment; preferably, wherein the ULK1-mediated disease is a cancer, preferably lung cancer, breast cancer, or pancreatic cancer, for example non-small cell lung cancer, triple negative breast cancer, or pancreatic ductal adenocarcinoma, or the ULK1-mediated disease is tuberous sclerosis (TSC) or lymphangioleiomyomatosis (LAM).

**25.** The use according to claim 24, wherein the medicament is used for co-administration with an additional therapeutic agent; preferably, wherein the additional therapeutic agent is a standard care therapy, for example an mTOR inhibitor, carboplatin, a MEK inhibitor, or a PARP inhibitor.

**26.** The use according to claim 24 or 25, wherein the medicament is used to degrade ATG13 in the subject.

**27.** A method for preparing the compound of claim 1, comprising the route A or route B as follows:

Synthetic route A:

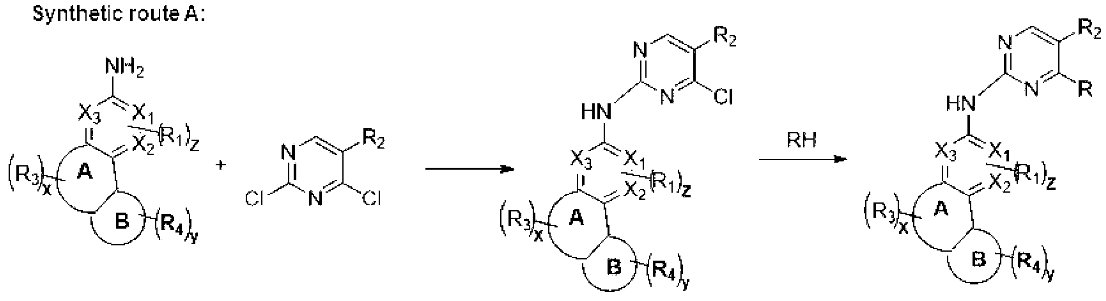


1

or,

Synthetic route B:

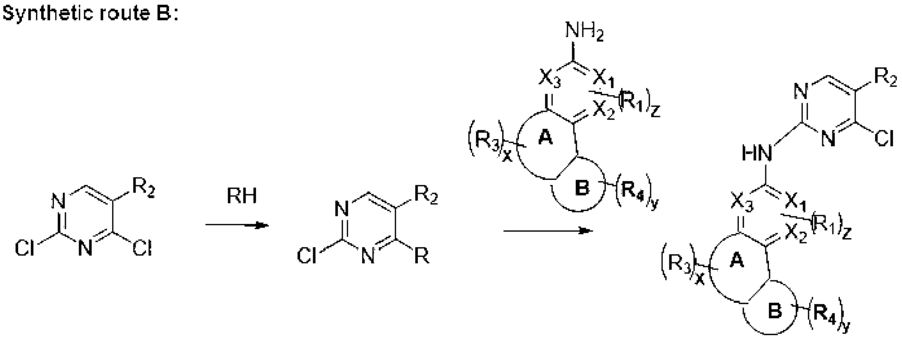


1

,

wherein $R_1$, $R_2$, $R_3$, $R_4$, x, y, z, $X_1$, $X_2$, and $X_3$ are as defined in claim 1.

## INTERNATIONAL SEARCH REPORT

International application No.
**PCT/CN2024/118472**

**A. CLASSIFICATION OF SUBJECT MATTER**

C07D498/04(2006.01)i; C07D519/00(2006.01)i; C07D413/04(2006.01)i; A61P35/00(2006.01)i; A61K31/506(2006.01)i; A61K31/551(2006.01)i

According to International Patent Classification (IPC) or to both national classification and IPC

**B. FIELDS SEARCHED**

Minimum documentation searched (classification system followed by classification symbols)

IPC: C07D A61P A61K

Documentation searched other than minimum documentation to the extent that such documents are included in the fields searched

Electronic data base consulted during the international search (name of data base and, where practicable, search terms used)

CNTXT, VEN, WOTXT, USTXT, EPTXT, CNKI, 万方, WANFANG, STN, ISI Web of Science: 众格生物, 花子皓, 孙宜娜, 赵传芳, 陈冲, ULK1抑制剂, 自噬活化激酶, 癌症, 肺癌, 乳腺癌, 胰腺癌, 非小细胞肺癌, 嘧啶 s (氨基 or 胺) s 哌嗪, ULK1 Inhibitors, Autophagy-activating kinase, Cancer, Tumor, Lung cancer, Breast cancer, Pancreatic cancer, Non-Small Cell Lung Cancer, +Pyrimidine + s (+amino+ or +amine+) s +piperazine+, 结构式

**C. DOCUMENTS CONSIDERED TO BE RELEVANT**

| Category* | Citation of document, with indication, where appropriate, of the relevant passages | Relevant to claim No. |
|---|---|---|
| PX | WO 2024153248 A1 (HANGZHOU INNOGATE PHARMA CO., LTD.) 25 July 2024 (2024-07-25)<br>claims 1-17, and embodiment 22 | 1-27 |
| X | CN 116456985 A (SHENZHEN IONOVA LIFE SCIENCE CO., LTD. et al.) 18 July 2023 (2023-07-18)<br>claims 1 and 23-29, and description, embodiments 203-205 | 1-26 |
| Y | CN 116456985 A (SHENZHEN IONOVA LIFE SCIENCE CO., LTD. et al.) 18 July 2023 (2023-07-18)<br>claims 1 and 23-29, and description, embodiments 203-205 | 27 |
| X | CN 110734454 A (CSPC ZHONGQI PHARMACEUTICAL TECHNOLOGY (SHIJIAZHUANG) CO., LTD. et al.) 31 January 2020 (2020-01-31)<br>claims 1, 5-7, 9, 12, and 13, and description, paragraphs 0006, 0009, and 0172, tables 1 and 3, and embodiments 2-11, 16-36, 40, 41, and 47 | 1-27 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **28 November 2024** | **12 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

## INTERNATIONAL SEARCH REPORT

International application No.
**PCT/CN2024/118472**

**C. DOCUMENTS CONSIDERED TO BE RELEVANT**

| Category* | Citation of document, with indication, where appropriate, of the relevant passages | Relevant to claim No. |
|---|---|---|
| Y | CN 110734454 A (CSPC ZHONGQI PHARMACEUTICAL TECHNOLOGY (SHIJIAZHUANG) CO., LTD. et al.) 31 January 2020 (2020-01-31)<br>claim 13, and description, paragraph 0172 | 27 |
| X | WO 2022131741 A1 (VORONOIBIO INC. et al.) 23 June 2022 (2022-06-23)<br>claims 1, 8, and 10, and description, paragraphs 74 and 229-234, table 2, and experiment 5 | 1-27 |
| Y | WO 2022131741 A1 (VORONOIBIO INC. et al.) 23 June 2022 (2022-06-23)<br>description, paragraphs 229-234 | 27 |
| A | KR 20220090443 A (B2SBIO INC. et al.) 29 June 2022 (2022-06-29)<br>entire document | 1-27 |
| A | WO 2023087027 A1 (ERASCA INC.) 19 May 2023 (2023-05-19)<br>entire document | 1-27 |

Form PCT/ISA/210 (second sheet) (July 2022)

**INTERNATIONAL SEARCH REPORT**
**Information on patent family members**

International application No.
**PCT/CN2024/118472**

| Patent document cited in search report | | | Publication date (day/month/year) | Patent family member(s) | | | Publication date (day/month/year) |
|---|---|---|---|---|---|---|---|
| WO | 2024153248 | A1 | 25 July 2024 | None | | | |
| CN | 116456985 | A | 18 July 2023 | WO | 2022063140 | A1 | 31 March 2022 |
| CN | 110734454 | A | 31 January 2020 | WO | 2018108084 | A1 | 21 June 2018 |
| | | | | CN | 109153687 | A | 04 January 2019 |
| | | | | US | 2019308993 | A1 | 10 October 2019 |
| | | | | EP | 3553064 | A1 | 16 October 2019 |
| | | | | HK | 1263157 | A0 | 24 January 2020 |
| | | | | CN | 110746443 | A | 04 February 2020 |
| | | | | EP | 3553064 | A4 | 10 June 2020 |
| | | | | US | 10730887 | B2 | 04 August 2020 |
| | | | | HK | 40018350 | A0 | 30 September 2020 |
| | | | | US | 2020354376 | A1 | 12 November 2020 |
| | | | | US | 10968234 | B2 | 06 April 2021 |
| | | | | JP | 6854496 | B2 | 07 April 2021 |
| | | | | CN | 110746443 | B | 27 April 2021 |
| | | | | CN | 109153687 | B | 20 July 2021 |
| | | | | CN | 110734454 | B | 17 August 2021 |
| | | | | CN | 113603707 | A | 05 November 2021 |
| | | | | HK | 40018350 | A1 | 07 January 2022 |
| | | | | HK | 1263157 | A1 | 08 April 2022 |
| | | | | CN | 113603707 | B | 31 March 2023 |
| | | | | EP | 3553064 | B1 | 07 June 2023 |
| | | | | JP | 2020503378 | A | 30 January 2020 |
| WO | 2022131741 | A1 | 23 June 2022 | KR | 20220085735 | A | 22 June 2022 |
| KR | 20220090443 | A | 29 June 2022 | WO | 2022139386 | A1 | 30 June 2022 |
| WO | 2023087027 | A1 | 19 May 2023 | EP | 4433059 | A1 | 25 September 2024 |
| | | | | TW | 202332448 | A | 16 August 2023 |
| | | | | AU | 2022383957 | A1 | 30 May 2024 |
| | | | | CA | 3238655 | A1 | 19 May 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311187688


### Non-patent literature cited in the description


- **STAHL** ; **WERMUTH**. Handbook of Pharmaceutical Salts: Properties, Selection, and Use. Wiley-VCH, 2002
- **T. L. GILCHRIST**. Comprehensive Organic Synthesis. Academic Press, vol. 7, 748-750
- **G. W. H. CHEESEMAN** ; **E. S. G. WERSTIUK**. Advances in Heterocyclic Chemistry. Academic Press, vol. 22, 390-392
- **T. HIGUCHI** ; **V. STELLA**. Pro-drugs as Novel Delivery Systems. *ACS Symposium Series*, vol. 14
- Bioreversible Carriers in Drug Design. Pergamon Press, 1987
- **H. BUNDGAARD**. Design of Prodrugs. Elsevier, 1985
- Protective Groups in Organic Chemistry. Plenum Press, 1973
- **T.W. GREENE** ; **P.G.M. WUTS**. Protective Groups in Organic Synthesis. John Wiley & Sons, 1991
- *Remington's Pharmaceutical Sciences*, 1990